# EUROPEAN PATENT APPLICATION

(11) **EP 4 782 445 A1**
(43) Date of publication of application: **29.07.2026**
(21) Application number: 24867038.2
(22) Date of filing: 11.07.2024
(51) Int. Cl.: C07D 405/14, C07D 403/14, A61K 31/435, A61K 31/538, A61K 31/395, A61K 31/33, A61P 35/00

(54) **CDK KINASE INHIBITOR AND USE THEREOF**

(30) Priority: 21.09.2023 CN 202311228053
(71) Applicant: Abbisko Therapeutics Co., Ltd., Shanghai 201203 (CN)
(72) Inventor: LI, Zheng, Shanghai 201203 (CN); DENG, Haibing, Shanghai 201203 (CN); PAN, Weiling, Shanghai 201203 (CN); YU, Hongping, Shanghai 201203 (CN); CHEN, Zhui, Shanghai 201203 (CN)
(74) Representative: dompatent
(86) International application number: PCT/CN2024/105018
(87) International publication number: WO 2025/060620

(57) **Abstract**

A CDK inhibitor having a structure of formula (I), a pharmaceutical composition thereof, the use thereof as a CDK inhibitor, and the use thereof in the preparation of a drug for treating cancers or tumors at least partially associated with CDK. The various substituents in formula (I) are as defined in the description.

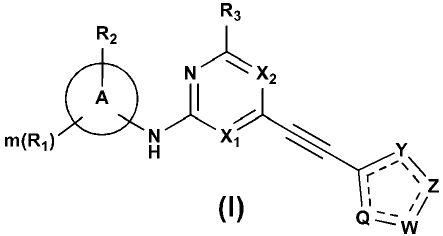

## Description

### TECHNICAL FIELD

The present invention pertains to the field of drug synthesis, and in particular relates to a CDK kinase inhibitor and the use thereof.

### BACKGROUND

Cyclin-dependent kinases (CDKs) are a group of important serine/threonine kinases that play crucial roles in cell cycle regulation and transcriptional processes. The human genome encodes more than 20 CDKs, which can be divided into two sub-families: cell cycle-related CDKs (CDK1-7 and 14-18) and transcription-related CDKs (CDK7-13, 19, and 20). Different CDKs interact with different cyclins to regulate multiple phases of the cell cycle or perform other functions.

Dysregulation of CDK activity is closely related to the occurrence and development of tumors. Among them, CDK4 and CDK6 are key regulators at the G1/S checkpoint of the cell cycle, and they are regulated by D-type cyclins and endogenous CDK inhibitors of the INK4 family (such as p16^{INK4a}). It has been reported that the dysregulation of the cyclin D-CDK4/6-INK4-retinoblastoma protein (Rb) signaling pathway is associated with resistance to endocrine therapy.

Breast cancer is the most prevalent malignancy in women, with over 2.3 million new cases diagnosed worldwide each year. Hormone receptor (HR)-positive, human epidermal growth factor receptor 2 (HER2)-negative breast cancer accounts for 60%-70% of all breast cancer subtypes. Endocrine therapy is one of the main treatment options, but resistance can occur. In recent years, with ongoing research into overcoming endocrine therapy resistance, CDK4/6 inhibitors have attracted considerable attention since their discovery. CDK4/6 inhibitors suppress the uncontrolled proliferation of tumor cells by selectively inhibiting CDK4/6 kinase activity and blocking the G1/S phase transition. Additionally, CDK4/6 inhibitors inhibit the expression of the estrogen receptor signaling pathway, creating a synergistic effect with endocrine therapy to delay and reverse resistance to endocrine therapy. The development of CDK4/6 inhibitors has altered the traditional model of endocrine therapy. The combination of CDK4/6 inhibitors and endocrine therapy has demonstrated significant efficacy in HR-positive HER2-negative breast cancer patients, establishing it as a new standard for first- and second-line treatment of HR-positive HER2-negative breast cancer.

However, clinical observations show that the treatment with CDK4/6 inhibitors may lead to adverse reactions such as gastrointestinal and/or hematological toxicity, and acquired resistance may develop with prolonged treatment. Increasing evidence shows that the hematological toxicity observed clinically is associated with CDK6/cyclin D3. Moreover, studies have identified CDK4 as a single oncogenic driver in many breast cancers. In summary, CDK4-selective inhibitors may offer better safety and efficacy than CDK4/6 inhibitors. Therefore, the development of CDK4-selective inhibitors has great clinical application value.

### SUMMARY

The purpose of the present invention is to provide a CDK kinase inhibitor and the use thereof. The compounds of the present invention exhibit potent inhibitory effects on CDK kinases, especially a selective inhibitory effect on CDK4 kinase, and can be widely used in the manufacture of a medicament for the treatment of cancers or tumors associated at least in part with CDK.

A first aspect of the present invention provides a compound of formula (I), a stereoisomer or pharmaceutically acceptable salt thereof: 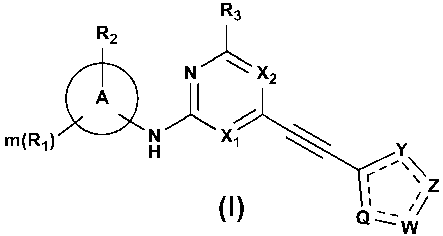
wherein, "-̅ -̅ -̅" represents a double or single bond;
X₁ is CR₄ or N; X₂ is CR₅ or N;
Y is N, CR₆ₐ, NR_{6b}, CR_{6c}R_{6d}, O or S;
Z is N, CR₇ₐ, NR_{7b}, CR_{7c}R_{7d}, O or S;
W is N, CR₈ₐ, NR_{8b}, CR_{8c}R_{8d}, O or S;
Q is N, CR₉ₐ, NR_{9b}, CR_{9c}R_{9d}, O or S;
ring A is C₃₋₁₀ cycloalkyl, 3-10 membered heterocyclyl, C₆₋₁₀ aryl or 5-10 membered heteroaryl;
each R₁ is independently selected from the group consisting of hydrogen, deuterium, halogen, cyano, nitro, azido, C₁₋₁₀ alkyl, C₂₋₁₀ alkenyl, C₂₋₁₀ alkynyl, C₃₋₁₀ cycloalkyl, 3-10 membered heterocyclyl, C₆₋₁₀ aryl, 5-10 membered heteroaryl, C₃₋₁₀ cycloalkyl-substituted C₁₋₄ alkyl, 3-10 membered heterocyclyl-substituted C₁₋₄ alkyl, C₆₋₁₀ aryl-substituted C₁₋₄ alkyl, 5-10 membered heteroaryl-substituted C₁₋₄ alkyl, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅, the above groups are independently optionally further substituted with one or more substituents selected from the group consisting of deuterium, halogen, cyano, nitro, azido, C₁₋₁₀ alkyl, C₂₋₁₀ alkenyl, C₂₋₁₀ alkynyl, C₃₋₁₀ cycloalkyl, 3-10 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, - C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅, the above groups are independently optionally more further substituted with one or more substituents selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₀ cycloalkyl, 3-10 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkylS(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, - C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅;
R₂ is selected from the group consisting of hydrogen, deuterium, halogen, cyano, nitro, azido, C₁₋₄ alkyl, C₁₋₄ haloalkyl, C₁₋₄ deuterioalkyl" C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkylS(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, - C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅, provided that R₂ is not hydrogen when ring A is C₃₋₆ cycloalkyl or 3-6 membered heterocyclyl;
R₃ is selected from the group consisting of hydrogen, deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₀ cycloalkyl, 3-10 membered heterocyclyl, C₆₋₁₀ aryl, 5-10 membered heteroaryl, -C₀₋₈ alkyl-SF₅, -C₀₋₈ alkylS(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅;
R₄ is selected from the group consisting of hydrogen, deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₀ cycloalkyl, 3-10 membered heterocyclyl, C₆₋₁₀ aryl, 5-10 membered heteroaryl, -C₀₋₈ alkyl-SF₅, -C₀₋₈ alkylS(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅;
R₅ is selected from the group consisting of hydrogen, deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₀ cycloalkyl, 3-10 membered heterocyclyl, C₆₋₁₀ aryl, 5-10 membered heteroaryl, -C₀₋₈ alkyl-SF₅, -C₀₋₈ alkylS(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅;
R₆ₐ, R₇ₐ, R₈ₐ and R₉ₐ are each independently selected from the group consisting of hydrogen, deuterium, halogen, cyano, nitro, azido, C₁₋₁₀ alkyl, C₂₋₁₀ alkenyl, C₂₋₁₀ alkynyl, C₃₋₁₀ cycloalkyl, 3-10 membered heterocyclyl, C₆₋₁₀ aryl, 5-10 membered heteroaryl, -C₀₋₈ alkyl-SF₅, -C₀₋₈ alkylS(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅, the above groups are independently optionally further substituted with one or more substituents selected from the group consisting of deuterium, halogen, cyano, nitro, azido, C₁₋₁₀ alkyl, C₂₋₁₀ alkenyl, C₂₋₁₀ alkynyl, C₃₋₁₀ cycloalkyl, 3-10 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, - C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅, the above groups are independently optionally further substituted with one or more substituents selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₀ cycloalkyl, 3-10 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅;
R_{6b}, R_{7b}, R_{8b} and R_{9b} are each independently selected from the group consisting of hydrogen, deuterium, C₁₋₁₀ alkyl, C₂₋₁₀ alkenyl, C₂₋₁₀ alkynyl, C₃₋₁₀ cycloalkyl, 3-10 membered heterocyclyl, C₆₋₁₀ aryl, 5-10 membered heteroaryl, -C₀₋₈ alkyl-S(O)(=N-R₁₀)R₁₁, -C₀₋₈ alkyl-S(O)ᵣR₁₃, -C₀₋₈ alkyl-C(O)OR₁₄, -C₀₋₈ alkyl-C(O)SR₁₄, -C₀₋₈ alkyl-C(O)R₁₅, -C₀₋₈ alkyl-P(O)(R₁₅)₂, -C₀₋₈ alkyl-C(=NR₁₆)R₁₅ and -C₀₋₈ alkyl-C(O)NR₁₆R₁₇, the above groups are independently optionally further substituted with one or more substituents selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₀ cycloalkyl, 3-10 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅;
or, R_{6b} and R₇ₐ together with a moiety to which they are directly attached form 3-10 membered heterocyclyl or 5-10 membered heteroaryl, the above 3-10 membered heterocyclyl or 5-10 membered heteroaryl is independently optionally further substituted with one or more substituents selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₀ cycloalkyl, 3-10 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, - C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅;
or, R₆ₐ and R_{7b} together a the moiety to which they are directly attached form 3-10 membered heterocyclyl or 5-10 membered heteroaryl, the above 3-10 membered heterocyclyl or 5-10 membered heteroaryl is independently optionally further substituted with one or more substituents selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₀ cycloalkyl, 3-10 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkylS(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, - C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅;
R_{6c}, R_{6d}, R_{7c}, R_{7d}, R_{8c}, R_{8d}, R_{9c} and R_{9d} are each independently selected from the group consisting of hydrogen, deuterium, halogen, cyano, nitro, azido, C₁₋₁₀ alkyl, C₂₋₁₀ alkenyl, C₂₋₁₀ alkynyl, C₃₋₁₀ cycloalkyl, 3-10 membered heterocyclyl, C₆₋₁₀ aryl, 5-10 membered heteroaryl, - C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, - C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅, the above groups are independently optionally further substituted with one or more substituents selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₀ cycloalkyl, 3-10 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅;
each R₁₀ is independently selected from the group consisting of hydrogen, deuterium, C₁₋₁₀ alkyl, C₂₋₁₀ alkenyl, C₂₋₁₀ alkynyl, C₃₋₁₀ cycloalkyl, 3-10 membered heterocyclyl, C₆₋₁₀ aryl, 5-10 membered heteroaryl, -C₀₋₈ alkyl-S(O)ᵣR₁₃, -C₀₋₈ alkyl-C(O)OR₁₄, -C₀₋₈ alkyl-C(O)R₁₅ and -C₀₋₈ alkyl-C(O)NR₁₆R₁₇, the above groups are optionally further substituted with one or more substituents selected from the group consisting of deuterium, halogen, cyano, nitro, azido, C₁₋₁₀ alkyl, C₂₋₁₀ alkenyl, C₂₋₁₀ alkynyl, C₁₋₁₀ haloalkyl, C₁₋₁₀ deuterioalkyl, C₃₋₁₀ cycloalkyl, 3-10 membered heterocyclyl, C₆₋₁₀ aryl, 5-10 membered heteroaryl, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)R₁₅, -C₀₋₈ alkyl-O-C(O)R₁₅, -C₀₋₈ alkyl-NR₁₆R₁₇, - C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅;
each R₁₁ and each R₁₂ are independently selected from the group consisting of hydrogen, deuterium, hydroxyl, C₁₋₁₀ alkyl, C₂₋₁₀ alkenyl, C₂₋₁₀ alkynyl, C₃₋₁₀ cycloalkyl, 3-10 membered heterocyclyl, C₆₋₁₀ aryl and 5-10 membered heteroaryl, or R₁₁ and R₁₂ together with a sulfur atom to which they are directly attached form 3-10 membered heterocyclyl, the above groups are optionally further substituted with one or more substituents selected from the group consisting of deuterium, halogen, cyano, nitro, azido, C₁₋₁₀ alkyl, C₂₋₁₀ alkenyl, C₂₋₁₀ alkynyl, C₁₋₁₀ haloalkyl, C₁₋₁₀ deuterioalkyl, C₃₋₁₀ cycloalkyl, 3-10 membered heterocyclyl, C₆₋₁₀ aryl, 5-10 membered heteroaryl, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)R₁₅, - C₀₋₈ alkyl-O-C(O)R₁₅, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅;
each R₁₃ is independently selected from the group consisting of hydrogen, deuterium, hydroxyl, C₁₋₁₀ alkyl, C₂₋₁₀ alkenyl, C₃₋₁₂ cycloalkyl, 3-12 membered heterocyclyl, C₆₋₁₀ aryl, 5-10 membered heteroaryl and -NR₁₆R₁₇, the above groups are independently optionally further substituted with one or more substituents selected from the group consisting of deuterium, halogen, cyano, hydroxyl, =O, C₁₋₁₀ alkyl, C₁₋₁₀ alkoxyl, C₃₋₁₂ cycloalkyl, C₃₋₁₂ cycloalkoxyl, 3-12 membered heterocyclyl, 3-12 membered heterocycloxyl, C₆₋₁₀ aryl, C₆₋₁₀ aryloxyl, 5-10 membered heteroaryl, 5-10 membered heteroaryloxyl and -NR₁₆R₁₇;
each R₁₄ is independently selected from the group consisting of hydrogen, deuterium, C₁₋₁₀ alkyl, C₂₋₁₀ alkenyl, C₃₋₁₂ cycloalkyl, 3-12 membered heterocyclyl, C₆₋₁₀ aryl and 5-10 membered heteroaryl, the above groups are independently optionally further substituted with one or more substituents selected from the group consisting of deuterium, halogen, hydroxyl, =O, cyano, C₁₋₁₀ alkyl, C₁₋₁₀ alkoxyl, C₃₋₁₂ cycloalkyl, C₃₋₁₂ cycloalkoxyl, 3-12 membered heterocyclyl, 3-12 membered heterocycloxyl, C₆₋₁₀ aryl, C₆₋₁₀ aryloxyl, 5-10 membered heteroaryl, 5-10 membered heteroaryloxyl and -NR₁₆R₁₇;
each R₁₅ is independently selected from the group consisting of hydrogen, deuterium, hydroxyl, C₁₋₁₀ alkyl, C₁₋₁₀ alkoxyl, C₂₋₁₀ alkenyl, C₂₋₁₀ alkynyl, C₃₋₁₂ cycloalkyl, C₃₋₁₂ cycloalkoxyl, 3-12 membered heterocyclyl, 3-12 membered heterocycloxyl, C₆₋₁₀ aryl, C₆₋₁₀ aryloxyl, 5-10 membered heteroaryl, 5-10 membered heteroaryloxyl and -NR₁₆R₁₇, the above groups are independently optionally further substituted with one or more substituents selected from the group consisting of deuterium, halogen, hydroxyl, =O, cyano, C₁₋₁₀ alkyl, C₁₋₁₀ alkoxyl, C₃₋₁₂ cycloalkyl, C₃₋₁₂ cycloalkoxyl, 3-12 membered heterocyclyl, 3-12 membered heterocycloxyl, C₆₋₁₀ aryl, C₆₋₁₀ aryloxyl, 5-10 membered heteroaryl, 5-10 membered heteroaryloxyl and -NR₁₆R₁₇;
each R₁₆ and each R₁₇ are independently selected from the group consisting of hydrogen, deuterium, hydroxyl, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, sulfinyl, sulfonyl, methylsulfonyl, isopropylsulfonyl, cyclopropylsulfonyl, p-toluenesulfonyl, aminosulfonyl, dimethylaminosulfonyl and C₁₋₁₀ alkanoyl, the above groups are independently optionally further substituted with one or more substituents selected from the group consisting of deuterium, halogen, hydroxyl, =O, C₁₋₁₀ alkyl, C₂₋₁₀ alkenyl, C₂₋₁₀ alkynyl, C₁₋₁₀ haloalkyl, C₁₋₁₀ deuterioalkyl, C₁₋₁₀ alkoxyl, C₃₋₁₂ cycloalkyl, C₃₋₁₂ cycloalkoxyl, 3-12 membered heterocyclyl, 3-12 membered heterocycloxyl, C₆₋₁₀ aryl, C₆₋₁₀ aryloxyl, 5-10 membered heteroaryl, 5-10 membered heteroaryloxyl, amino, mono(C₁₋₁₀ alkyl)amino, di(C₁₋₁₀ alkyl)amino and C₁₋₁₀ alkanoyl;
or, R₁₆ and R₁₇ together with a nitrogen atom to which they are directly attached form 4-10 membered heterocyclyl or 5-10 membered heteroaryl, the above 4-10 membered heterocyclyl or 5-10 membered heteroaryl is optionally further substituted with one or more substituents selected from the group consisting of deuterium, halogen, hydroxyl, =O, C₁₋₁₀ alkyl, C₂₋₁₀ alkenyl, C₂₋₁₀ alkynyl, C₁₋₁₀ haloalkyl, C₁₋₁₀ deuterioalkyl, C₁₋₁₀ alkoxyl, C₃₋₁₂ cycloalkyl, C₃₋₁₂ cycloalkoxyl, 3-12 membered heterocyclyl, 3-12 membered heterocycloxyl, C₆₋₁₀ aryl, C₆₋₁₀ aryloxyl, 5-10 membered heteroaryl, 5-10 membered heteroaryloxyl, amino, mono(C₁₋₁₀ alkyl)amino, di(C₁₋₁₀ alkyl)amino and C₁₋₁₀ alkanoyl;
m is 0, 1, 2 or 3; and each r is independently 0, 1 or 2.

As a preferred embodiment, in the compound of formula (I), the stereoisomer or pharmaceutically acceptable salt thereof, each R₁ is independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, C₃₋₆ cycloalkyl-substituted C₁₋₄ alkyl, 3-6 membered heterocyclyl-substituted C₁₋₄ alkyl, C₆₋₈ aryl-substituted C₁₋₄ alkyl, 5-8 membered heteroaryl-substituted C₁₋₄ alkyl, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkylS(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, - C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅, the above groups are independently optionally further substituted with one or more substituents selected from the group consisting of deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅, the above groups are independently optionally more further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅;
R₂ is selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -OR₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, - C(=NR₁₆)R₁₅, -N(R₁₆)-C(=NR₁₇)R₁₅, -C(O)NR₁₆R₁₇ and -N(R₁₆)-C(O)R₁₅, provided that R₂ is not hydrogen when ring A is C₃₋₆ cycloalkyl or 3-6 membered heterocyclyl;
R₃ is selected from the group consisting of hydrogen, deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-SF₅, -C₀₋₄ alkyl-S(O)(=N-R₁₀)R₁₁, - C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, - C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅;
R₄ is selected from the group consisting of hydrogen, deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-SF₅, -C₀₋₄ alkyl-S(O)(=N-R₁₀)R₁₁, - C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, - C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₈, -C₀₋₄ alkyl-C(O)NR₁₆R₁₇ and -C₀₋₄ alkyl-N(R₁₆)-C(O)R₁₅;
R₅ is selected from the group consisting of hydrogen, deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-SF₅, -C₀₋₄ alkyl-S(O)(=N-R₁₀)R₁₁, - C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, - C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₈, -C₀₋₄ alkyl-C(O)NR₁₆R₁₇ and -C₀₋₄ alkyl-N(R₁₆)-C(O)R₁₅-
R₆ₐ, R₇ₐ, R₈ₐ and R₉ₐ are each independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-SF₅, -C₀₋₄ alkyl-S(O)(=N-R₁₀)R₁₁, - C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, - C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₈, -C₀₋₄ alkyl-C(O)NR₁₆R₁₇ and -C₀₋₄ alkyl-N(R₁₆)-C(O)R₁₅, the above groups are independently optionally further substituted with one or more substituents selected from the group consisting of deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅, the above groups are independently optionally more further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅;
R_{6b}, R_{7b}, R_{8b} and R_{9b} are each independently selected from the group consisting of hydrogen, deuterium, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-S(O)(=N-R₁₀)R₁₁, -C₀₋₄ alkyl-S(O)ᵣR₁₃, -C₀₋₄ alkyl-C(O)OR₁₄, -C₀₋₄ alkyl-C(O)SR₁₄, -C₀₋₄ alkyl-C(O)R₁₅, -C₀₋₄ alkyl-P(O)(R₁₅)₂, -C₀₋₄ alkyl-C(=NR₁₆)R₁₅ and -C₀₋₄ alkyl-C(O)NR₁₆R₁₇, the above groups are independently optionally further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅;
or, R_{6b} and R₇ₐ together with a moiety to which they are directly attached form 3-8 membered heterocyclyl or 5-8 membered heteroaryl, the above 3-8 membered heterocyclyl or 5-8 membered heteroaryl is independently optionally further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, - C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₈, -C₀₋₄ alkyl-C(O)NR₁₆R₁₇ and -C₀₋₄ alkyl-N(R₁₆)-C(O)R₁₅;
or, R₆ₐ and R_{7b} together with a moiety to which they are directly attached form 3-8 membered heterocyclyl or 5-8 membered heteroaryl, the above 3-8 membered heterocyclyl or 5-8 membered heteroaryl is independently optionally further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, - C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₈, -C₀₋₄ alkyl-C(O)NR₁₆R₁₇ and -C₀₋₄ alkyl-N(R₁₆)-C(O)R₁₅;
wherein, R₁₀, R₁₁, R₁₂, R₁₃, R₁₄, R₁₅, R₁₆, R₁₇ and r are defined as those in the compound of formula (I).

As a preferred embodiment, in the compound of formula (I), the stereoisomer or pharmaceutically acceptable salt thereof, each R₁₀ is independently selected from the group consisting of hydrogen, deuterium, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-S(O)ᵣR₁₃, -C₀₋₄ alkyl-C(O)OR₁₄, -C₀₋₄ alkyl-C(O)R₁₅ and -C₀₋₄ alkyl-C(O)NR₁₆R₁₇, the above groups are independently optionally further substituted with one or more substituents selected from the group consisting of deuterium, halogen, cyano, C₁₋₄ alkyl, C₂₋₄ alkenyl, C₂₋₄ alkynyl, C₁₋₄ haloalkyl, C₁₋₄ deuterioalkyl, C₃₋₆ cycloalkyl, 3-6 membered heterocyclyl, C₆₋₈ aryl, 5-8 membered heteroaryl, -C₀₋₄ alkylS(O)ᵣR₁₃, -C₀₋₄ alkyl-O-R₁₄, -C₀₋₄ alkyl-C(O)OR₁₄, -C₀₋₄ alkyl-C(O)R₁₅, -C₀₋₄ alkyl-O-C(O)R₁₅, - C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅;
each R₁₁ and each R₁₂ are independently selected from the group consisting of hydrogen, deuterium, hydroxyl, C₁₋₄ alkyl, C₂₋₄ alkenyl, C₂₋₄ alkynyl, C₃₋₆ cycloalkyl, 3-6 membered heterocyclyl, C₆₋₈ aryl and 5-8 membered heteroaryl, or R₁₁ and R₁₂ together with a sulfur atom to which they are directly attached form 3-8 membered heterocyclyl, the above groups are optionally further substituted with one or more substituents selected from the group consisting of deuterium, halogen, cyano, C₁₋₄ alkyl, C₂₋₄ alkenyl, C₂₋₄ alkynyl, C₁₋₄ haloalkyl, C₁₋₄ deuterioalkyl, C₃₋₆ cycloalkyl, 3-6 membered heterocyclyl, C₆₋₈ aryl, 5-8 membered heteroaryl, -C₀₋₄ alkylS(O)ᵣR₁₃, -C₀₋₄ alkyl-O-R₁₄, -C₀₋₄ alkyl-C(O)OR₁₄, -C₀₋₄ alkyl-C(O)R₁₅, -C₀₋₄ alkyl-O-C(O)R₁₅, - C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅-
each R₁₃ is independently selected from the group consisting of hydrogen, deuterium, hydroxyl, C₁₋₄ alkyl, C₂₋₄ alkenyl, C₃₋₆ cycloalkyl, 3-6 membered heterocyclyl, C₆₋₈ aryl, 5-8 membered heteroaryl and -NR₁₆R₁₇, the above groups are independently optionally further substituted with one or more substituents selected from the group consisting of deuterium, halogen, cyano, hydroxyl, =O, C₁₋₄ alkyl, C₁₋₄ alkoxyl, C₃₋₆ cycloalkyl, C₃₋₆ cycloalkoxyl, 3-6 membered heterocyclyl, 3-6 membered heterocycloxyl, C₆₋₈ aryl, C₆₋₈ aryloxyl, 5-8 membered heteroaryl, 5-8 membered heteroaryloxyl and -NR₁₆R₁₇;
each R₁₄ is independently selected from the group consisting of hydrogen, deuterium, C₁₋₄ alkyl, C₂₋₄ alkenyl, C₃₋₆ cycloalkyl, 3-6 membered heterocyclyl, C₆₋₈ aryl and 5-8 membered heteroaryl, the above groups are independently optionally further substituted with one or more substituents selected from the group consisting of deuterium, halogen, hydroxyl, =O, cyano, C₁₋₄ alkyl, C₁₋₄ alkoxyl, C₃₋₆ cycloalkyl, C₃₋₆ cycloalkoxyl, 3-6 membered heterocyclyl, 3-6 membered heterocycloxyl, C₆₋₈ aryl, C₆₋₈ aryloxyl, 5-8 membered heteroaryl, 5-8 membered heteroaryloxyl and -NR₁₆R₁₇;
each R₁₅ is independently selected from the group consisting of hydrogen, deuterium, hydroxyl, C₁₋₄ alkyl, C₁₋₄ alkoxyl, C₂₋₄ alkenyl, C₂₋₄ alkynyl, C₃₋₆ cycloalkyl, C₃₋₆ cycloalkoxyl, 3-6 membered heterocyclyl, 3-6 membered heterocycloxyl, C₆₋₈ aryl, C₆₋₈ aryloxyl, 5-8 membered heteroaryl, 5-8 membered heteroaryloxyl and -NR₁₆R₁₇, the above groups are independently optionally further substituted with one or more substituents selected from the group consisting of deuterium, halogen, hydroxyl, =O, cyano, C₁₋₄ alkyl, C₁₋₄ alkoxyl, C₃₋₆ cycloalkyl, C₃₋₆ cycloalkoxyl, 3-6 membered heterocyclyl, 3-6 membered heterocycloxyl, C₆₋₈ aryl, C₆₋₈ aryloxyl, 5-8 membered heteroaryl, 5-8 membered heteroaryloxyl and -NR₁₆R₁₇;
each R₁₆ and each R₁₇ are independently selected from the group consisting of hydrogen, deuterium, hydroxyl, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, sulfinyl, sulfonyl, methylsulfonyl, isopropylsulfonyl, cyclopropylsulfonyl, p-toluenesulfonyl, aminosulfonyl, dimethylaminosulfonyl and C₁₋₄ alkanoyl, the above groups are independently optionally further substituted with one or more substituents selected from the group consisting of deuterium, halogen, hydroxyl, =O, C₁₋₄ alkyl, C₂₋₄ alkenyl, C₂₋₄ alkynyl, C₁₋₄ haloalkyl, C₁₋₄ deuterioalkyl, C₁₋₄ alkoxyl, C₃₋₆ cycloalkyl, C₃₋₆ cycloalkoxyl, 3-6 membered heterocyclyl, 3-6 membered heterocycloxyl, C₆₋₈ aryl, C₆₋₈ aryloxyl, 5-8 membered heteroaryl, 5-8 membered heteroaryloxyl, amino, mono(C₁₋₄ alkyl)amino, di(C₁₋₄ alkyl)amino and C₁₋₄ alkanoyl;
or, R₁₆ and R₁₇ together with a nitrogen atom to which they are directly attached form 4-6 membered heterocyclyl or 5-8 membered heteroaryl, the above 4-6 membered heterocyclyl or 5-8 membered heteroaryl is optionally further substituted with one or more substituents selected from the group consisting of deuterium, halogen, hydroxyl, =O, C₁₋₄ alkyl, C₂₋₄ alkenyl, C₂₋₄ alkynyl, C₁₋₄ haloalkyl, C₁₋₄ deuterioalkyl, C₁₋₄ alkoxyl, C₃₋₆ cycloalkyl, C₃₋₆ cycloalkoxyl, 3-6 membered heterocyclyl, 3-6 membered heterocycloxyl, C₆₋₈ aryl, C₆₋₈ aryloxyl, 5-8 membered heteroaryl, 5-8 membered heteroaryloxyl, amino, mono(C₁₋₄ alkyl)amino, di(C₁₋₄ alkyl)amino and C₁₋₄ alkanoyl.

As a further preferred embodiment, in the compound of formula (I), the stereoisomer or pharmaceutically acceptable salt thereof, the compound of formula (I) is a compound of formula (IIa1), (IIa2), (IIa3) or (IIa4) as follows: 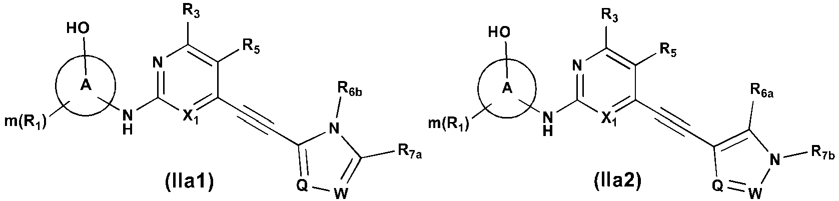 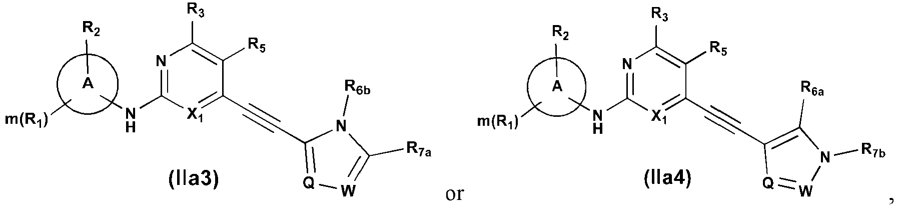
wherein, each X₁ is independently CR₄ or N;
each W is independently N or CR₈ₐ;
each Q is independently N or CR₉ₐ;
each ring A is independently C₅₋₈ cycloalkyl or 5-8 membered heterocyclyl;
each R₁ is independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, - P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅, the above groups are independently optionally further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, - P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅;
each R₂ is independently selected from the group consisting of halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₁₋₄ alkoxyl, C₁₋₄ haloalkoxyl, C₁₋₄ deuterioalkoxyl, -SF₅, -C(O)OR₁₄, -C(O)R₁₅, - O-C(O)R₁₅, -NR₁₆R₁₇, -C(O)NR₁₆R₁₇ and -N(R₁₆)-C(O)R₁₅;
each R₃ is independently selected from the group consisting of hydrogen, deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, -SF₅, -O-R₁₄ and -NR₁₆R₁₇;
each R₄ is independently selected from the group consisting of hydrogen, deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, -SF₅, -O-R₁₄ and -NR₁₆R₁₇;
each R₅ is independently selected from the group consisting of hydrogen, deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, -SF₅, -O-R₁₄ and -NR₁₆R₁₇;
each R₆ₐ and each R₇ₐ are independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -S(O)(=N-R₁₀)R₁₁, -N=S(O)R₁₁R₁₂, - N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and - N(R₁₆)-C(O)R₁₅, the above groups are independently optionally further substituted with one or more substituents selected from the group consisting of deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₁₀)R₁₁, -N=S(O)R₁₁R₁₂, -N=SR₁₁R₁₂, -O-S(O)₂R₁₃, - S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, - NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅, the above groups are independently optionally more further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, - P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅;
each R_{6b} and each R_{7b} are independently selected from the group consisting of hydrogen, deuterium, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -S(O)(=N-R₁₀)R₁₁, -S(O)ᵣR₁₃, -C(O)OR₁₄, -C(O)SR₁₄, -C(O)R₁₅, -P(O)(R₁₅)₂, -C(=NR₁₆)R₁₅ and -C(O)NR₁₆R₁₇, the above groups are independently optionally further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, - S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, - C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅;
or, in the compound of formula (IIa1) or the compound of formula (IIa3), R_{6b} and R₇ₐ together with the moiety to which they are directly attached form 3-8 membered heterocyclyl or 5-8 membered heteroaryl, the above 3-8 membered heterocyclyl or 5-8 membered heteroaryl is independently optionally further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, =O, =S, -SF₅, -S(O)ᵣR₁₃, -O-R₁₄, - C(O)OR₁₄, -C(O)R₁₅, -O-C(O)R₁₅ and -NR₁₆R₁₇;
or, in the compound of formula (IIa2) or the compound of formula (IIa4), R₆ₐ and R_{7b} together with the moiety to which they are directly attached form 3-8 membered heterocyclyl or 5-8 membered heteroaryl, the above 3-8 membered heterocyclyl or 5-8 membered heteroaryl is independently optionally further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, =O, =S, -SF₅, -S(O)ᵣR₁₃, -O-R₁₄, - C(O)OR₁₄, -C(O)R₁₅, -O-C(O)R₁₅ and -NR₁₆R₁₇;
each R₈ₐ and each R₉ₐ are independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -S(O)(=N-R₁₀)R₁₁, -N=S(O)R₁₁R₁₂, - N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and - N(R₁₆)-C(O)R₁₅, the above groups are independently optionally further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₁₀)R₁₁, - N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, - C(O)NR₁₆R₁₇ and -N(R₁₆)-C(O)R₁₅;
wherein, R₁₀, R₁₁, R₁₂, R₁₃, R₁₄, R₁₅, R₁₆, R₁₇, m and r are defined as those in the compound of formula (I).

As a further preferred embodiment, in the compound of formula (I), the stereoisomer or pharmaceutically acceptable salt thereof, the compound of formula (I) is a compound of formula (IIIa1), (IIIa2), (IIIa3) or (IIIa4) as follows: 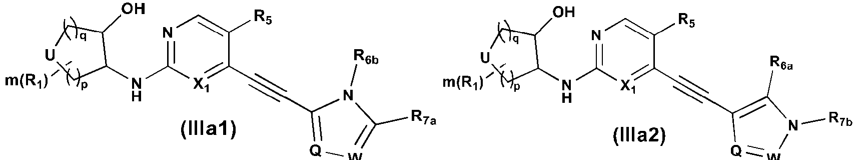 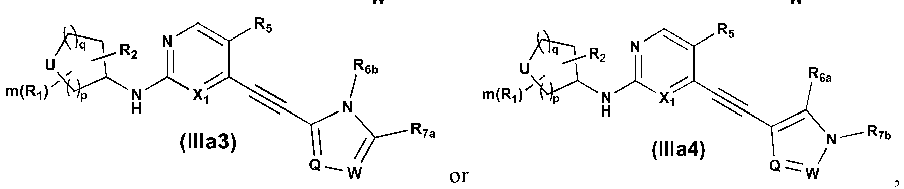 wherein, each X₁ isindependently CR₄ or N;
each W is independently N or CR₈ₐ;
each Q is independently N or CR₉ₐ;
each U is independently NR₁ₐ, CR_{1b}R_{1c}, O or S;
each p is independently 1, 2 or 3; each q is independently 0, 1, 2 or 3;
each R₁ is independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, -SF₅, -S(O)(=N-R₁₀)R₁₁, -N=S(O)R₁₁R₁₂, -S(O)ᵣR₁₃, -O-R₁₄, -C(O)OR₁₄, -C(O)R₁₅, -NR₁₆R₁₇, - C(O)NR₁₆R₁₇ and -N(R₁₆)-C(O)R₁₅, the above groups are independently optionally further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, =O, =S, -SF₅, -S(O)(=N-R₁₀)R₁₁, -N=S(O)R₁₁R₁₂, - S(O)ᵣR₁₃, -O-R₁₄, -C(O)OR₁₄, -C(O)R₁₅, -NR₁₆R₁₇, -C(O)NR₁₆R₁₇ and -N(R₁₆)-C(O)R₁₅;
each R₁ₐ is independently selected from the group consisting of hydrogen, deuterium, C₁₋₄ alkyl, C₂₋₄ alkenyl, C₂₋₄ alkynyl, C₃₋₆ cycloalkyl, 3-6 membered heterocyclyl, -S(O)(=N-R₁₀)R₁₁, -S(O)ᵣR₁₃, -C(O)OR₁₄, -C(O)R₁₅ and -C(O)NR₁₆R₁₇, the above groups are independently optionally further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, =O, =S, -SF₅, -S(O)(=N-R₁₀)R₁₁, -N=S(O)R₁₁R₁₂, - S(O)ᵣR₁₃, -O-R₁₄, -C(O)OR₁₄, -C(O)R₁₅, -NR₁₆R₁₇, -C(O)NR₁₆R₁₇ and -N(R₁₆)-C(O)R₁₅;
each R_{1b} and each R_{1c} are independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, -SF₅, -S(O)(=N-R₁₀)R₁₁, -N=S(O)R₁₁R₁₂, -S(O)ᵣR₁₃, -O-R₁₄, -C(O)OR₁₄, -C(O)R₁₅, -NR₁₆R₁₇, -C(O)NR₁₆R₁₇ and -N(R₁₆)-C(O)R₁₅, the above groups are independently optionally further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, =O, =S, -SF₅, -S(O)(=N-R₁₀)R₁₁, -N=S(O)R₁₁R₁₂, - S(O)ᵣR₁₃, -O-R₁₄, -C(O)OR₁₄, -C(O)R₁₅, -NR₁₆R₁₇, -C(O)NR₁₆R₁₇ and -N(R₁₆)-C(O)R₁₅;
each R₂ is independently selected from the group consisting of halogen, cyano, C₁₋₄ alkyl, C₁₋₄ haloalkyl, C₁₋₄ deuterioalkyl, C₁₋₄ alkoxyl, C₁₋₄ haloalkoxyl, C₁₋₄ deuterioalkoxyl, -NR₁₆R₁₇, -C(O)NR₁₆R₁₇ and -N(R₁₆)-C(O)R₁₅;
each R₄ is independently selected from the group consisting of hydrogen, deuterium, fluoro, chloro, cyano, methyl, ethyl, n-propyl, isopropyl, methoxy, ethoxy, n-propoxy, isopropoxy, trifluoromethyl, trideuteromethyl, trifluoromethoxy, trideuteromethoxy, vinyl, ethynyl, cyclopropyl, cyclobutyl, cyclopropoxy, cyclobutoxy, oxetanyl, azetidinyl, -SF₅, hydroxyl, amino, methylamino and dimethylamino;
each R₅ is independently selected from the group consisting of hydrogen, deuterium, fluoro, chloro, cyano, methyl, ethyl, n-propyl, isopropyl, methoxy, ethoxy, n-propoxy, isopropoxy, trifluoromethyl, trideuteromethyl, trifluoromethoxy, trideuteromethoxy, vinyl, ethynyl, cyclopropyl, cyclobutyl, cyclopropoxy, cyclobutoxy, oxetanyl, azetidinyl, -SF₅, hydroxyl, amino, methylamino and dimethylamino;
each R₆ₐ and each R₇ₐ are independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, -SF₅, -S(O)ᵣR₁₃, -O-R₁₄, -C(O)OR₁₄, -C(O)R₁₅, -O-C(O)R₁₅, -NR₁₆R₁₇, - C(O)NR₁₆R₁₇ and -N(R₁₆)-C(O)R₁₅, the above groups are independently optionally further substituted with one or more substituents selected from the group consisting of deuterium, halogen, cyano, C₁₋₄ alkyl, C₂₋₄ alkenyl, C₂₋₄ alkynyl, C₃₋₆ cycloalkyl, 3-6 membered heterocyclyl, =O, =S, -SF₅, -S(O)ᵣR₁₃, -O-R₁₄, -C(O)OR₁₄, -C(O)R₁₅, -O-C(O)R₁₅, -NR₁₆R₁₇, -C(O)NR₁₆R₁₇ and -N(R₁₆)-C(O)R₁₅, the above groups are independently optionally more further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, =O, =S, -SF₅, -S(O)ᵣR₁₃, -O-R₁₄, -C(O)OR₁₄, -C(O)R₁₅, -O-C(O)R₁₅, -NR₁₆R₁₇, - C(O)NR₁₆R₁₇ and -N(R₁₆)-C(O)R₁₅;
R_{6b} and R_{7b} are each independently selected from the group consisting of hydrogen, deuterium, C₁₋₄ alkyl, C₂₋₄ alkenyl, C₂₋₄ alkynyl, C₃₋₆ cycloalkyl, 3-6 membered heterocyclyl, - S(O)ᵣR₁₃, -C(O)OR₁₄, -C(O)R₁₅ and -C(O)NR₁₆R₁₇, the above groups are independently optionally further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, =O, =S, -SF₅, -S(O)ᵣR₁₃, -O-R₁₄, -C(O)OR₁₄, - C(O)R₁₅, -O-C(O)R₁₅, -NR₁₆R₁₇, -C(O)NR₁₆R₁₇ and -N(R₁₆)-C(O)R₁₅;
or, in the compound of formula (IIIa1) or the compound of formula (IIIa3), R_{6b} and R₇ₐ together with the moiety to which they are directly attached form 3-8 membered heterocyclyl or 5-8 membered heteroaryl, the above 3-8 membered heterocyclyl or 5-8 membered heteroaryl is independently optionally further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, =O, =S, -SF₅, -S(O)ᵣR₁₃, -O-R₁₄, - C(O)OR₁₄, -C(O)R₁₅, -O-C(O)R₁₅ and -NR₁₆R₁₇;
or, in the compound of formula (IIIa2) or the compound of formula (IIIa4), R₆ₐ and R_{7b} together with the moiety to which they are directly attached form 3-8 membered heterocyclyl or 5-8 membered heteroaryl, the above 3-8 membered heterocyclyl or 5-8 membered heteroaryl is independently optionally further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, =O, =S, -SF₅, -S(O)ᵣR₁₃, -O-R₁₄, - C(O)OR₁₄, -C(O)R₁₅, -O-C(O)R₁₅ and -NR₁₆R₁₇;
each R₈ₐ and each R₉ₐ are independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, -SF₅, -S(O)ᵣR₁₃, -O-R₁₄, -C(O)OR₁₄, -C(O)R₁₅, -O-C(O)R₁₅, -NR₁₆R₁₇, - C(O)NR₁₆R₁₇ and -N(R₁₆)-C(O)R₁₅, the above groups are independently optionally further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, =O, =S, -SF₅, -S(O)ᵣR₁₃, -O-R₁₄, -C(O)OR₁₄, -C(O)R₁₅, - O-C(O)R₁₅, -NR₁₆R₁₇, -C(O)NR₁₆R₁₇ and -N(R₁₆)-C(O)R₁₅;
wherein, R₁₀, R₁₁, R₁₂, R₁₃, R₁₄, R₁₅, R₁₆, R₁₇, m and r are defined as those in the compound of formula (I).

As a still further preferred embodiment, in the compound of formula (I), the stereoisomer or pharmaceutically acceptable salt thereof, the compound of formula (I) is a compound of formula (IVa1), (IVa2), (IVa3) or (IVa4) as follows: 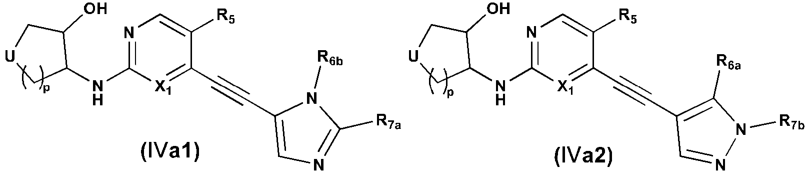 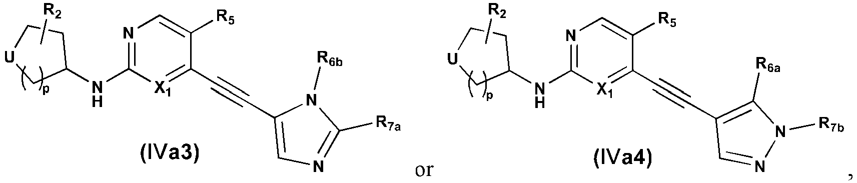
wherein, each X₁ is independently CH or N;
each U is independently NR₁ₐ, CH₂, O or S; each p is independently 1 or 2;
each R₁ₐ is independently selected from the group consisting of hydrogen, deuterium, C₁₋₄ alkyl, C₃₋₆ cycloalkyl, 3-6 membered heterocyclyl, methylsulfinyl, methylsulfonyl, isopropylsulfonyl, cyclopropylsulfonyl, aminosulfonyl and C₁₋₄ alkanoyl, the above groups are independently optionally further substituted with one or more substituents selected from the group consisting of deuterium, halogen, cyano, hydroxyl, carboxyl, C₁₋₄ alkyl, C₁₋₄ alkoxyl, C₁₋₄ haloalkyl, C₁₋₄ haloalkoxyl, C₁₋₄ deuterioalkyl, C₁₋₄ deuterioalkoxyl, C₃₋₆ cycloalkyl, C₃₋₆ cycloalkoxyl, 3-6 membered heterocyclyl, 3-6 membered heterocycloxyl, =O, =S, -NR₁₆R₁₇ and -C(O)NR₁₆R₁₇;
each R₂ is independently selected from the group consisting of halogen, cyano, C₁₋₄ alkyl, C₁₋₄ haloalkyl, C₁₋₄ deuterioalkyl, C₁₋₄ alkoxyl, C₁₋₄ haloalkoxyl, C₁₋₄ deuterioalkoxyl, -NR₁₆R₁₇, -C(O)NR₁₆R₁₇ and -N(R₁₆)-C(O)R₁₅;
each R₅ is independently selected from the group consisting of hydrogen, deuterium, fluoro, chloro, cyano, methyl, methoxy, trifluoromethyl, trifluoromethoxy, trifluoroethoxy, trideuteromethyl, trideuteromethoxy, cyclopropyl, cyclopropoxy, hydroxyl, amino, methylamino and dimethylamino;
each R₆ₐ and each R₇ₐ are independently selected from the group consisting of hydrogen, deuterium, halogen, cyano, C₁₋₄ alkyl, C₃₋₆ cycloalkyl, 3-6 membered heterocyclyl, methylsulfinyl, methylsulfonyl, isopropylsulfonyl, cyclopropylsulfonyl, aminosulfonyl, amino, methylamino, dimethylamino, C₁₋₄ alkanoyl and -C(O)NR₁₆R₁₇, the above groups are independently optionally further substituted with one or more substituents selected from the group consisting of deuterium, halogen, cyano, hydroxyl, C₁₋₄ alkyl, C₁₋₄ alkoxyl, C₃₋₆ cycloalkyl, C₃₋₆ cycloalkoxyl, 3-6 membered heterocyclyl, 3-6 membered heterocycloxyl, =O, =S, -NR₁₆R₁₇ and -C(O)NR₁₆R₁₇, the above groups are independently optionally more further substituted with one or more substituents selected from the group consisting of deuterium, halogen, cyano, hydroxyl, C₁₋₄ alkyl, C₁₋₄ alkoxyl, C₁₋₄ haloalkyl, C₁₋₄ haloalkoxyl, C₁₋₄ deuterioalkyl, C₁₋₄ deuterioalkoxyl, C₃₋₆ cycloalkyl, C₃₋₆ cycloalkoxyl, 3-6 membered heterocyclyl, 3-6 membered heterocycloxyl, =O, =S, -NR₁₆R₁₇ and -C(O)NR₁₆R₁₇;
each R_{6b} and each R_{7b} are independently selected from the group consisting of hydrogen, deuterium, C₁₋₄ alkyl, C₃₋₆ cycloalkyl, 3-6 membered heterocyclyl, methylsulfinyl, methylsulfonyl, isopropylsulfonyl, cyclopropylsulfonyl, aminosulfonyl and C₁₋₄ alkanoyl, the above groups are independently optionally further substituted with one or more substituents selected from the group consisting of deuterium, halogen, cyano, hydroxyl, C₁₋₄ alkyl, C₁₋₄ alkoxyl, C₁₋₄ haloalkyl, C₁₋₄ haloalkoxyl, C₁₋₄ deuterioalkyl, C₁₋₄ deuterioalkoxyl, C₃₋₆ cycloalkyl, C₃₋₆ cycloalkoxyl, 3-6 membered heterocyclyl, 3-6 membered heterocycloxyl, =O, =S, -NR₁₆R₁₇ and -C(O)NR₁₆R₁₇;
or, in the compound of formula (IVa1) or the compound of formula (IVa3), R_{6b} and R₇ₐ together with the moiety to which they are directly attached form 3-8 membered heterocyclyl or 5-8 membered heteroaryl, the above 3-8 membered heterocyclyl or 5-8 membered heteroaryl is independently optionally further substituted with one or more substituents selected from the group consisting of deuterium, halogen, cyano, hydroxyl, C₁₋₄ alkyl, C₁₋₄ alkoxyl, C₁₋₄ haloalkyl, C₁₋₄ haloalkoxyl, C₁₋₄ deuterioalkyl, C₁₋₄ deuterioalkoxyl, C₃₋₆ cycloalkyl, C₃₋₆ cycloalkoxyl, 3-6 membered heterocyclyl, 3-6 membered heterocycloxyl, =O, =S and -NR₁₆R₁₇;
or, in the compound of formula (IVa2) or the compound of formula (IVa4), R₆ₐ and R_{7b} together with the moiety to which they are directly attached form 3-8 membered heterocyclyl or 5-8 membered heteroaryl, the above 3-8 membered heterocyclyl or 5-8 membered heteroaryl is independently optionally further substituted with one or more substituents selected from the group consisting of deuterium, halogen, cyano, hydroxyl, C₁₋₄ alkyl, C₁₋₄ alkoxyl, C₁₋₄ haloalkyl, C₁₋₄ haloalkoxyl, C₁₋₄ deuterioalkyl, C₁₋₄ deuterioalkoxyl, C₃₋₆ cycloalkyl, C₃₋₆ cycloalkoxyl, 3-6 membered heterocyclyl, 3-6 membered heterocycloxyl, =O, =S and -NR₁₆R₁₇;
wherein, R₁₃, R₁₄, R₁₅, R₁₆ and R₁₇ are defined as those in the compound of formula (I).

As a still further preferred embodiment, in the compound of formula (I), the stereoisomer or pharmaceutically acceptable salt thereof, each U is independently NR₁ₐ, CH₂, O or S;
each R₁ₐ is independently selected from the group consisting of hydrogen, deuterium, C₁₋₄ alkyl, C₃₋₆ cycloalkyl, methylsulfonyl and isopropylsulfonyl, the above groups are independently optionally further substituted with one or more substituents selected from the group consisting of deuterium, halogen, C₁₋₄ alkyl, C₁₋₄ haloalkyl, C₁₋₄ deuterioalkyl, C₃₋₆ cycloalkyl, -C(O)NH₂, - C(O)NHCH₃ and -C(O)N(CH₃)₂.

As a still further preferred embodiment, in the compound of formula (I), the stereoisomer or pharmaceutically acceptable salt thereof, each R₅ is independently selected from the group consisting of hydrogen, deuterium, fluoro, chloro, cyano, methyl, methoxy, trifluoromethyl, trifluoromethoxy, trifluoroethoxy, trideuteromethyl, trideuteromethoxy and cyclopropyl.

As a still further preferred embodiment, in the compound of formula (I), the stereoisomer or pharmaceutically acceptable salt thereof, R_{6b} is selected from the group consisting of hydrogen, deuterium, C₁₋₄ alkyl, C₃₋₆ cycloalkyl, 3-6 membered heterocyclyl, methylsulfonyl and C₁₋₄ alkanoyl, the above groups are independently optionally further substituted with one or more substituents selected from the group consisting of deuterium, halogen, cyano, hydroxyl, C₁₋₄ alkyl, C₁₋₄ alkoxyl, C₁₋₄ haloalkyl, C₁₋₄ haloalkoxyl, C₁₋₄ deuterioalkyl, C₁₋₄ deuterioalkoxyl, C₃₋₆ cycloalkyl, C₃₋₆ cycloalkoxyl, 3-6 membered heterocyclyl, 3-6 membered heterocycloxyl and - NR₁₆R₁₇;
R₇ₐ is selected from the group consisting of hydrogen, deuterium, halogen, cyano, C₁₋₄ alkyl, C₃₋₆ cycloalkyl, 3-6 membered heterocyclyl, methylsulfonyl, amino, methylamino, dimethylamino, C₁₋₄ alkanoyl and -C(O)NR₁₆R₁₇, the above groups are independently optionally further substituted with one or more substituents selected from the group consisting of deuterium, halogen, cyano, hydroxyl, C₁₋₄ alkyl, C₁₋₄ alkoxyl, C₃₋₆ cycloalkyl, C₃₋₆ cycloalkoxyl, 3-6 membered heterocyclyl, 3-6 membered heterocycloxyl and -NR₁₆R₁₇, the above groups are independently optionally more further substituted with one or more substituents selected from the group consisting of deuterium, halogen, cyano, hydroxyl, C₁₋₄ alkyl, C₁₋₄ alkoxyl, C₁₋₄ haloalkyl, C₁₋₄ haloalkoxyl, C₁₋₄ deuterioalkyl, C₁₋₄ deuterioalkoxyl, C₃₋₆ cycloalkyl, C₃₋₆ cycloalkoxyl, 3-6 membered heterocyclyl, 3-6 membered heterocycloxyl and -NR₁₆R₁₇;
or, R_{6b} and R₇ₐ together with the moiety to which they are directly attached form 3-8 membered heterocyclyl, the above 3-8 membered heterocyclyl is optionally further substituted with one or more substituents selected from the group consisting of deuterium, halogen, cyano, hydroxyl, C₁₋₄ alkyl, C₁₋₄ alkoxyl, C₁₋₄ haloalkyl, C₁₋₄ haloalkoxyl, C₁₋₄ deuterioalkyl, C₁₋₄ deuterioalkoxyl, C₃₋₆ cycloalkyl, C₃₋₆ cycloalkoxyl, 3-6 membered heterocyclyl, 3-6 membered heterocycloxyl and -NR₁₆R₁₇;
wherein, R₁₆ and R₁₇ are defined as those in the compound of formula (I).

As a still further preferred embodiment, in the compound of formula (I), the stereoisomer or pharmaceutically acceptable salt thereof, R₆ₐ is selected from the group consisting of hydrogen, deuterium, halogen, cyano, C₁₋₄ alkyl, C₃₋₆ cycloalkyl, 3-6 membered heterocyclyl, methylsulfonyl, amino, methylamino, dimethylamino, C₁₋₄ alkanoyl and -C(O)NR₁₆R₁₇, the above groups are independently optionally further substituted with one or more substituents selected from the group consisting of deuterium, halogen, cyano, hydroxyl, C₁₋₄ alkyl, C₁₋₄ alkoxyl, C₃₋₆ cycloalkyl, C₃₋₆ cycloalkoxyl, 3-6 membered heterocyclyl, 3-6 membered heterocycloxyl and - NR₁₆R₁₇, the above groups are independently optionally more further substituted with one or more substituents selected from the group consisting of deuterium, halogen, cyano, hydroxyl, C₁₋₄ alkyl, C₁₋₄ alkoxyl, C₁₋₄ haloalkyl, C₁₋₄ haloalkoxyl, C₁₋₄ deuterioalkyl, C₁₋₄ deuterioalkoxyl, C₃₋₆ cycloalkyl, C₃₋₆ cycloalkoxyl, 3-6 membered heterocyclyl, 3-6 membered heterocycloxyl and - NR₁₆R₁₇;
R_{7b} is selected from the group consisting of hydrogen, deuterium, C₁₋₄ alkyl, C₃₋₆ cycloalkyl, 3-6 membered heterocyclyl, methylsulfonyl and C₁₋₄ alkanoyl, the above groups are independently optionally further substituted with one or more substituents selected from the group consisting of deuterium, halogen, cyano, hydroxyl, C₁₋₄ alkyl, C₁₋₄ alkoxyl, C₁₋₄ haloalkyl, C₁₋₄ haloalkoxyl, C₁₋₄ deuterioalkyl, C₁₋₄ deuterioalkoxyl, C₃₋₆ cycloalkyl, C₃₋₆ cycloalkoxyl, 3-6 membered heterocyclyl, 3-6 membered heterocycloxyl and -NR₁₆R₁₇;
or, R₆ₐ and R_{7b} together with the moiety to which they are directly attached form 3-8 membered heterocyclyl, the above 3-8 membered heterocyclyl is optionally further substituted with one or more substituents selected from the group consisting of deuterium, halogen, cyano, hydroxyl, C₁₋₄ alkyl, C₁₋₄ alkoxyl, C₁₋₄ haloalkyl, C₁₋₄ haloalkoxyl, C₁₋₄ deuterioalkyl, C₁₋₄ deuterioalkoxyl, C₃₋₆ cycloalkyl, C₃₋₆ cycloalkoxyl, 3-6 membered heterocyclyl, 3-6 membered heterocycloxyl and -NR₁₆R₁₇;
wherein, R₁₆ and R₁₇ are defined as those in the compound of formula (I).

As a still further preferred embodiment, in the compound of formula (I), the stereoisomer or pharmaceutically acceptable salt thereof, each R₁₆ and each R₁₇ are independently selected from the group consisting of hydrogen, deuterium, hydroxyl, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, sulfinyl, sulfonyl, methylsulfonyl, isopropylsulfonyl, cyclopropylsulfonyl, p-toluenesulfonyl, aminosulfonyl, dimethylaminosulfonyl and C₁₋₄ alkanoyl;
or, R₁₆ and R₁₇ together with a nitrogen atom to which they are directly attached form 4-6 membered heterocyclyl, the above 4-6 membered heterocyclyl is optionally further substituted with one or more substituents selected from the group consisting of deuterium, halogen, hydroxyl, =O, C₁₋₄ alkyl, C₁₋₄ haloalkyl, C₁₋₄ deuterioalkyl, C₃₋₆ cycloalkyl and 3-6 membered heterocyclyl.

As a preferred embodiment, in the compound of formula (I), the stereoisomer or pharmaceutically acceptable salt thereof, the compound of formula (I) is a compound of formula (IIb1) or (IIb2) as follows: 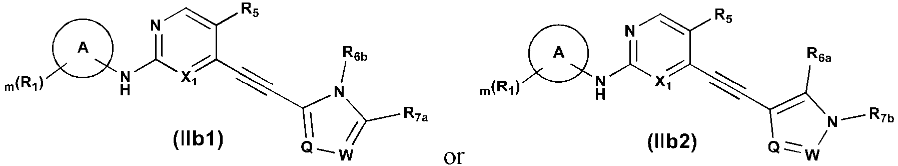
wherein, each X₁ is independently CR₄ or N;
each W is independently N or CR₈ₐ;
each Q is independently N or CR₉ₐ;
each ring A is independently phenyl or 5-6 membered heteroaryl;
each R₁ is independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-8 membered heterocyclyl, C₃₋₆ cycloalkyl-substituted C₁₋₄ alkyl, 3-6 membered heterocyclyl-substituted C₁₋₄ alkyl, -SF₅, - S(O)ᵣR₁₃, -O-R₁₄, -C(O)OR₁₄, -C(O)R₁₅, -O-C(O)R₁₅, -NR₁₆R₁₇, -C(O)NR₁₆R₁₇ and -N(R₁₆)-C(O)R₁₅, the above groups are independently optionally further substituted with one or more substituents selected from the group consisting of deuterium, halogen, cyano, C₁₋₄ alkyl, C₂₋₄ alkenyl, C₂₋₄ alkynyl, C₃₋₆ cycloalkyl, 3-6 membered heterocyclyl, =O, =S, -SF₅, -S(O)ᵣR₁₃, -OR₁₄, -C(O)OR₁₄, -C(O)R₁₅, -O-C(O)R₁₅, -NR₁₆R₁₇, -C(O)NR₁₆R₁₇ and -N(R₁₆)-C(O)R₁₅, the above groups are independently optionally more further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)ᵣR₁₃, -O-R₁₄, -C(O)OR₁₄, -C(O)R₁₅, -O-C(O)R₁₅, - NR₁₆R₁₇, -C(O)NR₁₆R₁₇ and -N(R₁₆)-C(O)R₁₅;
each R₄ is independently selected from the group consisting of hydrogen, deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, -SF₅, -O-R₁₄ and -NR₁₆R₁₇;
each R₅ is independently selected from the group consisting of hydrogen, deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, -SF₅, -O-R₁₄ and -NR₁₆R₁₇;
R₆ₐ and R₇ₐ are each independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, -SF₅, -S(O)ᵣR₁₃, -O-R₁₄, -C(O)OR₁₄, -C(O)R₁₅, -O-C(O)R₁₅, -NR₁₆R₁₇, - C(O)NR₁₆R₁₇ and -N(R₁₆)-C(O)R₁₅, the above groups are independently optionally further substituted with one or more substituents selected from the group consisting of deuterium, halogen, cyano, C₁₋₄ alkyl, C₂₋₄ alkenyl, C₂₋₄ alkynyl, C₃₋₆ cycloalkyl, 3-6 membered heterocyclyl, =O, =S, -SF₅, -S(O)ᵣR₁₃, -O-R₁₄, -C(O)OR₁₄, -C(O)R₁₅, -O-C(O)R₁₅, -NR₁₆R₁₇, -C(O)NR₁₆R₁₇ and -N(R₁₆)-C(O)R₁₅, the above groups are independently optionally more further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, =O, =S, -SF₅, -S(O)ᵣR₁₃, -O-R₁₄, -C(O)OR₁₄, -C(O)R₁₅, -O-C(O)R₁₅, -NR₁₆R₁₇, - C(O)NR₁₆R₁₇ and -N(R₁₆)-C(O)R₁₅;
R_{6b} and R_{7b} are each independently selected from the group consisting of hydrogen, deuterium, C₁₋₄ alkyl, C₂₋₄ alkenyl, C₂₋₄ alkynyl, C₃₋₆ cycloalkyl, 3-6 membered heterocyclyl, - S(O)ᵣR₁₃, -C(O)OR₁₄, -C(O)R₁₅ and -C(O)NR₁₆R₁₇, the above groups are independently optionally further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, =O, =S, -SF₅, -S(O)ᵣR₁₃, -O-R₁₄, -C(O)OR₁₄, - C(O)R₁₅, -O-C(O)R₁₅, -NR₁₆R₁₇, -C(O)NR₁₆R₁₇ and -N(R₁₆)-C(O)R₁₅;
or, in the compound of formula (IIb1), R_{6b} and R₇ₐ together with the moiety to which they are directly attached form 3-8 membered heterocyclyl or 5-8 membered heteroaryl, the above 3-8 membered heterocyclyl or 5-8 membered heteroaryl is independently optionally further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, =O, =S, -SF₅, -S(O)ᵣR₁₃, -O-R₁₄, -C(O)OR₁₄, -C(O)R₁₅, - O-C(O)R₁₅ and -NR₁₆R₁₇;
or, in the compound of formula (IIb2), R₆ₐ and R_{7b} together with the moiety to which they are directly attached form 3-8 membered heterocyclyl or 5-8 membered heteroaryl, the above 3-8 membered heterocyclyl or 5-8 membered heteroaryl is independently optionally further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, =O, =S, -SF₅, -S(O)ᵣR₁₃, -O-R₁₄, -C(O)OR₁₄, -C(O)R₁₅, - O-C(O)R₁₅ and -NR₁₆R₁₇;
each R₈ₐ and each R₉ₐ are independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, -SF₅, -S(O)ᵣR₁₃, -O-R₁₄, -C(O)OR₁₄, -C(O)R₁₅, -O-C(O)R₁₅, -NR₁₆R₁₇, - C(O)NR₁₆R₁₇ and -N(R₁₆)-C(O)R₁₅, the above groups are independently optionally further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, =O, =S, -SF₅, -S(O)ᵣR₁₃, -O-R₁₄, -C(O)OR₁₄, -C(O)R₁₅, - O-C(O)R₁₅, -NR₁₆R₁₇, -C(O)NR₁₆R₁₇ and -N(R₁₆)-C(O)R₁₅;
wherein, R₁₃, R₁₄, R₁₅, R₁₆, R₁₇, m and r are defined as those in the compound of formula (I).

As a further preferred embodiment, in the compound of formula (I), the stereoisomer or pharmaceutically acceptable salt thereof, the compound of formula (I) is a compound of formula (IIIb 1) or (IIIb2) as follows: 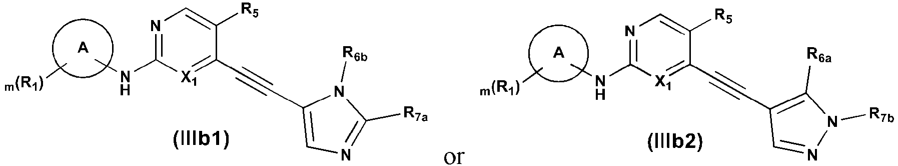 wherein, each X₁ is independently CH or N;
ring A is selected from the group consisting of phenyl, pyridinyl, pyrimidinyl, pyridazinyl, triazinyl, furanyl, thienyl, imidazolyl, triazolyl, pyrrolyl, oxazolyl, isoxazolyl, thiazolyl and isothiazolyl;
each R₁ is independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-8 membered heterocyclyl, C₃₋₆ cycloalkyl-substituted C₁₋₄ alkyl, 3-6 membered heterocyclyl-substituted C₁₋₄ alkyl, - C(O)R₁₅, -NR₁₆R₁₇ and -C(O)NR₁₆R₁₇, the above groups are independently optionally further substituted with one or more substituents selected from the group consisting of deuterium, halogen, cyano, C₁₋₄ alkyl, C₁₋₄ haloalkyl, C₁₋₄ deuterioalkyl, hydroxyl-substituted C₁₋₄ alkyl, cyano-substituted C₁₋₄ alkyl, C₂₋₄ alkenyl, C₂₋₄ alkynyl, C₃₋₆ cycloalkyl, 3-6 membered heterocyclyl, =O, =S, -SF₅, -S(O)ᵣR₁₃, -O-R₁₄, -C(O)OR₁₄, -C(O)R₁₅, -O-C(O)R₁₅, -NR₁₆R₁₇, - C(O)NR₁₆R₁₇ and -N(R₁₆)-C(O)R₁₅;
each R₅ is independently selected from the group consisting of hydrogen, deuterium, fluoro, chloro, cyano, methyl, methoxy, trifluoromethyl, trifluoromethoxy, trifluoroethoxy, trideuteromethyl, trideuteromethoxy, cyclopropyl, cyclopropoxy, hydroxyl, amino, methylamino and dimethylamino;
wherein, R₆ₐ, R_{6b}, R₇ₐ, R_{7b}, R₁₃, R₁₄, R₁₅, R₁₆, R₁₇, m and r are defined as those in the compound of formula (I).

As a still further preferred embodiment, in the compound of formula (I), the stereoisomer or pharmaceutically acceptable salt thereof, R_{6b} is selected from the group consisting of hydrogen, deuterium, C₁₋₄ alkyl, C₃₋₆ cycloalkyl, 3-6 membered heterocyclyl, methylsulfinyl, methylsulfonyl, isopropylsulfonyl, cyclopropylsulfonyl, aminosulfonyl and C₁₋₄ alkanoyl, the above groups are independently optionally further substituted with one or more substituents selected from the group consisting of deuterium, halogen, cyano, hydroxyl, C₁₋₄ alkyl, C₁₋₄ alkoxyl, C₁₋₄ haloalkyl, C₁₋₄ haloalkoxyl, C₁₋₄ deuterioalkyl, C₁₋₄ deuterioalkoxyl, C₃₋₆ cycloalkyl, C₃₋₆ cycloalkoxyl, 3-6 membered heterocyclyl, 3-6 membered heterocycloxyl, =O, =S, -NR₁₆R₁₇ and -C(O)NR₁₆R₁₇;
R₇ₐ is selected from the group consisting of hydrogen, deuterium, halogen, cyano, C₁₋₄ alkyl, C₃₋₆ cycloalkyl, 3-6 membered heterocyclyl, methylsulfinyl, methylsulfonyl, isopropylsulfonyl, cyclopropylsulfonyl, aminosulfonyl, amino, methylamino, dimethylamino, C₁₋₄ alkanoyl and - C(O)NR₁₆R₁₇, the above groups are independently optionally further substituted with one or more substituents the group consisting of deuterium, halogen, cyano, hydroxyl, C₁₋₄ alkyl, C₁₋₄ alkoxyl, C₃₋₆ cycloalkyl, C₃₋₆ cycloalkoxyl, 3-6 membered heterocyclyl, 3-6 membered heterocycloxyl, =O, =S, -NR₁₆R₁₇ and -C(O)NR₁₆R₁₇, the above groups are independently optionally more further substituted with one or more substituents selected from the group consisting of deuterium, halogen, cyano, hydroxyl, C₁₋₄ alkyl, C₁₋₄ alkoxyl, C₁₋₄ haloalkyl, C₁₋₄ haloalkoxyl, C₁₋₄ deuterioalkyl, C₁₋₄ deuterioalkoxyl, C₃₋₆ cycloalkyl, C₃₋₆ cycloalkoxyl, 3-6 membered heterocyclyl, 3-6 membered heterocycloxyl, =O, =S, -NR₁₆R₁₇ and -C(O)NR₁₆R₁₇;
or, R_{6b} and R₇ₐ together with the moiety to which they are directly attached form 3-8 membered heterocyclyl, the above 3-8 membered heterocyclyl is independently optionally further substituted with one or more substituents selected from the group consisting of deuterium, halogen, cyano, hydroxyl, C₁₋₄ alkyl, C₁₋₄ alkoxyl, C₁₋₄ haloalkyl, C₁₋₄ haloalkoxyl, C₁₋₄ deuterioalkyl, C₁₋₄ deuterioalkoxyl, C₃₋₆ cycloalkyl, C₃₋₆ cycloalkoxyl, 3-6 membered heterocyclyl, 3-6 membered heterocycloxyl, =O, =S and -NR₁₆R₁₇;
wherein, R₁₆ and R₁₇ are defined as those in the compound of formula (I).

As a still further preferred embodiment, in the compound of formula (I), the stereoisomer or pharmaceutically acceptable salt thereof, R₆ₐ is selected from the group consisting of hydrogen, deuterium, halogen, cyano, C₁₋₄ alkyl, C₃₋₆ cycloalkyl, 3-6 membered heterocyclyl, methylsulfinyl, methylsulfonyl, isopropylsulfonyl, cyclopropylsulfonyl, aminosulfonyl, amino, methylamino, dimethylamino, C₁₋₄ alkanoyl and -C(O)NR₁₆R₁₇, the above groups are independently optionally further substituted with one or more substituents selected from the group consisting of deuterium, halogen, cyano, hydroxyl, C₁₋₄ alkyl, C₁₋₄ alkoxyl, C₃₋₆ cycloalkyl, C₃₋₆ cycloalkoxyl, 3-6 membered heterocyclyl, 3-6 membered heterocycloxyl, =O, =S, -NR₁₆R₁₇ and -C(O)NR₁₆R₁₇, the above groups are independently optionally more further substituted with one or more substituents selected from the group consisting of deuterium, halogen, cyano, hydroxyl, C₁₋₄ alkyl, C₁₋₄ alkoxyl, C₁₋₄ haloalkyl, C₁₋₄ haloalkoxyl, C₁₋₄ deuterioalkyl, C₁₋₄ deuterioalkoxyl, C₃₋₆ cycloalkyl, C₃₋₆ cycloalkoxyl, 3-6 membered heterocyclyl, 3-6 membered heterocycloxyl, =O, =S, -NR₁₆R₁₇ and -C(O)NR₁₆R₁₇;
R_{7b} is selected from the group consisting of hydrogen, deuterium, C₁₋₄ alkyl, C₃₋₆ cycloalkyl, 3-6 membered heterocyclyl, methylsulfinyl, methylsulfonyl, isopropylsulfonyl, cyclopropylsulfonyl, aminosulfonyl and C₁₋₄ alkanoyl, the above groups are independently optionally further substituted with one or more substituents selected from the group consisting of deuterium, halogen, cyano, hydroxyl, C₁₋₄ alkyl, C₁₋₄ alkoxyl, C₁₋₄ haloalkyl, C₁₋₄ haloalkoxyl, C₁₋₄ deuterioalkyl, C₁₋₄ deuterioalkoxyl, C₃₋₆ cycloalkyl, C₃₋₆ cycloalkoxyl, 3-6 membered heterocyclyl, 3-6 membered heterocycloxyl, =O, =S, -NR₁₆R₁₇ and -C(O)NR₁₆R₁₇;
or, R₆ₐ and R_{7b} together with the moiety to which they are directly attached form 3-8 membered heterocyclyl, the above 3-8 membered heterocyclyl is independently optionally further substituted with one or more substituents selected from the group consisting of deuterium, halogen, cyano, hydroxyl, C₁₋₄ alkyl, C₁₋₄ alkoxyl, C₁₋₄ haloalkyl, C₁₋₄ haloalkoxyl, C₁₋₄ deuterioalkyl, C₁₋₄ deuterioalkoxyl, C₃₋₆ cycloalkyl, C₃₋₆ cycloalkoxyl, 3-6 membered heterocyclyl, 3-6 membered heterocycloxyl, =O, =S and -NR₁₆R₁₇;
wherein, R₁₆ and R₁₇ are defined as those in the compound of formula (I).

As a still further preferred embodiment, in the compound of formula (I), the stereoisomer or pharmaceutically acceptable salt thereof, each R₁₃ is independently selected from the group consisting of hydrogen, deuterium, hydroxyl, C₁₋₄ alkyl, 3-6 membered heterocyclyl and - NR₁₆R₁₇, the above groups are independently optionally further substituted with one or more substituents selected from the group consisting of deuterium, halogen, cyano, hydroxyl, =O, C₁₋₄ alkyl, C₁₋₄ alkoxyl, C₃₋₆ cycloalkyl, C₃₋₆ cycloalkoxyl, 3-6 membered heterocyclyl, 3-6 membered heterocycloxyl and -NR₁₆R₁₇;
each R₁₄ is independently selected from the group consisting of hydrogen, deuterium, C₁₋₄ alkyl and 3-6 membered heterocyclyl, the above groups are independently optionally further substituted with one or more substituents selected from the group consisting of deuterium, halogen, hydroxyl, =O, cyano, C₁₋₄ alkyl, C₁₋₄ alkoxyl, C₃₋₆ cycloalkyl, C₃₋₆ cycloalkoxyl, 3-6 membered heterocyclyl, 3-6 membered heterocycloxyl and -NR₁₆R₁₇;
each R₁₅ is independently selected from the group consisting of hydrogen, deuterium, hydroxyl, C₁₋₄ alkyl, C₁₋₄ alkoxyl, C₂₋₄ alkenyl, C₃₋₆ cycloalkyl, C₃₋₆ cycloalkoxyl, 3-6 membered heterocyclyl, 3-6 membered heterocycloxyl and -NR₁₆R₁₇, the above groups are independently optionally further substituted with one or more substituents selected from the group consisting of deuterium, halogen, hydroxyl, =O, cyano, C₁₋₄ alkyl, C₁₋₄ alkoxyl, C₃₋₆ cycloalkyl, C₃₋₆ cycloalkoxyl, 3-6 membered heterocyclyl, 3-6 membered heterocycloxyl and -NR₁₆R₁₇;
wherein, R₁₆ and R₁₇ are defined as those in the compound of formula (I).

As a still further preferred embodiment, in the compound of formula (I), the stereoisomer or pharmaceutically acceptable salt thereof, each R₁₆ and each R₁₇ are independently selected from the group consisting of hydrogen, deuterium, hydroxyl, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, sulfinyl, sulfonyl, methylsulfonyl, isopropylsulfonyl, cyclopropylsulfonyl, p-toluenesulfonyl, aminosulfonyl, dimethylaminosulfonyl and C₁₋₄ alkanoyl;
or, R₁₆ and R₁₇ together a the nitrogen atom to which they are directly attached form 4-6 membered heterocyclyl, the above 4-6 membered heterocyclyl is optionally further substituted with one or more substituents selected from the group consisting of deuterium, halogen, hydroxyl, =O, C₁₋₄ alkyl, C₁₋₄ haloalkyl, C₁₋₄ deuterioalkyl, C₃₋₆ cycloalkyl and 3-6 membered heterocyclyl.

As a most preferred embodiment, the compound of formula (I), the stereoisomer or pharmaceutically acceptable salt thereof includes, but is not limited to, the following compounds:

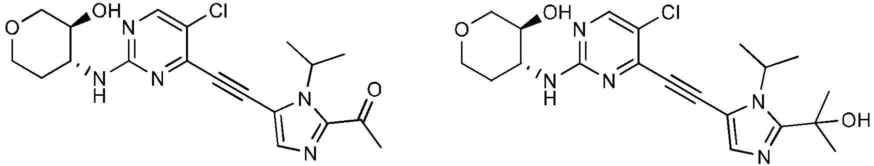

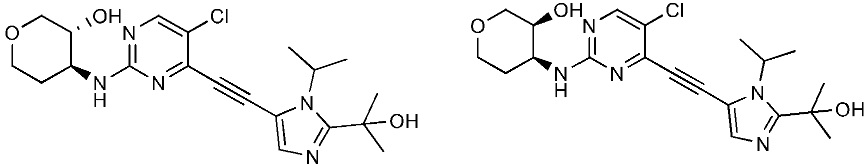

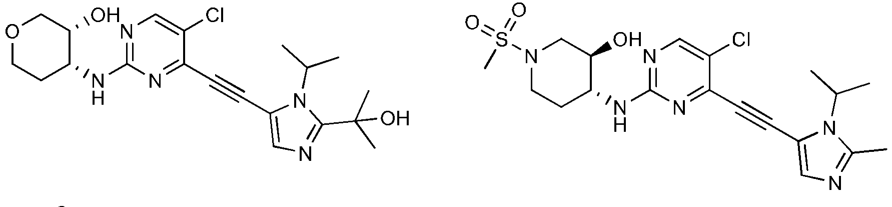

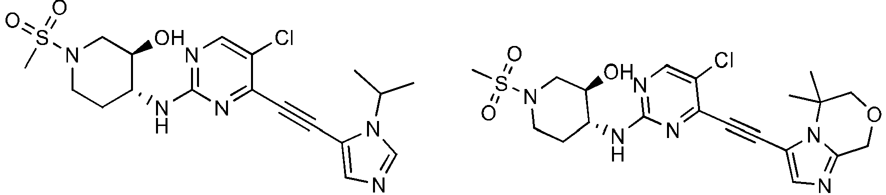

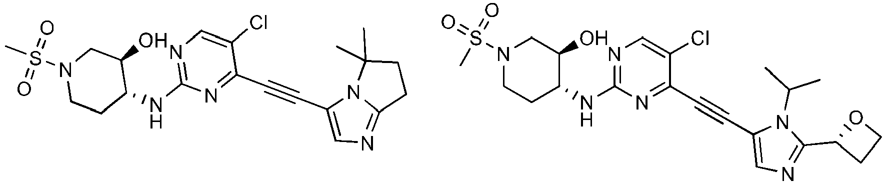

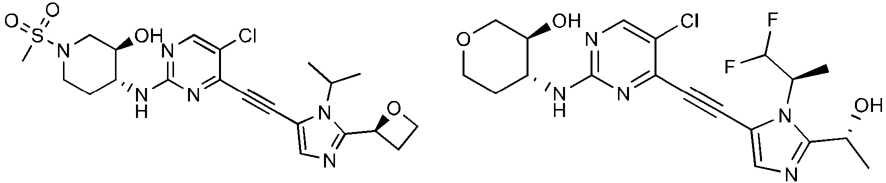

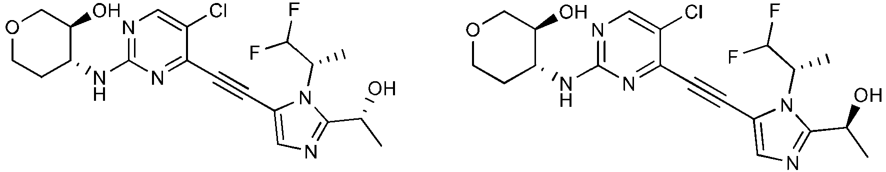

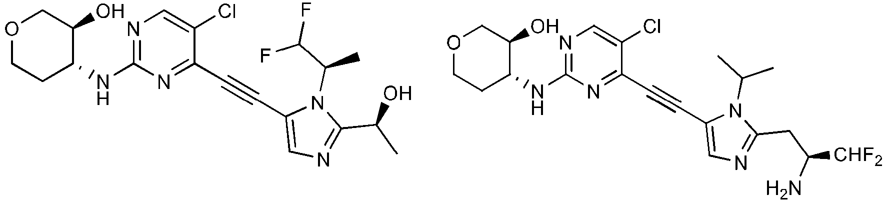

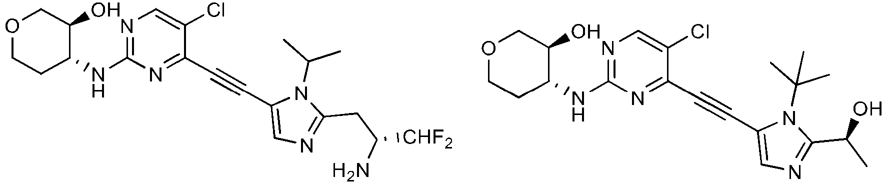

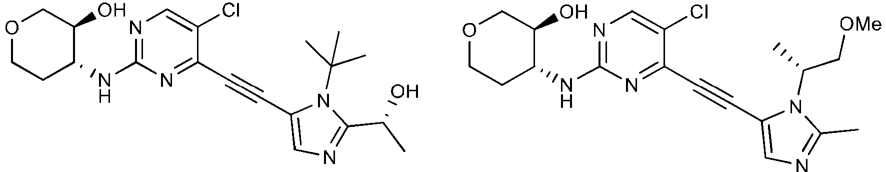

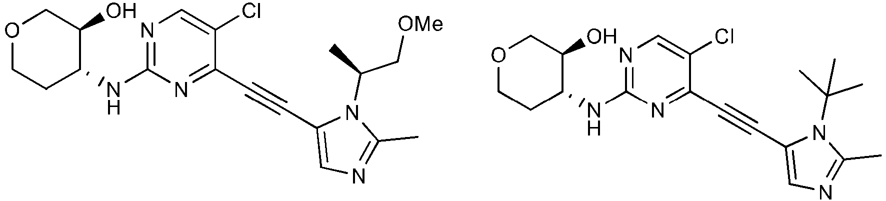

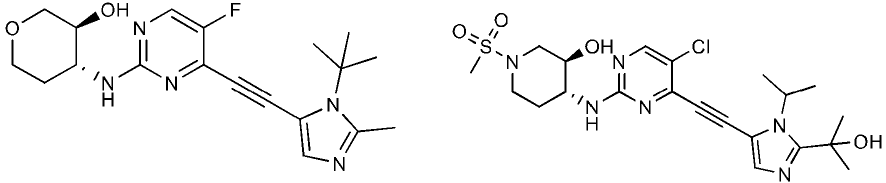

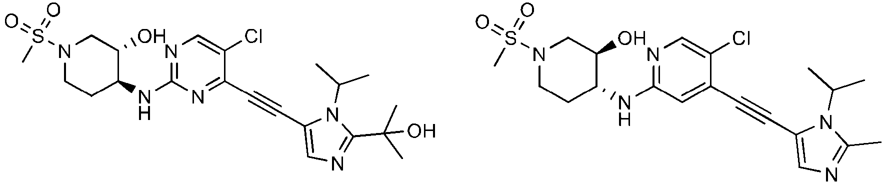

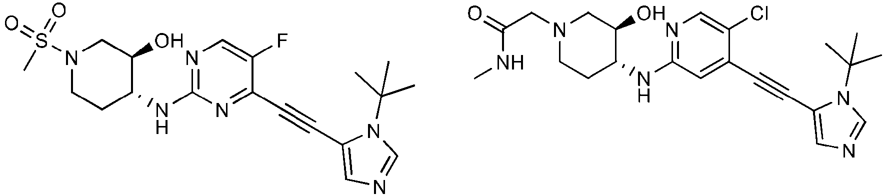

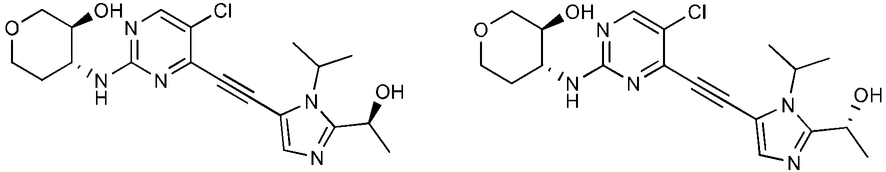

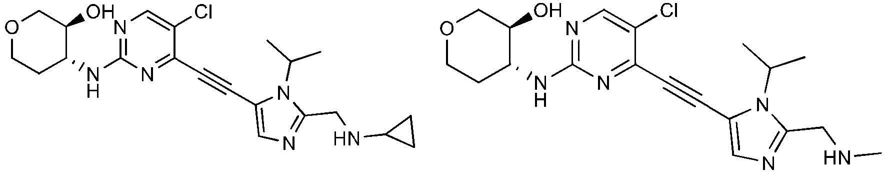

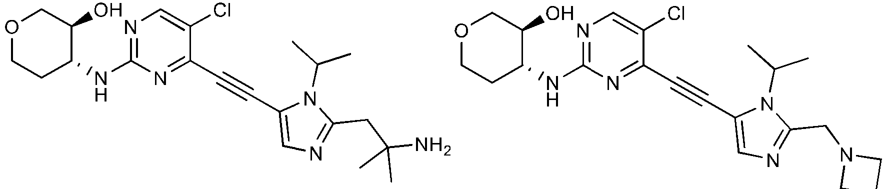

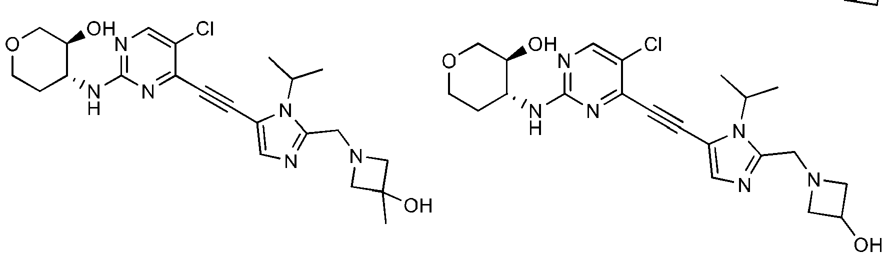

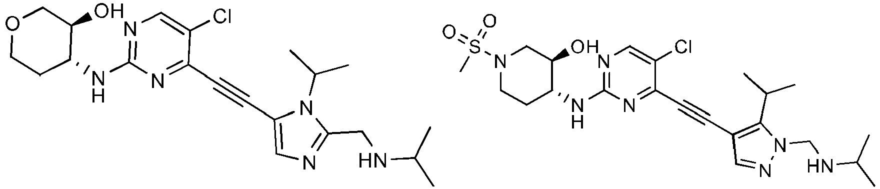

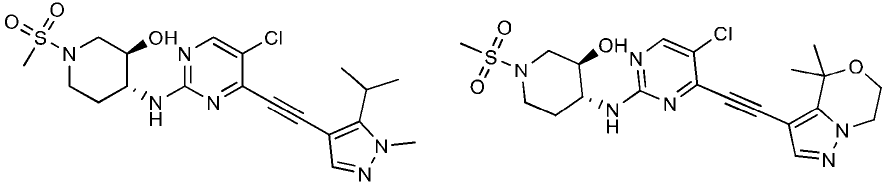

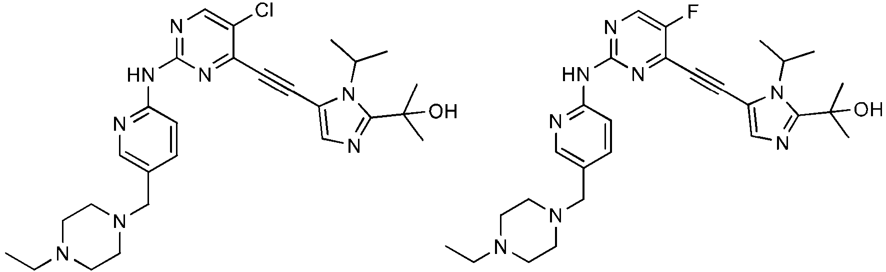

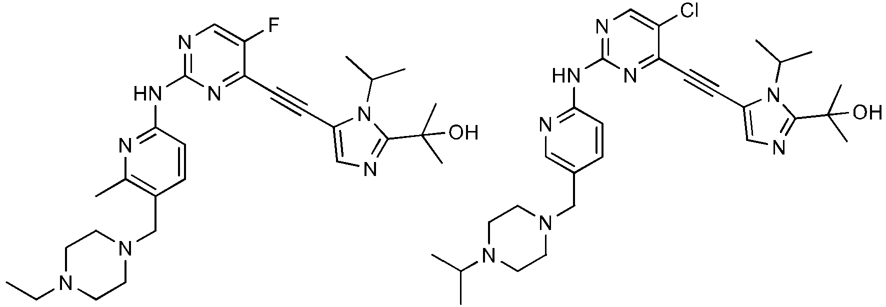

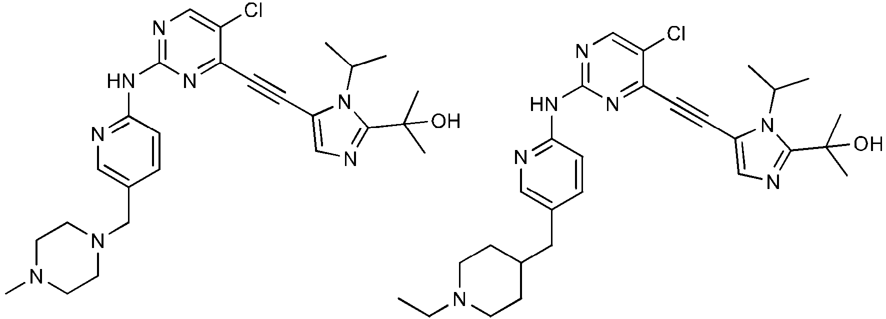

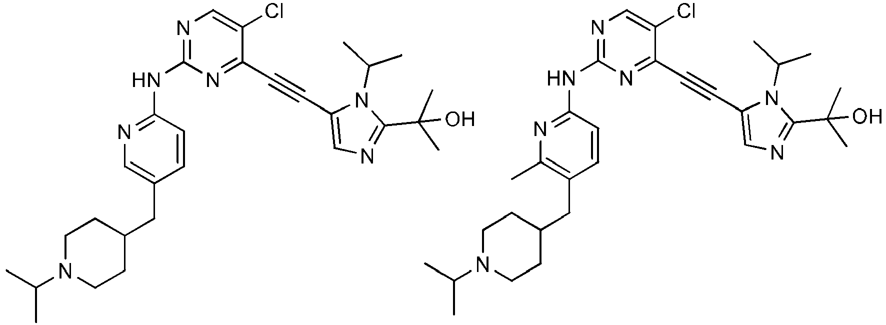

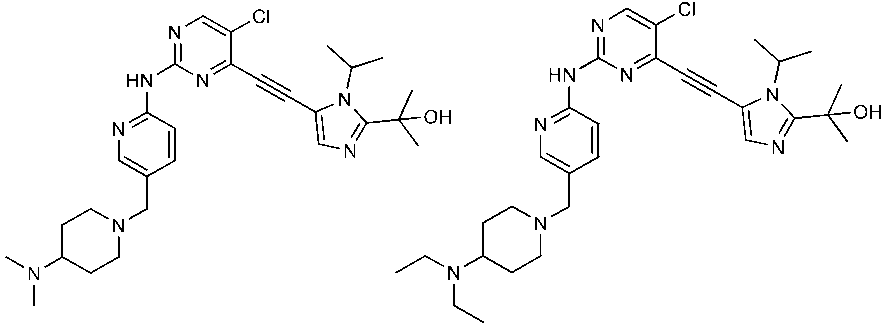

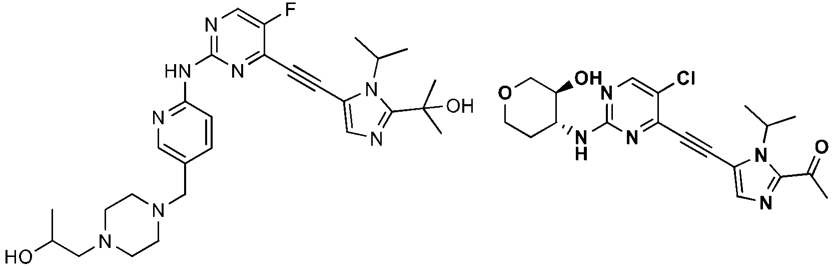

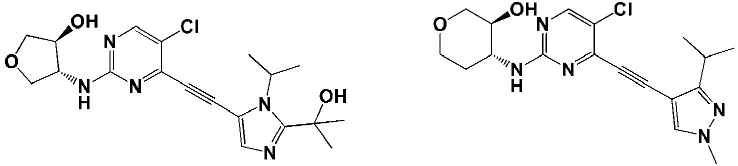

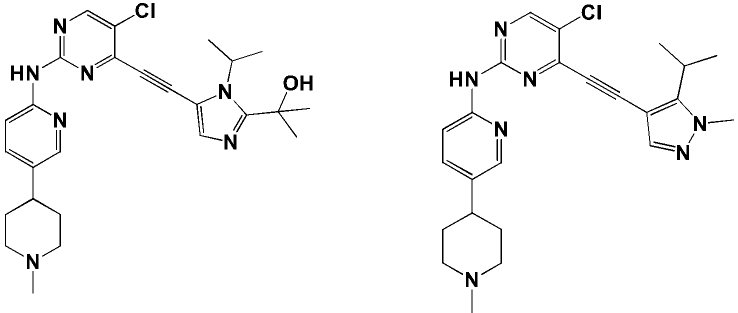

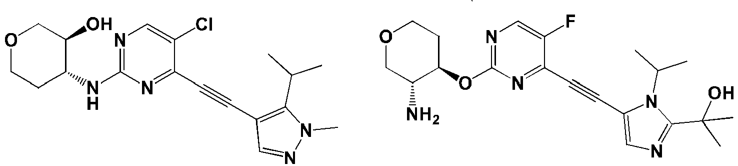

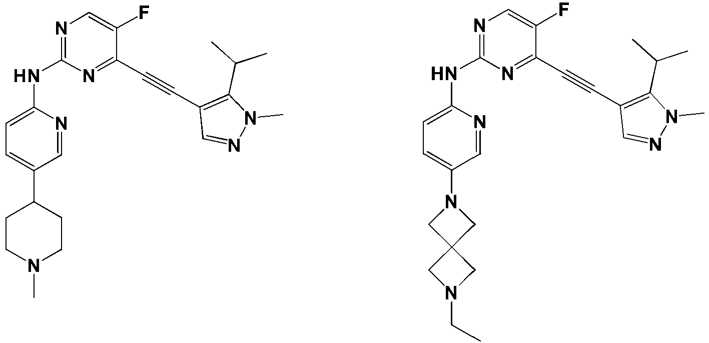

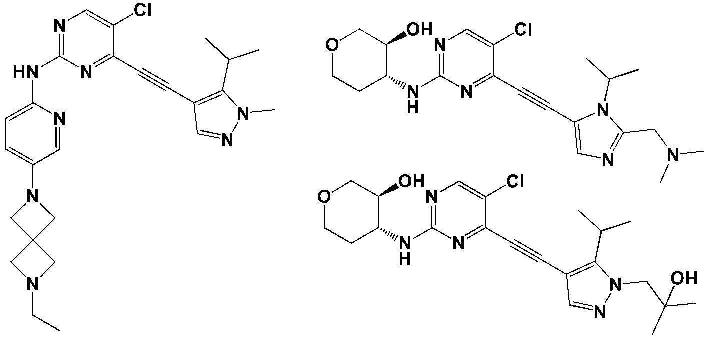

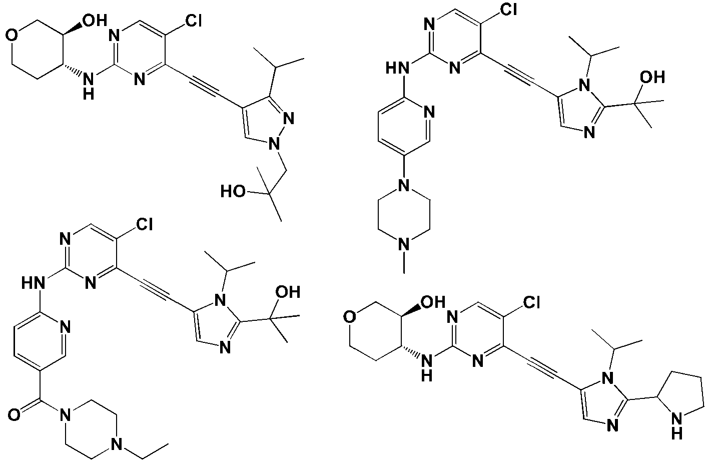

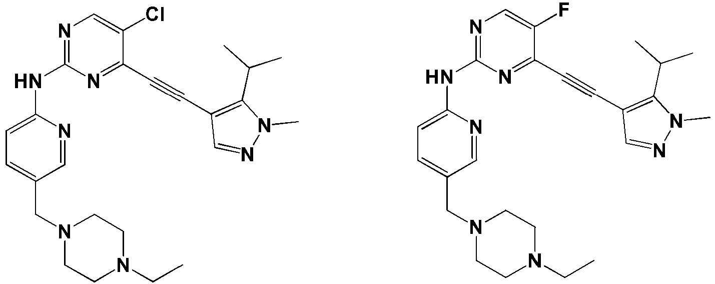

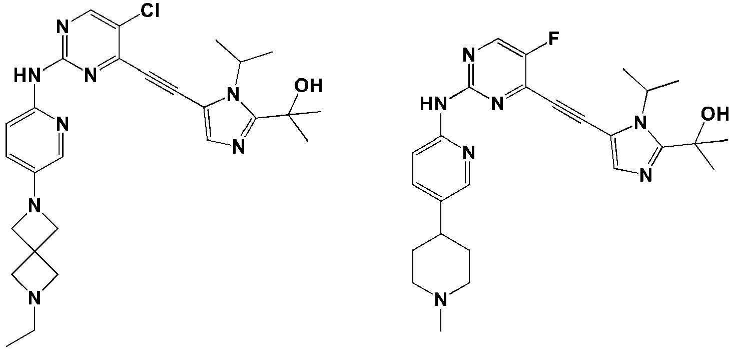

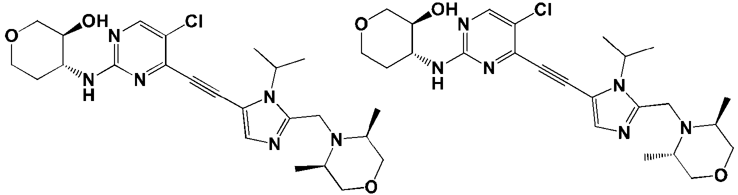

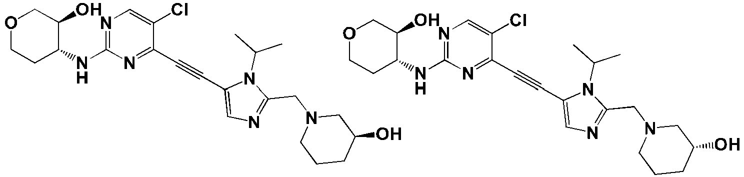

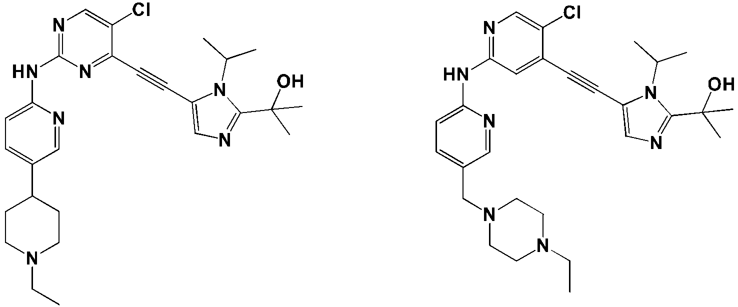

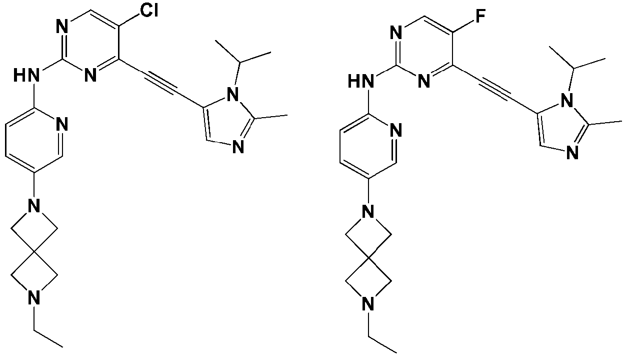

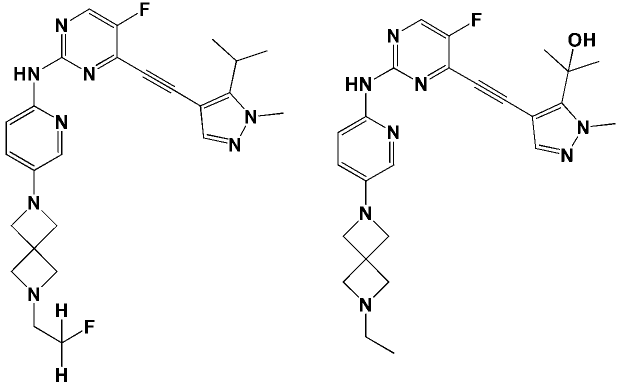

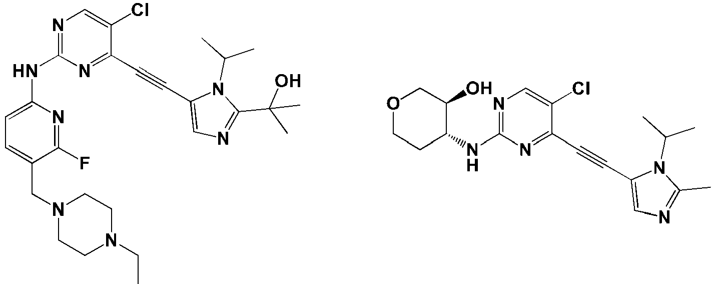

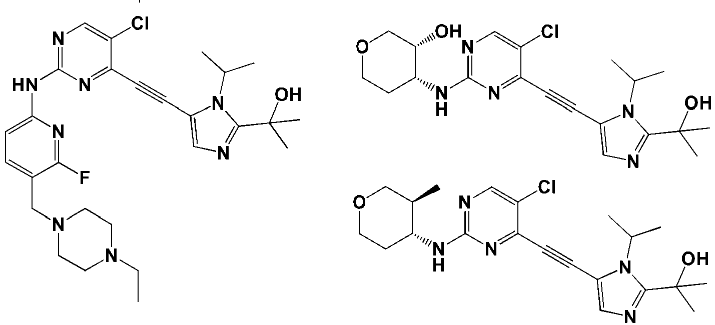

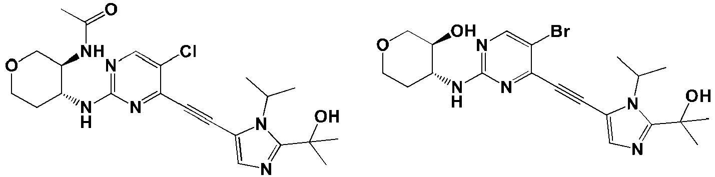

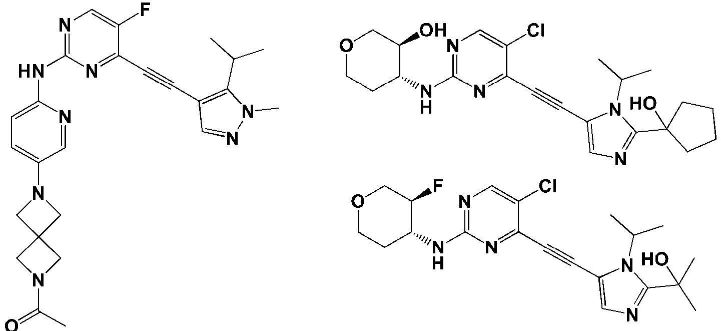

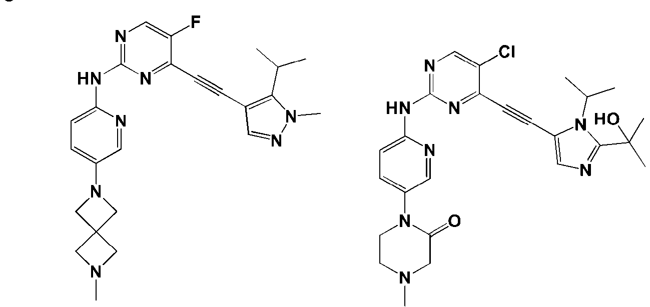

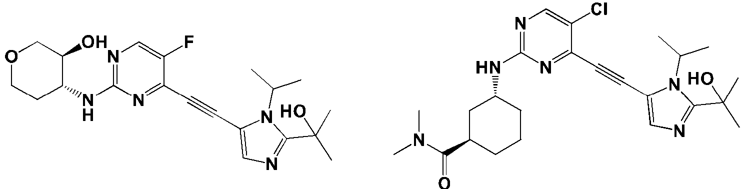

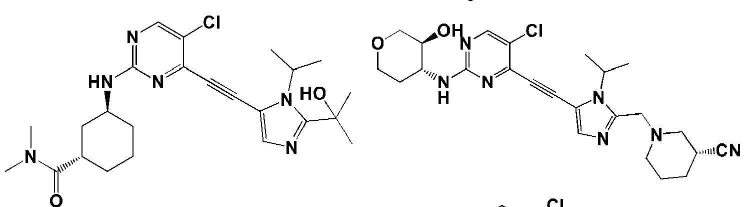

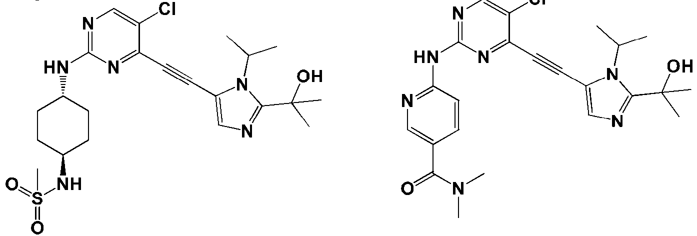

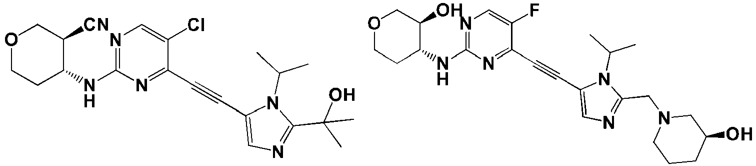

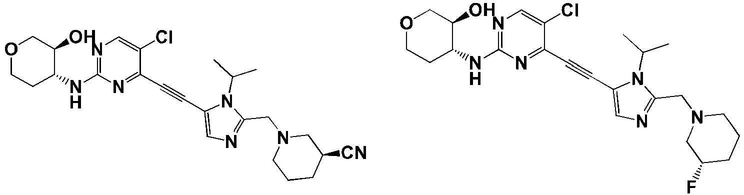

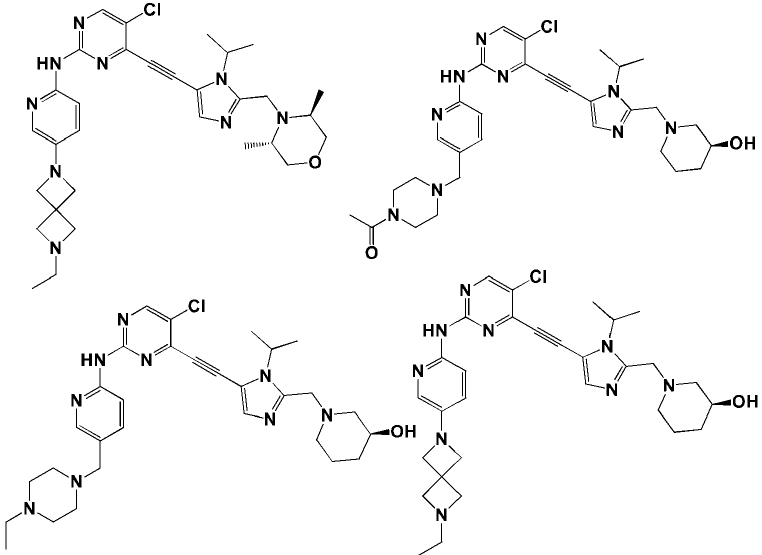

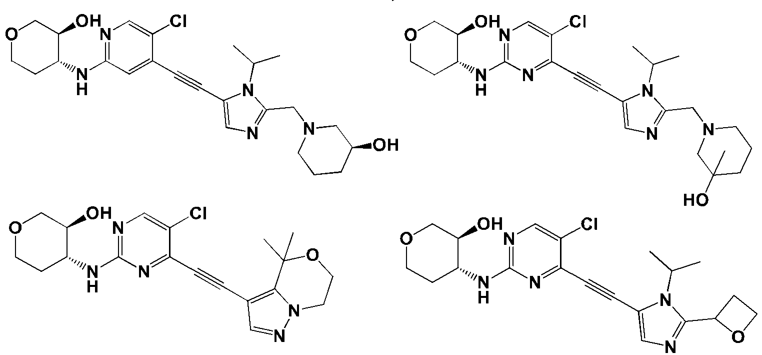

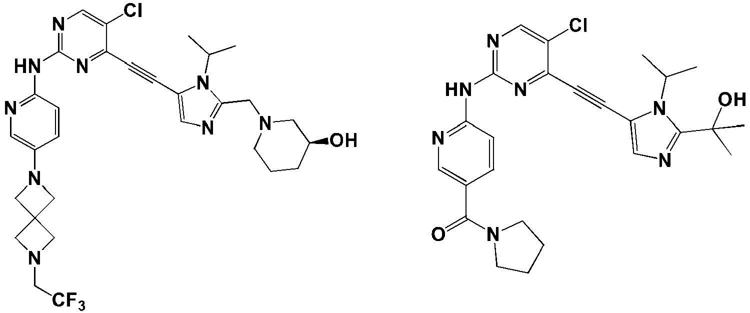

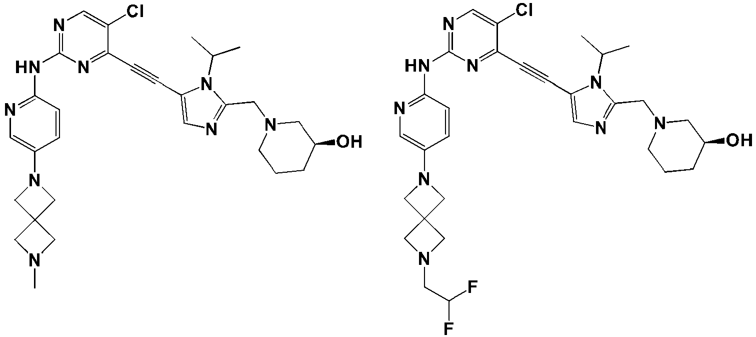

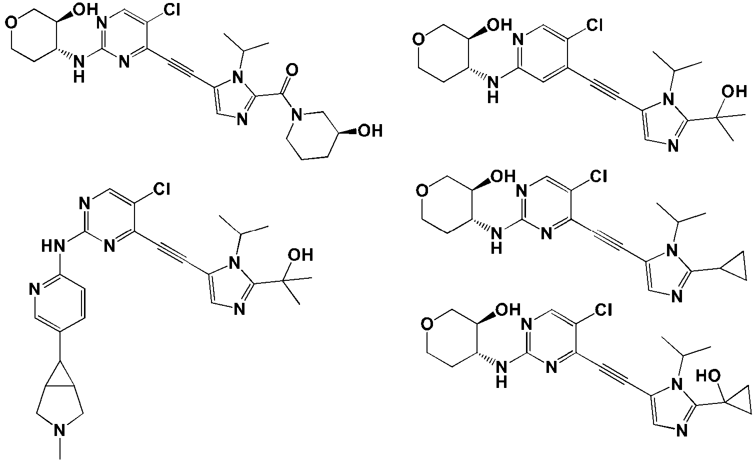

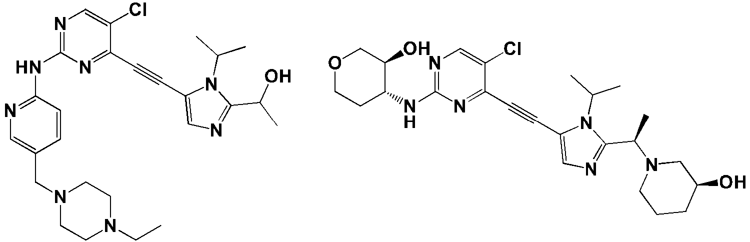

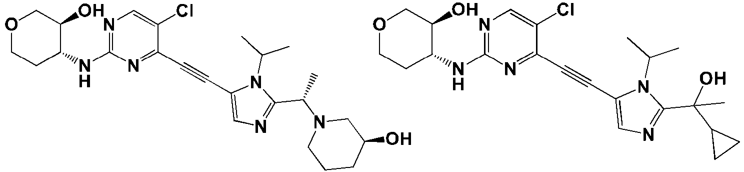

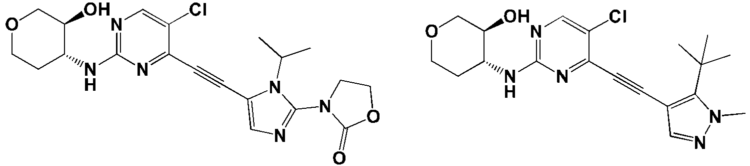

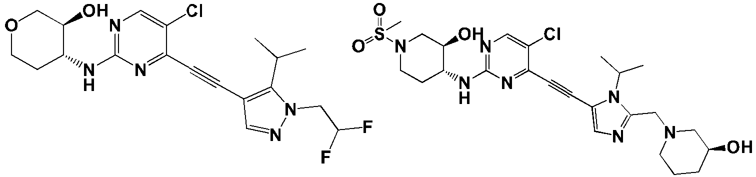

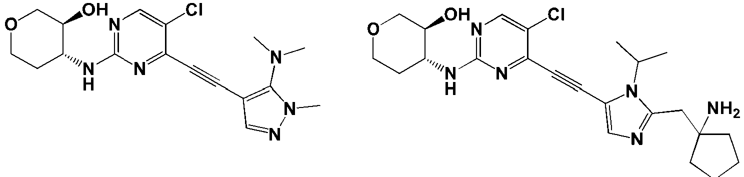

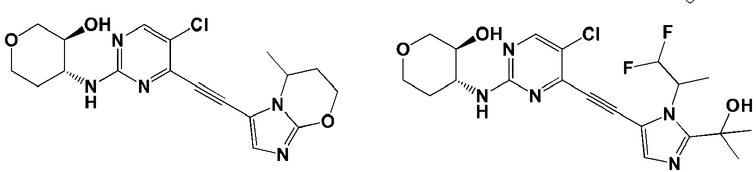

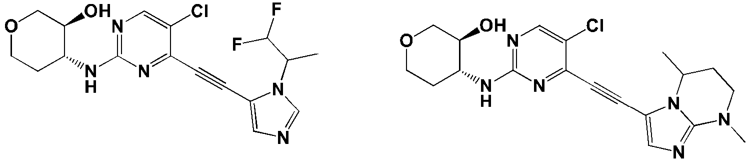

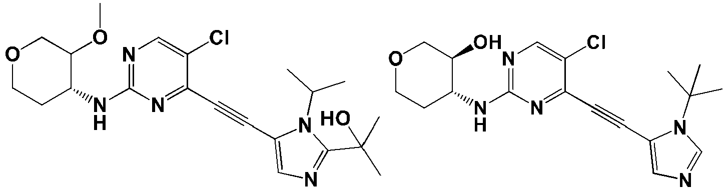

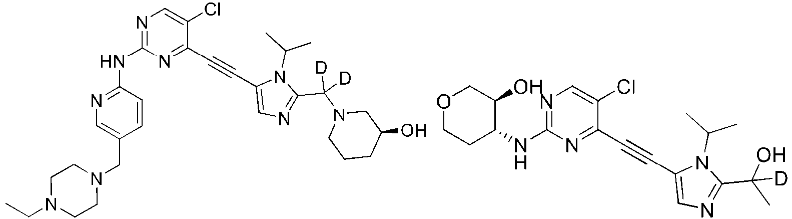

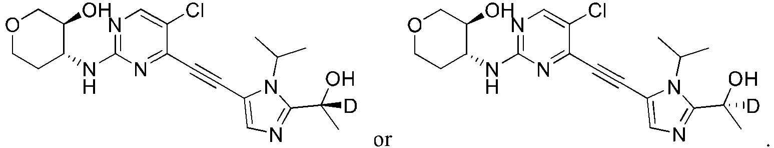

A second aspect of the present invention provides a pharmaceutical composition comprising a compound of formula (I), the stereoisomer or pharmaceutically acceptable salt thereof, and a pharmaceutically acceptable carrier.

The present invention also relates to the use of a compound of formula (I), the stereoisomer or pharmaceutically acceptable salt thereof in the preparation of a medicament for the treatment of a tumor at least partially associated with CDK.

As a preferred embodiment, the present invention also relates to the use of the compound of formula (I), the stereoisomer or pharmaceutically acceptable salt thereof in the preparation of a medicament for the treatment of a tumor at least partially associated with CDK; wherein, the tumor is a cancer.

The present invention also relates to the use of a compound of formula (I), the stereoisomer or pharmaceutically acceptable salt thereof in the preparation of a medicament for the treatment of breast cancer, malignant brain tumor, colon cancer, small cell lung cancer, non-small cell lung cancer, bladder cancer, ovarian cancer, prostate cancer, chronic lymphatic leukemia, lymphoma, myeloma, acute myeloid leukemia, secondary pancreatic cancer or secondary brain metastasis at least partially associated with CDK.

The present invention also relates to the compound of formula (I), the stereoisomer the pharmaceutically acceptable salt thereof for use in the treatment of a tumo at least partially associated with CDK.

As a preferred embodiment, the present invention also relates to the compound of formula (I), the stereoisomer or pharmaceutically acceptable salt thereof for use in the treatment of a tumor at least partially associated with CDK.

The present invention also relates to the compound of formula (I), the stereoisomer or pharmaceutically acceptable salt thereof for use in the treatment of breast cancer, malignant brain tumor, colon cancer, small cell lung cancer, non-small cell lung cancer, bladder cancer, ovarian cancer, prostate cancer, chronic lymphatic leukemia, lymphoma, myeloma, acute myeloid leukemia, secondary pancreatic cancer or secondary brain metastasis at least partially associated with CDK.

### DETAILED DESCRIPTION OF THE INVENTION

After extensive and in-depth research, the inventors of the present application have developed a CDK inhibitor having the following structure of formula (I) for the first time. The compounds of the present invention can be widely used in the manufacture of a medicament for the treatment of cancers or tumors associated at least in part with CDK, and are expected to be developed into a new generation of CDK inhibitors. On this basis, the present invention is completed.

Detailed description: Unless stated to the contrary or otherwise specified, the following terms used in the description and claims have the meanings set forth below.

The term "alkyl" refers to a straight or branched saturated aliphatic hydrocarbon group, preferably a straight or branched alkyl group of 1 to 10, 1 to 6, or 1 to 4 carbon atoms. Non-limiting examples of alkyl include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl or various branched isomers thereof. The term "C₁₋₁₀ alkyl" refers to a straight or branched alkyl group of 1 to 10 carbon atoms; "C₁₋₄ alkyl" refers to a straight or branched alkyl group of 1 to 4 carbon atoms; "C₀₋₈ alkyl" refers to a straight or branched alkyl group of 0 to 8 carbon atoms; "C₀₋₄ alkyl" refers to a straight or branched alkyl group of 0 to 4 carbon atoms.

Alkyl may be optionally substituted or unsubstituted, and when substituted, the substituent is preferably one or more (preferably 1, 2, 3 or 4) groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, - C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅.

The term "cycloalkyl" or "carbocycle" refers to a saturated or partially unsaturated monocyclic or polycyclic aliphatic hydrocarbon substituent, wherein the partially unsaturated cyclic hydrocarbon means that the cyclic hydrocarbon may contain one or more (preferably 1, 2 or 3) double bonds, but none of the rings has a fully conjugated π-electron system. Cycloalkyl includes monocyclic cycloalkyl and polycyclic cycloalkyl, preferably a cycloalkyl group of 3 to 12, 3 to 8, or 3 to 6 carbon atoms. For example, "C₃₋₁₂ cycloalkyl" refers to a cycloalkyl group of 3 to 12 carbon atoms; "C₄₋₈ cycloalkyl" refers to a cycloalkyl group of 4 to 8 carbon atoms; "C₃₋₈ cycloalkyl" refers to a cycloalkyl group of 3 to 8 carbon atoms; "C₃₋₆ cycloalkyl" refers to a cycloalkyl group of 3 to 6 carbon atoms, wherein:
Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, and the like.

Polycyclic cycloalkyl includes spirocycloalkyl, fused cycloalkyl and bridged cycloalkyl. The term "spirocycloalkyl" refers to a polycyclic group in which monocyclic rings share one carbon atom (referred to as a spiro atom), which may contain one or more (preferably 1, 2 or 3) double bonds, but none of the rings has a fully conjugated π-electron system. According to the number of spiro atoms shared between rings, it may be monospirocycloalkyl, bispirocycloalkyl or polyspirocycloalkyl. Non-limiting examples of spirocycloalkyl include:

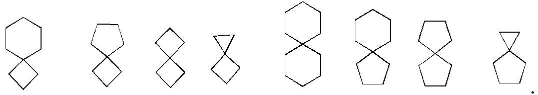

The term "fused cycloalkyl" refers to an all-carbon polycyclic group in which each ring in the system shares an adjacent pair of carbon atoms with the other rings in the system, wherein one or more rings may contain one or more (preferably 1, 2 or 3) double bonds, but none of the rings has a fully conjugated π-electron system. According to the number of constituent rings, it may be bicyclic, tricyclic, tetracyclic or polycyclic fused cycloalkyl. Non-limiting examples of fused cycloalkyl include:

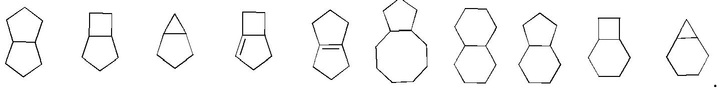

The term "bridged cycloalkyl" refers to an all-carbon polycyclic group in which any two rings share two carbon atoms that are not directly connected to each other, which may contain one or more (preferably 1, 2 or 3) double bonds, but none of the rings has a fully conjugated π-electron system. According to the number of constituent rings, it may be bicyclic, tricyclic, tetracyclic or polycyclic bridged cycloalkyl. Non-limiting examples of bridged cycloalkyl include:

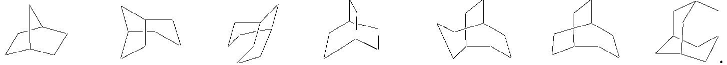

The cycloalkyl ring may be fused to an aryl, heteroaryl or heterocycloalkyl ring, wherein the ring attached to the parent structure is cycloalkyl; non-limiting examples include indanyl, tetrahydronaphthyl, benzocycloheptyl, and the like.

Cycloalkyl may be optionally substituted or unsubstituted, and when substituted, the substituent is preferably one or more (preferably 1, 2, 3 or 4) groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, - C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅.
The term "heterocyclyl" or "heterocycle" refers to a saturated or partially unsaturated monocyclic or polycyclic aliphatic hydrocarbon substituent, wherein the partially unsaturated cyclic hydrocarbon means that the cyclic hydrocarbon may contain one or more (preferably 1, 2 or 3) double bonds, but none of the rings has a fully conjugated π-electron system; wherein one or more (preferably 1, 2, 3 or 4) ring atoms in the heterocyclyl group are heteroatoms selected from N, O, N•O or S(O)ᵣ (where r is an integer from 0, 1 or 2), but does not contain a ring moiety of -O-O-, - O-S- or -S-S-, and the other ring atoms are carbon. Heterocyclyl preferably contains 3 to 12, 3 to 8, or 3 to 6 ring atoms. For example, "3-6 membered heterocyclyl" refers to a heterocyclyl group of 3 to 6 ring atoms; "3-8 membered heterocyclyl" refers to a heterocyclyl group of 3 to 8 ring atoms; "4-8 membered heterocyclyl" refers to a heterocyclyl group of 4 to 8 ring atoms; "4-10 membered heterocyclyl" refers to a heterocyclyl group of 4 to 10 ring atoms; "5-8 membered heterocyclyl" refers to a heterocyclyl group of 5 to 8 ring atoms; and" 3-12 membered heterocyclyl" refers to a heterocyclyl group of 3 to 12 ring atoms.

Non-limiting examples of monocyclic heterocyclyl include pyrrolidinyl, piperidinyl, piperazinyl, morpholinyl, thiomorpholinyl, homopiperazinyl, oxetanyl, tetrahydrofuranyl, and the like.

Polycyclic heterocyclyl includes spiroheterocyclyl, fused heterocyclyl and bridged heterocyclyl. The term "spiroheterocyclyl" refers to a polycyclic heterocyclyl group in which monocyclic rings share one atom (referred to as a spiro atom), wherein one or more (preferably 1, 2, 3 or 4) ring atoms are heteroatoms selected from N, O, N•O or S(O)ᵣ (where r is an integer from 0, 1 or 2) and the other ring atoms are carbon. These may contain one or more (preferably 1, 2 or 3) double bonds, but none of the rings has a fully conjugated π-electron system. According to the number of spiro atoms shared between rings, it may be monospiroheterocyclyl, bispiroheterocyclyl or polyspiroheterocyclyl. Non-limiting examples of spiroheterocyclyl include:

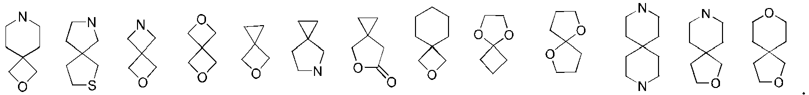

The term "fused heterocyclyl" refers to a polycyclic heterocyclyl group in which each ring in the system shares an adjacent pair of atoms with the other rings in the system, wherein one or more (preferably 1, 2, 3 or 4) rings may contain one or more (preferably 1, 2 or 3) double bonds, but none of the rings has a fully conjugated π-electron system; wherein one or more (preferably 1, 2, 3 or 4) ring atoms are heteroatoms selected from N, O, N•O or S(O)ᵣ (where r is an integer from 0, 1 or 2) and the other ring atoms are carbon. According to the number of constituent rings, it may be bicyclic, tricyclic, tetracyclic or polycyclic fused heterocyclyl. Non-limiting examples of fused heterocyclyl include:

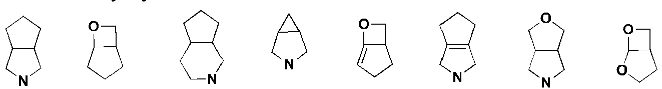

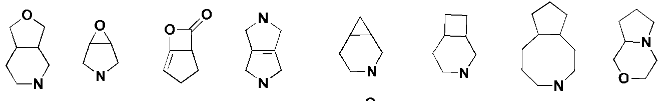

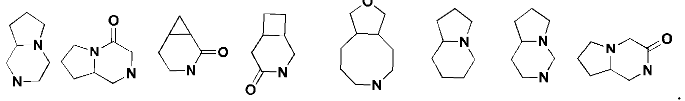

The term "bridged heterocyclyl" refers to a polycyclic heterocyclyl group in which any two rings share two atoms that are not directly connected to each other, which may contain one or more (preferably 1, 2 or 3) double bonds, but none of the rings has a fully conjugated π-electron system; wherein one or more (preferably 1, 2, 3 or 4) ring atoms are heteroatoms selected from N, O, N•O or S(O)ᵣ (where r is an integer from 0, 1 or 2) and the other ring atoms are carbon. According to the number of constituent rings, it may be bicyclic, tricyclic, tetracyclic or polycyclic bridged heterocyclyl. Non-limiting examples of bridged heterocyclyl include:

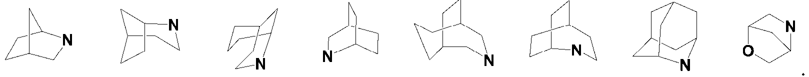

The heterocyclyl ring may be fused to an aryl, heteroaryl or cycloalkyl ring, wherein the ring attached to the parent structure is heterocyclyl; non-limiting examples include:

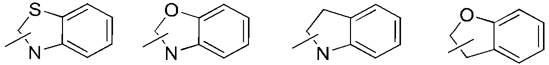

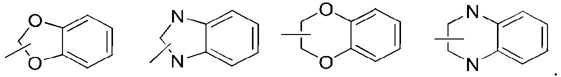

Heterocycly may be optionally substituted or unsubstituted, and when substituted, the substituent is preferably one or more (preferably 1, 2, 3 or 4) groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, - C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅.

The term "aryl" or "aromatic ring" refers to an all-carbon monocyclic or fused polycyclic (i.e., rings that share an adjacent pair of carbon atoms) group having a conjugated π-electron system, preferably an all-carbon aryl group of 6 to 10 or 6 to 8 carbon atoms. For example, "C₆₋₁₀ aryl" refers to an all-carbon aryl group of 6 to 10 carbon atoms, including but not limited to phenyl and naphthyl; "C₆₋₈ aryl" refers to an all-carbon aryl group of 6 to 8 carbon atoms. The aryl ring may be fused to a heteroaryl, heterocyclyl or cycloalkyl ring, wherein the ring attached to the parent structure is the aryl ring; non-limiting examples include:

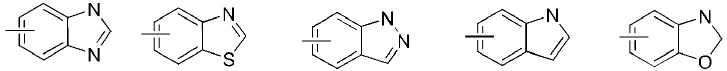

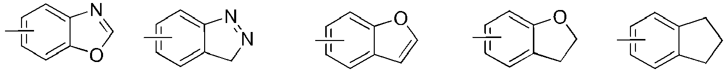

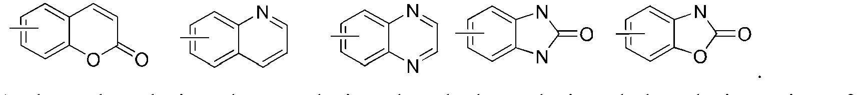

Aryl may be substituted or unsubstituted, and when substituted, the substituent is preferably one or more (preferably 1, 2, 3 or 4) groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅.

The term "heteroaryl" refers to a heteroaromatic system containing one or more (preferably 1, 2, 3 or 4) heteroatoms selected from N, O, N•O and S(O)ᵣ (where r is an integer from 0, 1 or 2), preferably a heteroaromatic system containing 5 to 10, 5 to 8, or 5 to 6 ring atoms. For example, "5-8 membered heteroaryl" refers to a heteroaromatic system containing 5 to 8 ring atoms; "5-10 membered heteroaryl" refers to a heteroaromatic system containing 5 to 10 ring atoms. Non-limiting examples include furanyl, thienyl, pyridyl, pyrrolyl, N-alkylpyrrolyl, pyrimidinyl, pyrazinyl, imidazolyl, tetrazolyl, and the like. The heteroaryl ring may be fused to an aryl, heterocyclyl or cycloalkyl ring, wherein the ring attached to the parent structure is the heteroaryl ring; non-limiting examples include:

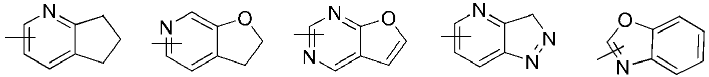

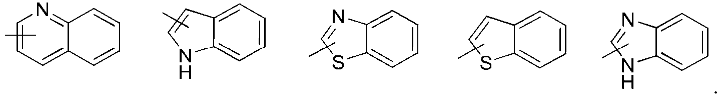

Heteroaryl may be optionally substituted or unsubstituted, and when substituted, the substituent is preferably one or more (preferably 1, 2, 3 or 4) groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, - C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅.

The term "alkenyl" refers to an alkyl group consisting of at least two carbon atoms and at least one carbon-carbon double bond as defined above, preferably a straight or branched alkenyl group of 2 to 10 or 2 to 4 carbon atoms. For example, "C₂₋₁₀ alkenyl" refers to a straight or branched alkenyl group of 2 to 10 carbon atoms; "C₂₋₄ alkenyl" refers to a straight or branched alkenyl group of 2 to 4 carbon atoms. Non-limiting examples include vinyl, 1-propenyl, 2-propenyl, 1-, 2- or 3-butenyl, and the like.

Alkenyl may be substituted or unsubstituted, and when substituted, the substituent is preferably one or more (preferably 1, 2, 3 or 4) groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, - C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅.

The term "alkynyl" refers to an alkyl group consisting of at least two carbon atoms and at least one carbon-carbon triple bond as defined above, preferably a straight or branched alkynyl group of 2 to 10 or 2 to 4 carbon atoms. For example, "C₂₋₁₀ alkynyl" refers to a straight or branched alkynyl group of 2 to 10 carbon atoms; "C₂₋₄ alkynyl" refers to a straight or branched alkynyl group of 2 to 4 carbon atoms. Non-limiting examples include ethynyl, 1-propynyl, 2-propynyl, 1-, 2- or 3-butynyl, and the like.

Alkynyl may be substituted or unsubstituted, and when substituted, the substituent is preferably one or more (preferably 1, 2, 3 or 4) groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, - C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅.

The term "alkoxyl" refers to -O-alkyl, wherein the alkyl is as defined above. For example, "C₁₋₁₀ alkoxyl" refers to an alkoxyl group of 1 to 10 carbon atoms; "C₁₋₄ alkoxyl" refers to an alkoxyl group of 1 to 4 carbon atoms; "C₁₋₂ alkoxyl" refers to an alkoxyl group of 1 to 2 carbon atoms. Non-limiting examples include methoxy, ethoxy, propoxy, butoxy, and the like.

Alkoxyl may be optionally substituted or unsubstituted, and when substituted, the substituent is preferably one or more (preferably 1, 2, 3 or 4) groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, - C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅.

The term "cycloalkoxyl" refers to -O-cycloalkyl, wherein the cycloalkyl is as defined above. For example, "C₃₋₁₂ cycloalkoxyl" refers to a cycloalkoxyl group of 3 to 12 carbon atoms; "C₃₋₆ cycloalkoxyl" refers to a cycloalkoxyl group of 3 to 6 carbon atoms. Non-limiting examples include cyclopropoxy, cyclobutoxy, cyclopentyloxy, cyclohexyloxy, and the like.

Cycloalkoxyl may be optionally substituted or unsubstituted, and when substituted, the substituent is preferably one or more (preferably 1, 2, 3 or 4) groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, - C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅.

The term "heterocycloxyl" refers to -O-heterocyclyl, wherein the heterocyclyl is as defined above. Non-limiting examples include azetidinyloxy, oxetanyloxy, azopentyloxy, nitrogen, oxhexyloxy, and the like.

Heterocycloxyl may be optionally substituted or unsubstituted, and when substituted, the substituent is preferably one or more (preferably 1, 2, 3 or 4) groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, - C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅.

The term "C₁₋₁₀ alkanoyl" refers to the monovalent atomic group remaining after the hydroxyl group is removed from a C₁₋₁₀ alkyl acid, and is also commonly represented as "C₀₋₉ alkyl-C(O)-". For example, "C₁ alkyl-C(O)-" refers to acetyl; "C₂ alkyl-C(O)-" refers to propionyl; "C₃ alkyl-C(O)-" refers to butyryl or isobutyryl.

The term "-C₀₋₈ alkyl-S(O)(=N-R₁₀)R₁₁" refers to that the sulfur atom in -S(O)(=N-R₁₀)R₁₁ is attached to a C₀₋₈ alkyl group, wherein the C₀₋₈ alkyl is as defined above.

The term "-C₀₋₈ alkyl-N=S(O)R₁₁R₁₂" refers to that the nitrogen atom in -N=S(O)R₁₁R₁₂ is attached to a C₀₋₈ alkyl group, wherein the C₀₋₈ alkyl is as defined above.

The term "-C₀₋₈ alkyl-N=SR₁₁R₁₂" refers to that the nitrogen atom in -N=SR₁₁R₁₂ is attached to a C₀₋₈ alkyl group, wherein the C₀₋₈ alkyl is as defined above.

The term "-C₀₋₈ alkyl-O-S(O)₂R₁₃" refers to that the oxygen atom in -O-S(O)₂R₁₃ is attached to a C₀₋₈ alkyl group, wherein the C₀₋₈ alkyl is as defined above.

The term "-C₀₋₈ alkyl-S(O)ᵣR₁₃" refers to that the sulfur atom in -S(O)ᵣR₁₃ is attached to a C₀₋₈ alkyl group, wherein the C₀₋₈ alkyl is as defined above.

The term "-C₀₋₈ alkyl-P(O)(R₁₅)₂" refers to that the phosphorus atom in -P(O)(R₁₅)₂ is attached to a C₀₋₈ alkyl group, wherein the C₀₋₈ alkyl is as defined above.

The term "-C₀₋₈ alkyl-O-R₁₄" refers to that the oxygen atom in -O-R₁₄ is attached to a C₀₋₈ alkyl group, wherein the C₀₋₈ alkyl is as defined above.

The term "-C₀₋₈ alkyl-C(O)OR₁₄" refers to that the carbonyl group in -C(O)OR₁₄ is attached to a C₀₋₈ alkyl group, wherein the C₀₋₈ alkyl is as defined above.

The term "-C₀₋₈ alkyl-C(O)SR₁₄" refers to that the carbonyl group in -C(O)SR₁₄ is attached to a C₀₋₈ alkyl group, wherein the C₀₋₈ alkyl is as defined above.

The term "-C₀₋₈ alkyl-S-C(O)R₁₅" refers to that the sulfur atom in -S-C(O)R₁₅ is attached to a C₀₋₈ alkyl group, wherein the C₀₋₈ alkyl is as defined above.

The term "-C₀₋₈ alkyl-C(O)R₁₅" refers to that the carbonyl group in -C(O)R₁₅ is attached to a C₀₋₈ alkyl group, wherein the C₀₋₈ alkyl is as defined above.

The term "-C₀₋₈ alkyl-O-C(O)R₁₅" refers to that the oxygen atom in -O-C(O)R₁₅ is attached to a C₀₋₈ alkyl group, wherein the C₀₋₈ alkyl is as defined above.

The term "-C₀₋₈ alkyl-NR₁₆R₁₇" refers to that the nitrogen atom in -NR₁₆R₁₇ is attached to a C₀₋₈ alkyl group, wherein the C₀₋₈ alkyl is as defined above.

The term "-C₀₋₈ alkyl-C(=NR₁₆)R₁₅" refers to that the carbon atom in -C(=NR₁₆)R₁₅ is attached to a C₀₋₈ alkyl group, wherein the C₀₋₈ alkyl is as defined above.

The term "-C₀₋₈ alkyl-N(R₁₆)-C(=NR₁₇)R₁₅" refers to that the nitrogen atom in -N(R₁₆)-C(=NR₁₇)R₁₅ is attached to a C₀₋₈ alkyl group, wherein the C₀₋₈ alkyl is as defined above.

The term "-C₀₋₈ alkyl-C(O)NR₁₆R₁₇" refers to that the carbonyl group in -C(O)NR₁₆R₁₇ is attached to a C₀₋₈ alkyl group, wherein the C₀₋₈ alkyl is as defined above.

The term "-C₀₋₈ alkyl-N(R₁₆)-C(O)R₁₅" refers to that the nitrogen atom in -N(R₁₆)-C(O)R₁₅ is attached to a C₀₋₈ alkyl group, wherein the C₀₋₈ alkyl is as defined above.

The term " C₁₋₁₀ haloalkyl" refers to an alkyl group of 1 to 10 carbon atoms in which the hydrogen on the alkyl group is optionally substituted with a fluorine, chlorine, bromine or iodine atom. Non-limiting examples include difluoromethyl, dichloromethyl, dibromomethyl, trifluoromethyl, trichloromethyl, tribromomethyl, and the like.

The term " C₁₋₁₀ haloalkoxyl" refers to an alkoxyl group of 1 to 10 carbon atoms in which the hydrogen on the alkyl group is optionally substituted with a fluorine, chlorine, bromine or iodine atom. Non-limiting examples include difluoromethoxy, dichloromethoxy, dibromomethoxy, trifluoromethoxy, trichloromethoxy, tribromomethoxy, and the like.

The term " C₁₋₁₀ deuterioalkyl" refers to an alkyl group of 1 to 10 carbon atoms in which the hydrogen on the alkyl group is optionally substituted with a deuterium atom. Non-limiting examples include monodeuteriomethyl, dideuteriomethyl, trideuteriomethyl, and the like.

The term "halogen" refers to fluorine, chlorine, bromine or iodine.

The term "optionally" refers to that the event or circumstance described subsequently may, but need not, occur, and the description includes occasions where the event or circumstance occurs or does not occur, that is, both substituted and unsubstituted situations are included. For example, "heterocyclyl optionally substituted with alkyl" means that an alkyl group may, but does not have to, be present, and the description includes the case where the heterocyclyl is substituted with an alkyl group and the case where the heterocyclyl is not substituted with an alkyl group.

The term "substituted" refers to that one or more "hydrogen atoms" in a group are substituted independently of each other with a corresponding number of substituents. It goes without saying that the substituents are only in their possible chemical positions and consistent with the valence bond theory in chemistry, and those skilled in the art can determine (by experiment or theory) possible or impossible substitutions without undue effort. For example, an amino or hydroxyl group having a free hydrogen may be unstable when bound to a carbon atom having an unsaturated bond (such as an olefin).

The term "stereoisomer" refers to an isomer produced by the different spatial arrangement of atoms in a molecule. It can be divided into two types: cis-trans isomers and enantiomers, or into two categories: enantiomers and diastereomers. Stereoisomers caused by the rotation of a single bond are called conformational stereoisomers, sometimes also called rotamers. Stereoisomers caused by bond length, bond angle, double bonds in the molecule, or rings are called configuration stereoisomers. Configuration stereoisomers are divided into two categories. Among them, isomers caused by the inability of double bonds or single bonds of ring-forming carbon atoms to rotate freely are called geometric isomers, also known as cis-trans isomers, which are divided into Z and E configurations. For example, cis-2-butene and trans-2-butene are a pair of geometric isomers. Stereoisomers with different optical properties due to the lack of anti-axis symmetry in the molecule are called optical isomers, which are divided into R and S configurations. The term "stereoisomer" in the present invention, unless otherwise specified, can be understood to include one or more of the enantiomers, conformational stereoisomers, and conformational stereoisomers described above.

The term "pharmaceutically acceptable salt" in the present invention refers to pharmaceutically acceptable acid addition salts, including inorganic and organic acid salts, and these salts can be prepared by methods known in the art.

The term "pharmaceutical composition" refers to a mixture containing one or more of the compounds described herein or a physiologically/pharmaceutically acceptable salt or prodrug thereof and other chemical components, as well as other components such as physiologically/pharmaceutically acceptable carriers and excipients. The purpose of a pharmaceutical composition is to facilitate administration to an organism, facilitate absorption of the active ingredient, and thus exert biological activity.

**Hereinafter, the present invention is further explained in detail below with reference to examples, which are not intended to limit the present invention, and the present invention is not merely 30 limited to the contents of the examples.**

The compound structure of the present invention was determined by nuclear magnetic resonance (NMR) and/or liquid chromatography-mass spectrometry (LC-MS). The NMR chemical shift (δ) is given in parts per million (ppm). The NMR measurement was performed using a Bruker AVANCE-400/500 spectrometer, with deuterated dimethyl sulfoxide (DMSO-*d₆*), deuterated methanol (CD₃OD/MeOH-*d*₄), and deuterated chloroform (CDCl₃) as solvents, and tetramethylsilane (TMS) as the internal standard.

The LC-MS analysis was performed using an Agilent 6120 mass spectrometer. HPLC analysis was performed using an Agilent 1200DAD HPLC system (Sunfire C18 150 × 4.6 mm column) and a Waters 2695-2996 HPLC system (Gemini C18 150 × 4.6 mm column).

The thin-layer chromatography (TLC) was performed using a Yantai Huanghai HSGF254 or Qingdao GF254 silica gel plate. The specification used for TLC was 0.15 to 0.20 mm, and for purification by TLC, it was 0.4 to 0.5 mm. The column chromatography was typically performed using a Yantai Huanghai silica gel (200-300 mesh) as the carrier.

The starting materials used in the examples of the present invention are known and commercially available, or can be synthesized by or according to methods known in the art.

Unless otherwise specified, all reactions of the present invention were carried out under a dry nitrogen or argon atmosphere with continuous magnetic stirring. The solvents were dry ones. The reaction temperature was in degrees centigrade (°C).

### Preparation of Examples

### Example 1: Preparation of 1-(5-((5-chloro-2-(((3S,4R)-3-hydroxytetrahydro-2H-pyran-4-yl)amino)pyrimidin-4-yl)ethynyl)-1-isopropyl-1H-imidazol-2-yl)ethan-1-one

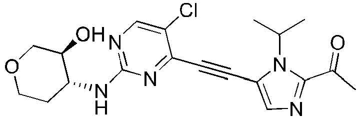

### Step 1: Synthesis of 2,5-diiodo-1-isopropyl-1H-imidazole

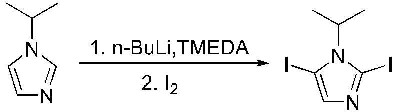

N,N,N,N-Tetramethylethylenediamine (10 mL, 66 mmol) was dissolved in n-pentane (28 mL). Under a nitrogen atmosphere at -78°C, a solution of n-butyllithium in n-hexane (26.400 mL, 2.5 M) was added while stirring, followed by dropwise addition of 1-isopropylimidazole (3.0 mL, 26 mmol). The solution was allowed to warm to room temperature and stirred for 1 h, then diluted with tetrahydrofuran (50 mL) and cooled to -78°C. Iodine (16.75 g, 66.00 mmol) was dissolved in tetrahydrofuran (80 mL) and added dropwise to the reaction mixture at -78°C, which was then stirred for 1.5 h. The reaction mixture was allowed to warm to room temperature and stirred overnight. Ethyl acetate (10 mL) was added to quench the reaction. The reaction mixture was concentrated and separated by silica gel column chromatography [eluent: 0%-60% ethyl acetate in petroleum ether] to obtain 2,5-diiodo-1-isopropyl-1H-imidazole (5.21 g, yield: 55%). MS (ESI): *m*/*z* 362.8 [M+H]⁺.

### Step 2: Synthesis of 1-(5-iodo-1-isopropyl-1H-imidazol-2-yl)ethan-1-one

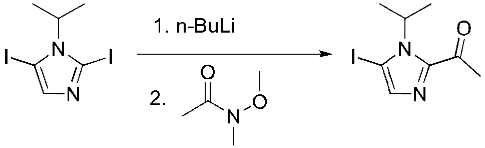

2,5-Diiodo-1-isopropyl-1H-imidazole (4.21 g, 11.631 mmol) was dissolved in tetrahydrofuran (40 mL) under a nitrogen atmosphere and cooled to -78°C. Then, a solution of n-butyllithium in n-hexane (5.1 mL, 2.5 M) was added and stirred at -78°C for 30 min. The reaction mixture was allowed to warm to room temperature and stirred for 30 min. N-methoxy-N-methylacetamide (1.2 mL, 11.631 mmol) was dissolved in tetrahydrofuran (40 mL) and added dropwise to the reaction mixture at -78°C. The reaction mixture was allowed to warm to room temperature and stirred overnight. Ethyl acetate (10 mL) was added to quench the reaction. The reaction mixture was concentrated and separated by silica gel column chromatography [eluent: 0%-4% ethyl acetate in petroleum ether] to obtain 1-(5-iodo-1-isopropyl-1H-imidazol-2-yl)ethan-1-one (2.20 g, yield: 68%). MS (ESI): *m*/*z* 279.0 [M+H]⁺.

### Step 3: Synthesis of 1-(1-isopropyl-5-((trimethylsilyl)ethynyl)-1H-imidazol-2-yl)ethan-1-one

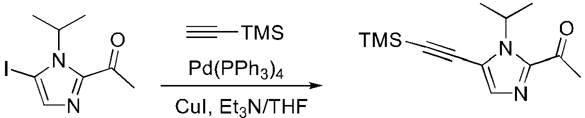

To a Schlenk tube, 1-(5-iodo-1-isopropyl-1H-imidazol-2-yl)ethan-1-one (1.60 g, 5.74 mmol), ethynyltrimethylsilane (2.5 mL, 17 mmol), tetrakis(triphenylphosphine)palladium (331.8 mg, 0.287 mmol), cuprous iodide (237.4 mg, 0.57 mmol), triethylamine (10 mL), and tetrahydrofuran (10 mL) were added. The reaction mixture was purged three times with nitrogen, then stirred at 70°C overnight, diluted with ethyl acetate (20 mL), filtered through silica gel (10 g) in a sand core funnel, and washed twice with ethyl acetate (10 mL). The filtrate was concentrated to give crude 1-(1-isopropyl-5-((trimethylsilyl)ethynyl)-1H-imidazol-2-yl)ethan-1-one, which was used directly in the next step.

### Step 4: Synthesis of 1-(5-ethynyl-1-isopropyl-1H-imidazol-2-yl)ethan-1-one

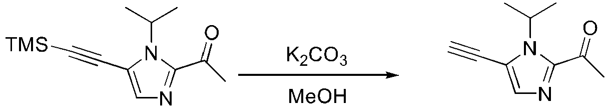

The 1-(1-isopropyl-5-((trimethylsilyl)ethynyl)-1H-imidazol-2-yl)ethan-1-one obtained in the previous step was dissolved in methanol (10 mL). Potassium carbonate (100 mg) was added, and the reaction mixture was stirred at room temperature for 3 h. The reaction mixture was diluted with dichloromethane (20 mL), filtered through celite in a sand core funnel, and washed with ethyl acetate (10 mL). The pooled filtrate was concentrated and separated by silica gel column chromatography [eluent: 0%-4% ethyl acetate in petroleum ether] to obtain 1-(5-ethynyl-1-isopropyl-1H-imidazol-2-yl)ethan-1-one (757.0 mg, two-step yield: 68%). MS (ESI): *m*/*z* 177.0 [M+H]⁺.

### Step 5: Synthesis of 1-(5-((2,5-dichloropyrimidin-4-yl)ethynyl)-1-isopropyl-1H-imidazol-2-yl)ethan-1-one

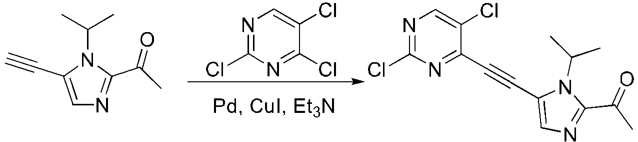

To a Schlenk tube, 1-(5-ethynyl-1-isopropyl-1H-imidazol-2-yl)ethan-1-one (264.2 mg, 1.50 mmol), 2,4,5-trichloropyrimidine (250 mg, 1.363 mmol), tetrakis(triphenylphosphine)palladium (78.75 mg, 0.068 mmol), cuprous iodide (0.011 mL, 0.136 mmol), and triethylamine (10 mL) were added. The reaction mixture was purged three times with nitrogen, then stirred at 100°C overnight, diluted with ethyl acetate (20 mL), filtered through silica gel (10 g) in a sand core funnel, and washed twice with ethyl acetate (10 mL). The filtrate was concentrated and separated by silica gel column chromatography [eluent: 0%-4% ethyl acetate in petroleum ether] to obtain 1-(5-((2,5-dichloropyrimidin-4-yl)ethynyl)-1-isopropyl-1H-imidazol-2-yl)ethan-1-one (286 mg, yield: 58.43%). MS (ESI): *m*/*z* 323.0 [M+H]⁺.

### Step 6: Synthesis of 1-(5-((5-chloro-2-(((3S,4R)-3-hydroxytetrahydro-2H-pyran-4-yl)amino)pyrimidin-4-yl)ethynyl)-1-isopropyl-1H-imidazol-2-yl)ethan-1-one

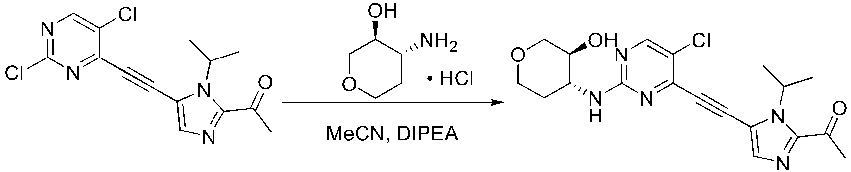

1-(5-((2,5-Dichloropyrimidin-4-yl)ethynyl)-1-isopropyl-1H-imidazol-2-yl)ethan-1-one (200 mg, 0.473 mmol), (3S,4R)-4-aminotetrahydropyran-3-ol hydrochloride (61.79 mg, 0.402 mmol), and diisopropylethylamine (0.2 mL) were dissolved in acetonitrile (2 mL) under a nitrogen atmosphere, and the reaction mixture was stirred at 110°C overnight. Subsequent separation by preparative chromatography gave 1-(5-((5-chloro-2-(((3S,4R)-3-hydroxytetrahydro-2H-pyran-4-yl)amino)pyrimidin-4-yl)ethynyl)-1-isopropyl-1H-imidazol-2-yl)ethan-1-one (30 mg, yield: 23%). MS (ESI): *m*/*z* 404.2 [M+H]⁺.

¹H NMR (400 MHz, CD₃OD) δ 8.36 (s, 1H), 7.60 (s, 1H), 5.86 (p, *J* = 6.9 Hz, 1H), 3.94 (ddd, *J* = 18.5, 10.6, 4.5 Hz, 3H), 3.59 (td, *J* = 9.4, 4.8 Hz, 1H), 3.49 (td, *J* = 11.7, 2.2 Hz, 1H), 3.21 (dd, *J =* 11.2, 9.7 Hz, 1H), 2.66 (d, *J* = 1.0 Hz, 3H), 2.10 (dd, *J =* 12.7, 3.2 Hz, 1H), 1.70 (d, *J =* 7.0 Hz, 6H), 1.62 (qd, *J =* 11.7, 4.6 Hz, 1H).

### Example 2: Preparation of (3S,4R)-4-((5-chloro-4-((2-(2-hydroxypropan-2-yl)-1-isopropyl-1H-imidazol-5-yl)ethynyl)pyrimidin-2-yl)amino)tetrahydro-2H-pyran-3-ol

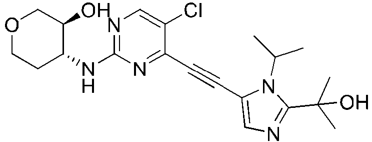

### Step 1: Synthesis of 2-(5-((2,5-dichloropyrimidin-4-yl)ethynyl)-1-isopropyl-1H-imidazol-2-yl)propan-2-ol

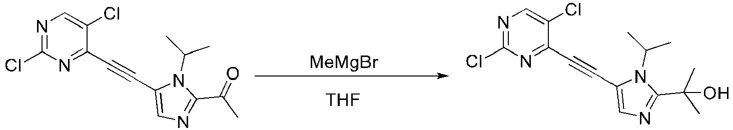

1-(5-((2,5-Dichloropyrimidin-4-yl)ethynyl)-1-isopropyl-1H-imidazol-2-yl)ethan-1-one (100 mg, 0.309 mmol) was dissolved in tetrahydrofuran (10 mL). Under a nitrogen atmosphere at 0°C, a solution of methylmagnesium bromide in methyltetrahydrofuran (0.5 mL, 3 M) was added, and the reaction mixture was stirred at 0°C for 1 h. The reaction mixture was allowed to warm to room temperature and stirred for an additional 30 min. Methanol (10 mL) was added to quench the reaction. The reaction mixture was concentrated and separated by preparative chromatography to obtain 2-(5-((2,5-dichloropyrimidin-4-yl)ethynyl)-1-isopropyl-1H-imidazol-2-yl)propan-2-ol (30.0 mg, yield: 28.6%). MS (ESI): *m*/*z* 339.0 [M+H]⁺.

¹H NMR (400 MHz, CD₃OD) δ 8.79 (s, 1H), 7.53 (s, 1H), 5.67 (hept, *J* = 6.9 Hz, 1H), 1.74 (d, *J =* 6.9 Hz, 6H), 1.66 (s, 6H).

### Step 2: Synthesis of (3S,4R)-4-((5-chloro-4-((2-(2-hydroxypropan-2-yl)-1-isopropyl-1H-imidazol-5-yl)ethynyl)pyrimidin-2-yl)amino)tetrahydro-2H-pyran-3-ol

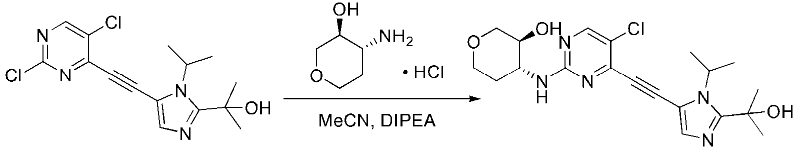

2-(5-((2,5-Dichloropyrimidin-4-yl)ethynyl)-1-isopropyl-1H-imidazol-2-yl)propan-2-ol (30 mg, 0.088 mmol), (3S,4R)-4-aminotetrahydropyran-3-ol hydrochloride (14.94 mg, 0.097 mmol), and diisopropylethylamine (0.055 mL) were dissolved in acetonitrile (1 mL) under a nitrogen atmosphere, and the reaction mixture was stirred at 80°C for 48 h. Subsequent separation by preparative chromatography gave (3 S,4R)-4-((5-chloro-4-((2-(2-hydroxypropan-2-yl)-1-isopropyl-1H-imidazol-5-yl)ethynyl)pyrimidin-2-yl)amino)tetrahydro-2H-pyran-3-ol (12 mg, yield: 32%). MS (ESI): *m*/*z* 420.2 [M+H]⁺.

¹H NMR (400 MHz, CD₃OD) δ 8.31 (s, 1H), 7.39 (s, 1H), 5.63 (hept, *J* = 6.9 Hz, 1H), 4.00-3.87 (m, 3H), 3.59 (td, *J =* 9.5, 4.8 Hz, 1H), 3.49 (td, *J =* 11.7, 2.3 Hz, 1H), 3.21 (dd, *J =* 11.2, 9.7 Hz, 1H), 2.10 (ddd, *J =* 13.5, 4.7, 2.3 Hz, 1H), 1.73 (dd, *J =* 7.0, 1.7 Hz, 6H), 1.68-1.54 (m, 1H), 1.65 (s, 6H).

### Example 57: Preparation of 5-chloro-4-((5-isopropyl-1-methyl-1H-pyrazol-4-yl)ethynyl)-N-(5-(1-methylpiperidin-4-yl)pyridin-2-yl)pyrimidin-2-amine

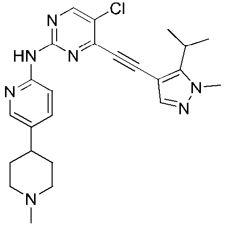

### Step 1: Synthesis of 5-chloro-4-((5-isopropyl-1-methyl-1H-pyrazol-4-yl)ethynyl)-N-(5-(1-methylpiperidin-4-yl)pyridin-2-yl)pyrimidin-2-amine

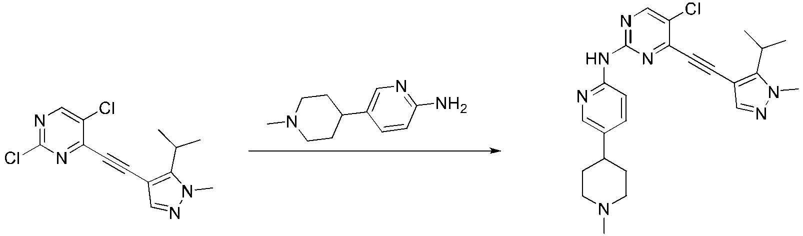

2,5-Dichloro-4-((5-isopropyl-1-methyl-1H-pyrazol-4-yl)ethynyl)pyrimidine (50 mg, 0.169 mmol) was dissolved in a mixture of toluene (2 mL) and tert-butanol (0.5 mL), followed by the addition of 5-(1-methylpiperidin-4-yl)pyridin-2-amine (21.23 mg, 0.138 mmol), cesium carbonate (110.38 mg, 0.339 mmol), Pd₂(dba)₃ (15.51 mg, 0.017 mmol), and 2-dicyclohexylphosphino-2',4',6'-triisopropylbiphenyl (8.08 mg, 0.017 mmol). The reaction mixture was stirred at 100°C overnight under a nitrogen atmosphere. The reaction mixture was filtered, and the filtrate was concentrated and separated by preparative liquid chromatography to obtain 5-chloro-4-((5-isopropyl-1-methyl-1H-pyrazol-4-yl)ethynyl)-N-(5-(1-methylpiperidin-4-yl)pyridin-2-yl)pyrimidin-2-amine (4.5 mg, yield: 8.84%). MS (ESI): *m*/*z* 450.2 [M+H]⁺.

¹H NMR (400 MHz, DMSO-*d*₆) δ 10.10 (s, 1H), 8.63 (s, 1H), 8.18 (s, 1H), 8.04 (d, J = 8.6 Hz, 1H), 7.74 (s, 1H), 7.66 (dd, J = 8.7, 2.5 Hz, 1H), 3.85 (s, 3H), 3.29-3.25 (m, 1H), 2.89-2.83 (m, 2H), 2.47-2.42 (m, 1H), 2.19 (s, 3H), 2.00-1.91 (m, 2H), 1.76-1.63 (m, 4H), 1.43 (d, J = 7.0 Hz, 6H).

### Example 126: Preparation of 3-(5-((5-chloro-2-(((3S,4R)-3-hydroxytetrahydro-2H-pyran-4-yl)amino)pyrimidin-4-yl)ethynyl)-1-isopropyl-1H-imidazol-2-yl)oxazolidin-2-one

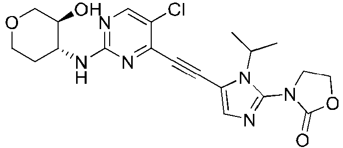

### Step 1: Synthesis of 5-chloro-2-(methylthio)-4-((triisopropylsilyl)ethynyl)pyrimidine

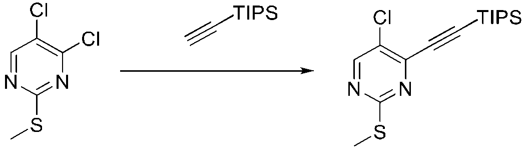

4,5-Dichloro-2-(methylthio)pyrimidine (3.5 g, 17.943 mmol) was dissolved in DMF (35 mL), followed by the addition of triisopropylsilylacetylene (9.82 g, 53.829 mmol), Pd(PPh₃)₂Cl₂ (1.26 g, 1.794 mmol), CuI (0.34 g, 1.794 mmol), and DIPEA (6.96 g, 53.830 mmol) in sequence. The mixture was sealed under a nitrogen atmosphere and heated to 80°C, and stirred overnight. After the reaction was completed to remove most of the acetonitrile, saturated ammonium chloride was added, and the reaction mixture was extracted three times with ethyl acetate. The pooled organic phases were washed with saturated brine, dried over anhydrous sodium sulfate, filtered, and concentrated. Subsequent separation by silica gel column chromatography (100% ethyl acetate in petroleum ether) gave 5-chloro-2-(methylthio)-4-((triisopropylsilyl)ethynyl)pyrimidine (5.4 g, yield: 88%). MS (ESI): *m*/*z* 341.2 [M+H]⁺.

### Step 2: Synthesis of 5-chloro-2-(methylsulfinyl)-4-((triisopropylsilyl)ethynyl)pyrimidine

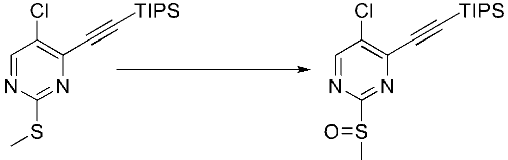

5-Chloro-2-(methylthio)-4-((triisopropylsilyl)ethynyl)pyrimidine (5.4 g, 15.836 mmol) was dissolved in DCM (100 mL), followed by the addition of mCPBA (5.14 g, 25.338 mmol), and the mixture was stirred overnight. After the reaction was completed, the reaction mixture was cooled to 0°C, and a saturated aqueous sodium bicarbonate solution was added to quench the reaction. The reaction mixture was then extracted three times with dichloromethane. The pooled organic phases were washed with saturated aqueous sodium bicarbonate solution and saturated brine, dried over anhydrous sodium sulfate, filtered, and concentrated. Subsequent separation by silica gel column chromatography (50% ethyl acetate in petroleum ether) gave 5-chloro-2-(methylsulfinyl)-4-((triisopropylsilyl)ethynyl)pyrimidine (4.71 g, yield: 83%). MS (ESI): *m*/*z* 357.2 [M+H]⁺.

### Step 3: Synthesis of (3S,4R)-4-((5-chloro-4-((triisopropylsilyl)ethynyl)pyrimidin-2-yl)amino)tetrahydro-2H-pyran-3-ol

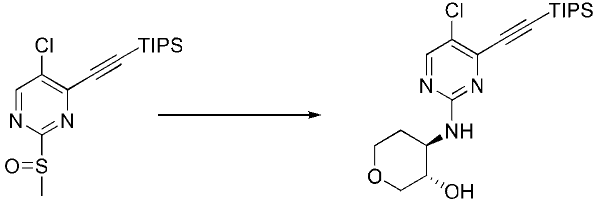

5-Chloro-2-(methylsulfinyl)-4-((triisopropylsilyl)ethynyl)pyrimidine (2.5 g, 7.003 mmol), (3S,4R)-4-aminotetrahydropyran-3-ol hydrochloride (1.61 g, 10.505 mmol), and DIPEA (3.62 g, 28.013 mmol) were dissolved in DMA (25 mL), and the mixture was heated to 90°C and stirred overnight. After the reaction was completed, the reaction mixture was cooled to room temperature, and water was added to quench the reaction. The reaction mixture was then extracted three times with ethyl acetate. The pooled organic phases were washed with saturated brine, dried over anhydrous sodium sulfate, filtered, and concentrated. Subsequent separation by silica gel column chromatography (40% ethyl acetate in petroleum ether) gave (3S,4R)-4-((5-chloro-4-((triisopropylsilyl)ethynyl)pyrimidin-2-yl)amino)tetrahydro-2H-pyran-3-ol (2.08 g, yield: 72%). MS (ESI): *m*/*z* 410.2 [M+H]⁺.

### Step 4: Synthesis of (3S,4R)-4-((5-chloro-4-ethynylpyrimidin-2-yl)amino)tetrahydro-2H-pyran-3-ol

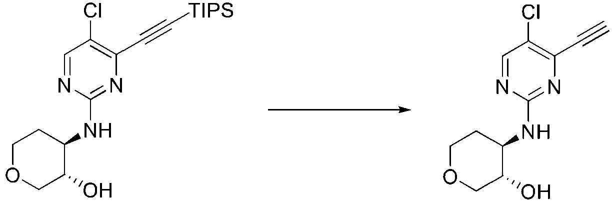

(3S,4R)-4-((5-Chloro-4-((triisopropylsilyl)ethynyl)pyrimidin-2-yl)amino)tetrahydro-2H-pyran-3-ol (2.08 g,5.073 mmol) was dissolved in THF (10 mL). To this solution, TBAF in THF (6.087 mL, 6.087 mmol, 1.0 mol/L) was added in an ice bath and stirred in the ice bath for 1 h. The reaction was then quenched with saturated ammonium chloride. The reaction mixture was extracted three times with ethyl acetate. The pooled organic phases were washed with saturated brine, dried over anhydrous sodium sulfate, filtered, and concentrated. Subsequent separation by silica gel column chromatography (50% ethyl acetate in petroleum ether) gave (3S,4R)-4-((5-chloro-4-ethynylpyrimidin-2-yl)amino)tetrahydro-2H-pyran-3-ol (1.2 g, yield: 93%). MS (ESI): *m*/*z* 254.2 [M+H]⁺.

### Step 5: Synthesis of 3-(5-iodo-1-isopropyl-1H-imidazol-2-yl)oxazolidin-2-one

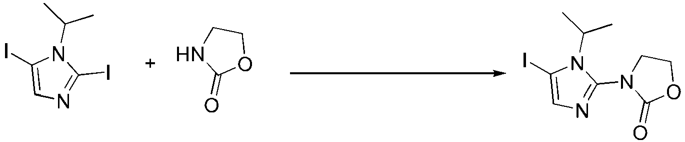

2,5-Diiodo-1-isopropyl-1H-imidazole (600 mg, 1.658 mmol) was dissolved in dioxane (3 mL), followed by the addition of oxazolidin-2-one (721.76 mg, 8.288 mmol), CuI (63.14 mg, 0.332 mmol), K₂CO₃ (458.18 mg, 3.315 mmol), and trans-(1R,2R)-N,N'-dimethyl-1,2-cyclohexanediamine (0.106 mL, 0.663 mmol) in sequence. The mixture was sealed under a nitrogen atmosphere and heated to 100°C, and stirred overnight. After the reaction was completed, the reaction mixture was filtered and concentrated. Subsequent separation by silica gel column chromatography (20% ethyl acetate in petroleum ether) gave 3-(5-iodo-1-isopropyl-1H-imidazol-2-yl)oxazolidin-2-one (320 mg, 0.997 mmol, 60.11%). MS (ESI): *m*/*z* 322.1 [M+H]⁺.

### Step 6: Synthesis of 3-(5-((5-chloro-2-(((3S,4R)-3-hydroxytetrahydro-2H-pyran-4-yl)amino)pyrimidin-4-yl)ethynyl)-1-isopropyl-1H-imidazol-2-yl)oxazolidin-2-one

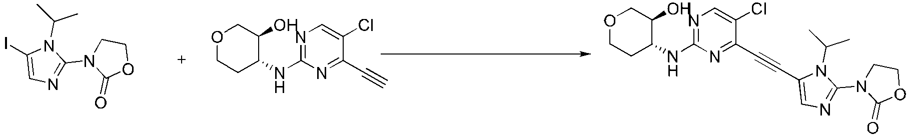

3-(5-Iodo-1-isopropyl-1H-imidazol-2-yl)oxazolidin-2-one (132.91 mg, 0.414 mmol) was dissolved in tetrahydrofuran (2 mL), followed by the addition of (3S,4R)-4-((5-chloro-4-ethynylpyrimidin-2-yl)amino)tetrahydro-2H-pyran-3-ol (70 mg, 0.276 mmol), CuI (5.26 mg, 0.028 mmol), tetrakis(triphenylphosphine)palladium (17.99 mg, 0.016 mmol), and DIPEA (0.137 mL, 0.828 mmol). The reaction mixture was stirred at 50°C overnight under a nitrogen atmosphere. After the reaction was completed, the reaction mixture was diluted with water and extracted three times with ethyl acetate. The pooled organic phases were dried over anhydrous sodium sulfate, filtered, and concentrated. Subsequent separation by silica gel column chromatography (20% methanol in dichloromethane) gave the crude product, which was then separated by preparative liquid chromatography to obtain 3-(5-((5-chloro-2-(((3 S,4R)-3-hydroxytetrahydro-2H-pyran-4-yl)amino)pyrimidin-4-yl)ethynyl)-1-isopropyl-1H-imidazol-2-yl)oxazolidin-2-one (41.6 mg, yield: 33.74%). MS (ESI): *m*/*z* 447.2 [M+H]⁺.

¹H NMR (400 MHz, DMSO-*d*₆) δ 8.43 (s, 1H), 7.59 (s, 2H), 4.94 (d, *J =* 5.3 Hz, 1H), 4.65-4.54 (m, 3H), 4.08-4.00 (m, 2H), 3.84-3.73 (m, 3H), 3.54-3.41 (m, 1H), 3.32-3.28 (m, 1H), 3.03 (t, *J =* 10.4 Hz, 1H), 1.93-1.83 (m, 1H), 1.60 (d, *J =* 6.8 Hz, 6H), 1.50-1.40 (m, 1H).

### Examples 3 to 138 can be prepared using appropriate starting materials with reference to all or part of the synthesis method described in Examples 1, 2, 57 or 126.

| **Example No.** | **Compound Structure** | **Compound Name** | **[M+H]⁺** |
|---|---|---|---|
| 3 | 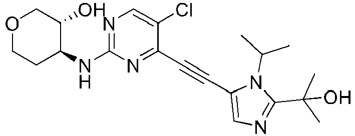 | (3R,4S)-4-((5-chloro-4-((2-(2-hydroxypropan-2-yl)-1-isopropyl-1H-imidazol-5-yl)ethynyl)pyrimidin-2-yl)amino)tetrahydro-2H-pyran-3-ol | 420.2 |
| 4 | 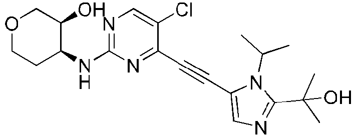 | (3S,4S)-4-((5-chloro-4-((2-(2-hydroxypropan-2-yl)-1-isopropyl-1H-imidazol-5-yl)ethynyl)pyrimidin-2-yl)amino)tetrahydro-2H-pyran-3-ol | 420.2 |
| 5 | 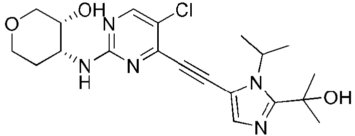 | (3R,4R)-4-((5-chloro-4-((2-(2-hydroxypropan-2-yl)-1-isopropyl-1H-imidazol-5-yl)ethynyl)pyrimidin-2-yl)amino)tetrahydro-2H-pyran-3-ol | 420.2 |
| 6 | 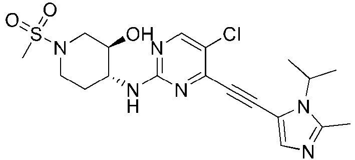 | (3R,4R)-4-((5-chloro-4-((1-isopropyl-2-methyl-1H-imidazol-5-yl)ethynyl)pyrimidin-2-yl)amino)-1-(methanesulfonyl)piperidin-3-ol | 453.1 |
| 7 | 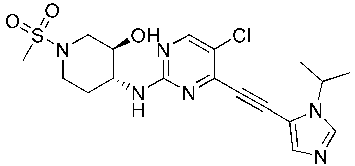 | (3R,4R)-4-((5-chloro-4-((1-isopropyl-1H-imidazol-5-yl)ethynyl)pyrimidin-2-yl)amino)-1-(methanesulfonyl)piperidin-3-ol | 439.1 |
| 8 | 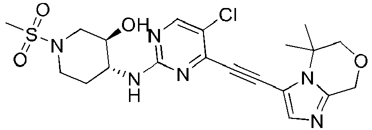 | (3R,4R)-4-((5-chloro-4-((5,5-dimethyl-5,6-dihydro-8H-imidazo[2,1-c][1,4]oxazin-3-yl)ethynyl)pyrimidin-2-yl)amino)-1-(methanesulfonyl)piperidin-3-ol | 481.1 |
| 9 | 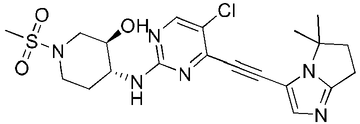 | (3R,4R)-4-((5-chloro-4-((5,5-dimethyl-6,7-dihydro-5H-pyrrolo[1,2-a]imidazol-3-yl)ethynyl)pyrimidin-2-yl)amino)-1-(methanesulfonyl)piperidin-3-ol | 465.1 |
| 10 | 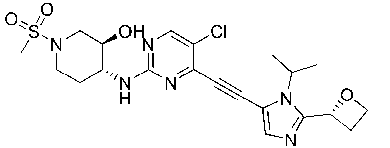 | (3R,4R)-4-((5-chloro-4-((1-isopropyl-2-((R)-oxetan-2-yl)-1H-imidazol-5-yl)ethynyl)pyrimidin-2-yl)amino)-1-(methanesulfonyl)piperidin-3-ol | 495.2 |
| 11 | 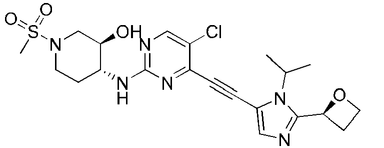 | (3R,4R)-4-((5-chloro-4-((1-isopropyl-2-((S)-oxetan-2-yl)-1H-imidazol-5-yl)ethynyl)pyrimidin-2-yl)amino)-1-(methanesulfonyl)piperidin-3-ol | 495.2 |
| 12 | 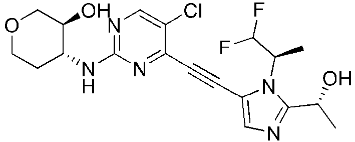 | (3S,4R)-4-((5-chloro-4-((1-((R)-1,1-difluoropropan-2-yl)-2-((R)-1-hydroxyethyl)-1H-imidazol-5-yl)ethynyl)pyrimidin-2-yl)amino)tetrahydro-2H-pyran-3-ol | 442.1 |
| 13 | 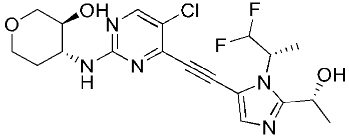 | (3 S,4R)-4-((5-chloro-4-((1-((S)-1,1-difluoropropan-2-yl)-2-((R)-1-hydroxyethyl)-1H-imidazol-5-yl)ethynyl)pyrimidin-2-yl)amino)tetrahydro-2H-pyran-3-ol | 442.1 |
| 14 | 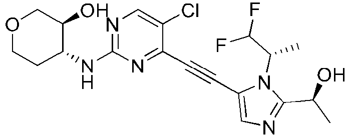 | (3 S,4R)-4-((5-chloro-4-((1-((S)-1,1-difluoropropan-2-yl)-2-((S)-1-hydroxyethyl)-1H-imidazol-5-yl)ethynyl)pyrimidin-2-yl)amino)tetrahydro-2H-pyran-3-ol | 442.1 |
| 15 | 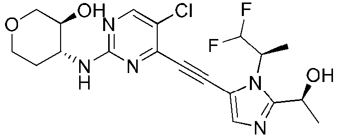 | (3S,4R)-4-((5-chloro-4-((1-((R)-1,1-difluoropropan-2-yl)-2-((S)-1-hydroxyethyl)-1H-imidazol-5-yl)ethynyl)pyrimidin-2-yl)amino)tetrahydro-2H-pyran-3-ol | 442.1 |
| 16 | 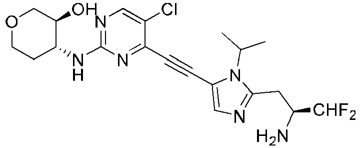 | (3 S,4R)-4-((4-((2-((S)-2-amino-3,3-difluoropropyl)-1-isopropyl-1H-imidazol-5-yl)ethynyl)-5-chloropyrimidin-2-yl)amino)tetrahydro-2H-pyran-3-ol | 455.2 |
| 17 | 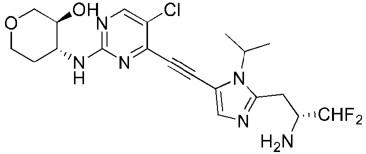 | (3 S,4R)-4-((4-((2-((R)-2-amino-3,3-difluoropropyl)-1-isopropyl-1H-imidazol-5-yl)ethynyl)-5-chloropyrimidin-2-yl)amino)tetrahydro-2H-pyran-3-ol | 455.2 |
| 18 | 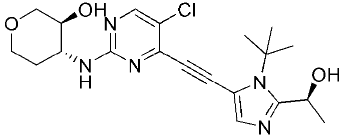 | (3 S,4R)-4-((4-((1-(tert-butyl)-2-((S)-1-hydroxyethyl)-1H-imidazol-5-yl)ethynyl)-5-chloropyrimidin-2-yl)amino)tetrahydro-2H-pyran-3-ol | 420.2 |
| 19 | 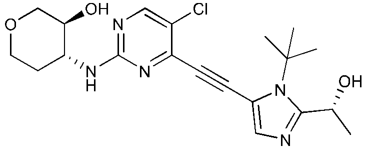 | (3 S,4R)-4-((4-((1-(tert-butyl)-2-((R)-1-hydroxyethyl)-1H-imidazol-5-yl)ethynyl)-5-chloropyrimidin-2-yl)amino)tetrahydro-2H-pyran-3-ol | 420.2 |
| 20 | 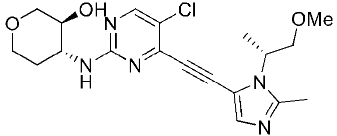 | (3 S,4R)-4-((5-chloro-4-((1-((R)-1-methoxypropan-2-yl)-2-methyl-1H-imidazol-5-yl)ethynyl)pyrimidin-2-yl)amino)tetrahydro-2H-pyran-3-ol | 406.2 |
| 21 | 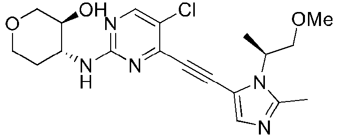 | (3 S,4R)-4-((5-chloro-4-((1-((S)-1-methoxypropan-2-yl)-2-methyl-1H-imidazol-5-yl)ethynyl)pyrimidin-2-yl)amino)tetrahydro-2H-pyran-3-ol | 406.2 |
| 22 | 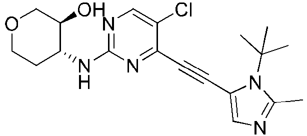 | (3 S,4R)-4-((4-((1-(tert-butyl)-2-methyl-1H-imidazol-5-yl)ethynyl)-5-chloropyrimidin-2-yl)amino)tetrahydro-2H-pyran-3-ol | 390.2 |
| 23 | 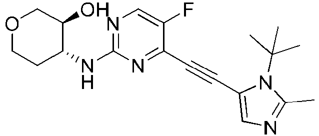 | (3 S,4R)-4-((4-((1-(tert-butyl)-2-methyl-1H-imidazol-5-yl)ethynyl)-5-fluoropyrimidin-2-yl)amino)tetrahydro-2H-pyran-3-ol | 374.2 |
| 24 | 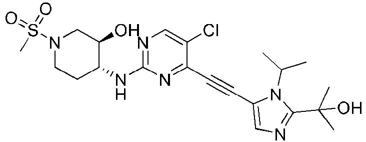 | (3R,4R)-4-((5-chloro-4-((2-(2-hydroxypropan-2-yl)-1-isopropyl-1H-imidazol-5-yl)ethynyl)pyrimidin-2-yl)amino)-1-(methanesulfonyl)piperidin-3-ol | 497.2 |
| 25 | 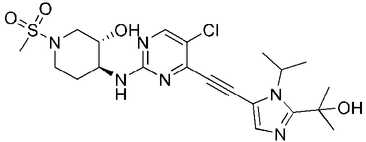 | (3S,4S)-4-((5-chloro-4-((2-(2-hydroxypropan-2-yl)-1-isopropyl-1H-imidazol-5-yl)ethynyl)pyrimidin-2-yl)amino)-1-(methanesulfonyl)piperidin-3-ol | 497.2 |
| 26 | 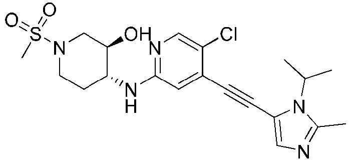 | (3R,4R)-4-((5-chloro-4-((1-isopropyl-2-methyl-1H-imidazol-5-yl)ethynyl)pyridin-2-yl)amino)-1-(methanesulfonyl)piperidin-3-ol | 452.1 |
| 27 | 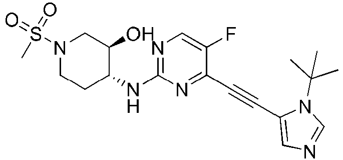 | (3R,4R)-4-((4-((1-(tert-butyl)-1H-imidazol-5-yl)ethynyl)-5-fluoropyrimidin-2-yl)amino)-1-(methanesulfonyl)piperidin-3-ol | 437.2 |
| 28 | 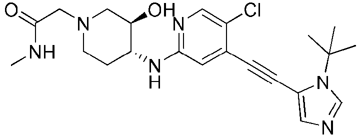 | 2-((3R,4R)-4-((4-((1-(tert-butyl)-1H-imidazol-5-yl)ethynyl)-5-chloropyridin-2-yl)amino)-3-hydroxypiperidin-1-yl)-N-methylacetamide | 445.2 |
| 29 | 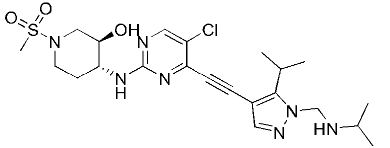 | (3R,4R)-4-((5-chloro-4-((5-isopropyl-1-((isopropylamino)methyl)-1H-pyrazol-4-yl)ethynyl)pyrimidin-2-yl)amino)-1-(methanesulfonyl)piperidin-3-ol | 510.2 |
| 30/31 | 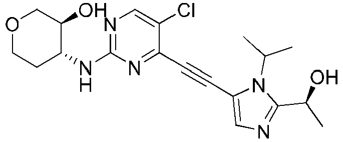 | (3 S,4R)-4-((5-chloro-4-((2-((S)-1-hydroxyethyl)-1-isopropyl-1H-imidazol-5-yl)ethynyl)pyrimidin-2-yl)amino)tetrahydro-2H-pyran-3-ol | 406.2 |
| | 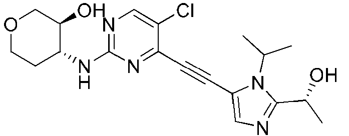 | (3 S,4R)-4-((5-chloro-4-((2-((R)-1-hydroxyethyl)-1-isopropyl-1H-imidazol-5-yl)ethynyl)pyrimidin-2-yl)amino)tetrahydro-2H-pyran-3-ol | 406.2 |
| 32 | 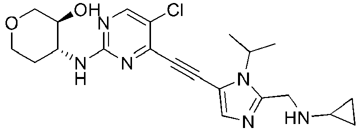 | (3 S,4R)-4-((5-chloro-4-((2-((cyclopropylamino)methyl)-1-isopropyl-1H-imidazol-5-yl)ethynyl)pyrimidin-2-yl)amino)tetrahydro-2H-pyran-3-ol | 431.2 |
| 33 | 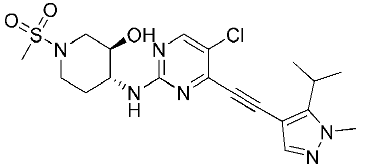 | (3R,4R)-4-((5-chloro-4-((5-isopropyl-1-methyl-1H-pyrazol-4-yl)ethynyl)pyrimidin-2-yl)amino)-1-(methanesulfonyl)piperidin-3-ol | 453.1 |
| 34 | 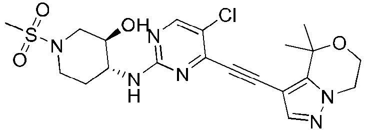 | (3R,4R)-4-((5-chloro-4-((4,4-dimethyl-6,7-dihydro-4H-pyrazolo[5,1-c][1,4]oxazin-3-yl)ethynyl)pyrimidin-2-yl)amino)-1-(methanesulfonyl)piperidin-3-ol | 481.1 |
| 35 | 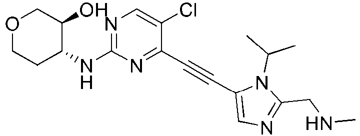 | (3 S,4R)-4-((5-chloro-4-((1-isopropyl-2-((methylamino)methyl)-1H-imidazol-5-yl)ethynyl)pyrimidin-2-yl)amino)tetrahydro-2H-pyran-3-ol | 405.2 |
| 36 | 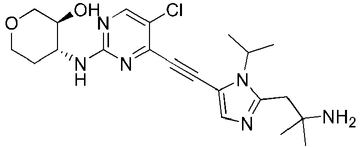 | (3 S,4R)-4-((4-((2-(2-amino-2-methylpropyl)-1-isopropyl-1H-imidazol-5-yl)ethynyl)-5-chloropyrimidin-2-yl)amino)tetrahydro-2H-pyran-3-ol | 433.2 |
| 37 | 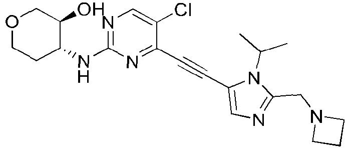 | (3S,4R)-4-((4-((2-(azetidin-1-ylmethyl)-1-isopropyl-1H-imidazol-5-yl)ethynyl)-5-chloropyrimidin-2-yl)amino)tetrahydro-2H-pyran-3-ol | 431.2 |
| 38 | 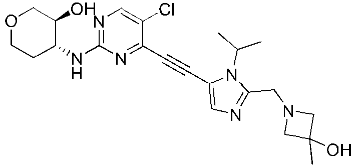 | 1-((5-((5-chloro-2-(((3 S,4R)-3-hydroxytetrahydro-2H-pyran-4-yl)amino)pyrimidin-4-yl)ethynyl)-1-isopropyl-1H-imidazol-2-yl)methyl)-3-methylazetidin-3-ol | 461.2 |
| 39 | 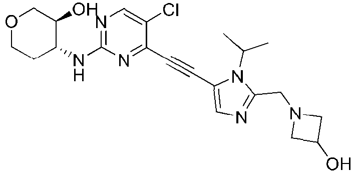 | 1-((5-((5-chloro-2-(((3 S,4R)-3-hydroxytetrahydro-2H-pyran-4-yl)amino)pyrimidin-4-yl)ethynyl)-1-isopropyl-1H-imidazol-2-yl)methyl)azetidin-3-ol | 447.2 |
| 40 | 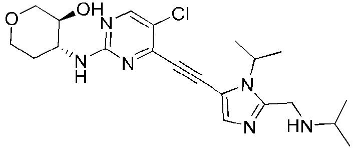 | (3 S,4R)-4-((5-chloro-4-((1-isopropyl-2-((isopropylamino)methyl)-1H-imidazol-5-yl)ethynyl)pyrimidin-2-yl)amino)tetrahydro-2H-pyran-3-ol | 433.2 |
| 41 | 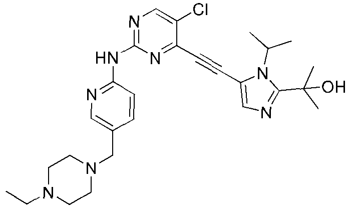 | 2-(5-((5-chloro-2-((5-((4-ethylpiperazin-1-yl)methyl)pyridin-2-yl)amino)pyrimidin-4-yl)ethynyl)-1-isopropyl-1H-imidazol-2-yl)propan-2-ol | 523.3 |
| 42 | 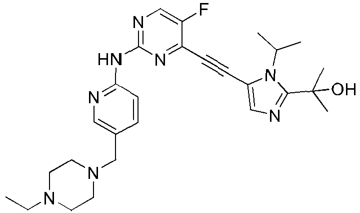 | 2-(5-((2-((5-((4-ethylpiperazin-1-yl)methyl)pyridin-2-yl)amino)-5-fluoropyrimidin-4-yl)ethynyl)-1-isopropyl-1H-imidazol-2-yl)propan-2-ol | 507.3 |
| 43 | 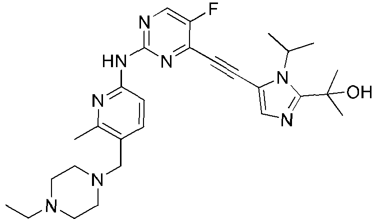 | 2-(5-((2-((5-((4-ethylpiperazin-1-yl)methyl)-6-methylpyridin-2-yl)amino)-5-fluoropyrimidin-4-yl)ethynyl)-1-isopropyl-1H-imidazol-2-yl)propan-2-ol | 521.3 |
| 44 | 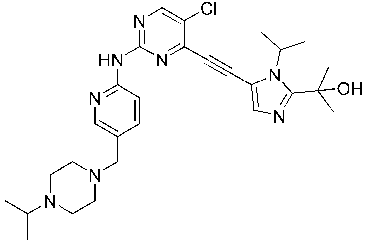 | 2-(5-((5-chloro-2-((5-((4-isopropylpiperazin-1-yl)methyl)pyridin-2-yl)amino)pyrimidin-4-yl)ethynyl)-1-isopropyl-1H-imidazol-2-yl)propan-2-ol | 537.3 |
| 45 | 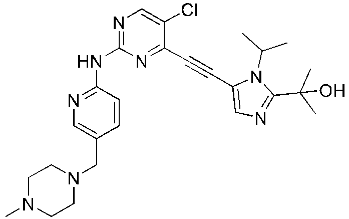 | 2-(5-((5-chloro-2-((5-((4-methylpiperazin-1-yl)methyl)pyridin-2-yl)amino)pyrimidin-4-yl)ethynyl)-1-isopropyl-1H-imidazol-2-yl)propan-2-ol | 509.3 |
| 46 | 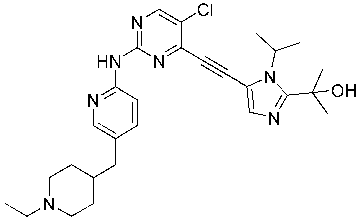 | 2-(5-((5-chloro-2-((5-((1-ethylpiperidin-4-yl)methyl)pyridin-2-yl)amino)pyrimidin-4-yl)ethynyl)-1-isopropyl-1H-imidazol-2-yl)propan-2-ol | 522.3 |
| 47 | 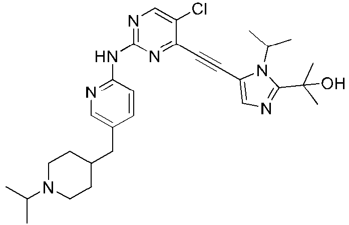 | 2-(5-((5-chloro-2-((5-((1-isopropylpiperidin-4-yl)methyl)pyridin-2-yl)amino)pyrimidin-4-yl)ethynyl)-1-isopropyl-1H-imidazol-2-yl)propan-2-ol | 536.3 |
| 48 | 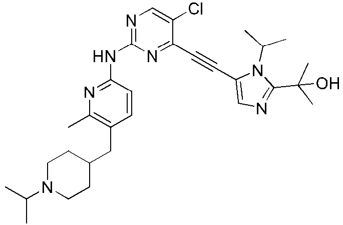 | 2-(5-((5-chloro-2-((5-((1-isopropylpiperidin-4-yl)methyl)-6-methylpyridin-2-yl)amino)pyrimidin-4-yl)ethynyl)-1-isopropyl-1H-imidazol-2-yl)propan-2-ol | 550.3 |
| 49 | 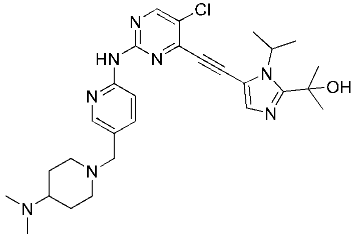 | 2-(5-((5-chloro-2-((5-((4-(dimethylamino)piperidin-1-yl)methyl)pyridin-2-yl)amino)pyrimidin-4-yl)ethynyl)-1-isopropyl-1H-imidazol-2-yl)propan-2-ol | 537.3 |
| 50 | 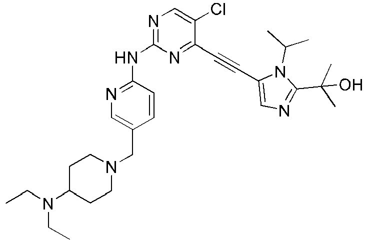 | 2-(5-((5-chloro-2-((5-((4-(diethylamino)piperidin-1-yl)methyl)pyridin-2-yl)amino)pyrimidin-4-yl)ethynyl)-1-isopropyl-1H-imidazol-2-yl)propan-2-ol | 565.3 |
| 51 | 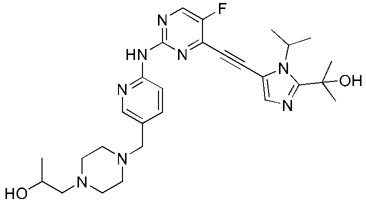 | 1-(4-((6-((5-fluoro-4-((2-(2-hydroxypropan-2-yl)-1-isopropyl-1H-imidazol-5-yl)ethynyl)pyrimidin-2-yl)amino)pyridin-3-yl)methyl)piperazin-1-yl)propan-2-ol | 537.3 |
| 52 | 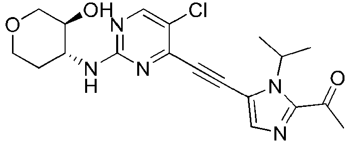 | 1-(5-((5-chloro-2-(((3 S,4R)-3-hydroxytetrahydro-2H-pyran-4-yl)amino)pyrimidin-4-yl)ethynyl)-1-isopropyl-1H-imidazol-2-yl)ethan-1-one | 404.2 |
| 53 | 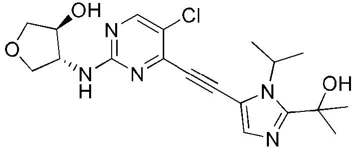 | (3 S,4R)-4-((5-chloro-4-((2-(2-hydroxypropan-2-yl)-1-isopropyl-1H-imidazol-5-yl)ethynyl)pyrimidin-2-yl)amino)tetrahydrofuran-3-ol | 406.2 |
| 54 | 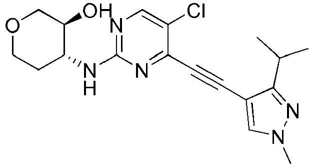 | (3 S,4R)-4-((5-chloro-4-((3-isopropyl-1-methyl-1H-pyrazol-4-yl)ethynyl)pyrimidin-2-yl)amino)tetrahydro-2H-pyran-3-ol | 376.2 |
| 55 | 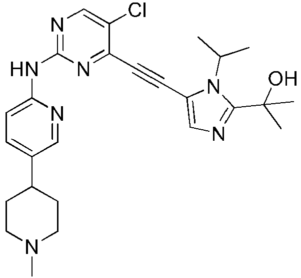 | 2-(5-((5-chloro-2-((5-(1-methylpiperidin-4-yl)pyridin-2-yl)amino)pyrimidin-4-yl)ethynyl)-1-isopropyl-1H-imidazol-2-yl)propan-2-ol | 494.2 |
| 56 | 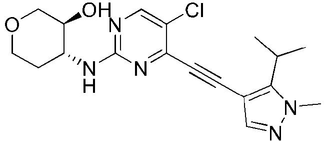 | (3 S,4R)-4-((5-chloro-4-((5-isopropyl-1-methyl-1H-pyrazol-4-yl)ethynyl)pyrimidin-2-yl)amino)tetrahydro-2H-pyran-3-ol | 376.2 |
| 58 | 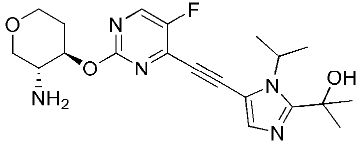 | 2-(5-((2-(((3R,4R)-3-aminotetrahydro-2H-pyran-4-yl)oxy)-5-fluoropyrimidin-4-yl)ethynyl)-1-isopropyl-1H-imidazol-2-yl)propan-2-ol | 404.2 |
| 59 | 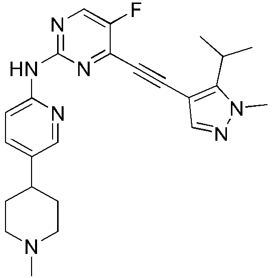 | 5-fluoro-4-((5-isopropyl-1-methyl-1H-pyrazol-4-yl)ethynyl)-N-(5-(1-methylpiperidin-4-yl)pyridin-2-yl)pyrimidin-2-amine | 434.2 |
| 60 | 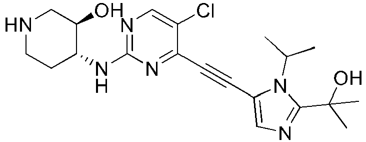 | (3R,4R)-4-((5-chloro-4-((2-(2-hydroxypropan-2-yl)-1-isopropyl-1H-imidazol-5-yl)ethynyl)pyrimidin-2-yl)amino)piperidin-3-ol | 419.2 |
| 61 | 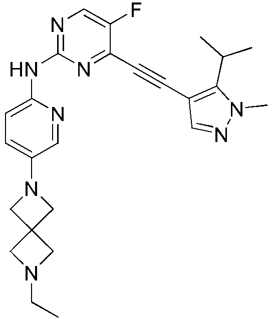 | N-(5-(6-ethyl-2,6-diazaspiro[3.3]heptan-2-yl)pyridin-2-yl)-5-fluoro-4-((5-isopropyl-1-methyl-1H-pyrazol-4-yl)ethynyl)pyrimidin-2-amine | 461.2 |
| 62 | 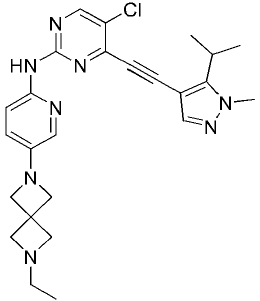 | 5-chloro-N-(5-(6-ethyl-2,6-diazaspiro[3.3]heptan-2-yl)pyridin-2-yl)-4-((5-isopropyl-1-methyl-1H-pyrazol-4-yl)ethynyl)pyrimidin-2-amine | 477.2 |
| 63 | 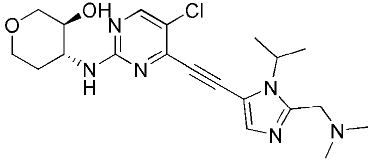 | (3 S,4R)-4-((5-chloro-4-((2-((dimethylamino)methyl)-1-isopropyl-1H-imidazol-5-yl)ethynyl)pyrimidin-2-yl)amino)tetrahydro-2H-pyran-3-ol | 419.2 |
| 64 | 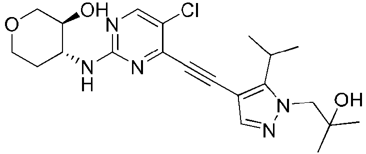 | (3 S,4R)-4-((5-chloro-4-((1-(2-hydroxy-2-methylpropyl)-5-isopropyl-1H-pyrazol-4-yl)ethynyl)pyrimidin-2-yl)amino)tetrahydro-2H-pyran-3-ol | 434.2 |
| 65 | 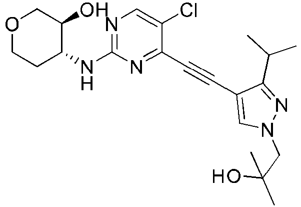 | (3 S,4R)-4-((5-chloro-4-((1-(2-hydroxy-2-methylpropyl)-3-isopropyl-1H-pyrazol-4-yl)ethynyl)pyrimidin-2-yl)amino)tetrahydro-2H-pyran-3-ol | 434.2 |
| 66 | 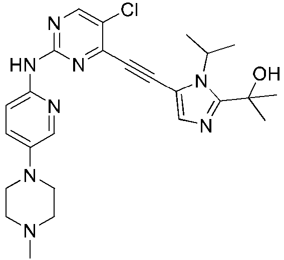 | 2-(5-((5-chloro-2-((5-(4-methylpiperazin-1-yl)pyridin-2-yl)amino)pyrimidin-4-yl)ethynyl)-1-isopropyl-1H-imidazol-2-yl)propan-2-ol | 495.2 |
| 67 | 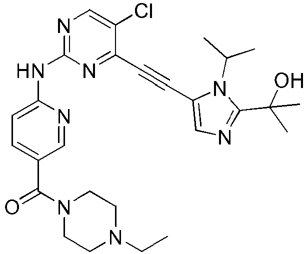 | (6-((5-chloro-4-((2-(2-hydroxypropan-2-yl)-1-isopropyl-1H-imidazol-5-yl)ethynyl)pyrimidin-2-yl)amino)pyridin-3-yl)(4-ethylpiperazin-1-yl)methanone | 537.2 |
| 68 | 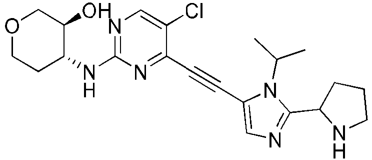 | (3 S,4R)-4-((5-chloro-4-((1-isopropyl-2-(pyrrolidin-2-yl)-1H-imidazol-5-yl)ethynyl)pyrimidin-2-yl)amino)tetrahydro-2H-pyran-3-ol | 431.2 |
| 69 | 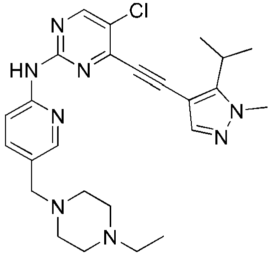 | 5-Chloro-N-(5-((4-ethylpiperazin-1-yl)methyl)pyridin-2-yl)-4-((5-isopropyl-1-methyl-1H-pyrazol-4-yl)ethynyl)pyrimidin-2-amine | 479.2 |
| 70 | 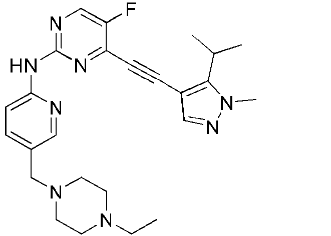 | N-(5-((4-ethylpiperazin-1-yl)methyl)pyridin-2-yl)-5-fluoro-4-((5-isopropyl-1-methyl-1H-pyrazol-4-yl)ethynyl)pyrimidin-2-amine | 463.2 |
| 71 | 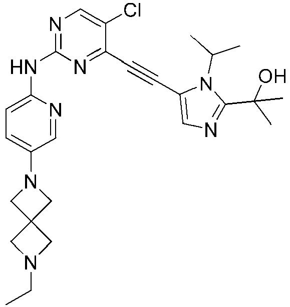 | 2-(5-((5-chloro-2-((5-(6-ethyl-2,6-diazaspiro[3.3]heptan-2-yl)pyridin-2-yl)amino)pyrimidin-4-yl)ethynyl)-1-isopropyl-1H-imidazol-2-yl)propan-2-ol | 521.2 |
| 72 | 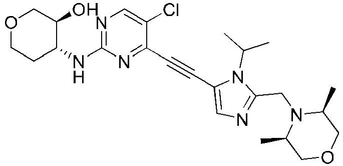 | (3S,4R)-4-((5-chloro-4-((2-(((3R,5S)-3,5-dimethylmorpholino)methyl)-1-isopropyl-1H-imidazol-5-yl)ethynyl)pyrimidin-2-yl)amino)tetrahydro-2H-pyran-3-ol | 489.2 |
| 73 | 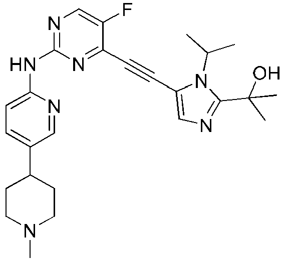 | 2-(5-((5-fluoro-2-((5-(1-methylpiperidin-4-yl)pyridin-2-yl)amino)pyrimidin-4-yl)ethynyl)-1-isopropyl-1H-imidazol-2-yl)propan-2-ol | 478.2 |
| 74 | 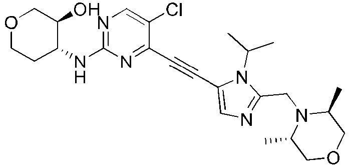 | (3 S,4R)-4-((5-chloro-4-((2-(((3 S,5 S)-3,5-dimethylmorpholino)methyl)-1-isopropyl-1H-imidazol-5-yl)ethynyl)pyrimidin-2-yl)amino)tetrahydro-2H-pyran-3-ol | 489.2 |
| 75 | 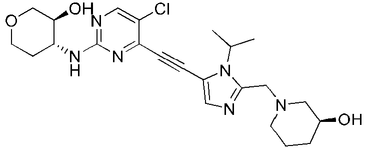 | (S)-1-((5-((5-chloro-2-(((3 S,4R)-3-hydroxytetrahydro-2H-pyran-4-yl)amino)pyrimidin-4-yl)ethynyl)-1-isopropyl-1H-imidazol-2-yl)methyl)piperidin-3-ol | 475.2 |
| 76 | 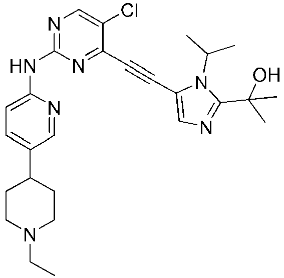 | 2-(5-((5-chloro-2-((5-(1-ethylpiperidin-4-yl)pyridin-2-yl)amino)pyrimidin-4-yl)ethynyl)-1-isopropyl-1H-imidazol-2-yl)propan-2-ol | 508.2 |
| 77 | 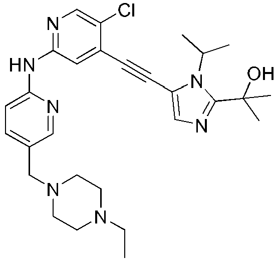 | 2-(5-((5-chloro-2-((5-((4-ethylpiperazin-1-yl)methyl)pyridin-2-yl)amino)pyridin-4-yl)ethynyl)-1-isopropyl-1H-imidazol-2-yl)propan-2-ol | 522.2 |
| 78 | 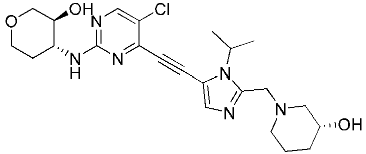 | (R)-1-((5-((5-chloro-2-(((3 S,4R)-3-hydroxytetrahydro-2H-pyran-4-yl)amino)pyrimidin-4-yl)ethynyl)-1-isopropyl-1H-imidazol-2-yl)methyl)piperidin-3-ol | 475.2 |
| 79 | 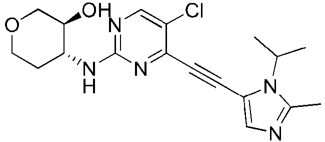 | (3 S,4R)-4-((5-chloro-4-((1-isopropyl-2-methyl-1H-imidazol-5-yl)ethynyl)pyrimidin-2-yl)amino)tetrahydro-2H-pyran-3-ol | 376.2 |
| 80 | 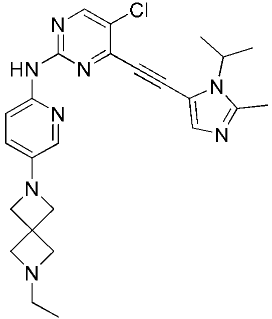 | 5-chloro-N-(5-(6-ethyl-2,6-diazaspiro[3.3]heptan-2-yl)pyridin-2-yl)-4-((1-isopropyl-2-methyl-1H-imidazol-5-yl)ethynyl)pyrimidin-2-amine | 477.2 |
| 81 | 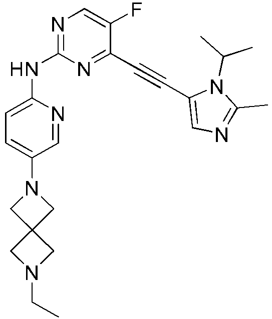 | N-(5-(6-ethyl-2,6-diazaspiro[3.3]heptan-2-yl)pyridin-2-yl)-5-fluoro-4-((1-isopropyl-2-methyl-1H-imidazol-5-yl)ethynyl)pyrimidin-2-amine | 461.2 |
| 82 | 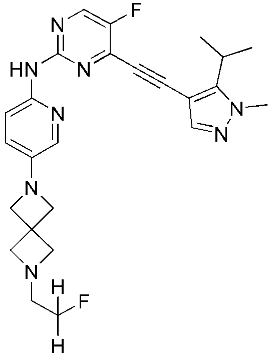 | 5-Fluoro-N-(5-(6-(2-fluoroethyl)-2,6-diazaspiro[3.3]heptan-2-yl)pyridin-2-yl)-4-((5-isopropyl-1-methyl-1H-pyrazol-4-yl)ethynyl)pyrimidin-2-amine | 479.2 |
| 83 | 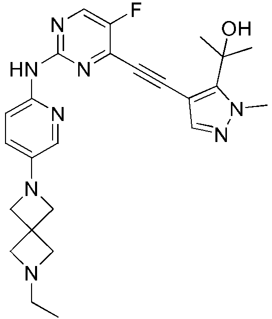 | 2-(4-((2-((5-(6-ethyl-2,6-diazaspiro[3.3]heptan-2-yl)pyridin-2-yl)amino)-5-fluoropyrimidin-4-yl)ethynyl)-1-methyl-1H-pyrazol-5-yl)propan-2-ol | 477.2 |
| 84 | 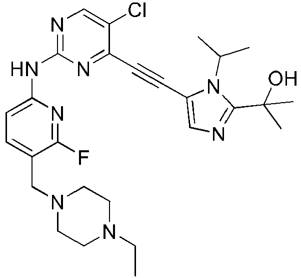 | 2-(5-((5-chloro-2-((5-((4-ethylpiperazin-1-yl)methyl)-6-fluoropyridin-2-yl)amino)pyrimidin-4-yl)ethynyl)-1-isopropyl-1H-imidazol-2-yl)propan-2-ol | 541.2 |
| 85 | 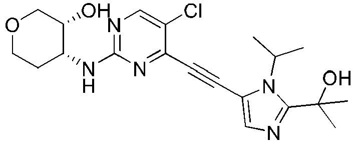 | (3R,4R)-4-((5-chloro-4-((2-(2-hydroxypropan-2-yl)-1-isopropyl-1H-imidazol-5-yl)ethynyl)pyrimidin-2-yl)amino)tetrahydro-2H-pyran-3-ol | 420.2 |
| 86 | 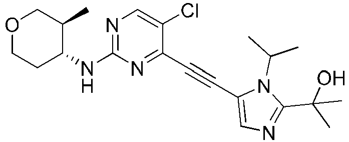 | 2-(5-((5-chloro-2-(((3 S,4R)-3-methyltetrahydro-2H-pyran-4-yl)amino)pyrimidin-4-yl)ethynyl)-1-isopropyl-1H-imidazol-2-yl)propan-2-ol | 418.2 |
| 87 | 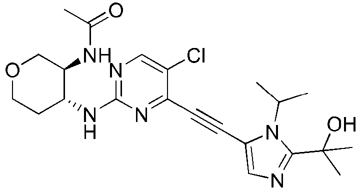 | N-((3 S,4R)-4-((5-chloro-4-((2-(2-hydroxypropan-2-yl)-1-isopropyl-1H-imidazol-5-yl)ethynyl)pyrimidin-2-yl)amino)tetrahydro-2H-pyran-3-yl)acetamide | 461.2 |
| 88 | 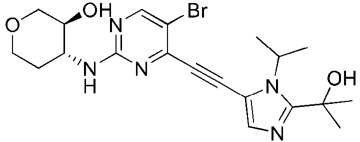 | (3S,4R)-4-((5-bromo-4-((2-(2-hydroxypropan-2-yl)-1-isopropyl-1H-imidazol-5-yl)ethynyl)pyrimidin-2-yl)amino)tetrahydro-2H-pyran-3-ol | 464.2 |
| 89 | 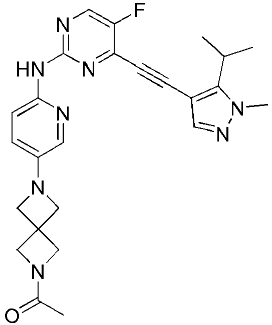 | 1-(6-(6-((5-fluoro-4-((5-isopropyl-1-methyl-1H-pyrazol-4-yl)ethynyl)pyrimidin-2-yl)amino)pyridin-3-yl)-2,6-diazaspiro[3.3]heptan-2-yl)ethan-1-one | 475.2 |
| 90 | 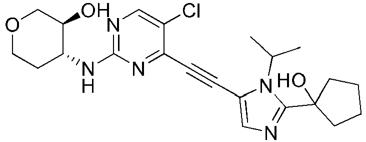 | (3 S,4R)-4-((5-chloro-4-((2-(1-hydroxycyclopentyl)-1-isopropyl-1H-imidazol-5-yl)ethynyl)pyrimidin-2-yl)amino)tetrahydro-2H-pyran-3-ol | 446.2 |
| 91 | 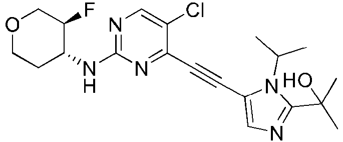 | 2-(5-((5-chloro-2-(((3 S,4R)-3-fluorotetrahydro-2H-pyran-4-yl)amino)pyrimidin-4-yl)ethynyl)-1-isopropyl-1H-imidazol-2-yl)propan-2-ol | 422.2 |
| 92 | 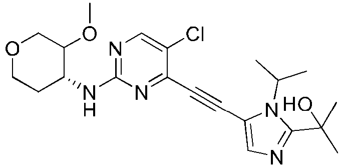 | 2-(5-((5-chloro-2-(((4R)-3-methoxytetrahydro-2H-pyran-4-yl)amino)pyrimidin-4-yl)ethynyl)-1-isopropyl-1H-imidazol-2-yl)propan-2-ol | 434.2 |
| 93 | 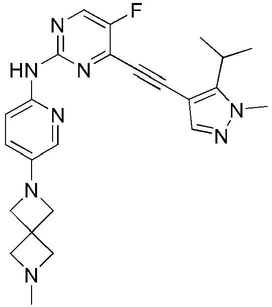 | 5-fluoro-4-((5-isopropyl-1-methyl-1H-pyrazol-4-yl)ethynyl)-N-(5-(6-methyl-2,6-diazaspiro[3 .3]heptan-2-yl)pyridin-2-yl)pyrimidin-2-amine | 447.2 |
| 94 | 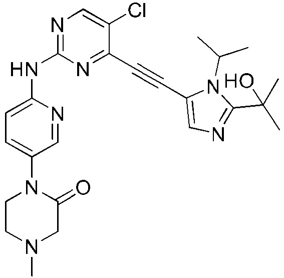 | 1-(6-((5-chloro-4-((2-(2-hydroxypropan-2-yl)-1-isopropyl-1H-imidazol-5-yl)ethynyl)pyrimidin-2-yl)amino)pyridin-3-yl)-4-methylpiperazin-2-one | 509.2 |
| 95 | 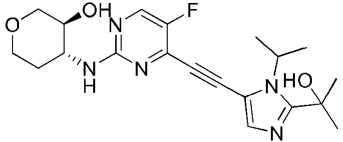 | (3 S,4R)-4-((5-fluoro-4-((2-(2-hydroxypropan-2-yl)-1-isopropyl-1H-imidazol-5-yl)ethynyl)pyrimidin-2-yl)amino)tetrahydro-2H-pyran-3-ol | 404.2 |
| 96 | 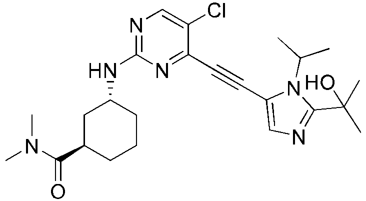 | (1R,3R)-3-((5-chloro-4-((2-(2-hydroxypropan-2-yl)-1-isopropyl-1H-imidazol-5-yl)ethynyl)pyrimidin-2-yl)amino)-N,N-dimethylcyclohexane-1-carboxamide | 473.2 |
| 97 | 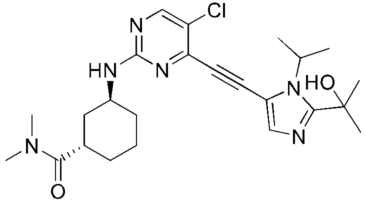 | (1S,3S)-3-((5-chloro-4-((2-(2-hydroxypropan-2-yl)-1-isopropyl-1H-imidazol-5-yl)ethynyl)pyrimidin-2-yl)amino)-N,N-dimethylcyclohexane-1-carboxamide | 473.2 |
| 98 | 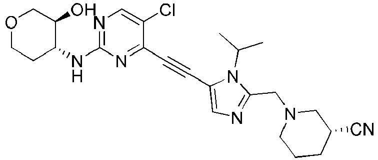 | (R)-1-((5-((5-chloro-2-(((3 S,4R)-3-hydroxytetrahydro-2H-pyran-4-yl)amino)pyrimidin-4-yl)ethynyl)-1-isopropyl-1H-imidazol-2-yl)methyl)piperidin-3-carbonitrile | 484.2 |
| 99 | 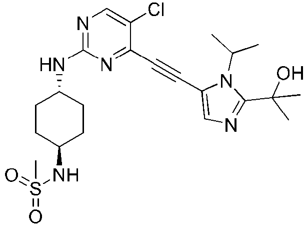 | N-((1R,4R)-4-((5-chloro-4-((2-(2-hydroxypropan-2-yl)-1-isopropyl-1H-imidazol-5-yl)ethynyl)pyrimidin-2-yl)amino)cyclohexyl)methanesulfona mide | 495.2 |
| 100 | 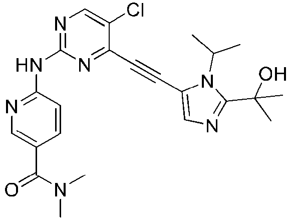 | 6-((5-chloro-4-((2-(2-hydroxypropan-2-yl)-1-isopropyl-1H-imidazol-5-yl)ethynyl)pyrimidin-2-yl)amino)-N,N-dimethylnicotinamide | 468.2 |
| 101 | 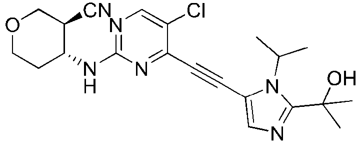 | (3R,4R)-4-((5-chloro-4-((2-(2-hydroxypropan-2-yl)-1-isopropyl-1H-imidazol-5-yl)ethynyl)pyrimidin-2-yl)amino)tetrahydro-2H-pyran-3-carbonitrile | 429.2 |
| 102 | 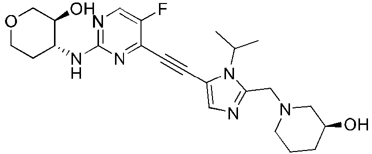 | (S)-1-((5-((5-fluoro-2-(((3 S,4R)-3-hydroxytetrahydro-2H-pyran-4-yl)amino)pyrimidin-4-yl)ethynyl)-1-isopropyl-1H-imidazol-2-yl)methyl)piperidin-3-ol | 459.2 |
| 103 | 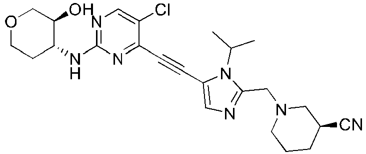 | (S)-1-((5-((5-chloro-2-(((3 S,4R)-3-hydroxytetrahydro-2H-pyran-4-yl)amino)pyrimidin-4-yl)ethynyl)-1-isopropyl-1H-imidazol-2-yl)methyl)piperidin-3-carbonitrile | 484.2 |
| 104 | 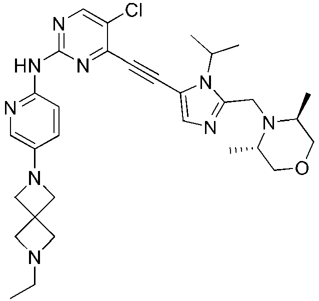 | 5-chloro-4-((2-(((3 S,5 S)-3,5-dimethylmorpholino)methyl)-1-isopropyl-1H-imidazol-5-yl)ethynyl)-N-(5-(6-ethyl-2,6-diazaspiro[3.3]heptan-2-yl)pyridin-2-yl)pyrimidin-2-amine | 590.3 |
| 105 | 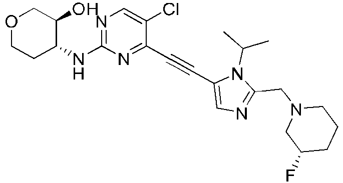 | (3S,4R)-4-((5-chloro-4-((2-(((S)-3-fluoropiperidin-1-yl)methyl)-1-isopropyl-1H-imidazol-5-yl)ethynyl)pyrimidin-2-yl)amino)tetrahydro-2H-pyran-3-ol | 477.2 |
| 106 | 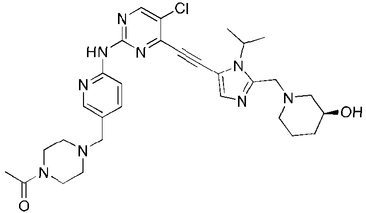 | (S)-1-(4-((6-((5-chloro-4-((2-((3-hydroxypiperidin-1-yl)methyl)-1-isopropyl-1H-imidazol-5-yl)ethynyl)pyrimidin-2-yl)amino)pyridin-3-yl)methyl)piperazin-1-yl)ethan-1-one | 592.3 |
| 107 | 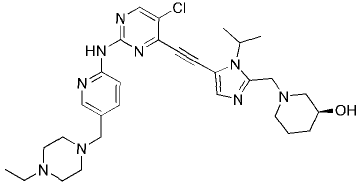 | (S)-1-((5-((5-chloro-2-((5-((4-ethylpiperazin-1-yl)methyl)pyridin-2-yl)amino)pyrimidin-4-yl)ethynyl)-1-isopropyl-1H-imidazol-2-yl)methyl)piperidin-3-ol | 578.3 |
| 108 | 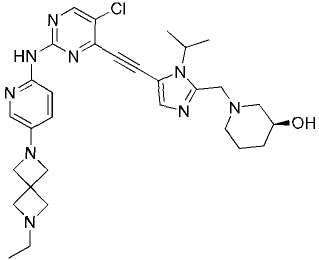 | (S)-1-((5-((5-chloro-2-((5-(6-ethyl-2,6-diazaspiro[3.3]heptan-2-yl)pyridin-2-yl)amino)pyrimidin-4-yl)ethynyl)-1-isopropyl-1H-imidazol-2-yl)methyl)piperidin-3-ol | 576.3 |
| 109 | 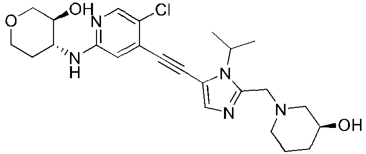 | (S)-1-((5-((5-chloro-2-(((3 S,4R)-3-hydroxytetrahydro-2H-pyran-4-yl)amino)pyridin-4-yl)ethynyl)-1-isopropyl-1H-imidazol-2-yl)methyl)piperidin-3-ol | 474.2 |
| 110 | 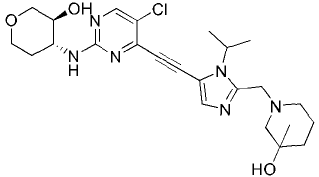 | 1-((5-((5-chloro-2-(((3 S,4R)-3-hydroxytetrahydro-2H-pyran-4-yl)amino)pyrimidin-4-yl)ethynyl)-1-isopropyl-1H-imidazol-2-yl)methyl)-3-methylpiperidin-3-ol | 489.2 |
| 111 | 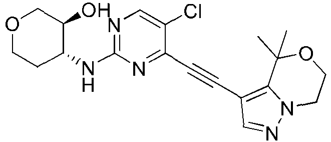 | (3 S,4R)-4-((5-chloro-4-((4,4-dimethyl-6,7-dihydro-4H-pyrazolo[5,1-c][1,4]oxazin-3-yl)ethynyl)pyrimidin-2-yl)amino)tetrahydro-2H-pyran-3-ol | 404.2 |
| 112 | 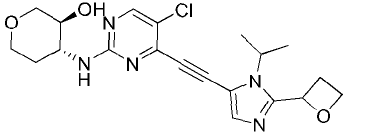 | (3S,4R)-4-((5-chloro-4-((1-isopropyl-2-(oxetan-2-yl)-1H-imidazol-5-yl)ethynyl)pyrimidin-2-yl)amino)tetrahydro-2H-pyran-3-ol | 418.2 |
| 113 | 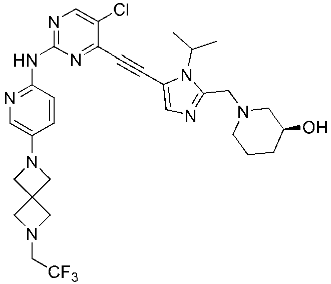 | (S)-1-((5-((5-chloro-2-((5-(6-(2,2,2-trifluoroethyl)-2,6-diazaspiro[3.3]heptan-2-yl)pyridin-2-yl)amino)pyrimidin-4-yl)ethynyl)-1-isopropyl-1H-imidazol-2-yl)methyl)piperidin-3-ol | 630.3 |
| 114 | 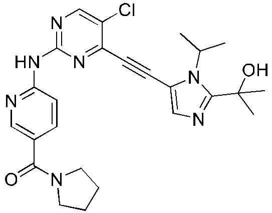 | (6-((5-chloro-4-((2-(2-hydroxypropan-2-yl)-1-isopropyl-1H-imidazol-5-yl)ethynyl)pyrimidin-2-yl)amino)pyridin-3-yl)(pyrrolidin-1-yl)methanone | 494.2 |
| 115 | 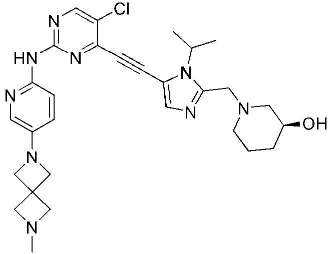 | (S)-1-((5-((5-chloro-2-((5-(6-methyl-2,6-diazaspiro[3.3]heptan-2-yl)pyridin-2-yl)amino)pyrimidin-4-yl)ethynyl)-1-isopropyl-1H-imidazol-2-yl)methyl)piperidin-3-ol | 562.3 |
| 116 | 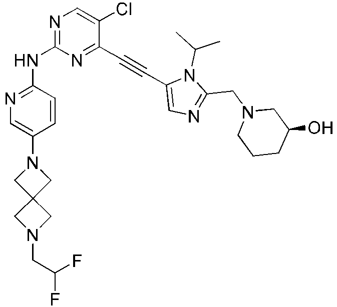 | (S)-1-((5-((5-chloro-2-((5-(6-(2,2-difluoroethyl)-2,6-diazaspiro[3.3]heptan-2-yl)pyridin-2-yl)amino)pyrimidin-4-yl)ethynyl)-1-isopropyl-1H-imidazol-2-yl)methyl)piperidin-3-ol | 612.3 |
| 117 | 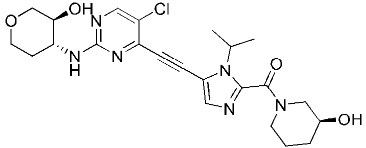 | (5-((5-chloro-2-(((3 S,4R)-3-hydroxytetrahydro-2H-pyran-4-yl)amino)pyrimidin-4-yl)ethynyl)-1-isopropyl-1H-imidazol-2-yl)((S)-3-hydroxypiperidin-1-yl)methanone | 489.2 |
| 118 | 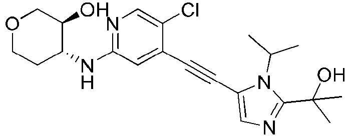 | (3 S,4R)-4-((5-chloro-4-((2-(2-hydroxypropan-2-yl)-1-isopropyl-1H-imidazol-5-yl)ethynyl)pyridin-2-yl)amino)tetrahydro-2H-pyran-3-ol | 419.2 |
| 119 | 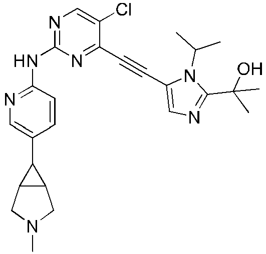 | 2-(5-((5-chloro-2-((5-(3-methyl-3-azabicyclo[3.1.0]hexan-6-yl)pyridin-2-yl)amino)pyrimidin-4-yl)ethynyl)-1-isopropyl-1H-imidazol-2-yl)propan-2-ol | 492.2 |
| 120 | 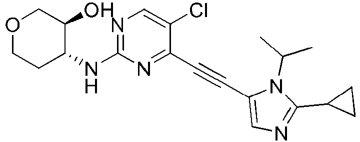 | (3 S,4R)-4-((5-chloro-4-((2-cyclopropyl-1-isopropyl-1H-imidazol-5-yl)ethynyl)pyrimidin-2-yl)amino)tetrahydro-2H-pyran-3-ol | 402.2 |
| 121 | 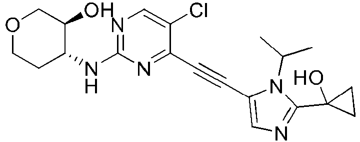 | (3 S,4R)-4-((5-chloro-4-((2-(1-hydroxycyclopropyl)-1-isopropyl-1H-imidazol-5-yl)ethynyl)pyrimidin-2-yl)amino)tetrahydro-2H-pyran-3-ol | 418.2 |
| 122 | 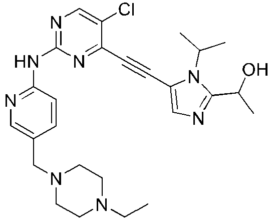 | 1-(5-((5-chloro-2-((5-((4-ethylpiperazin-1-yl)methyl)pyridin-2-yl)amino)pyrimidin-4-yl)ethynyl)-1-isopropyl-1H-imidazol-2-yl)ethan-1-ol | 509.3 |
| 123 | 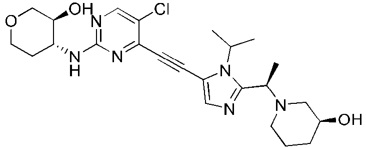 | (S)-1-((R)-1-(5-((5-chloro-2-(((3 S,4R)-3-hydroxytetrahydro-2H-pyran-4-yl)amino)pyrimidin-4-yl)ethynyl)-1-isopropyl-1H-imidazol-2-yl)ethyl)piperidin-3-ol | 489.2 |
| 124 | 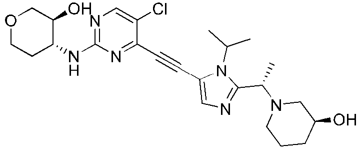 | (S)-1-((S)-1-(5-((5-chloro-2-(((3 S,4R)-3-hydroxytetrahydro-2H-pyran-4-yl)amino)pyrimidin-4-yl)ethynyl)-1-isopropyl-1H-imidazol-2-yl)ethyl)piperidin-3-ol | 489.2 |
| 125 | 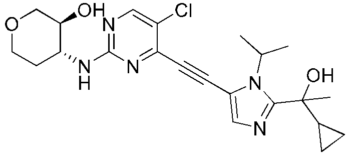 | (3 S,4R)-4-((5-chloro-4-((2-(1-cyclopropyl-1-hydroxyethyl)-1-isopropyl-1H-imidazol-5-yl)ethynyl)pyrimidin-2-yl)amino)tetrahydro-2H-pyran-3-ol | 446.2 |
| 127 | 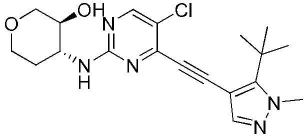 | (3 S,4R)-4-((4-((5-(tert-butyl)-1-methyl-1H-pyrazol-4-yl)ethynyl)-5-chloropyrimidin-2-yl)amino)tetrahydro-2H-pyran-3-ol | 390.2 |
| 128 | 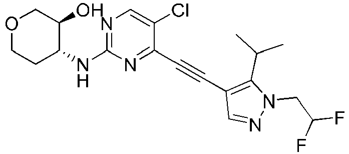 | (3 S,4R)-4-((5-chloro-4-((1-(2,2-difluoroethyl)-5-isopropyl-1H-pyrazol-4-yl)ethynyl)pyrimidin-2-yl)amino)tetrahydro-2H-pyran-3-ol | 426.2 |
| 129 | 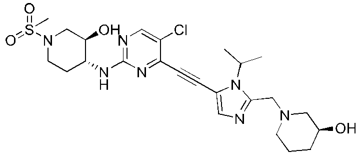 | (3R,4R)-4-((5-chloro-4-((2-(((S)-3-hydroxypiperidin-1-yl)methyl)-1-isopropyl-1H-imidazol-5-yl)ethynyl)pyrimidin-2-yl)amino)-1-(methanesulfonyl)piperidin-3-ol | 552.2 |
| 130 | 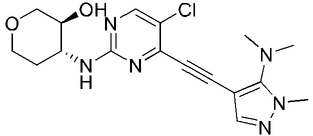 | (3 S,4R)-4-((5-chloro-4-((5-(dimethylamino)-1-methyl-1H-pyrazol-4-yl)ethynyl)pyrimidin-2-yl)amino)tetrahydro-2H-pyran-3-ol | 377.2 |
| 131 | 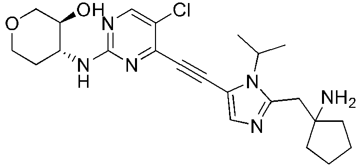 | (3S,4R)-4-((4-((2-((1-aminocyclopentyl)methyl)-1-isopropyl-1H-imidazol-5-yl)ethynyl)-5-chloropyrimidin-2-yl)amino)tetrahydro-2H-pyran-3-ol | 459.2 |
| 132 | 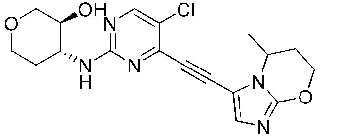 | (3 S,4R)-4-((5-chloro-4-((5-methyl-6,7-dihydro-5H-imidazo[2,1-o][1,3]oxazin-3-yl)ethynyl)pyrimidin-2-yl)amino)tetrahydro-2H-pyran-3-ol | 390.2 |
| 133 | 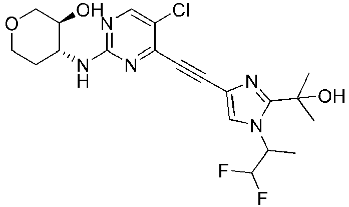 | (3S,4R)-4-((5-chloro-4-((1-(1, 1-difluoropropan-2-yl)-2-(2-hydroxypropan-2-yl)-1H-imidazol-5-yl)ethynyl)pyrimidin-2-yl)amino)tetrahydro-2H-pyran-3-ol | 456.2 |
| 134 | 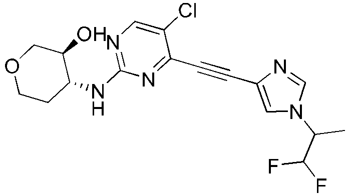 | (3 S,4R)-4-((5-chloro-4-((1-(1, 1-difluoropropan-2-yl)-1H-imidazol-5-yl)ethynyl)pyrimidin-2-yl)amino)tetrahydro-2H-pyran-3-ol | 398.1 |
| 135 | 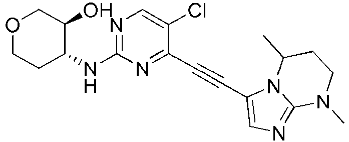 | (3 S,4R)-4-((5-chloro-4-((5,8-dimethyl-5,6,7,8-tetrahydroimidazo[1,2-a]pyrimidin-3-yl)ethynyl)pyrimidin-2-yl)amino)tetrahydro-2H-pyran-3-ol | 403.2 |
| 136 | 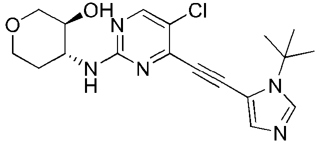 | (3S,4R)-4-((4-((1-(tert-butyl)-1H-imidazol-5-yl)ethynyl)-5-chloropyrimidin-2-yl)amino)tetrahydro-2H-pyran-3-ol | 376.2 |
| 137 | 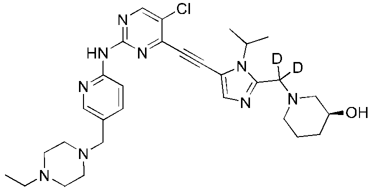 | (S)-1-((5-((5-chloro-2-((5-((4-ethylpiperazin-1-yl)methyl)pyridin-2-yl)amino)pyrimidin-4-yl)ethynyl)-1-isopropyl-1H-imidazol-2-yl)methyl-*d₂*)piperidin-3-ol | 580.4 |
| 138 | 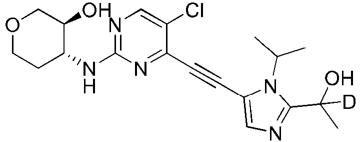 | (3 S,4R)-4-((S-chloro-4-((2-(1-hydroxyethyl-1-*d*)-1-isopropyl-1H-imidazol-5-yl)ethynyl)pyrimidin-2-yl)amino)tetrahydro-2H-pyran-3-ol | 407.2 |
| 138-(R) | 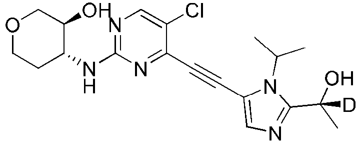 | (3 S,4R)-4-((5-chloro-4-((2-((R)-1-hydroxyethyl-1-*d*)-1-isopropyl-1H-imidazol-5-yl)ethynyl)pyrimidin-2-yl)amino)tetrahydro-2H-pyran-3-ol | 407.2 |
| 138-(S) | 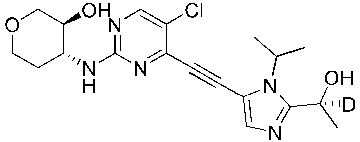 | (3 S,4R)-4-((5-chloro-4-((2-((S)-1-hydroxyethyl-1-*d*)-1-isopropyl-1H-imidazol-5-yl)ethynyl)pyrimidin-2-yl)amino)tetrahydro-2H-pyran-3-ol | 407.2 |

**The ¹H NMR data of the compounds prepared in the above examples are as follows:**

| **Example No.** | **¹H NMR** |
|---|---|
| 3 | (400 MHz, MeOH-*d*₄) δ 8.31 (s, 1H), 7.39 (s, 1H), 5.63 (hept, *J =* 6.9 Hz, 1H), 4.00-3.87 (m, 3H), 3.59 (td, *J* = 9.5, 4.8 Hz, 1H), 3.49 (td, *J* = 11.7, 2.3 Hz, 1H), 3.21 (dd, *J* = 11.2, 9.7 Hz, 1H), 2.10 (ddd, *J =* 13.5, 4.7, 2.3 Hz, 1H), 1.73 (dd, *J =* 7.0, 1.7 Hz, 6H), 1.68-1.54 (m, 1H), 1.65 (s, 6H). |
| 24 | (400 MHz, MeOH-*d*₄) δ 8.32 (s, 1H), 7.39 (s, 1H), 5.63 (p, *J =* 6.9 Hz, 1H), 3.92-3.79 (m, 2H), 3.75-3.65 (m, 2H), 2.96 (dd, *J* = 11.9, 2.8 Hz, 1H), 2.90 (s, 3H), 2.74 (dd, *J =* 11.7, 9.4 Hz, 1H), 2.27-2.13 (m, 1H), 2.04 (s, 1H), 1.73 (d, *J =* 7.0 Hz, 6H), 1.65 (s, 6H). |
| 30 | (400 MHz, MeOH-*d*₄) δ 8.33 (s, 1H), 7.51 (s, 1H), 5.13-5.01 (m, 2H), 4.02-3.87 (m, 3H), 3.59 (td, *J =* 9.4, 4.8 Hz, 1H), 3.54-3.44 (m, 1H), 3.21 (dd, *J =* 11.1, 9.6 Hz, 1H), 2.14-2.06 (m, 1H), 1.74 (dd, *J =* 6.9, 3.1 Hz, 6H), 1.69-1.54 (m, 4H). |
| 31 | (400 MHz, MeOH-*d*₄) δ 8.32 (s, 1H), 7.47 (s, 1H), 5.09 (p, *J =* 7.0 Hz, 1H), 5.03 (q, *J* = 6.6 Hz, 1H), 4.01-3.88 (m, 3H), 3.59 (td, *J* = 9.4, 4.8 Hz, 1H), 3.50 (td, *J* = 11.7, 2.3 Hz, 1H), 3.21 (dd, *J* = 11.2, 9.7 Hz, 1H), 2.15-2.06 (m, 1H), 1.73 (t, *J =* 6.7 Hz, 6H), 1.69-1.55 (m, 4H). |
| 33 | (400 MHz, MeOH-*d*₄) δ 8.27 (s, 1H), 7.62 (s, 1H), 4.60 (br s, 1H), 3.86 (s, 3H), 3.85 - 3.78 (m, 2H), 3.72-3.63 (m, 2H), 2.98-2.90 (m, 1H), 2.88 (s, 3H), 2.73 (dd, *J =* 11.7, 9.4 Hz, 1H), 2.22-2.14 (m, 1H), 1.69-1.59 (m, 1H), 1.48 (d, *J =* 7.0 Hz, 6H). |
| 35 | (400 MHz, MeOH-*d*₄) δ 8.31 (s, 1H), 7.48 (s, 1H), 3.97-3.89 (m, 5H), 3.62-3.54 (m, 1H), 3.52-3.44 (m, 2H), 3.20 (t, *J =* 10.4 Hz, 1H), 2.46 (s, 3H), 2.08 (d, *J =* 13.4 Hz, 1H), 1.70 (d, *J* = 6.8 Hz, 6H), 1.59 (dd, *J* = 8.9, 13.2 Hz, 1H). |
| 38 | (400 MHz, CDCl₃) δ 8.25 (s, 1H), 7.50 (s, 1H), 5.29 (d, *J* = 6.4 Hz, 1H), 4.81 (p, *J* = 6.9 Hz, 1H), 4.48 (br s, 1H), 4.06 (dd, *J =* 11.4, 4.9 Hz, 1H), 4.02- 3.93 (m, 3H), 3.89-3.78 (m, 1H), 3.66- 3.54 (m, 3H), 3.54-3.42 (m, 3H), 3.20 (dd, *J =* 11.4, 9.8 Hz, 1H), 2.07-2.01 (m, 1H), 1.70-1.65 (m, 6H), 1.51 (s, 3H). |
| 40 | (400 MHz, MeOH-*d*₄) δ 8.30 (s, 1H), 7.45 (s, 1H), 4.00-3.86 (m, 5H), 3.63-3.53 (m, 1H), 3.48 (td, *J* = 2.3*,* 11.7 Hz, 1H), 3.20 (dd, *J* = 9.7*,* 11.2 Hz, 1H), 2.88-2.75 (m, 1H), 2.15-2.03 (m, 1H), 1.73-1.67 (m, 6H), 1.67-1.50 (m, 2H), 1.11 (d, *J =* 6.2 Hz, 6H). |
| 41 | (400 MHz, MeOH-*d*₄) δ 8.58 (s, 1H), 8.29 (d, *J* = 8.7 Hz, 1H), 8.23 (d, *J=* 2.2 Hz, 1H), 7.78 (dd, *J* = 8.7*,* 2.4 Hz, 1H), 7.46 (s, 1H), 5.68 (p, *J* = 6.9 Hz, 1H), 3.55 (s, 2H), 2.85-2.52 (m, 8H), 2.47 (q, *J =* 7.3 Hz, 2H), 1.77 (d, *J =* 7.0 Hz, 6H), 1.67 (s, 6H), 1.11 (t, *J* = 7.2 Hz, 3H). |
| 52 | (400 MHz, MeOH-*d*₄) δ 8.36 (s, 1H), 7.60 (s, 1H), 5.86 (p, *J=* 6.9 Hz, 1H), 3.94 (ddd, *J =* 18.5, 10.6, 4.5 Hz, 3H), 3.59 (td, *J =* 9.4, 4.8 Hz, 1H), 3.49 (td, *J =* 11.7, 2.2 Hz, 1H), 3.21 (dd, *J=* 11.2, 9.7 Hz, 1H), 2.66 (d, *J=* 1.0 Hz, 3H), 2.10 (dd, *J=* 12.7, 3.2 Hz, 1H), 1.70 (d, *J=* 7.0 Hz, 6H), 1.62 (qd, *J=* 11.7, 4.6 Hz, 1H). |
| 53 | (400 MHz, MeOH-*d*₄) δ 8.34 (s, 1H), 7.37 (s, 1H), 5.62 (p, *J=* 6.9 Hz, 1H), 4.30-4.26 (m, 1H), 4.25-4.22 (m, 1H), 4.16 (dd, *J* = 9.2, 5.4 Hz, 1H), 4.01 (dd, *J* = 9.7, 4.5 Hz, 1H), 3.75 (dd, *J =* 9.2, 2.7 Hz, 1H), 3.70 (dd, *J =* 9.7, 2.0 Hz, 1H), 1.71 (d, *J* = 6.9 Hz, 6H), 1.63 (s, 6H). |
| 54 | (400 MHz, DMSO-*d*₆) δ 8.37 (s, 1H), 8.13 (s, 1H), 7.46 (s, 1H), 4.93 (d, *J=* 5.3 Hz, 1H), 3.88-3.68 (m, 6H), 3.51-3.40 (m, 1H), 3.29-3.19 (m, 1H), 3.11-2.92 (m, 2H), 1.94-1.83 (m, 1H), 1.52-1.39 (m, 1H), 1.29 (d, *J* = 6.9 Hz, 6H). |
| 55 | (400 MHz, MeOH-*d*₄) δ 8.55 (s, 1H), 8.21 (d, *J =* 8.7 Hz, 1H), 8.16 (s, 1H), 7.69 (d, *J* = 7.7 Hz, 1H), 7.44 (s, 1H), 5.72-5.59 (m, 1H), 3.03 (d, *J* = 11.5 Hz, 2H), 2.35 (s, 3H), 2.21 (t*, J =* 11.7 Hz, 2H), 1.93-1.78 (m, 5H), 1.75 (d, *J =* 7.0 Hz, 6H), 1.65 (s, 6H). |
| 56 | (400 MHz, DMSO-*d*₆) δ 8.37 (s, 1H), 7.70 (d, *J=* 6.3 Hz, 1H), 7.46 (s, 1H), 4.93 (d, *J* = 5.4 Hz, 1H), 3.88-3.72 (m, 6H), 3.48-3.44 (m, 1H), 3.29-3.23 (m, 1H), 3.03 (t, *J* = 10.4 Hz, 2H), 1.94-1.81 (m, 1H), 1.53-1.33 (m, 7H). |
| 58 | (400 MHz, MeOH-*d*₄) δ 8.58 (d, *J =* 1.2 Hz, 1H), 7.45 (s, 1H), 5.65 (p, *J =* 6.9 Hz, 1H), 4.76 (td, *J* = 9.4, 4.7 Hz, 1H), 4.24 (dd, *J =* 10.9, 4.7 Hz, 1H), 3.95 *(d, J =* 11.7 Hz, 1H), 3.51 (dd, *J* = 11.9, 2.3 Hz, 1H), 3.23 (dd, *J* = 10.9, 9.5 Hz, 1H), 3.17-3.07 (m, 1H), 1.70 (dd, *J =* 6.9,1.7 Hz, 7H), 1.64 (s, 7H). |
| 59 | (400 MHz, DMSO-*d*₆) δ 10.10 (s, 1H), 8.63 (s, 1H), 8.18 (d, *J =* 2.5 Hz, 1H), 8.04 (d, *J =* 8.6 Hz, 1H), 7.74 (s, 1H), 7.66 (dd, *J =* 8.7, 2.5 Hz, 1H), 3.85 (s, 3H), 3.29-3.26 (m, 1H), 2.89-2.83 (m, 2H), 2.47-2.41 (m, 1H), 2.19 (s, 3H), 2.01-1.91 (m, 2H), 1.77-1.63 (m, 4H), 1.44 (d, *J* = 7.0 Hz, 6H). |
| 60 | (400 MHz, MeOH-*d*₄) δ 8.33 (s, 1H), 7.40 (s, 1H), 5.65 (p, *J* = 6.8 Hz, 1H), 3.90-3.86 (m, 1H), 3.69-3.56 (m, 1H), 3.23 (d, *J* = 12.4 Hz, 1H), 3.06 (d, *J* = 12.9 Hz, 1H), 2.82-2.65 (m, 2H), 2.68-2.51 (m, 1H), 2.16 (d, *J* = 13.2 Hz, 1H), 1.74 (dd, *J* = 7.0, 1.6 Hz, 6H), 1.67 (s, 6H). |
| 61 | (400 MHz, DMSO-*d*₆)δ 9.64 (s, 1H), 8.56 (d*, J =* 1.5 Hz, 1H), 7.86 (d, *J =* 8.8 Hz, 1H), 7.74 (s, 1H), 7.55 (d, *J =* 3.0 Hz, 1H), 6.90 (dd, *J* = 8.8*,* 3.0 Hz, 1H), 3.87 (s, 4H), 3.84 (s, 3H), 3.30-3.27 (m, 1H), 3.23 (s, 4H), 2.37-2.29 (m, 2H), 1.40 (d, *J =* 7.0 Hz, 6H), 0.86 (t*, J =* 7.1 Hz, 3H). |
| 62 | (400 MHz, DMSO-*d*₆) δ 9.79 (s, 1H), 8.55 (s, 1H), 7.84 (d, *J =* 8.9 Hz, 1H), 7.73 (s, 1H), 7.56 (d, *J =* 3.0 Hz, 1H), 6.90 (dd, *J =* 8.8, 3.0 Hz, 1H), 3.88 (s, 4H), 3.84 (s, 3H), 3.30-3.26 (m, 1H), 3.21 (s, 4H), 2.37-2.31 (m, 2H), 1.43 (d, J = 7.0 Hz, 6H), 0.85 (t, J = 7.2 Hz, 3H). |
| 63 | (400 MHz, MeOH-*d*₄) δ 8.31 (s, 1H), 7.44 (s, 1H), 5.00-4.95 (m, 1H), 3.98-3.88 (m, 3H), 3.62-3.56 (m, 3H), 3.48 (td, *J =* 2.2, 11.6 Hz, 1H), 3.20 (t, *J =* 9.6 Hz, 1H), 2.24 (s, 6H), 2.13-2.05 (m, 1H), 1.69 (dd, *J* = 1.3, 6.9 Hz, 6H), 1.63-1.57 (m, 1H). |
| 64 | (400 MHz, DMSO-*d*₆) δ 8.36 (s, 1H), 7.72 (s, 1H), 7.46 (s, 1H), 4.92 (s, 1H), 4.68 (s, 1H), 4.04 (s, 2H), 3.83-3.73 (m, 3H), 3.43-3.38 (m, 1H), 3.30-3.26 (m, 1H), 3.03 (t, *J* = 10.4 Hz, 1H), 1.93-1.85 (m, 1H), 1.52-1.29 (m, 8H), 1.11 (s, 6H). |
| 65 | (400 MHz, DMSO-*d*₆) δ 8.37 (s, 1H), 8.03 (s, 1H), 7.44 (d, *J* = 7.3 Hz, 1H), 4.92 (d, *J* = 5.4 Hz, 1H), 4.73 (s, 1H), 3.98 (s, 2H), 3.85-3.71 (m, 3H), 3.51-3.41 (m, 1H), 3.30-3.27 (m, 1H), 3.13-3.01 (m, 2H), 1.93-1.83 (m, 1H), 1.52-1.41 (m, 1H), 1.29 (d, *J* = 6.9 Hz, 6H), 1.06 (s, 6H). |
| 66 | (400 MHz, MeOH-*d*₄) δ 8.41 (s, 1H), 8.40 (d, *J=* 2.7 Hz, 1H), 7.91 (dd, *J=* 9.0, 2.8 Hz, 1H), 7.40 (s, 1H), 6.85 (d, *J* = 9.2 Hz, 1H), 5.64 (p, *J* = 7.0 Hz, 1H), 3.57-3.45 (m, 4H), 2.60 (t, *J* = 5.1 Hz, 4H), 2.36 (s, 3H), 1.73 (d, *J* = 6.8 Hz, 6H), 1.65 (s, 6H). |
| 67 | (400 MHz, MeOH-*d*₄) δ 8.61 (s, 1H), 8.42 (d, *J=* 8.8 Hz, 1H), 8.39 (d, *J* = 2.3 Hz, 1H), 7.86 (dd, *J=* 8.8, 2.4 Hz, 1H), 7.45 (s, 1H), 5.66 (p, *J =* 7.0 Hz, 1H), 3.84-3.60 (m, 4H), 2.68-2.49 (m, 7H), 1.76 (d, *J* = 6.9 Hz, 6H), 1.65 (s, 6H), 1.15 (t, *J* = 7.2 Hz, 3H). |
| 68 | (400 MHz, DMSO-*d*₆) δ 8.40 (s, 1H), 7.53 (d, *J=* 8.2 Hz, 1H), 7.46 (s, 1H), 4.94 (p, *J* = 6.8 Hz, 2H), 4.23 (t, *J =* 7.2 Hz, 1H), 3.84-3.74 (m, 3H), 3.44 (d, *J =* 4.6 Hz, 1H), 3.03 (t, *J=* 10.4 Hz, 1H), 2.89 (dt, *J=* 10.6, 6.5 Hz, 1H), 2.82-2.75 (m, 1H), 2.13-1.97 (m, 2H), 1.89 (dd, *J =* 13.3, 4.3 Hz, 1H), 1.83-1.68 (m, 2H), 1.60 (d, *J =* 6.9Hz, 6H), 1.45 (dt*, J =* 11.3, 6.0 Hz, 1H). |
| 69 | (400 MHz, CDCl₃) δ 8.45 (s, 1H), 8.36-8.09 (m, 3H), 7.78-7.56 (m, 2H), 3.87 (s, 3H), 3.50 (s, 2H), 3.31-3.10 (m, 1H), 2.78-2.15 (m, 10H), 1.52 (d, *J =* 7.1 Hz, 6H), 1.19-1.06 (m, 3H). |
| 70 | (400 MHz, CDCl₃) δ 8.40-8.26 (m, 2H), 8.21 (d, *J =* 2.3 Hz, 1H), 8.05 (s, 1H), 7.72-7.58 (m, 2H), 3.86 (s, 3H), 3.53 (s, 2H), 3.20 (p, *J =* 7.0 Hz, 1H), 3.05-2.24 (m, 10H), 1.50 (d, *J* = 7.0 Hz, 6H), 1.32-1.16 (m, 3H). |
| 71 | (400 MHz, MeOH-*d*₄) δ 8.49 (s, 1H), 8.01 (d, *J=* 8.9 Hz, 1H), 7.57 (d, *J =* 2.9 Hz, 1H), 7.44 (s, 1H), 7.01 (dd, *J* = 8.9*,* 3.0 Hz, 1H), 5.66 (p, *J* = 6.8 Hz, 1H), 3.99 (s, 4H), 3.54-3.44 (m, 4H), 2.57 (d, *J =* 7.8 Hz, 2H), 1.76 (d, *J=* 6.9 Hz, 6H), 1.66 (s, 6H), 1.02 (t, *J* = 7.2 Hz, 3H). |
| 72 | (400 MHz, DMSO-*d*₆) δ 8.40 (s, 1H), 7.51 (d, *J =* 14.5 Hz, 2H), 5.07 (p, *J =* 6.9 Hz, 1H), 4.92 (d, *J* = 5.3 Hz, 1H), 3.94 (s, 2H), 3.80 (dd, *J* = 11.8, 6.0 Hz, 3H), 3.61 (dd, *J* = 11.2, 3.2 Hz, 2H), 3.49-3.43 (m, 1H), 3.06 (dt, *J* = 26.5, 10.2 Hz, 4H), 2.73 (d, *J* = 9.6Hz, 2H), 1.89 (d, *J* = 13.1 Hz, 1H), 1.61 (dd, *J* = 6.9, 1.8 Hz, 6H), 1.45 (dd, *J* = 12.8, 8.5 Hz, 1H), 0.87 (d, *J* = 6.3 Hz, 6H). |
| 73 | (400 MHz, DMSO-*d*₆) δ 10.00 (s, 1H), 8.68 (d, *J =* 1.4 Hz, 1H), 8.17 (d, *J =* 2.4 Hz, 1H), 8.06 (d, *J =* 8.6 Hz, 1H), 7.66 (dd, *J =* 8.7, 2.5 Hz, 1H), 7.49 (s, 1H), 5.63 (s, 1H), 5.58 (m, 1H), 2.90-2.82(m, 2H), 2.49-2.41 (m, 2H), 2.19 (s, 3H), 2.00-1.95 (m, 2H), 1.76-1.66 (m, 3H), 1.63 (d, *J =* 6.8 Hz, 6H), 1.56 (s, 6H) |
| 74 | (400 MHz, DMSO-*d*₆) δ 8.40 (s, 1H), 7.50 (d, *J* = 22.0 Hz, 2H), 4.93 (dd, *J=* 12.5, 6.1 Hz, 2H), 4.07 (d, *J* = 13.7 Hz, 1H), 3.80 (dd, *J* = 12.1, 6.4 Hz, 3H), 3.58-3.51 (m, 3H), 3.49-3.43 (m, 1H), 3.27-3.22 (m, 2H), 3.03 (t, *J =* 10.4 Hz, 1H), 2.57 (s, 2H), 1.89 (d, *J=* 12.9 Hz, 1H), 1.62 (dd, *J* = 13.1, 6.8 Hz, 6H), 1.45 (dd, *J* = 12.3, 8.3 Hz, 1H), 0.98 (d, *J* = 6.4 Hz, 6H). |
| 75 | (400 MHz, DMSO-*d*₆) δ 8.41 (s, 1H), 7.51 (d, *J =* 20.8 Hz, 2H), 4.96-4.82 (m, 2H), 4.62 (d, *J =* 4.7 Hz, 1H), 3.84-3.74 (m, 3H), 3.68-3.54 (m, 2H), 3.45 (s, 1H), 3.03 (t, *J* = 10.4 Hz, 1H), 2.73-2.66 (m, 1H), 2.57 (d, *J* = 11.2 Hz, 1H), 1.94-1.84 (m, 2H), 1.74 (d, *J =* 10.2 Hz, 2H), 1.65-1.57 (m, 7H), 1.46 (d, *J* = 9.0 Hz, 1H), 1.35 (d, *J =* 12.4 Hz, 1H), 1.07 (dd, *J =* 13.9, 10.1 Hz, 1H). |
| 76 | (400 MHz, DMSO-*d*₆) δ 10.15 (s, 1H), 8.67 (s, 1H), 8.19 (d, *J* = 2.4 Hz, 1H), 8.05 (d, *J=* 8.7 Hz, 1H), 7.69 (dd, *J =* 8.6, 2.5 Hz, 1H), 7.49 (s, 1H), 5.64 (s, 1H), 5.62-5.51(m, 1H), 3.02-2.91 (m, 2H), 2.34 (q, *J* = 7.1 Hz, 2H), 1.94 (td, *J* = 11.5, 2.6 Hz, 2H), 1.78-1.60 (m, 11H), 1.56 (s, 6H), 1.02 (t, *J* = 7.1 Hz, 3H). |
| 77 | (400 MHz, MeOH-*d*₄) δ 8.25 (s, 1H), 8.19 (d, *J =* 2.3 Hz, 1H), 8.04 (s, 1H), 7.66 (dd, *J* = 8.6, 2.3 Hz, 1H), 7.46 (d, *J* = 8.5 Hz, 1H), 7.34 (s, 1H), 5.63 (p, *J* = 6.9 Hz, 1H), 3.51 (s, 2H), 2.55 (s, 8H), 2.46 (q, *J =* 7.2 Hz, 2H), 1.74 (d, *J =* 6.9 Hz, 6H), 1.65 (s, 6H), 1.10 (t, *J* = 7.2 Hz, 3H). |
| 78 | (400 MHz, DMSO-*d*₆) δ 8.41 (s, 1H), 7.51 (d, *J=* 31.8 Hz, 2H), 4.93 (d, *J* = 5.3 Hz, 1H), 4.86 (q, *J =* 6.9 Hz, 1H), 4.62 (d, *J=* 4.7 Hz, 1H), 3.83-3.75 (m, 3H), 3.66 (d, *J =* 13.4 Hz, 1H), 3.56 (d, *J =* 13.4 Hz, 1H), 3.49-3.39 (m, 2H), 3.03 (t, *J =* 10.4 Hz, 1H), 2.69 (d, *J* = 10.0 Hz, 1H), 2.57 (d, *J* = 10.0 Hz, 1H), 1.92-1.84 (m, 2H), 1.81-1.70 (m, 2H), 1.59 (dd, *J* = 6.9, 1.6 Hz, 6H), 1.51-1.44 (m, 1H), 1.35 (d, *J* = 12.3 Hz, 1H), 1.06 (d, *J =* 12.6 Hz, 1H). |
| 79 | (400 MHz, MeOH-*d*₄) δ 8.29 (s, 1H), 7.38 (s, 1H), 4.77-4.65 (m, 2H), 3.98-3.85 (m, 3H), 3.61-3.53 (m, 1H), 3.48 (t, *J* = 11.5 Hz, 1H), 3.19 (t, *J* = 10.4 Hz, 1H), 2.46 (s, 3H), 2.13-2.04 (m, 1H), 1.67 (d, *J =* 6.9 Hz, 6H). |
| 80 | (400 MHz, CDCl₃) δ 13.31 (s, 1H), 8.48-8.38 (m, 1H), 8.26-8.06 (m, 2H), 7.69-7.58 (s, 1H), 7.58-7.48 (s, 1H), 6.89 (d, *J* = 8.6 Hz, 1H), 4.70-4.51 (m, 3H), 4.29 (s, 2H), 4.00 (s, 2H), 3.86 (s, 2H), 3.14 (d, *J* = 7.7 Hz, 2H), 2.56 (s, 3H), 1.71 (d, *J* = 6.9 Hz, 6H), 1.36 (t, *J =* 7.1 Hz, 3H). |
| 81 | (400 MHz, CDCl₃) δ 8.33 (d, *J =* 1.3 Hz, 1H), 8.14 (d, *J* = 8.9 Hz, 1H), 8.04 (s, 1H), 7.62 (d, *J =* 2.9 Hz, 1H), 7.48 (s, 1H), 6.87 (dd, J = 9.0, 3.0 Hz, 1H), 4.57 (p, *J* = 6.9 Hz, 1H), 3.98 (s, 4H), 3.47 (s, 4H), 2.61-2.50 (m, 2H), 2.48 (s, 3H), 1.67 (d, *J =* 6.9 Hz, 6H), 1.03 (t, *J =* 7.2 Hz, 3H). |
| 82 | (400 MHz, DMSO-*d*₆) δ 9.62 (s, 1H), 8.55 (d, *J* = 1.3 Hz, 1H), 7.86 (d, *J =* 8.9 Hz, 1H), 7.73 (s, 1H), 7.55 (d, *J* = 3.0 Hz, 1H), 6.90 (dd, *J* = 8.9, 3.0 Hz, 1H), 4.44 (t, *J =* 4.9 Hz, 1H), 4.32 (t, *J* = 4.8 Hz, 1H), 3.86 (d, *J* = 18.9 Hz, 7H), 3.34 (s, 4H), 3.28 (d, *J* = 6.9 Hz, 1H), 2.68 (t, *J* = 4.8 Hz, 1H), 2.61 (t, *J* = 4.8 Hz, 1H), 1.40 (d, *J* = 7.0 Hz, 6H), 1.24 (s, 1H). |
| 83 | (400 MHz, DMSO-*d*₆) δ 9.13 (s, 1H), 8.20 (d, *J =* 2.6 Hz, 1H), 8.09 (s, 1H), 7.70 (d, *J* = 8.8 Hz, 1H), 7.56 (d, *J* = 3.0 Hz, 1H), 6.87 (dd, *J* = 8.8, 3.0 Hz, 1H), 5.69 (d, *J* = 1.9 Hz, 1H), 3.87 (d, *J =* 4.8 Hz, 7H), 3.22 (s, 4H), 2.34 (d, *J =* 7.2 Hz, 2H), 1.66 (s, 6H), 0.85 (t, *J* = 7.1 Hz, 3H). |
| 84 | (400 MHz, DMSO-*d*₆) δ 10.52 (s, 1H), 8.73 (s, 1H), 8.05 (dd, *J* = 8.2, 1.7 Hz, 1H), 7.97-7.80 (m, 1H), 7.51 (s, 1H), 5.69-5.52 (m, 2H), 3.44 (s, 2H), 2.44-2.19 (m, 10H), 1.66 (d, *J =* 6.9 Hz, 6H), 1.56 (s, 6H), 0.96 (t, *J =* 7.1 Hz, 3H). |
| 85 | (400 MHz, CDCl₃) δ 8.22 (s, 1H), 7.41 (s, 1H), 5.42 (d, *J* = 7.5 Hz, 1H), 5.32 (p, *J* = 6.9 Hz, 1H), 4.13 (dd, *J* = 11.2, 4.3 Hz, 1H), 4.01-3.93 (m, 1H), 3.92-3.85 (m, 1H), 3.80-3.71 (m, 1H), 3.54-3.42 (m, 1H), 3.23 (dd, *J =* 11.2, 8.3 Hz, 1H), 3.03 (s, 1H), 2.13-2.01 (m, 1H), 1.72-1.66 (m, 12H). |
| 86 | (400 MHz, CDCl₃) δ 8.24 (s, 1H), 7.47 (s, 1H), 5.32 (p, *J* = 6.9 Hz, 1H), 5.01 (d, *J =* 9.0 Hz, 1H), 4.04-3.96 (m, 1H), 3.95-3.87 (m, 1H), 3.80-3.69 (m, 1H), 3.53 (td, *J =* 12.0, 2.2 Hz, 1H), 3.15 (t, *J =* 11.1 Hz, 1H), 2.82 (s, 1H), 2.10-2.00 (m, 1H), 1.76-1.68 (m, 12H), 1.57-1.45 (m, 1H), 0.91 (d, *J* = 6.6 Hz, 3H). |
| 87 | (400 MHz, CDCl₃) δ 8.17 (s, 1H), 7.37 (s, 1H), 6.46 (br s, 1H), 5.36 (d, *J=* 7.4 Hz, 1H), 5.33-5.22 (m, 1H), 4.13 (dd, *J* = 11.2, 4.4 Hz, 1H), 4.00-3.90 (m, 1H), 3.90-3.73 (m, 2H), 3.41 (td, *J* = 11.8, 2.2 Hz, 1H), 3.03 (t, *J* = 10.4 Hz, 1H), 2.93 (s, 1H), 2.07-1.96 (m, 1H), 1.80 (s, 3H), 1.72-1.59 (m, 12H). |
| 88 | (400 MHz, MeOH-*d*₄) δ 8.38 (s, 1H), 7.38 (s, 1H), 5.62 (hept, *J* = 6.8 Hz, 1H), 3.98-3.83 (m, 3H), 3.57 (td, *J* = 9.4, 4.8 Hz, 1H), 3.48 (td, *J =* 11.7, 2.3 Hz, 1H), 3.19 (dd, *J* = 11.2, 9.7 Hz, 1H), 2.12-2.03 (m, 1H), 1.73 (dd, *J=* 6.9, 1.8 Hz, 6H), 1.64 (s, 6H), 1.58 (ddd, *J=* 13.2, 11.5, 4.5 Hz, 1H). |
| 89 | (400 MHz, DMSO-*d*₆) δ 9.66 (s, 1H), 8.56 (d, *J =* 1.5 Hz, 1H), 7.88 (d*, J =* 8.8 Hz, 1H), 7.73 (s, 1H), 7.57 (d, *J =* 2.9 Hz, 1H), 6.93 (dd, *J =* 9.0, 3.0 Hz, 1H), 4.29 (s, 2H), 4.01 (s, 2H), 3.96 (s, 4H), 3.84 (s, 3H), 3.29-3.20 (m, 1H), 1.75 (s, 3H), 1.40 (d, *J =* 7.0 Hz, 6H). |
| 90 | (400 MHz, DMSO-*d*₆) δ 8.40 (s, 1H), 7.52 (s, 1H), 7.43 (s, 1H), 5.49 (s, 1H), 5.40-5.29 (m, 1H), 4.92 (d, *J* = 5.3 Hz, 1H), 3.85-3.70 (m, 3H), 3.52-3.41 (m, 1H), 3.03 (t, *J* = 10.4 Hz, 1H), 2.27-2.15 (m, 2H), 1.99-1.86 (m, 3H), 1.82-1.72 (m, 2H), 1.69-1.61 (m, 2H), 1.61 (d, *J* = 6.8, 6H), 1.57-1.36 (m, 2H). |
| 91 | (400 MHz, CDCl₃) δ 8.28 (s, 1H), 7.47 (s, 1H), 5.37-5.18 (m, 2H), 4.58-4.35 (m, 1H), 4.32-4.18 (m, 1H), 4.13-4.01 (m, 1H), 3.92-3.80 (m, 1H), 3.65-3.50 (m, 2H), 2.65 (s, 1H), 2.38-2.26 (m, 1H), 1.74-1.68 (m, 12H). |
| 92 | (400 MHz, CDCl₃) δ 8.24 (d, *J =* 6.0 Hz, 1H), 7.46 (d, *J =* 2.0 Hz, 1H), 6.47-4.95 (m, 2H), 4.86-4.68 (m, 1H), 4.40-4.20(m, 1H), 4.05-3.83 (m, 1H), 3.75-3.51 (m, 1H), 3.47-3.33 (m, 3H), 2.22-2.00 (m, 2H), 1.75-1.65 (m, 12H), 1.59-1.52 (m, 1H). |
| 93 | (400 MHz, DMSO-*d*₆) δ 9.65 (s, 1H), 8.56 (s, 1H), 7.86 (d, *J =* 8.8 Hz, 1H), 7.74 (s, 1H), 7.55 (d, *J* = 3.0 Hz, 1H), 6.90 (dd, *J =* 9.0, 3.1 Hz, 1H), 3.85 (d, *J* = 10.6 Hz, 7H), 3.25 (s, 5H), 2.18 (s, 3H), 1.40 (d, *J =* 7.0 Hz, 6H). |
| 94 | (400 MHz, DMSO-*d*₆) δ 10.38 (s, 1H), 8.70 (s, 1H), 8.29 (d, *J =* 2.7 Hz, 1H), 8.16 (d, *J =* 9.0 Hz, 1H), 7.78 (dd, *J=* 8.8, 2.7 Hz, 1H), 7.50 (s, 1H), 5.64 (s, 1H), 5.58 (p, *J =* 6.9 Hz, 1H), 3.72-3.64 (m, 2H), 3.13 (s, 2H), 2.74 (t, *J =* 5.5 Hz, 2H), 2.30 (s, 3H), 1.66 (d, *J =* 6.9 Hz, 6H), 1.56 (s, 6H). |
| 95 | (400 MHz, MeOH-*d*₄) δ 8.30 (d, *J=* 1.6 Hz, 1H), 7.38 (s, 1H), 5.64 (p, *J= 6.9* Hz, 1H), 3.97 (dd, *J* = 11.2, 4.8 Hz, 1H), 3.90 (ddd, *J* = 14.9, 10.2, 4.8 Hz, 2H), 3.59 (td, *J* = 9.4, 4.8 Hz, 1H), 3.50 (td, *J* = 11.7, 2.3 Hz, 1H), 3.22 (dd, *J* = 11.2, 9.6 Hz, 1H), 2.16-2.09 (m, 1H), 1.70 (dd, *J* = 6.9, 1.2 Hz, 6H), 1.66 (s, 6H), 1.59 (td*, J =* 12.4, 4.7 Hz, 1H). |
| 96 | (400 MHz, DMSO-*d*₆) δ 8.42 (s, 1H), 7.73 (s, 1H), 7.42 (s, 1H), 5.61 (s, 1H), 5.54 (p, *J=* 6.7 Hz, 1H), 4.19 (s, 1H), 3.12 (d, *J =* 8.5 Hz, 1H), 2.99 (s, 3H), 2.78 (s, 3H), 1.71-1.59 (m, 12H), 1.54 (s, 8H). |
| 97 | (400 MHz, DMSO-*d*₆) δ 8.42 (s, 1H), 7.73 (s, 1H), 7.42 (s, 1H), 5.61 (s, 1H), 5.54 (p, *J=* 7.0 Hz, 1H), 4.19 (s, 1H), 3.13 (d, *J =* 8.6 Hz, 1H), 2.99 (s, 3H), 2.78 (s, 3H), 1.70-1.60 (m, 12H), 1.54 (s, 8H). |
| 98 | (400 MHz, MeOH-*d*₄) δ 8.31 (s, 1H), 7.44 (s, 1H), 5.01 (pent, *J* = 6.9 Hz, 1H), 3.99-3.87 (m, 3H), 3.76-3.62 (m, 2H), 3.58 (td, *J* = 4.8, 9.5 Hz, 1H), 3.48 (td, *J* = 2.0, 11.6 Hz, 1H), 3.23-3.17 (m, 1H), 3.04-2.96 (m, 1H), 2.85-2.73 (m, 1H), 2.61-2.46 (m, 2H), 2.31-2.19 (m, 1H), 2.14-2.05 (m, 1H), 1.89-1.75 (m, 3H), 1.72 (t, *J =* 7.1 Hz, 6H), 1.66-1.54 (m, 2H). |
| 99 | (400 MHz, DMSO-*d*₆) δ 8.40 (s, 1H), 7.54 (s, 1H), 7.42 (s, 1H), 7.01 (s, 1H), 5.61 (s, 1H), 5.58-5.49 (m, 1H), 3.57 (s, 1H), 3.09 (s, 1H), 2.91 (s, 3H), 1.92 (d, *J =* 9.8 Hz, 4H), 1.62 (d, *J* = 6.8 Hz, 6H), 1.54 (s, 6H), 1.37-1.28 (m, 4H). |
| 100 | (400 MHz, MeOH-*d*₄) δ 8.61 (s, 1H), 8.44-8.37 (m, 2H), 7.88 (dd, *J =* 2.5, 8.6 Hz, 1H), 7.46 (s, 1H), 5.67 (d, *J* = 7.0 Hz, 1H), 3.11 (s, 6H), 1.77 (s, 3H), 1.75 (s, 3H), 1.66 (s, 6H). |
| 101 | (400 MHz, CDCl₃) δ 8.39 (s, 1H), 7.51 (s, 1H), 5.44-5.33 (m, 1H), 5.32 (s, 1H), 4.00 (d, *J =* 4.2 Hz, 1H), 3.97 (t, *J =* 4.0 Hz, 1H), 3.87-3.77 (m, 2H), 3.06 (s, 1H), 2.58 (d, *J* = 13.2 Hz, 2H), 2.04-1.95 (m, 2H), 1.74-1.69 (m, 12H). |
| 102 | (400 MHz, DMSO-*d*₆) δ 8.45 (s, 1H), 7.53 (d, *J =* 20.7 Hz, 2H), 4.95-4.82 (m, 2H), 4.62 (d, *J =* 4.7 Hz, 1H), 3.86-3.74 (m, 3H), 3.69-3.54 (m, 2H), 3.45 (s, 1H), 3.05 (t, *J* = 10.4 Hz, 1H), 2.74-2.66 (m, 1H), 2.58 (d, *J* = 11.2 Hz, 1H), 1.94-1.84 (m, 2H), 1.75 (d, *J =* 10.2 Hz, 2H), 1.63-1.57 (m, 7H), 1.48 (d, *J =* 9.0 Hz, 1H), 1.36 (d, *J =* 12.4 Hz, 1H), 1.08 (dd, *J* = 13.9, 10.1 Hz, 1H). |
| 103 | (400 MHz, MeOH-*d*₄) δ 8.31 (s, 1H), 7.44 (s, 1H), 5.01 (d, *J* = 6.9 Hz, 1H), 3.98-3.87 (m, 3H), 3.75-3.63 (m, 2H), 3.58 (td, *J* = 4.8, 9.5 Hz, 1H), 3.48 (td, *J* = 2.0, 11.7 Hz, 1H), 3.24-3.16 (m, 1H), 3.05-2.95 (m, 1H), 2.87-2.74 (m, 1H), 2.62-2.47 (m, 2H), 2.32-2.19 (m, 1H), 2.14-2.04 (m, 1H), 1.87-1.75 (m, 3H), 1.72 (dd, *J =* 6.9, 10.5 Hz, 6H), 1.67-1.54 (m, 2H). |
| 104 | (400 MHz, DMSO-*d*₆) δ 9.89 (s, 1H), 8.59 (s, 1H), 7.83 (d, *J =* 8.8 Hz, 1H), 7.59-7.49 (m, 2H), 6.91 (dd, *J* = 9.0, 3.0 Hz, 1H), 5.03-4.88 (m, 1H), 4.09 (d, *J* = 13.8 Hz, 1H), 3.88 (s, 4H), 3.60-3.51 (m, 3H), 3.21 (s, 6H), 2.62-2.54 (m, 2H), 2.33 (q, *J* = 7.2 Hz, 2H), 1.64 (dd, *J* = 12.3, 6.8 Hz, 6H), 0.98 (d, *J* = 6.4 Hz, 6H), 0.85 (t, *J* = 7.1 Hz, 3H). |
| 105 | (400 MHz, MeOH-*d*₄) δ 8.30 (s, 1H), 7.43 (s, 1H), 5.02-4.92 (m, 1H), 4.69-4.47(m, 1H), 3.99-3.85 (m, 3H), 3.67 (s, 2H), 3.61-3.53(m, 1H), 3.52-3.44 (m, 1H), 3.27-3.16 (m, 2H), 2.75-2.55(m, 1H), 2.48 (s, 1H), 2.38 (s, 2H), 2.12-2.05 (m, 1H), 1.89-1.74(m, 2H), 1.69 (d, *J* = 6.8 Hz, 6H), 1.63-1.58 (m, 1H), 1.56-1.47 (m, 1H). |
| 106 | (400 MHz, DMSO-*d*₆) δ 10.28 (s, 1H), 8.70 (s, 1H), 8.20 (d, *J =* 2.3 Hz, 1H), 8.10 (d, *J =* 8.6 Hz, 1H), 7.71 (dd, *J* = 8.6, 2.4 Hz, 1H), 7.54 (s, 1H), 4.90 (p, *J* = 6.8 Hz, 1H), 4.63 (d, *J =* 4.7 Hz, 1H), 3.68 (d, *J =* 13.4 Hz, 1H), 3.58 (d, *J =* 13.4 Hz, 1H), 3.48 (s, 2H), 3.45-3.37 (m, 5H), 2.76-2.66 (m, 1H), 2.63-2.55 (m, 1H), 2.41-2.26 (m, 4H), 1.97 (s, 3H), 1.93-1.84 (m, 1H), 1.83-1.70 (m, 2H), 1.63 (d, *J* = 6.8 Hz, 7H), 1.44-1.30 (m, 1H), 1.14-1.01 (m, 1H). |
| 107 | (400 MHz, DMSO-*d*₆) δ 10.25 (s, 1H), 8.69 (s, 1H), 8.18 (d, *J =* 2.3 Hz, 1H), 8.09 (d, *J =* 8.5 Hz, 1H), 7.68 (dd, *J =* 8.5, 2.4 Hz, 1H), 7.54 (s, 1H), 4.90 (p, *J =* 6.8 Hz, 1H), 4.63 (d, *J =* 4.7 Hz, 1H), 3.68 (d, *J* = 13.5 Hz, 1H), 3.58 (d, *J* = 13.4 Hz, 1H), 3.47-3.41 (m, 2H), 3.23-3.30 (m, 1H), 2.75-2.68 (m, 1H), 2.62-2.56 (m, 1H), 2.46-2.15 (m, 10H), 1.93-1.85 (m, 1H), 1.82-1.72 (m, 2H), 1.63 (d, *J =* 7.0 Hz, 7H), 1.41-1.31 (m, 1H), 1.13-1.04 (m, 1H), 0.97 (t, *J =* 7.2 Hz, 3H). |
| 108 | (400 MHz, DMSO-*d*₆) δ 9.86 (s, 1H), 8.58 (s, 1H), 7.83 (d, *J=* 8.8 Hz, 1H), 7.59-7.48 (m, 2H), 6.91 (dd, *J* = 8.9, 3.0 Hz, 1H), 4.96-4.82 (m, 1H), 4.61 (d, *J* = 4.8 Hz, 1H), 3.88 (s, 4H), 3.72-3.53 (m, 2H), 3.39 (dd, *J* = 9.3, 4.9 Hz, 1H), 3.22 (s, 4H), 2.70 (d, *J* = 11.5 Hz, 1H), 2.57 *(d, J=* 11.7 Hz, 1H), 2.34 (q, *J =* 7.1 Hz, 2H), 1.87 (d, *J=* 10.8 Hz, 1H), 1.75 (q, *J =* 11.5 Hz, 2H), 1.61 (d, *J* = 6.9 Hz, 7H), 1.36 (d, *J* = 12.5 Hz, 1H), 1.06 (d, *J=* 12.4 Hz, 1H), 0.85 (t, *J=* 7.2 Hz, 3H). |
| 109 | (400 MHz, DMSO-*d*₆) δ 8.05 (s, 1H), 7.35 (s, 1H), 6.88 (d, *J =* 7.2 Hz, 1H), 6.73 (s, 1H), 5.00 (d, *J* = 5.3 Hz, 1H), 4.85 (p, *J* = 6.9 Hz, 1H), 4.60 (t, *J* = 5.2 Hz, 1H), 3.84-3.71 (m, 3H), 3.64 (d, *J* = 13.4 Hz, 1H), 3.54 (d, *J =* 13.4 Hz, 1H), 3.42-3.36 (m, 2H), 3.06 (dd, *J =* 11.0, 9.3 Hz, 1H), 2.73-2.66 (m, 1H), 2.57 (d, *J* = 11.0 Hz, 1H), 2.01-1.94 (m, 1H), 1.91-1.83 (m, 1H), 1.82-1.75 (m, 1H), 1.71 (d, *J* = 9.7 Hz, 1H), 1.58 (d, *J =* 6.8 Hz, 6H), 1.41-1.31 (m, 2H), 1.06 (dd, *J* = 10.8, 4.1 Hz, 1H). |
| 110 | (400 MHz, MeOH-*d*₄) δ 8.31 (s, 1H), 7.44 (s, 1H), 5.00 (s, 1H), 3.99-3.85 (m, 3H), 3.75-3.43 (m, 4H), 3.25-3.10 (m, 2H), 2.31 (s, 3H), 2.13-2.03 (m, 1H), 1.70 (d, *J* = 5.9 Hz, 6H), 1.63-1.43 (m, 4H), 1.22 (s, 3H). |
| 111 | (400 MHz, CDCl₃) δ 8.17 (s, 1H), 7.66 (s, 1H), 5.17 (d, *J* = 6.3 Hz, 1H), 4.52 (d, *J* = 3.4 Hz, 1H), 4.15-4.07 (m, 2H), 4.07-4.02 (m, 2H), 4.02-3.96 (m, 1H), 3.95-3.85 (m, 1H), 3.81-3.69 (m, 1H), 3.60-3.48 (m, 1H), 3.45-3.34 (m, 1H), 3.13 (t, *J=* 10.6 Hz, 1H), 2.01-1.89 (m, 1H), 1.66 (s, 6H). |
| 112 | (400 MHz, CDCl₃) δ 8.27 (s, 1H), 7.62 (s, 1H), 5.88 (s, 1H), 5.25 (s, 1H), 4.80 (s, 1H), 4.75-4.62 (m, 2H), 4.35 (br s, 1H), 4.06 (dd, *J =* 11.3, 4.9 Hz, 1H), 4.02-3.94 (m, 1H), 3.89-3.78 (m, 1H), 3.65-3.55 (m, 2H), 3.52-3.41 (m, 2H), 3.20 (t, *J* = 10.6 Hz, 1H), 3.05 (s, 1H), 2.07-1.99 (m, 1H), 1.67 (t, *J =* 6.5 Hz, 6H). |
| 113 | (400 MHz, DMSO-*d*₆) δ 9.86 (s, 1H), 8.58 (s, 1H), 7.83 (d, *J =* 8.8 Hz, 1H), 7.57 (d, *J* = 3.0 Hz, 1H), 7.51 (s, 1H), 6.92 (dd, *J* = 8.9, 3.0 Hz, 1H), 4.89 (p, *J* = 6.8 Hz, 1H), 4.61 (d, *J* = 4.6 Hz, 1H), 3.92 (s, 4H), 3.67 (d, *J =* 13.5 Hz, 1H), 3.57 (d, *J =* 13.4 Hz, 1H), 3.51 (s, 4H), 3.40 (dd, *J =* 9.5, 4.9 Hz, 2H), 3.19 (dd, *J=* 11.8, 8.6 Hz, 2H), 2.70 (d, *J =* 10.7 Hz, 1H), 2.57 *(d, J =* 9.3 Hz, 1H), 1.88 (t*, J =* 10.9 Hz, 1H), 1.75 (d, *J =* 10.3 Hz, 1H), 1.62 (d, *J =* 6.9 Hz, 6H), 1.36 (d, *J =* 12.9 Hz, 1H), 1.06 (d, *J =* 12.9 Hz, 1H). |
| 114 | (400 MHz, MeOH-*d*₄) δ 8.61 (s, 1H), 8.51 (d, *J =* 1.8 Hz, 1H), 8.41 (d, *J =* 8.7 Hz, 1H), 7.98 (dd, *J* = 2.4, 8.8 Hz, 1H), 7.46 (s, 1H), 5.67 (d, *J* = 7.0 Hz, 1H), 3.61 (q, *J =* 6.4 Hz, 4H), 2.03-1.92 (m, 4H), 1.76 (d, *J* = 6.9 Hz, 6H), 1.66 (s, 6H). |
| 115 | (400 MHz, CDCl₃) δ 8.39 (s, 1H), 8.12 (d*, J =* 8.9 Hz, 1H), 7.86 (s, 1H), 7.65-7.44 (m, 2H), 6.86 (dd, *J* = 8.8*,* 3.0 Hz, 1H), 4.86 (p, *J =* 6.9 Hz, 1H), 3.97 (s, 4H), 3.84-3.78 (m, 1H), 3.64 (s, 2H), 3.46 (s, 4H), 2.62-2.53 (m, 1H), 2.49-2.18 (m, 7H), 1.78-1.68 (m, 10H). |
| 116 | (400 MHz, CDCl₃) δ 8.40 (s, 1H), 8.13 (d, *J =* 8.9 Hz, 1H), 7.90 (s, 1H), 7.67-7.47 (m, 2H), 6.86 (dd, *J =* 8.9, 2.9 Hz, 1H), 5.93-5.60 (m, 1H), 4.88 (p, *J =* 6.8 Hz, 1H), 3.98 (s, 4H), 3.92-3.72 (m, 3H), 3.55 (s, 4H), 2.87-2.78 (m, 2H), 2.76-2.34 (m, 5H), 1.91-1.83 (m, 1H), 1.72 (d, *J* = 7.0 Hz, 6H), 1.68-1.55 (m, 3H). |
| 117 | (400 MHz, MeOH-*d*₄) δ 8.33 (s, 1H), 7.55 (s, 1H), 4.85-4.77 (m, 1H), 3.98-3.87 (m, 3H), 3.87-3.70 (m, 2H), 3.63-3.53 (m, 2H), 3.52-3.40 (m, 2H), 3.38-3.32 (m, 1H), 3.25-3.15 (m, 1H), 2.13-2.05 (m, 1H), 2.02-1.78 (m, 2H), 1.74-1.68 (m, 6H), 1.67-1.50 (m, 3H). |
| 118 | (400 MHz, MeOH-*d*₄) δ 8.00 (s, 1H), 7.29 (s, 1H), 6.76 (s, 1H), 5.62 (d, *J* = 6.9 Hz, 1H), 3.96 (dd*, J =* 11.3, 4.9 Hz, 1H), 3.93-3.88 (m, 1H), 3.81 (ddd, *J* = 10.7, 8.9, 4.5 Hz, 1H), 3.57-3.45 (m, 2H), 3.22 (dd, *J* = 11.2, 9.5 Hz, 1H), 2.15-2.06 (m, 1H), 1.73 (d, *J=* 7.0 Hz, 6H), 1.66 (s, 6H), 1.62-1.49 (m, 1H). |
| 119 | (400 MHz, CDCl₃) δ 8.42 (s, 1H), 8.29 (d*, J =* 8.9 Hz, 1H), 8.15 (s, 1H), 7.67-7.55 (m, 2H), 5.36-5.25 (m, 1H), 4.13-4.05 (m, 2H), 3.38-3.25 (m,2H), 3.18 (s, 1H), 2.92 (s, 3H), 2.07-1.88 (m, 2H), 1.72 (d, *J* = 7.0 Hz, 6H), 1.68 (s, 6H). |
| 120 | (400 MHz, DMSO-*d*₆) δ 8.39 (d, *J =* 3.4 Hz, 1H), 7.51 (s, 1H), 7.40 (s, 1H), 4.92 (q, *J* = 5.4 Hz, 2H), 3.79 (d, *J =* 11.4 Hz, 3H), 3.45 (s, 2H), 3.03 (t, *J =* 10.6 Hz, 1H), 2.10 (d, *J=* 11.2Hz, 1H), 1.88 *(d, J=* 13.3 Hz, 1H), 1.67-1.56 (m, 6H), 1.46 *(d, J=* 12.1 Hz, 1H), 0.97 (d, *J* = 8.5 Hz, 2H), 0.90 (d, *J* = 6.8Hz, 2H). |
| 121 | (400 MHz, DMSO-*d*₆) δ 8.41 (s, 1H), 7.52 (s, 1H), 7.43 (s, 1H), 6.40 (s, 1H), 5.29-5.15 (m, 1H), 4.92 (d, *J =* 5.3 Hz, 1H), 3.84-3.73 (m, 3H), 3.51-3.39 (m, 1H), 3.26 (s, 1H), 3.03 (t, *J =* 10.4 Hz, 1H), 1.95-1.82 (m, 1H), 1.63 (d*, J =* 6.9 Hz, 6H), 1.51-1.43 (m, 1H), 1.03 (dt, *J* = 15.1, 2.5 Hz, 4H). |
| 122 | (400 MHz, MeOH-*d*₄) δ 8.58 (s, 1H), 8.28 (d, *J =* 8.6 Hz, 1H), 8.23 (d, *J* = 2.3 Hz, 1H), 7.78 (dd, *J =* 8.6, 2.4 Hz, 1H), 7.50 (s, 1H), 5.12 (p, *J* = 6.9 Hz, 1H), 5.04 (q, *J* = 6.6 Hz, 1H), 3.55 (s, 2H), 2.57 (s, 8H), 2.48 (q, *J =* 7.3 Hz, 2H), 1.76 (dd, *J =* 6.9, 5.1 Hz, 6H), 1.63 (d, *J =* 6.6 Hz, 3H), 1.12 (t, *J =* 7.2 Hz, 3H). |
| 123 | (400 MHz, MeOH-*d*₄) δ 8.30 (s, 1H), 7.43 (s, 1H), 5.08 (q, *J =* 6.9 Hz, 1H), 4.60 (s, 2H), 4.03 (q, *J=* 6.8 Hz, 1H), 3.99-3.86 (m, 3H), 3.68-3.54 (m, 2H), 3.52-3.44 (m, 1H), 3.23-3.17 (m, 1H), 2.92-2.85 (m, 1H), 2.41 (d, *J =* 11.4 Hz, 1H), 2.14-2.04 (m, 1H), 2.08 (t, *J =* 10.1 Hz, 2H), 1.96-1.87 (m, 1H), 1.69 (t, *J =* 7.2 Hz, 6H), 1.64-1.54 (m, 1H), 1.43 (d*, J =* 6.8 Hz, 3H), 1.25-1.13 (m, 1H). |
| 124 | (400 MHz, MeOH-*d*₄) δ 8.30 (s, 1H), 7.43 (s, 1H), 5.03 (p, *J =* 6.9 Hz, 1H), 4.61 (s, 2H), 4.10 (q, *J =* 6.8 Hz, 1H), 3.99-3.86 (m, 3H), 3.62-3.54 (m, 1H), 3.54-3.42 (m, 2H), 3.20 (dd, *J =* 11.1, 9.6 Hz, 1H), 2.69 (d, *J* = 11.1 Hz, 1H), 2.61 (dd, *J =* 10.6, 4.0 Hz, 1H), 2.33 (td, *J =* 11.0, 2.8 Hz, 1H), 2.13-2.05 (m, 1H), 2.01 (t, *J =* 9.8 Hz, 1H), 1.93-1.85 (m, 1H), 1.69 (dd, *J* = 11.1, 6.9 Hz, 6H), 1.63-1.54 (m, 1H), 1.42 (d, *J* = 6.8 Hz, 3H), 1.25-1.14 (m, 1H). |
| 125 | (400 MHz, MeOH-*d*₄) δ 8.30 (s, 1H), 7.40 (s, 1H), 5.75-5.64 (m, 1H), 4.02-3.84 (m, 3H), 3.63-3.53 (m, 1H), 3.52-3.43 (m, 1H), 3.20 (t, *J* = 10.4 Hz, 1H), 2.73 (s, 1H), 2.09 (d, *J* = 13.5 Hz, 1H), 1.71 (t, *J =* 7.6 Hz, 6H), 1.60 (s, 3H), 1.35-1.25 (m, 1H), 0.70-0.60 (m, 1H), 0.60-0.47 (m, 2H), 0.47-0.36 (m, 1H). |
| 127 | (400 MHz, DMSO-*d*₆) δ 8.36 (s, 1H), 8.15 (s, 1H), 7.44 (s, 1H), 4.91 (d, *J =* 5.3 Hz, 1H), 3.78 (d, *J* = 5.8 Hz, 7H), 3.44 (d, *J* = 9.8Hz, 1H), 3.02 (t, *J* = 10.4 Hz, 1H), 1.89 (d*, J =* 12.7 Hz, 1H), 1.39 (s, 9H) |
| 128 | (400 MHz, DMSO-*d*₆) δ 8.38 (s, 1H), 8.23 (s, 1H), 7.47 (s, 1H), 6.38 (t, *J =* 8.0Hz, 1H), 4.92 (d, *J =* 5.3 Hz, 1H), 4.61 (t*, J =* 15.0Hz, 2H), 3.80 (dd, *J* = 12.6, 6.7 Hz, 3H), 3.45 (t, *J =* 9.7Hz, 1H), 3.12 (p, *J =* 6.9 Hz, 1H), 3.03 (t, *J =* 10.4 Hz, 1H), 1.88 (dd, *J =* 13.6, 4.3 Hz, 1H), 1.49-1.44 (m, 1H), 1.30 (d, *J =* 6.9 Hz, 6H). |
| 129 | (400 MHz, DMSO-*d*₆) δ 8.42 (s, 1H), 7.58 (s, 1H), 7.47 (s, 1H), 5.21 (d, *J* = 4.1 Hz, 1H), 4.94-4.80 (m, 1H), 4.62 (d, *J =* 4.8 Hz, 1H), 3.73 (s, 1H), 3.66 (d, *J =* 13.4 Hz, 1H), 3.62-3.52 (m, 3H), 3.47 (d, *J =* 11.7 Hz, 1H), 3.40 (dt, *J* = 9.5*,* 4.7 Hz, 1H), 3.30 (d, *J =* 9.8 Hz, 1H), 2.92-2.81 (m, 4H), 2.66 (dt, *J =* 12.7, 9.8 Hz, 2H), 2.57 (d, *J =* 10.7 Hz, 2H), 1.98 (dd, *J =* 13.8, 4.0 Hz, 1H), 1.93-1.83 (m, 1H), 1.75 (dt, *J =* 20.0, 9.9 Hz, 2H), 1.59 (d, *J =* 6.8 Hz, 6H), 1.35 (d, *J =* 12.7 Hz, 1H), 1.12-1.00 (m, 1H). |
| 130 | (400 MHz, MeOH-*d*₄) δ 8.25 (s, 1H), 7.56 (s, 1H), 3.97-3.86 (m, 3H), 3.70 (s, 3H), 3.59-3.53 (m, 1H), 3.51-3.44 (m, 1H), 3.21-3.16 (m, 1H), 2.99 (s, 6H), 2.11-2.04 (m, 1H), 1.63-1.53 (m, 1H). |
| 131 | (400 MHz, MeOH-*d*₄) δ 8.20 (s, 1H), 7.38 (s, 1H), 4.71-4.66 (m, 1H), 3.8-3.76 (m, 3H), 3.52-3.44 (m, 1H), 3.42-3.34 (m, 1H), 3.10 (d, *J =* 1.5 Hz, 1H), 2.88 (s, 2H), 2.64 (br s, 1H), 2.04-1.96 (m, 1H), 1.77-1.68 (m, 2H), 1.64-1.55 (m, 9H), 1.55-1.46 (m, 3H). |
| 132 | (400 MHz, DMSO-*d*₆) δ 8.38 (s, 1H), 7.47 (s, 1H), 7.34 (s, 1H), 4.96-4.89 (m, 1H), 4.49-4.41 (m, 3H), 3.80 (dd, *J =* 12.1, 6.1 Hz, 3H), 3.44 (d, *J =* 9.4Hz, 1H), 3.03 (t, *J =* 10.4 Hz, 1H), 2.32 (q, *J* = 7.2 Hz, 1H), 1.99-1.92 (m, 1H), 1.88 (d, *J* = 12.4 Hz, 1H), 1.54 (d, *J =* 6.5 Hz, 3H), 1.49-1.40 (m, 1H). |
| 133 | (400 MHz, MeOH-*d*₄) δ 8.28 (s, 1H), 7.73 (s, 1H), 6.14 (td, *J =* 55.5, 3.2 Hz, 1H), 5.69 (s, 1H), 4.59 (s, 1H), 4.00-3.83 (m, 3H), 3.61-3.52 (m, 1H), 3.52-3.43 (m, 1H), 3.19 (t, *J =* 10.4 Hz, 1H), 2.14-2.02 (m, 1H), 1.63 (s, 6H), 1.56 (d, *J =* 7.0 Hz, 3H). |
| 134 | (400 MHz, MeOH-*d*₄) δ 8.31 (d, *J =* 15.2 Hz, 1H), 8.08-7.81 (m, 1H), 7.80-7.49 (m, 1H), 6.17 (qd, *J* = 55.1, 3.2 Hz, 1H), 5.05-4.93(m, 1H), 4.60 (s, 1H), 4.00-3.86 (m, 3H), 3.62-3.53 (m, 1H), 3.48 (t, *J =* 11.6 Hz, 1H), 3.20 (t, *J* = 10.4 Hz, 1H), 2.13-2.01 (m, 1H), 1.76-1.61 (m, 3H). |
| 135 | (400 MHz, DMSO-*d*₆) δ 8.33 (s, 1H), 7.62 (s, 1H), 7.34 (d, *J* = 35.9 Hz, 1H), 4.91 (s, 1H), 4.35 (s, 1H), 3.78 (s, 3H), 3.00 (s, 5H), 1.89 (s, 2H), 1.41 (s, 4H), 1.27 (s, 3H). |
| 136 | (400 MHz, DMSO-*d*₆) δ 8.31 (s, 1H), 7.91 (d, *J =* 1.0 Hz, 1H), 7.55 (d*, J =* 1.0 Hz, 1H), 3.99-3.86 (m, 3H), 3.62-3.53 (m, 1H), 3.52-3.44 (m, 1H), 3.23-3.16 (m, 1H), 2.12-2.04 (m, 1H), 1.80 (s, 9H), 1.65-1.57 (m, 1H). |
| 137 | (400 MHz, DMSO-*d*₆) δ 10.25 (s, 1H), 8.69 (s, 1H), 8.18 (d, *J =* 2.3 Hz, 1H), 8.09 (d, *J* = 8.6 Hz, 1H), 7.68 (dd, *J* = 8.5, 2.4 Hz, 1H), 7.54 (s, 1H), 4.96-4.83 (m, 1H), 4.62 (d, *J =* 4.5 Hz, 1H), 3.43 (s, 2H), 3.44-3.35 (m, 1H), 2.71 (d, *J =* 10.7 Hz, 1H), 2.58 (d, *J =* 10.9 Hz, 1H), 2.43-2.16 (m, 10H), 1.89 (t, *J* = 10.9 Hz, 1H), 1.80-1.71 (m, 1H), 1.63 (d, *J=* 6.9 Hz, 6H), 1.64-1.57 (m, 2H), 1.42-1.30 (m, 1H), 1.13-1.01 (m, 1H), 0.97 (t, *J* = 7.1 Hz, 3H). |
| 138/138-(R)/138-(S) | (400 MHz, DMSO-*d*₆) δ 8.41 (s, 1H), 7.52 (s, 1H), 7.47 (s, 1H), 5.52 (s, 1H), 5.05-4.95 (m, 1H), 4.92 (d, *J =* 5.3 Hz, 1H), 3.87-3.71 (m, 3H), 3.50-3.40 (m, 1H), 3.37-3.28 (m, 1H), 3.03 (t, *J =* 10.4 Hz, 1H), 1.94-1.83 (m, 1H), 1.61 (d*, J =* 6.9 Hz, 6H), 1.49 (s, 3H), 1.50-1.42 (m, 1H). |

### Biological Assays and Evaluation

### I. In Vitro Biochemical Kinase Activity Assay for CDK2/Cyclin E1

The inhibitory effects of the compounds of the present invention on CDK2/cyclin E1 kinase activity were determined using a homogeneous time-resolved fluorescence (HTRF) assay. The experimental process was as follows:
**1.** The kinase reaction was carried out in a 384-well plate. 15 nM CDK2/cyclin E1 kinase (Carna), 1 µM ATP, and 1 µM biotin-labeled substrate protein ATF2-biotin (Cisbio) were incubated at room temperature for 90 min in a buffer system composed of 10 mM MgCl₂, 1 mM MnCl₂, 1 mM DTT, and basal kinase buffer (Cisbio).
**2.** The detection solution (Cisbio, containing reagents that specifically recognize phosphorylated ATF2 and ATF2-biotin proteins, respectively) was added. The plate was then sealed with a film (to protect from light and volatilization) and incubated at 25°C for 60 min.
**3.** The plate was transferred to an Envision plate reader to detect fluorescence resonance energy transfer (FRET) signals. (The 665/615 ratio was calculated by reading the emission at 665 nM and 615 nM, respectively.)
**4.** The IC₅₀ values were determined by analyzing the inhibition at varying compound concentrations using Model 205 in the XLfit software. The enzymatic activities of the specific exemplary compounds are shown in Table 1.

### II. In Vitro Biochemical Kinase Activity Assay for CDK4/Cyclin D3

The inhibitory effects of the compounds of the present invention on CDK4/cyclin D3 kinase activity were determined using an HTRF assay. The experimental process was as follows:
**1.** The kinase reaction was carried out in a 384-well plate. 4.9 nM CDK4/cyclin D3 (Carna), 1 mM (or 80 µM) ATP, and 0.3 µM biotin-labeled substrate protein Rb-biotin (Cisbio) were incubated at 30°C for 90 min in a buffer system composed of 50 mM MgCl₂, 1 mM DTT, and basal kinase buffer (Cisbio).
**2.** The detection solution (Cisbio, containing reagents that specifically recognize phosphorylated Rb and Rb-biotin proteins, respectively) was added. The plate was then sealed with a film (to protect from light and volatilization) and incubated at 25°C for 60 min.
**3.** The plate was transferred to an Envision plate reader to detect FRET signals. (The 665/615 ratio was calculated by reading the emission at 665 nM and 615 nM, respectively.)
**4.** The IC₅₀ values were determined by analyzing the inhibition at varying compound concentrations using Model 205 in the XLfit software. The enzymatic activities of the specific exemplary compounds are shown in Table 1.

### III. In Vitro Biochemical Kinase Activity Assay for CDK6/Cyclin D3

The inhibitory effects of the compounds of the present invention on CDK6/cyclin D3 kinase activity were determined using an HTRF assay. The experimental process was as follows:
**1.** The kinase reaction was carried out in a 384-well plate. 0.02 nM CDK6/cyclin D3 (Carna), 1 mM (or 100 µM) ATP, and 0.3 µM biotin-labeled substrate protein Rb-biotin (Cisbio) were incubated at 30°C for 60 min in a buffer system composed of 5 mM MgCl₂, 1 mM DTT, and basal kinase buffer (Cisbio).
**2.** The detection solution (Cisbio, containing reagents that specifically recognize phosphorylated Rb and Rb-biotin proteins, respectively) was added. The plate was then sealed with a film (to protect from light and volatilization) and incubated at 25°C for 60 min.
**3.** The plate was transferred to an Envision plate reader to detect FRET signals. (The 665/615 ratio was calculated by reading the emission at 665 nM and 615 nM, respectively.)
**4.** The IC₅₀ values were determined by analyzing the inhibition at varying compound concentrations using Model 205 in the XLfit software. The enzymatic activities of the specific exemplary compounds are shown in the table below.

**Table 1. Activity Test Results of Compounds (Km ATP)**

| **Example No.** | **CDK2 IC₅₀ (nM)** | **CDK4 IC₅₀ (nM)** | **CDK6 IC₅₀ (nM)** |
|---|---|---|---|
| 1 | 3632.6 | 6.2 | 24.2 |
| 2 | 1323.7 | 1.4 | 12.7 |
| 3 | 467.48 | 238.44 | 518.41 |
| 24 | 235.51 | 0.56 | 3.04 |
| 30 | 125.08 | 1.14 | 11.45 |
| 31 | 590.44 | 7.85 | 47.07 |
| 35 | 538.99 | 18.96 | 131.19 |
| 40 | 1973.71 | 30.77 | 298.80 |
| 41 | 1853.58 | 1.83 | 10.95 |
| 33 | NT | 1.35* | 10.34* |
| 38 | NT | 171.96* | 2115.92* |
| 52 | 2926.35 | 6.22 | 24.21 |
| 53 | 493.16 | 60.89 | 191.97 |
| 54 | 10000 | 59.00 | 164.42 |
| 55 | 2115.19 | 0.33 | 1.90 |
| 56 | 1370.62 | 3.07 | 49.02 |
| 57 | 2828.01 | 0.68 | 3.70 |
| 58 | 10000 | 10000* | 10000* |
| 59 | 10000 | 9.64 | 38.32 |
| 60 | 10000 | 47.64 | 161.54 |
| 61 | 617.26 | 0.94 | 8.46 |
| 62 | 129.07 | 0.36 | 4.86 |
| 63 | 692.59 | 23.80 | 129.90 |
| 64 | NT | 13.15 | 131.97 |
| 65 | NT | 118.83 | 1204.49 |
| 66 | NT | 1.03 | 1.90 |
| 67 | NT | 1.17 | 5.88 |
| 68 | NT | 76.75* | 2597.67* |
| 69 | NT | 4.71* | 38.41* |
| 70 | NT | 15.06* | 130.56* |
| 71 | NT | 2.52* | 6.31* |
| 72 | NT | 16.98* | 262.81* |
| 73 | NT | 6.43* | 26.75* |
| 74 | NT | 20.13* | 341.47* |
| 75 | NT | 21.20* | 1164.62* |
| 76 | NT | 2.90* | 6.53* |
| 77 | NT | 1.62* | 5.94* |
| 78 | NT | 75.52* | 392.82* |
| 79 | NT | 17.17* | 179.12* |
| 80 | NT | 2.65* | 12.05* |
| 81 | NT | 9.86* | 59.10* |
| 82 | NT | 3.25* | 29.47* |
| 83 | NT | 2860.87* | 10000 |
| 84 | NT | 7.44* | 48.88* |
| 85 | NT | 10000* | 10000* |
| 86 | NT | 479.99 | 1970.52 |
| 87 | NT | 3969.16* | 9360.63* |
| 88 | NT | 12.56* | 51.11 |
| 89 | NT | 16.20* | 44.92* |
| 90 | NT | 22.99* | 252.52* |
| 91 | NT | 88.16* | 332.68* |
| 92 | NT | 99.72* | 520.49* |
| 93 | NT | 7.28* | 33.68* |
| 94 | NT | 5.53* | 17.64* |
| 95 | NT | 116.67* | 828.78* |
| 96 | NT | 3286.73* | 10000 |
| 97 | NT | 10000* | 10000* |
| 98 | NT | 8.08* | 197.77* |
| 99 | NT | 28.06* | 190.46* |
| 100 | NT | 6.69* | 25.89* |
| 101 | NT | 786.34* | 4765.90* |
| 102 | NT | 1041.48* | 10000 |
| 103 | NT | 11.95* | 256.12* |
| 104 | NT | 5.57* | 45.17* |
| 105 | NT | 26.36* | 412.69* |
| 106 | NT | 33.25* | 280.38* |
| 107 | NT | 8.86* | 115.88* |
| 108 | NT | 5.25* | 51.95* |
| 109 | 3189.72 | 4.50 | 223.32 |
| 110 | NT | 36.08* | 451.40* |
| 111 | NT | 47.09* | 335.23* |
| 112 | NT | 179.14* | 1237.85* |
| 113 | NT | 5.95* | 45.58* |
| 114 | NT | 7.99* | 28.36* |
| 115 | NT | 2.28* | 17.94* |
| 116 | NT | 5.70* | 87.84* |
| 117 | NT | 615.12* | 5552.49* |
| 118 | NT | 21.67* | 135.32* |
| 119 | NT | 5.37* | 15.38* |
| 120 | NT | 26.15* | 130.06* |
| 121 | NT | 9.24* | 75.88* |
| 122 | NT | 2.07* | 7.84* |
| 123 | NT | 36.93* | 544.56* |
| 124 | NT | 181.54* | 1155.43* |
| 125 | NT | 11.62* | 65.41* |
| 126 | NT | 17.01* | 75.82* |
| 127 | NT | 136.59* | 455.08* |
| 128 | NT | 53.26* | 253.98* |
| 129 | NT | 1.48* | 21.00* |
| 130 | NT | 23.53* | 222.43* |
| 131 | NT | 23.19* | 684.23* |
| 132 | NT | 93.74* | 242.52* |
| 133 | NT | 259.80* | 1137.13* |
| 134 | NT | 36.13* | 230.36* |
| 135 | NT | 177.47* | 275.37* |
| 136 | NT | 10.34 | 39.31 |
| 137 | NT | 3.17* | 53.05* |
| 138 | NT | 5.92* | 38.38* |
| Remarks | ● "NT" means "not tested". | | |
| | ● Data marked with "*" indicate CDK4 or CDK6 IC₅₀ values measured at 1 mM ATP in Step 1. Unmarked data indicate CDK4 or CDK6 IC₅₀ values measured at 80 µM ATP or 100 µM ATP in Step 1. | | |

Based on the biological activity data of the compounds in the above specific examples, the compounds of the present invention exhibit potent inhibition on CDK4 kinase, and some compounds demonstrate high selectivity for CDK2 and/or CDK6 kinase.

All literatures mentioned in the present invention are incorporated herein by reference as if individually incorporated by reference. In addition, it should be understood that those skilled in the art can make various alterations or modifications to the present invention after reading the above disclosure of the present invention, and these equivalent forms shall also fall within the scope defined by the claims appended to the present invention.

## Claims

1. A compound of formula (I), a stereoisomer or pharmaceutically acceptable salt thereof: 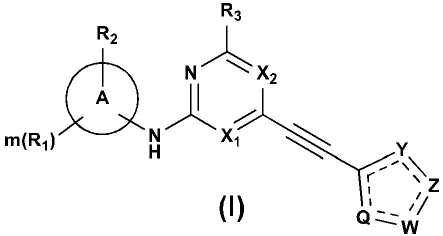
wherein, "-̅ -̅ -̅"represents a double or single bond;
X₁ is CR₄ or N; X₂ is CR₅ or N;
Y is N, CR₆ₐ, NR_{6b}, CR_{6c}R_{6d}, O or S;
Z is N, CR₇ₐ, NR_{7b}, CR_{7c}R_{7d}, O or S;
W is N, CR₈ₐ, NR_{8b}, CR_{8c}R_{8d}, O or S;
Q is N, CR₉ₐ, NR_{9b}, CR_{9c}R_{9d}, O or S;
ring A is C₃₋₁₀ cycloalkyl, 3-10 membered heterocyclyl, C₆₋₁₀ aryl or 5-10 membered heteroaryl;
each R₁ is independently selected from the group consisting of hydrogen, deuterium, halogen, cyano, nitro, azido, C₁₋₁₀ alkyl, C₂₋₁₀ alkenyl, C₂₋₁₀ alkynyl, C₃₋₁₀ cycloalkyl, 3-10 membered heterocyclyl, C₆₋₁₀ aryl, 5-10 membered heteroaryl, C₃₋₁₀ cycloalkyl-substituted C₁₋₄ alkyl, 3-10 membered heterocyclyl-substituted C₁₋₄ alkyl, C₆₋₁₀ aryl-substituted C₁₋₄ alkyl, 5-10 membered heteroaryl-substituted C₁₋₄ alkyl, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅, the above groups are independently optionally further substituted with one or more substituents selected from the group consisting of deuterium, halogen, cyano, nitro, azido, C₁₋₁₀ alkyl, C₂₋₁₀ alkenyl, C₂₋₁₀ alkynyl, C₃₋₁₀ cycloalkyl, 3-10 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, - C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅, the above groups are independently optionally more further substituted with one or more substituents selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₀ cycloalkyl, 3-10 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkylS(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, - C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅;
R₂ is selected from the group consisting of hydrogen, deuterium, halogen, cyano, nitro, azido, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkylS(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, - C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅, provided that R₂ is not hydrogen when ring A is C₃₋₆ cycloalkyl or 3-6 membered heterocyclyl;
R₃ is selected from the group consisting of hydrogen, deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₀ cycloalkyl, 3-10 membered heterocyclyl, C₆₋₁₀ aryl, 5-10 membered heteroaryl, -C₀₋₈ alkyl-SF₅, -C₀₋₈ alkylS(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅;
R₄ is selected from the group consisting of hydrogen, deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₀ cycloalkyl, 3-10 membered heterocyclyl, C₆₋₁₀ aryl, 5-10 membered heteroaryl, -C₀₋₈ alkyl-SF₅, -C₀₋₈ alkylS(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅;
R₅ is selected from the group consisting of hydrogen, deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₀ cycloalkyl, 3-10 membered heterocyclyl, C₆₋₁₀ aryl, 5-10 membered heteroaryl, -C₀₋₈ alkyl-SF₅, -C₀₋₈ alkylS(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅;
R₆ₐ, R₇ₐ, R₈ₐ and R₉ₐ are each independently selected from the group consisting of hydrogen, deuterium, halogen, cyano, nitro, azido, C₁₋₁₀ alkyl, C₂₋₁₀ alkenyl, C₂₋₁₀ alkynyl, C₃₋₁₀ cycloalkyl, 3-10 membered heterocyclyl, C₆₋₁₀ aryl, 5-10 membered heteroaryl, -C₀₋₈ alkyl-SF₅, -C₀₋₈ alkylS(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅, the above groups are independently optionally further substituted with one or more substituents selected from the group consisting of deuterium, halogen, cyano, nitro, azido, C₁₋₁₀ alkyl, C₂₋₁₀ alkenyl, C₂₋₁₀ alkynyl, C₃₋₁₀ cycloalkyl, 3-10 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, - C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅, the above groups are independently optionally further substituted with one or more substituents selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₀ cycloalkyl, 3-10 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅;
R_{6b}, R_{7b}, R_{8b} and R_{9b} are each independently selected from the group consisting of hydrogen, deuterium, C₁₋₁₀ alkyl, C₂₋₁₀ alkenyl, C₂₋₁₀ alkynyl, C₃₋₁₀ cycloalkyl, 3-10 membered heterocyclyl, C₆₋₁₀ aryl, 5-10 membered heteroaryl, -C₀₋₈ alkyl-S(O)(=N-R₁₀)R₁₁, -C₀₋₈ alkyl-S(O)ᵣR₁₃, -C₀₋₈ alkyl-C(O)OR₁₄, -C₀₋₈ alkyl-C(O)SR₁₄, -C₀₋₈ alkyl-C(O)R₁₅, -C₀₋₈ alkyl-P(O)(R₁₅)₂, -C₀₋₈ alkyl-C(=NR₁₆)R₁₅ and -C₀₋₈ alkyl-C(O)NR₁₆R₁₇, the above groups are independently optionally further substituted with one or more substituents selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₀ cycloalkyl, 3-10 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅;
or, R_{6b} and R₇ₐ together with the moiety to which they are directly attached form 3-10 membered heterocyclyl or 5-10 membered heteroaryl, the above 3-10 membered heterocyclyl or 5-10 membered heteroaryl is independently optionally further substituted with one or more substituents selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₀ cycloalkyl, 3-10 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, - C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅;
or, R₆ₐ and R_{7b} together with the moiety to which they are directly attached form 3-10 membered heterocyclyl or 5-10 membered heteroaryl, the above 3-10 membered heterocyclyl or 5-10 membered heteroaryl is independently optionally further substituted with one or more substituents selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₀ cycloalkyl, 3-10 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, - C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅;
R_{6c}, R_{6d}, R_{7c}, R_{7d}, R_{8c}, R_{8d}, R_{9c} and R_{9d} are each independently selected from the group consisting of hydrogen, deuterium, halogen, cyano, nitro, azido, C₁₋₁₀ alkyl, C₂₋₁₀ alkenyl, C₂₋₁₀ alkynyl, C₃₋₁₀ cycloalkyl, 3-10 membered heterocyclyl, C₆₋₁₀ aryl, 5-10 membered heteroaryl, - C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, - C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅, the above groups are independently optionally further substituted with one or more substituents selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₀ cycloalkyl, 3-10 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₈, -C₀₋₈ alkyl-C(O)NR₁₆R₁₇ and -C₀₋₈ alkyl-N(R₁₆)-C(O)R₁₅;
each R₁₀ is independently selected from the group consisting of hydrogen, deuterium, C₁₋₁₀ alkyl, C₂₋₁₀ alkenyl, C₂₋₁₀ alkynyl, C₃₋₁₀ cycloalkyl, 3-10 membered heterocyclyl, C₆₋₁₀ aryl, 5-10 membered heteroaryl, -C₀₋₈ alkyl-S(O)ᵣR₁₃, -C₀₋₈ alkyl-C(O)OR₁₄, -C₀₋₈ alkyl-C(O)R₁₅ and -C₀₋₈ alkyl-C(O)NR₁₆R₁₇, the above groups are optionally further substituted with one or more substituents selected from the group consisting of deuterium, halogen, cyano, nitro, azido, C₁₋₁₀ alkyl, C₂₋₁₀ alkenyl, C₂₋₁₀ alkynyl, C₁₋₁₀ haloalkyl, C₁₋₁₀ deuterioalkyl, C₃₋₁₀ cycloalkyl, 3-10 membered heterocyclyl, C₆₋₁₀ aryl, 5-10 membered heteroaryl, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)R₁₅, -C₀₋₈ alkyl-O-C(O)R₁₅, -C₀₋₈ alkyl-NR₁₆R₁₇, - C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅;
each R₁₁ and each R₁₂ are independently selected from the group consisting of hydrogen, deuterium, hydroxyl, C₁₋₁₀ alkyl, C₂₋₁₀ alkenyl, C₂₋₁₀ alkynyl, C₃₋₁₀ cycloalkyl, 3-10 membered heterocyclyl, C₆₋₁₀ aryl and 5-10 membered heteroaryl, or R₁₁ and R₁₂ together with a sulfur atom to which they are directly attached form 3-10 membered heterocyclyl, the above groups are optionally further substituted with one or more substituents selected from the group consisting of deuterium, halogen, cyano, nitro, azido, C₁₋₁₀ alkyl, C₂₋₁₀ alkenyl, C₂₋₁₀ alkynyl, C₁₋₁₀ haloalkyl, C₁₋₁₀ deuterioalkyl, C₃₋₁₀ cycloalkyl, 3-10 membered heterocyclyl, C₆₋₁₀ aryl, 5-10 membered heteroaryl, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)R₁₅, - C₀₋₈ alkyl-O-C(O)R₁₅, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅;
each R₁₃ is independently selected from the group consisting of hydrogen, deuterium, hydroxyl, C₁₋₁₀ alkyl, C₂₋₁₀ alkenyl, C₃₋₁₂ cycloalkyl, 3-12 membered heterocyclyl, C₆₋₁₀ aryl, 5-10 membered heteroaryl and -NR₁₆R₁₇, the above groups are independently optionally further substituted with one or more substituents selected from the group consisting of deuterium, halogen, cyano, hydroxyl, =O, C₁₋₁₀ alkyl, C₁₋₁₀ alkoxyl, C₃₋₁₂ cycloalkyl, C₃₋₁₂ cycloalkoxyl, 3-12 membered heterocyclyl, 3-12 membered heterocycloxyl, C₆₋₁₀ aryl, C₆₋₁₀ aryloxyl, 5-10 membered heteroaryl, 5-10 membered heteroaryloxyl and -NR₁₆R₁₇;
each R₁₄ is independently selected from the group consisting of hydrogen, deuterium, C₁₋₁₀ alkyl, C₂₋₁₀ alkenyl, C₃₋₁₂ cycloalkyl, 3-12 membered heterocyclyl, C₆₋₁₀ aryl and 5-10 membered heteroaryl, the above groups are independently optionally further substituted with one or more substituents selected from the group consisting of deuterium, halogen, hydroxyl, =O, cyano, C₁₋₁₀ alkyl, C₁₋₁₀ alkoxyl, C₃₋₁₂ cycloalkyl, C₃₋₁₂ cycloalkoxyl, 3-12 membered heterocyclyl, 3-12 membered heterocycloxyl, C₆₋₁₀ aryl, C₆₋₁₀ aryloxyl, 5-10 membered heteroaryl, 5-10 membered heteroaryloxyl and -NR₁₆R₁₇;
each R₁₅ is independently selected from the group consisting of hydrogen, deuterium, hydroxyl, C₁₋₁₀ alkyl, C₁₋₁₀ alkoxyl, C₂₋₁₀ alkenyl, C₂₋₁₀ alkynyl, C₃₋₁₂ cycloalkyl, C₃₋₁₂ cycloalkoxyl, 3-12 membered heterocyclyl, 3-12 membered heterocycloxyl, C₆₋₁₀ aryl, C₆₋₁₀ aryloxyl, 5-10 membered heteroaryl, 5-10 membered heteroaryloxyl and -NR₁₆R₁₇, the above groups are independently optionally further substituted with one or more substituents selected from the group consisting of deuterium, halogen, hydroxyl, =O, cyano, C₁₋₁₀ alkyl, C₁₋₁₀ alkoxyl, C₃₋₁₂ cycloalkyl, C₃₋₁₂ cycloalkoxyl, 3-12 membered heterocyclyl, 3-12 membered heterocycloxyl, C₆₋₁₀ aryl, C₆₋₁₀ aryloxyl, 5-10 membered heteroaryl, 5-10 membered heteroaryloxyl and -NR₁₆R₁₇;
each R₁₆ and each R₁₇ are independently selected from the group consisting of hydrogen, deuterium, hydroxyl, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, sulfinyl, sulfonyl, methylsulfonyl, isopropylsulfonyl, cyclopropylsulfonyl, p-toluenesulfonyl, aminosulfonyl, dimethylaminosulfonyl and C₁₋₁₀ alkanoyl, the above groups are independently optionally further substituted with one or more substituents selected from the group consisting of deuterium, halogen, hydroxyl, =O, C₁₋₁₀ alkyl, C₂₋₁₀ alkenyl, C₂₋₁₀ alkynyl, C₁₋₁₀ haloalkyl, C₁₋₁₀ deuterioalkyl, C₁₋₁₀ alkoxyl, C₃₋₁₂ cycloalkyl, C₃₋₁₂ cycloalkoxyl, 3-12 membered heterocyclyl, 3-12 membered heterocycloxyl, C₆₋₁₀ aryl, C₆₋₁₀ aryloxyl, 5-10 membered heteroaryl, 5-10 membered heteroaryloxyl, amino, mono(C₁₋₁₀ alkyl)amino, di(C₁₋₁₀ alkyl)amino and C₁₋₁₀ alkanoyl;
or, R₁₆ and R₁₇ together with a nitrogen atom to which they are directly attached form 4-10 membered heterocyclyl or 5-10 membered heteroaryl, the above 4-10 membered heterocyclyl or 5-10 membered heteroaryl is optionally further substituted with one or more substituents selected from the group consisting of deuterium, halogen, hydroxyl, =O, C₁₋₁₀ alkyl, C₂₋₁₀ alkenyl, C₂₋₁₀ alkynyl, C₁₋₁₀ haloalkyl, C₁₋₁₀ deuterioalkyl, C₁₋₁₀ alkoxyl, C₃₋₁₂ cycloalkyl, C₃₋₁₂ cycloalkoxyl, 3-12 membered heterocyclyl, 3-12 membered heterocycloxyl, C₆₋₁₀ aryl, C₆₋₁₀ aryloxyl, 5-10 membered heteroaryl, 5-10 membered heteroaryloxyl, amino, mono(C₁₋₁₀ alkyl)amino, di(C₁₋₁₀ alkyl)amino and C₁₋₁₀ alkanoyl;
m is 0, 1, 2 or 3; and
each r is independently 0, 1 or 2.

2. The compound of formula (I) according to claim 1, the stereoisomer or pharmaceutically acceptable salt thereof, wherein, each R₁ is independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, C₃₋₆ cycloalkyl-substituted C₁₋₄ alkyl, 3-6 membered heterocyclyl-substituted C₁₋₄ alkyl, C₆₋₈ aryl-substituted C₁₋₄ alkyl, 5-8 membered heteroaryl-substituted C₁₋₄ alkyl, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, - C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅, the above groups are independently optionally further substituted with one or more substituents selected from the group consisting of deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₅, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅, the above groups are independently optionally more further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅;
R₂ is selected from the group consisting of hydrogen, deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, **-O**R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, - C(=NR₁₆)R₁₅, -N(R₁₆)-C(=NR₁₇)R₁₅, -C(O)NR₁₆R₁₇ and -N(R₁₆)-C(O)R₁₅, provided that R₂ is not hydrogen when ring A is C₃₋₆ cycloalkyl or 3-6 membered heterocyclyl;
R₃ is selected from the group consisting of hydrogen, deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-SF₅, -C₀₋₄ alkyl-S(O)(=N-R₁₀)R₁₁, - C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, - C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅;
R₄ is selected from the group consisting of hydrogen, deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-SF₅, -C₀₋₄ alkyl-S(O)(=N-R₁₀)R₁₁, - C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, - C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅;
R₅ is selected from the group consisting of hydrogen, deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-SF₅, -C₀₋₄ alkyl-S(O)(=N-R₁₀)R₁₁, - C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, - C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅;
R₆ₐ, R₇ₐ, R₈ₐ and R₉ₐ are each independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-SF₅, -C₀₋₄ alkyl-S(O)(=N-R₁₀)R₁₁, - C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, - C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅, the above groups are independently optionally further substituted with one or more substituents selected from the group consisting of deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅, the above groups are independently optionally more further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅;
R_{6b}, R_{7b}, R_{8b} and R_{9b} are each independently selected from the group consisting of hydrogen, deuterium, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-S(O)(=N-R₁₀)R₁₁, -C₀₋₄ alkyl-S(O)ᵣR₁₃, -C₀₋₄ alkyl-C(O)OR₁₄, -C₀₋₄ alkyl-C(O)SR₁₄, -C₀₋₄ alkyl-C(O)R₁₅, -C₀₋₄ alkyl-P(O)(R₁₅)₂, -C₀₋₄ alkyl-C(=NR₁₆)R₁₅ and -C₀₋₄ alkyl-C(O)NR₁₆R₁₇, the above groups are independently optionally further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₅, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅;
or, R_{6b} and R₇ₐ together with the moiety to which they are directly attached form 3-8 membered heterocyclyl or 5-8 membered heteroaryl, the above 3-8 membered heterocyclyl or 5-8 membered heteroaryl is independently optionally further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkylS(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, - C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅;
or, R₆ₐ and R_{7b} together with the moiety to which they are directly attached form 3-8 membered heterocyclyl or 5-8 membered heteroaryl, the above 3-8 membered heterocyclyl or 5-8 membered heteroaryl is independently optionally further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkylS(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, - C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅;
wherein, R₁₀, R₁₁, R₁₂, R₁₃, R₁₄, R₁₅, R₁₆, R₁₇ and r are defined as in claim 1.

3. The compound of formula (I) according to claim 1, the stereoisomer or pharmaceutically acceptable salt thereof, wherein, each R₁₀ is independently selected from the group consisting of hydrogen, deuterium, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-S(O)ᵣR₁₃, -C₀₋₄ alkyl-C(O)OR₁₄, -C₀₋₄ alkyl-C(O)R₁₅ and -C₀₋₄ alkyl-C(O)NR₁₆R₁₇, the above groups are independently optionally further substituted with one or more substituents selected from the group consisting of deuterium, halogen, cyano, C₁₋₄ alkyl, C₂₋₄ alkenyl, C₂₋₄ alkynyl, C₁₋₄ haloalkyl, C₁₋₄ deuterioalkyl, C₃₋₆ cycloalkyl, 3-6 membered heterocyclyl, C₆₋₈ aryl, 5-8 membered heteroaryl, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)R₁₅, -C₀₋₄ alkyl-O-C(O)R₁₅, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅;
each R₁₁ and each R₁₂ are independently selected from the group consisting of hydrogen, deuterium, hydroxyl, C₁₋₄ alkyl, C₂₋₄ alkenyl, C₂₋₄ alkynyl, C₃₋₆ cycloalkyl, 3-6 membered heterocyclyl, C₆₋₈ aryl and 5-8 membered heteroaryl, or R₁₁ and R₁₂ together with a sulfur atom to which they are directly attached form 3-8 membered heterocyclyl, the above groups are optionally further substituted with one or more substituents selected from the group consisting of deuterium, halogen, cyano, C₁₋₄ alkyl, C₂₋₄ alkenyl, C₂₋₄ alkynyl, C₁₋₄ haloalkyl, C₁₋₄ deuterioalkyl, C₃₋₆ cycloalkyl, 3-6 membered heterocyclyl, C₆₋₈ aryl, 5-8 membered heteroaryl, -C₀₋₄ alkylS(O)ᵣR₁₃, -C₀₋₄ alkyl-O-R₁₄, -C₀₋₄ alkyl-C(O)OR₁₄, -C₀₋₄ alkyl-C(O)R₁₅, -C₀₋₄ alkyl-O-C(O)R₁₅, - C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅;
each R₁₃ is independently selected from the group consisting of hydrogen, deuterium, hydroxyl, C₁₋₄ alkyl, C₂₋₄ alkenyl, C₃₋₆ cycloalkyl, 3-6 membered heterocyclyl, C₆₋₈ aryl, 5-8 membered heteroaryl and -NR₁₆R₁₇, the above groups are independently optionally further substituted with one or more substituents selected from the group consisting of deuterium, halogen, cyano, hydroxyl, =O, C₁₋₄ alkyl, C₁₋₄ alkoxyl, C₃₋₆ cycloalkyl, C₃₋₆ cycloalkoxyl, 3-6 membered heterocyclyl, 3-6 membered heterocycloxyl, C₆₋₈ aryl, C₆₋₈ aryloxyl, 5-8 membered heteroaryl, 5-8 membered heteroaryloxyl and -NR₁₆R₁₇;
each R₁₄ is independently selected from the group consisting of hydrogen, deuterium, C₁₋₄ alkyl, C₂₋₄ alkenyl, C₃₋₆ cycloalkyl, 3-6 membered heterocyclyl, C₆₋₈ aryl and 5-8 membered heteroaryl, the above groups are independently optionally further substituted with one or more substituents selected from the group consisting of deuterium, halogen, hydroxyl, =O, cyano, C₁₋₄ alkyl, C₁₋₄ alkoxyl, C₃₋₆ cycloalkyl, C₃₋₆ cycloalkoxyl, 3-6 membered heterocyclyl, 3-6 membered heterocycloxyl, C₆₋₈ aryl, C₆₋₈ aryloxyl, 5-8 membered heteroaryl, 5-8 membered heteroaryloxyl and -NR₁₆R₁₇;
each R₁₅ is independently selected from the group consisting of hydrogen, deuterium, hydroxyl, C₁₋₄ alkyl, C₁₋₄ alkoxyl, C₂₋₄ alkenyl, C₂₋₄ alkynyl, C₃₋₆ cycloalkyl, C₃₋₆ cycloalkoxyl, 3-6 membered heterocyclyl, 3-6 membered heterocycloxyl, C₆₋₈ aryl, C₆₋₈ aryloxyl, 5-8 membered heteroaryl, 5-8 membered heteroaryloxyl and -NR₁₆R₁₇, the above groups are independently optionally further substituted with one or more substituents selected from the group consisting of deuterium, halogen, hydroxyl, =O, cyano, C₁₋₄ alkyl, C₁₋₄ alkoxyl, C₃₋₆ cycloalkyl, C₃₋₆ cycloalkoxyl, 3-6 membered heterocyclyl, 3-6 membered heterocycloxyl, C₆₋₈ aryl, C₆₋₈ aryloxyl, 5-8 membered heteroaryl, 5-8 membered heteroaryloxyl and -NR₁₆R₁₇;
each R₁₆ and each R₁₇ are independently selected from the group consisting of hydrogen, deuterium, hydroxyl, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, sulfinyl, sulfonyl, methylsulfonyl, isopropylsulfonyl, cyclopropylsulfonyl, p-toluenesulfonyl, aminosulfonyl, dimethylaminosulfonyl and C₁₋₄ alkanoyl, the above groups are independently optionally further substituted with one or more substituents selected from the group consisting of deuterium, halogen, hydroxyl, =O, C₁₋₄ alkyl, C₂₋₄ alkenyl, C₂₋₄ alkynyl, C₁₋₄ haloalkyl, C₁₋₄ deuterioalkyl, C₁₋₄ alkoxyl, C₃₋₆ cycloalkyl, C₃₋₆ cycloalkoxyl, 3-6 membered heterocyclyl, 3-6 membered heterocycloxyl, C₆₋₈ aryl, C₆₋₈ aryloxyl, 5-8 membered heteroaryl, 5-8 membered heteroaryloxyl, amino, mono(C₁₋₄ alkyl)amino, di(C₁₋₄ alkyl)amino and C₁₋₄ alkanoyl;
or, R₁₆ and R₁₇ together with the nitrogen atom to which they are directly attached form 4-6 membered heterocyclyl or 5-8 membered heteroaryl, the above 4-6 membered heterocyclyl or 5-8 membered heteroaryl is optionally further substituted with one or more substituents selected from the group consisting of deuterium, halogen, hydroxyl, =O, C₁₋₄ alkyl, C₂₋₄ alkenyl, C₂₋₄ alkynyl, C₁₋₄ haloalkyl, C₁₋₄ deuterioalkyl, C₁₋₄ alkoxyl, C₃₋₆ cycloalkyl, C₃₋₆ cycloalkoxyl, 3-6 membered heterocyclyl, 3-6 membered heterocycloxyl, C₆₋₈ aryl, C₆₋₈ aryloxyl, 5-8 membered heteroaryl, 5-8 membered heteroaryloxyl, amino, mono(C₁₋₄ alkyl)amino, di(C₁₋₄ alkyl)amino and C₁₋₄ alkanoyl.

4. The compound of formula (I) according to claim 1, the stereoisomer or pharmaceutically acceptable salt thereof, wherein the compound of formula (I) is a compound of formula (IIa1), (IIa2), (IIa3) or (IIa4) as follows: 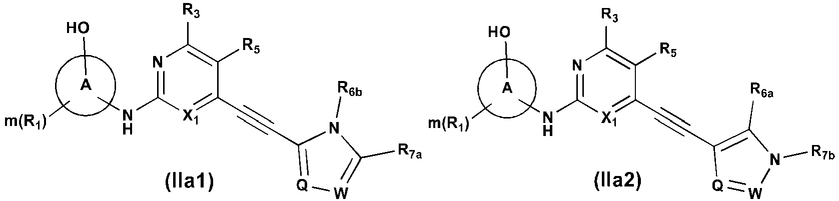 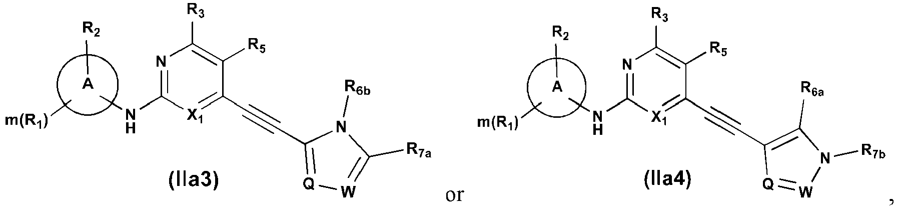
wherein, each X₁ is independently CR₄ or N;
each W is independently N or CR₈ₐ;
each Q is independently N or CR₉ₐ;
each ring A is independently C₅₋₈ cycloalkyl or 5-8 membered heterocyclyl;
each R₁ is independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, - P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅, the above groups are independently optionally further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, - P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅;
each R₂ is independently selected from the group consisting of halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₁₋₄ alkoxyl, C₁₋₄ haloalkoxyl, C₁₋₄ deuterioalkoxyl, -SF₅, -C(O)OR₁₄, -C(O)R₁₅, - O-C(O)R₁₅, -NR₁₆R₁₇, -C(O)NR₁₆R₁₇ and -N(R₁₆)-C(O)R₁₅;
each R₃ is independently selected from the group consisting of hydrogen, deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, -SF₅, -O-R₁₄ and -NR₁₆R₁₇;
each R₄ is independently selected from the group consisting of hydrogen, deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, -SF₅, -O-R₁₄ and -NR₁₆R₁₇;
each R₅ is independently selected from the group consisting of hydrogen, deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, -SF₅, -O-R₁₄ and -NR₁₆R₁₇;
each R₆ₐ and each R₇ₐ are independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -S(O)(=N-R₁₀)R₁₁, -N=S(O)R₁₁R₁₂, - N=SR₁₁R₁₂, -O-S(I)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and - N(R₁₆)-C(O)R₁₅, the above groups are independently optionally further substituted with one or more substituents selected from the group consisting of deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₁₀)R₁₁, -N=S(O)R₁₁R₁₂, -N=SR₁₁R₁₂, -O-S(O)₂R₁₅, - S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, - NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅, the above groups are independently optionally more further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, - P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅;
each R_{6b} and each R_{7b} are independently selected from the group consisting of hydrogen, deuterium, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -S(O)(=N-R₁₀)R₁₁, -S(O)ᵣR₁₃, -C(O)OR₁₄, -C(O)SR₁₄, -C(O)R₁₅, -P(O)(R₁₅)₂, -C(=NR₁₆)R₁₅ and -C(O)NR₁₆R₁₇, the above groups are independently optionally further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, - S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, - C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅;
or, in the compound of formula (IIa1) or the compound of formula (IIa3), R_{6b} and R₇ₐ together with the moiety to which they are directly attached form 3-8 membered heterocyclyl or 5-8 membered heteroaryl, the above 3-8 membered heterocyclyl or 5-8 membered heteroaryl is independently optionally further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, =O, =S, -SF₅, -S(O)ᵣR₁₃, -O-R₁₄, - C(O)OR₁₄, -C(O)R₁₅, -O-C(O)R₁₅ and -NR₁₆R₁₇;
or, in the compound of formula (IIa2) or the compound of formula (IIa4), R₆ₐ and R_{7b} together with the moiety to which they are directly attached form 3-8 membered heterocyclyl or 5-8 membered heteroaryl, the above 3-8 membered heterocyclyl or 5-8 membered heteroaryl is independently optionally further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, =O, =S, -SF₅, -S(O)ᵣR₁₃, -O-R₁₄, - C(O)OR₁₄, -C(O)R₁₅, -O-C(O)R₁₅ and -NR₁₆R₁₇;
each R₈ₐ and each R₉ₐ are independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -S(O)(=N-R₁₀)R₁₁, -N=S(O)R₁₁R₁₂, - N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and - N(R₁₆)-C(O)R₁₅, the above groups are independently optionally further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₁₀)R₁₁, - N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, - C(O)NR₁₆R₁₇ and -N(R₁₆)-C(O)R₁₅;
wherein, R₁₀, R₁₁, R₁₂, R₁₃, R₁₄, R₁₅, R₁₆, R₁₇, m and r are defined as in claim 1.

5. The compound of formula (I) according to claim 1, the stereoisomer or pharmaceutically acceptable salt thereof, wherein the compound of formula (I) is a compound of formula (IIIa1), (IIIa2), (IIIa3) or (IIIa4) as follows: 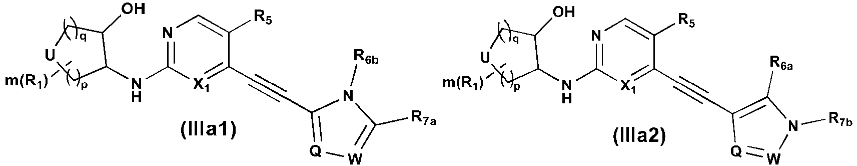 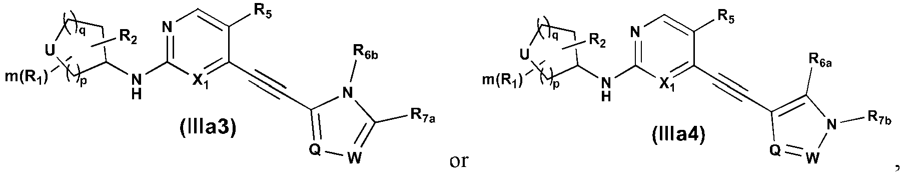
wherein, each X₁ is independently CR₄ or N;
each W is independently N or CR₈ₐ;
each Q is independently N or CR₉ₐ;
each U is independently NR₁ₐ, CR_{1b}R_{1c}, O or S;
each p is independently 1, 2 or 3; each q is independently 0, 1, 2 or 3;
each R₁ is independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, -SF₅, -S(O)(=N-R₁₀)R₁₁, -N=S(O)R₁₁R₁₂, -S(O)ᵣR₁₃, -O-R₁₄, -C(O)OR₁₄, -C(O)R₁₅, -NR₁₆R₁₇, - C(O)NR₁₆R₁₇ and -N(R₁₆)-C(O)R₁₅, the above groups are independently optionally further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, =O, =S, -SF₅, -S(O)(=N-R₁₀)R₁₁, -N=S(O)R₁₁R₁₂, - S(O)ᵣR₁₃, -O-R₁₄, -C(O)OR₁₄, -C(O)R₁₅, -NR₁₆R₁₇, -C(O)NR₁₆R₁₇ and -N(R₁₆)-C(O)R₁₅;
each R₁ₐ is independently selected from the group consisting of hydrogen, deuterium, C₁₋₄ alkyl, C₂₋₄ alkenyl, C₂₋₄ alkynyl, C₃₋₆ cycloalkyl, 3-6 membered heterocyclyl, -S(O)(=N-R₁₀)R₁₁, -S(O)ᵣR₁₃, -C(O)OR₁₄, -C(O)R₁₅ and -C(O)NR₁₆R₁₇, the above groups are independently optionally further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, =O, =S, -SF₅, -S(O)(=N-R₁₀)R₁₁, -N=S(O)R₁₁R₁₂, - S(O)ᵣR₁₃, -O-R₁₄, -C(O)OR₁₄, -C(O)R₁₅, -NR₁₆R₁₇, -C(O)NR₁₆R₁₇ and -N(R₁₆)-C(O)R₁₅;
each R_{1b} and each R_{1c} are independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, -SF₅, -S(O)(=N-R₁₀)R₁₁, -N=S(O)R₁₁R₁₂, -S(O)ᵣR₁₃, -O-R₁₄, -C(O)OR₁₄, -C(O)R₁₅, -NR₁₆R₁₇, -C(O)NR₁₆R₁₇ and -N(R₁₆)-C(O)R₁₅, the above groups are independently optionally further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, =O, =S, -SF₅, -S(O)(=N-R₁₀)R₁₁, -N=S(O)R₁₁R₁₂, - S(O)ᵣR₁₃, -O-R₁₄, -C(O)OR₁₄, -C(O)R₁₅, -NR₁₆R₁₇, -C(O)NR₁₆R₁₇ and -N(R₁₆)-C(O)R₁₅;
each R₂ is independently selected from the group consisting of halogen, cyano, C₁₋₄ alkyl, C₁₋₄ haloalkyl, C₁₋₄ deuterioalkyl, C₁₋₄ alkoxyl, C₁₋₄ haloalkoxyl, C₁₋₄ deuterioalkoxyl, -NR₁₆R₁₇, -C(O)NR₁₆R₁₇ and -N(R₁₆)-C(O)R₁₅;
each R₄ is independently selected from the group consisting of hydrogen, deuterium, fluoro, chloro, cyano, methyl, ethyl, n-propyl, isopropyl, methoxy, ethoxy, n-propoxy, isopropoxy, trifluoromethyl, trideuteromethyl, trifluoromethoxy, trideuteromethoxy, vinyl, ethynyl, cyclopropyl, cyclobutyl, cyclopropoxy, cyclobutoxy, oxetanyl, azetidinyl, -SF₅, hydroxyl, amino, methylamino and dimethylamino;
each R₅ is independently selected from the group consisting of hydrogen, deuterium, fluoro, chloro, cyano, methyl, ethyl, n-propyl, isopropyl, methoxy, ethoxy, n-propoxy, isopropoxy, trifluoromethyl, trideuteromethyl, trifluoromethoxy, trideuteromethoxy, vinyl, ethynyl, cyclopropyl, cyclobutyl, cyclopropoxy, cyclobutoxy, oxetanyl, azetidinyl, -SF₅, hydroxyl, amino, methylamino and dimethylamino;
each R₆ₐ and each R₇ₐ are independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, -SF₅, -S(O)ᵣR₁₃, -O-R₁₄, -C(O)OR₁₄, -C(O)R₁₅, -O-C(O)R₁₅, -NR₁₆R₁₇, - C(O)NR₁₆R₁₇ and -N(R₁₆)-C(O)R₁₅, the above groups are independently optionally further substituted with one or more substituents selected from the group consisting of deuterium, halogen, cyano, C₁₋₄ alkyl, C₂₋₄ alkenyl, C₂₋₄ alkynyl, C₃₋₆ cycloalkyl, 3-6 membered heterocyclyl, =O, =S, -SF₅, -S(O)ᵣR₁₃, -O-R₁₄, -C(O)OR₁₄, -C(O)R₁₅, -O-C(O)R₁₅, -NR₁₆R₁₇, -C(O)NR₁₆R₁₇ and -N(R₁₆)-C(O)R₁₅, the above groups are independently optionally more further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, =O, =S, -SF₅, -S(O)ᵣR₁₃, -O-R₁₄, -C(O)OR₁₄, -C(O)R₁₅, -O-C(O)R₁₅, -NR₁₆R₁₇, - C(O)NR₁₆R₁₇ and -N(R₁₆)-C(O)R₁₅;
each R_{6b} and each R_{7b} are independently selected from the group consisting of hydrogen, deuterium, C₁₋₄ alkyl, C₂₋₄ alkenyl, C₂₋₄ alkynyl, C₃₋₆ cycloalkyl, 3-6 membered heterocyclyl, - S(O)ᵣR₁₃, -C(O)OR₁₄, -C(O)R₁₅ and -C(O)NR₁₆R₁₇, the above groups are independently optionally further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, =O, =S, -SF₅, -S(O)ᵣR₁₃, -O-R₁₄, -C(O)OR₁₄, - C(O)R₁₅, -O-C(O)R₁₅, -NR₁₆R₁₇, -C(O)NR₁₆R₁₇ and -N(R₁₆)-C(O)R₁₅;
or, in the compound of formula (IIIa1) or the compound of formula (IIIa3), R_{6b} and R₇ₐ together with the moiety to which they are directly attached form 3-8 membered heterocyclyl or 5-8 membered heteroaryl, the above 3-8 membered heterocyclyl or 5-8 membered heteroaryl is independently optionally further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, =O, =S, -SF₅, -S(O)ᵣR₁₃, -O-R₁₄, - C(O)OR₁₄, -C(O)R₁₅, -O-C(O)R₁₅ and -NR₁₆R₁₇;
or, in the compound of formula (IIIa2) or the compound of formula (IIIa4), R₆ₐ and R_{7b} together with the moiety to which they are directly attached form 3-8 membered heterocyclyl or 5-8 membered heteroaryl, the above 3-8 membered heterocyclyl or 5-8 membered heteroaryl is independently optionally further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, =O, =S, -SF₅, -S(O)ᵣR₁₃, -O-R₁₄, - C(O)OR₁₄, -C(O)R₁₅, -O-C(O)R₁₅ and -NR₁₆R₁₇;
each R₈ₐ and each R₉ₐ are independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, -SF₅, -S(O)ᵣR₁₃, -O-R₁₄, -C(O)OR₁₄, -C(O)R₁₅, -O-C(O)R₁₅, -NR₁₆R₁₇, - C(O)NR₁₆R₁₇ and -N(R₁₆)-C(O)R₁₅, the above groups are independently optionally further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, =O, =S, -SF₅, -S(O)ᵣR₁₃, -O-R₁₄, -C(O)OR₁₄, -C(O)R₁₅, - O-C(O)R₁₅, -NR₁₆R₁₇, -C(O)NR₁₆R₁₇ and -N(R₁₆)-C(O)R₁₅;
wherein, R₁₀, R₁₁, R₁₂, R₁₃, R₁₄, R₁₅, R₁₆, R₁₇, m and r are defined as in claim 1.

6. The compound of formula (I) according to claim 1, the stereoisomer or pharmaceutically acceptable salt thereof, wherein the compound of formula (I) is a compound of formula (IVa1), (IVa2), (IVa3) or (IVa4) as follows: 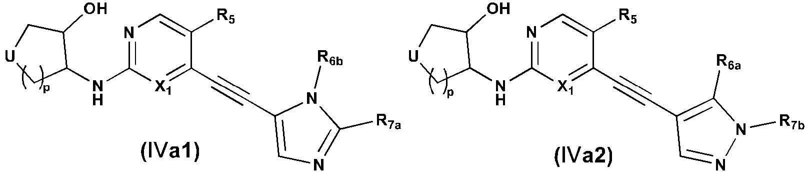 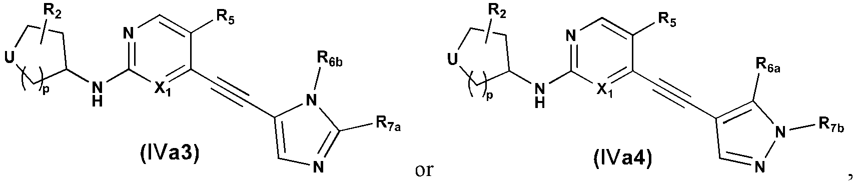
wherein, each X₁ is independently CH or N;
each U is independently NR₁ₐ, CH₂, O or S; each p is independently 1 or 2;
each R₁ₐ is independently selected from the group consisting of hydrogen, deuterium, C₁₋₄ alkyl, C₃₋₆ cycloalkyl, 3-6 membered heterocyclyl, methylsulfinyl, methylsulfonyl, isopropylsulfonyl, cyclopropylsulfonyl, aminosulfonyl and C₁₋₄ alkanoyl, the above groups are independently optionally further substituted with one or more substituents selected from the group consisting of deuterium, halogen, cyano, hydroxyl, carboxyl, C₁₋₄ alkyl, C₁₋₄ alkoxyl, C₁₋₄ haloalkyl, C₁₋₄ deuterioalkyl, C₁₋₄ haloalkoxyl, C₁₋₄ deuterioalkoxyl, C₃₋₆ cycloalkyl, C₃₋₆ cycloalkoxyl, 3-6 membered heterocyclyl, 3-6 membered heterocycloxyl, =O, =S, -NR₁₆R₁₇ and -C(O)NR₁₆R₁₇;
each R₂ is independently selected from the group consisting of halogen, cyano, C₁₋₄ alkyl, C₁₋₄ haloalkyl, C₁₋₄ deuterioalkyl, C₁₋₄ alkoxyl, C₁₋₄ haloalkoxyl, C₁₋₄ deuterioalkoxyl, -NR₁₆R₁₇, -C(O)NR₁₆R₁₇ and -N(R₁₆)-C(O)R₁₅;
each R₅ is independently selected from the group consisting of hydrogen, deuterium, fluoro, chloro, cyano, methyl, methoxy, trifluoromethyl, trifluoromethoxy, trifluoroethoxy, trideuteromethyl, trideuteromethoxy, cyclopropyl, cyclopropoxy, hydroxyl, amino, methylamino and dimethylamino;
each R₆ₐ and each R₇ₐ are independently selected from the group consisting of hydrogen, deuterium, halogen, cyano, C₁₋₄ alkyl, C₃₋₆ cycloalkyl, 3-6 membered heterocyclyl, methylsulfinyl, methylsulfonyl, isopropylsulfonyl, cyclopropylsulfonyl, aminosulfonyl, amino, methylamino, dimethylamino, C₁₋₄ alkanoyl and -C(O)NR₁₆R₁₇, the above groups are independently optionally further substituted with one or more substituents selected from the group consisting of deuterium, halogen, cyano, hydroxyl, C₁₋₄ alkyl, C₁₋₄ alkoxyl, C₃₋₆ cycloalkyl, C₃₋₆ cycloalkoxyl, 3-6 membered heterocyclyl, 3-6 membered heterocycloxyl, =O, =S, -NR₁₆R₁₇ and -C(O)NR₁₆R₁₇, the above groups are independently optionally more further substituted with one or more substituents selected from the group consisting of deuterium, halogen, cyano, hydroxyl, C₁₋₄ alkyl, C₁₋₄ alkoxyl, C₁₋₄ haloalkyl, C₁₋₄ haloalkoxyl, C₁₋₄ deuterioalkyl, C₁₋₄ deuterioalkoxyl, C₃₋₆ cycloalkyl, C₃₋₆ cycloalkoxyl, 3-6 membered heterocyclyl, 3-6 membered heterocycloxyl, =O, =S, -NR₁₆R₁₇ and -C(O)NR₁₆R₁₇;
each R_{6b} and each R_{7b} are independently selected from the group consisting of hydrogen, deuterium, C₁₋₄ alkyl, C₃₋₆ cycloalkyl, 3-6 membered heterocyclyl, methylsulfinyl, methylsulfonyl, isopropylsulfonyl, cyclopropylsulfonyl, aminosulfonyl and C₁₋₄ alkanoyl, the above groups are independently optionally further substituted with one or more substituents selected from the group consisting of deuterium, halogen, cyano, hydroxyl, C₁₋₄ alkyl, C₁₋₄ alkoxyl, C₁₋₄ haloalkyl, C₁₋₄ haloalkoxyl, C₁₋₄ deuterioalkyl, C₁₋₄ deuterioalkoxyl, C₃₋₆ cycloalkyl, C₃₋₆ cycloalkoxyl, 3-6 membered heterocyclyl, 3-6 membered heterocycloxyl, =O, =S, -NR₁₆R₁₇ and -C(O)NR₁₆R₁₇;
or, in the compound of formula (IVa1) or the compound of formula (IVa3), R_{6b} and R₇ₐ together with the moiety to which they are directly attached form 3-8 membered heterocyclyl or 5-8 membered heteroaryl, the above 3-8 membered heterocyclyl or 5-8 membered heteroaryl is independently optionally further substituted with one or more substituents selected from the group consisting of deuterium, halogen, cyano, hydroxyl, C₁₋₄ alkyl, C₁₋₄ alkoxyl, C₁₋₄ haloalkyl, C₁₋₄ haloalkoxyl, C₁₋₄ deuterioalkyl, C₁₋₄ deuterioalkoxyl, C₃₋₆ cycloalkyl, C₃₋₆ cycloalkoxyl, 3-6 membered heterocyclyl, 3-6 membered heterocycloxyl, =O, =S and -NR₁₆R₁₇;
or, in the compound of formula (IVa2) or the compound of formula (IVa4), R₆ₐ and R_{7b} together with the moiety to which they are directly attached form 3-8 membered heterocyclyl or 5-8 membered heteroaryl, the above 3-8 membered heterocyclyl or 5-8 membered heteroaryl is independently optionally further substituted with one or more substituents selected from the group consisting of deuterium, halogen, cyano, hydroxyl, C₁₋₄ alkyl, C₁₋₄ alkoxyl, C₁₋₄ haloalkyl, C₁₋₄ haloalkoxyl, C₁₋₄ deuterioalkyl, C₁₋₄ deuterioalkoxyl, C₃₋₆ cycloalkyl, C₃₋₆ cycloalkoxyl, 3-6 membered heterocyclyl, 3-6 membered heterocycloxyl, =O, =S and -NR₁₆R₁₇;
wherein, R₁₃, R₁₄, R₁₅, R₁₆ and R₁₇ are defined as in claim 1.

7. The compound of formula (I) according to claim 6, the stereoisomer or pharmaceutically acceptable salt thereof, wherein, each U is independently NR₁ₐ, CH₂, O or S;
each R₁ₐ is independently selected from the group consisting of hydrogen, deuterium, C₁₋₄ alkyl, C₃₋₆ cycloalkyl, methylsulfonyl and isopropylsulfonyl, the above groups are independently optionally further substituted with one or more substituents selected from the group consisting of deuterium, halogen, C₁₋₄ alkyl, C₁₋₄ haloalkyl, C₁₋₄ deuterioalkyl, C₃₋₆ cycloalkyl, -C(O)NH₂, - C(O)NHCH₃ and -C(O)N(CH₃)₂.

8. The compound of formula (I) according to claim 6, the stereoisomer or pharmaceutically acceptable salt thereof, wherein, each R₅ is independently selected from the group consisting of hydrogen, deuterium, fluoro, chloro, cyano, methyl, methoxy, trifluoromethyl, trifluoromethoxy, trifluoroethoxy, trideuteromethyl, trideuteromethoxy and cyclopropyl.

9. The compound of formula (I) according to claim 6, the stereoisomer or pharmaceutically acceptable salt thereof, wherein, R_{6b} is selected from the group consisting of hydrogen, deuterium, C₁₋₄ alkyl, C₃₋₆ cycloalkyl, 3-6 membered heterocyclyl, methylsulfonyl and C₁₋₄ alkanoyl, the above groups are independently optionally further substituted with one or more substituents selected from the group consisting of deuterium, halogen, cyano, hydroxyl, C₁₋₄ alkyl, C₁₋₄ alkoxyl, C₁₋₄ haloalkyl, C₁₋₄ haloalkoxyl, C₁₋₄ deuterioalkyl, C₁₋₄ deuterioalkoxyl, C₃₋₆ cycloalkyl, C₃₋₆ cycloalkoxyl, 3-6 membered heterocyclyl, 3-6 membered heterocycloxyl and -NR₁₆R₁₇;
R₇ₐ is selected from the group consisting of hydrogen, deuterium, halogen, cyano, C₁₋₄ alkyl, C₃₋₆ cycloalkyl, 3-6 membered heterocyclyl, methylsulfonyl, amino, methylamino, dimethylamino, C₁₋₄ alkanoyl and -C(O)NR₁₆R₁₇, the above groups are independently optionally further substituted with one or more substituents selected from the group consisting of deuterium, halogen, cyano, hydroxyl, C₁₋₄ alkyl, C₁₋₄ alkoxyl, C₃₋₆ cycloalkyl, C₃₋₆ cycloalkoxyl, 3-6 membered heterocyclyl, 3-6 membered heterocycloxyl and -NR₁₆R₁₇, the above groups are independently optionally more further substituted with one or more substituents selected from the group consisting of deuterium, halogen, cyano, hydroxyl, C₁₋₄ alkyl, C₁₋₄ alkoxyl, C₁₋₄ haloalkyl, C₁₋₄ haloalkoxyl, C₁₋₄ deuterioalkyl, C₁₋₄ deuterioalkoxyl, C₃₋₆ cycloalkyl, C₃₋₆ cycloalkoxyl, 3-6 membered heterocyclyl, 3-6 membered heterocycloxyl and -NR₁₆R₁₇;
or, R_{6b} and R₇ₐ together with the moiety to which they are directly attached form 3-8 membered heterocyclyl, the above 3-8 membered heterocyclyl is optionally further substituted with one or more substituents selected from the group consisting of deuterium, halogen, cyano, hydroxyl, C₁₋₄ alkyl, C₁₋₄ alkoxyl, C₁₋₄ haloalkyl, C₁₋₄ haloalkoxyl, C₁₋₄ deuterioalkyl, C₁₋₄ deuterioalkoxyl, C₃₋₆ cycloalkyl, C₃₋₆ cycloalkoxyl, 3-6 membered heterocyclyl, 3-6 membered heterocycloxyl and -NR₁₆R₁₇;
wherein, R₁₆ and R₁₇ are defined as in claim 6.

10. The compound of formula (I) according to claim 6, the stereoisomer or pharmaceutically acceptable salt thereof, wherein, R₆ₐ is selected from the group consisting of hydrogen, deuterium, halogen, cyano, C₁₋₄ alkyl, C₃₋₆ cycloalkyl, 3-6 membered heterocyclyl, methylsulfonyl, amino, methylamino, dimethylamino, C₁₋₄ alkanoyl and -C(O)NR₁₆R₁₇, the above groups are independently optionally further substituted with one or more substituents selected from the group consisting of deuterium, halogen, cyano, hydroxyl, C₁₋₄ alkyl, C₁₋₄ alkoxyl, C₃₋₆ cycloalkyl, C₃₋₆ cycloalkoxyl, 3-6 membered heterocyclyl, 3-6 membered heterocycloxyl and -NR₁₆R₁₇, the above groups are independently optionally more further substituted with one or more substituents selected from the group consisting of deuterium, halogen, cyano, hydroxyl, C₁₋₄ alkyl, C₁₋₄ alkoxyl, C₁₋₄ haloalkyl, C₁₋₄ haloalkoxyl, C₁₋₄ deuterioalkyl, C₁₋₄ deuterioalkoxyl, C₃₋₆ cycloalkyl, C₃₋₆ cycloalkoxyl, 3-6 membered heterocyclyl, 3-6 membered heterocycloxyl and -NR₁₆R₁₇;
R_{7b} is selected from the group consisting of hydrogen, deuterium, C₁₋₄ alkyl, C₃₋₆ cycloalkyl, 3-6 membered heterocyclyl, methylsulfonyl and C₁₋₄ alkanoyl, the above groups are independently optionally further substituted with one or more substituents selected from the group consisting of deuterium, halogen, cyano, hydroxyl, C₁₋₄ alkyl, C₁₋₄ alkoxyl, C₁₋₄ haloalkyl, C₁₋₄ haloalkoxyl, C₁₋₄ deuterioalkyl, C₁₋₄ deuterioalkoxyl, C₃₋₆ cycloalkyl, C₃₋₆ cycloalkoxyl, 3-6 membered heterocyclyl, 3-6 membered heterocycloxyl and -NR₁₆R₁₇;
or, R₆ₐ and R_{7b} together with the moiety to which they are directly attached form 3-8 membered heterocyclyl, the above 3-8 membered heterocyclyl is optionally further substituted with one or more substituents selected from the group consisting of deuterium, halogen, cyano, hydroxyl, C₁₋₄ alkyl, C₁₋₄ alkoxyl, C₁₋₄ haloalkyl, C₁₋₄ haloalkoxyl, C₁₋₄ deuterioalkyl, C₁₋₄ deuterioalkoxyl, C₃₋₆ cycloalkyl, C₃₋₆ cycloalkoxyl, 3-6 membered heterocyclyl, 3-6 membered heterocycloxyl and -NR₁₆R₁₇;
wherein, R₁₆ and R₁₇ are defined as in claim 6.

11. The compound of formula (I) according to claim 6, the stereoisomer or pharmaceutically acceptable salt thereof, wherein, each R₁₆ and each R₁₇ are independently selected from the group consisting of hydrogen, deuterium, hydroxyl, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, sulfinyl, sulfonyl, methylsulfonyl, isopropylsulfonyl, cyclopropylsulfonyl, p-toluenesulfonyl, aminosulfonyl, dimethylaminosulfonyl and C₁₋₄ alkanoyl;
or, R₁₆ and R₁₇ together with the nitrogen atom to which they are directly attached form 4-6 membered heterocyclyl, the above 4-6 membered heterocyclyl is optionally further substituted with one or more substituents selected from the group consisting of deuterium, halogen, hydroxyl, =O, C₁₋₄ alkyl, C₁₋₄ haloalkyl, C₁₋₄ deuterioalkyl, C₃₋₆ cycloalkyl and 3-6 membered heterocyclyl.

12. The compound of formula (I) according to claim 1, the stereoisomer or pharmaceutically acceptable salt thereof, wherein the compound of formula (I) is a compound of formula (IIb 1) or (IIb2) as follows: 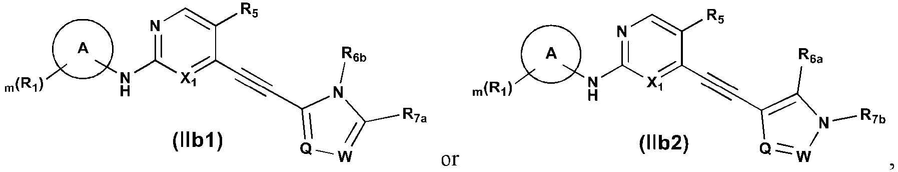
wherein, each X₁ is independently CR₄ or N;
each W is independently N or CR₈ₐ;
each Q is independently N or CR₉ₐ;
each ring A is independently phenyl or 5-6 membered heteroaryl;
each R₁ is independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-8 membered heterocyclyl, C₃₋₆ cycloalkyl-substituted C₁₋₄ alkyl, 3-6 membered heterocyclyl-substituted C₁₋₄ alkyl, -SF₅, - S(O)ᵣR₁₃, -O-R₁₄, -C(O)OR₁₄, -C(O)R₁₅, -O-C(O)R₁₅, -NR₁₆R₁₇, -C(O)NR₁₆R₁₇ and -N(R₁₆)-C(O)R₁₅, the above groups are independently optionally further substituted with one or more substituents selected from the group consisting of deuterium, halogen, cyano, C₁₋₄ alkyl, C₂₋₄ alkenyl, C₂₋₄ alkynyl, C₃₋₆ cycloalkyl, 3-6 membered heterocyclyl, =O, =S, -SF₅, -S(O)ᵣR₁₃, -OR₁₄, -C(O)OR₁₄, -C(O)R₁₅, -O-C(O)R₁₅, -NR₁₆R₁₇, -C(O)NR₁₆R₁₇ and -N(R₁₆)-C(O)R₁₅, the above groups are independently optionally more further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)ᵣR₁₃, -O-R₁₄, -C(O)OR₁₄, -C(O)R₁₅, -O-C(O)R₁₅, - NR₁₆R₁₇, -C(O)NR₁₆R₁₇ and -N(R₁₆)-C(O)R₁₅;
each R₄ is independently selected from the group consisting of hydrogen, deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, -SF₅, -O-R₁₄ and -NR₁₆R₁₇;
each R₅ is independently selected from the group consisting of hydrogen, deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, -SF₅, -O-R₁₄ and -NR₁₆R₁₇;
R₆ₐ and R₇ₐ are each independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, -SF₅, -S(O)ᵣR₁₃, -O-R₁₄, -C(O)OR₁₄, -C(O)R₁₅, -O-C(O)R₁₅, -NR₁₆R₁₇, - C(O)NR₁₆R₁₇ and -N(R₁₆)-C(O)R₁₅, the above groups are independently optionally further substituted with one or more substituents selected from the group consisting of deuterium, halogen, cyano, C₁₋₄ alkyl, C₂₋₄ alkenyl, C₂₋₄ alkynyl, C₃₋₆ cycloalkyl, 3-6 membered heterocyclyl, =O, =S, -SF₅, -S(O)ᵣR₁₃, -O-R₁₄, -C(O)OR₁₄, -C(O)R₁₅, -O-C(O)R₁₅, -NR₁₆R₁₇, -C(O)NR₁₆R₁₇ and -N(R₁₆)-C(O)R₁₅, the above groups are independently optionally more further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, =O, =S, -SF₅, -S(O)ᵣR₁₃, -O-R₁₄, -C(O)OR₁₄, -C(O)R₁₅, -O-C(O)R₁₅, -NR₁₆R₁₇, - C(O)NR₁₆R₁₇ and -N(R₁₆)-C(O)R₁₅;
R_{6b} and R_{7b} are each independently selected from the group consisting of hydrogen, deuterium, C₁₋₄ alkyl, C₂₋₄ alkenyl, C₂₋₄ alkynyl, C₃₋₆ cycloalkyl, 3-6 membered heterocyclyl, - S(O)ᵣR₁₃, -C(O)OR₁₄, -C(O)R₁₅ and -C(O)NR₁₆R₁₇, the above groups are independently optionally further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, =O, =S, -SF₅, -S(O)ᵣR₁₃, -O-R₁₄, -C(O)OR₁₄, - C(O)R₁₅, -O-C(O)R₁₅, -NR₁₆R₁₇, -C(O)NR₁₆R₁₇ and -N(R₁₆)-C(O)R₁₅;
or, in the compound of formula (IIb1), R_{6b} and R₇ₐ together with the moiety to which they are directly attached form 3-8 membered heterocyclyl or 5-8 membered heteroaryl, the above 3-8 membered heterocyclyl or 5-8 membered heteroaryl is independently optionally further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, =O, =S, -SF₅, -S(O)ᵣR₁₃, -O-R₁₄, -C(O)OR₁₄, -C(O)R₁₅, - O-C(O)R₁₅ and -NR₁₆R₁₇;
or, in the compound of formula (IIb2), R₆ₐ and R_{7b} together with the moiety to which they are directly attached form 3-8 membered heterocyclyl or 5-8 membered heteroaryl, the above 3-8 membered heterocyclyl or 5-8 membered heteroaryl is independently optionally further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, =O, =S, -SF₅, -S(O)ᵣR₁₃, -O-R₁₄, -C(O)OR₁₄, -C(O)R₁₅, - O-C(O)R₁₅ and -NR₁₆R₁₇;
each R₈ₐ and each R₉ₐ are independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, -SF₅, -S(O)ᵣR₁₃, -O-R₁₄, -C(O)OR₁₄, -C(O)R₁₅, -O-C(O)R₁₅, -NR₁₆R₁₇, - C(O)NR₁₆R₁₇ and -N(R₁₆)-C(O)R₁₅, the above groups are independently optionally further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, =O, =S, -SF₅, -S(O)ᵣR₁₃, -O-R₁₄, -C(O)OR₁₄, -C(O)R₁₅, - O-C(O)R₁₅, -NR₁₆R₁₇, -C(O)NR₁₆R₁₇ and -N(R₁₆)-C(O)R₁₅;
wherein, R₁₃, R₁₄, R₁₅, R₁₆, R₁₇, m and r are defined as in claim 1.

13. The compound of formula (I) according to claim 12, the stereoisomer or pharmaceutically acceptable salt thereof, wherein the compound of formula (I) is a compound of formula (IIIb1) or (IIIb2) as follows: 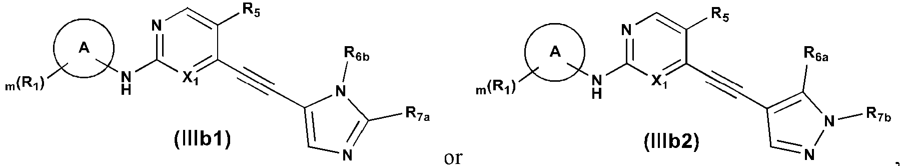
wherein, each X₁ is independently CH or N;
ring A is selected from the group consisting of phenyl, pyridinyl, pyrimidinyl, pyridazinyl, triazinyl, furanyl, thienyl, imidazolyl, triazolyl, pyrrolyl, oxazolyl, isoxazolyl, thiazolyl and isothiazolyl;
each R₁ is independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-8 membered heterocyclyl, C₃₋₆ cycloalkyl-substituted C₁₋₄ alkyl, 3-6 membered heterocyclyl-substituted C₁₋₄ alkyl, - C(O)R₁₅, -NR₁₆R₁₇ and -C(O)NR₁₆R₁₇, the above groups are independently optionally further substituted with one or more substituents selected from the group consisting of deuterium, halogen, cyano, C₁₋₄ alkyl, C₁₋₄ haloalkyl, C₁₋₄ deuterioalkyl, hydroxyl-substituted C₁₋₄ alkyl, cyano-substituted C₁₋₄ alkyl, C₂₋₄ alkenyl, C₂₋₄ alkynyl, C₃₋₆ cycloalkyl, 3-6 membered heterocyclyl, =O, =S, -SF₅, -S(O)ᵣR₁₃, -O-R₁₄, -C(O)OR₁₄, -C(O)R₁₅, -O-C(O)R₁₅, -NR₁₆R₁₇, - C(O)NR₁₆R₁₇ and -N(R₁₆)-C(O)R₁₅;
each R₅ is independently selected from the group consisting of hydrogen, deuterium, fluoro, chloro, cyano, methyl, methoxy, trifluoromethyl, trifluoromethoxy, trifluoroethoxy, trideuteromethyl, trideuteromethoxy, cyclopropyl, cyclopropoxy, hydroxyl, amino, methylamino and dimethylamino;
wherein, R₆ₐ, R_{6b}, R₇ₐ, R_{7b}, R₁₃, R₁₄, R₁₅, R₁₆, R₁₇, m and r are defined as in claim 12.

14. The compound of formula (I) according to claim 13, the stereoisomer or pharmaceutically acceptable salt thereof, wherein, R_{6b} is selected from the group consisting of hydrogen, deuterium, C₁₋₄ alkyl, C₃₋₆ cycloalkyl, 3-6 membered heterocyclyl, methylsulfinyl, methylsulfonyl, isopropylsulfonyl, cyclopropylsulfonyl, aminosulfonyl and C₁₋₄ alkanoyl, the above groups are independently optionally further substituted with one or more substituents selected from the group consisting of deuterium, halogen, cyano, hydroxyl, C₁₋₄ alkyl, C₁₋₄ alkoxyl, C₁₋₄ haloalkyl, C₁₋₄ haloalkoxyl, C₁₋₄ deuterioalkyl, C₁₋₄ deuterioalkoxyl, C₃₋₆ cycloalkyl, C₃₋₆ cycloalkoxyl, 3-6 membered heterocyclyl, 3-6 membered heterocycloxyl, =O, =S, -NR₁₆R₁₇ and -C(O)NR₁₆R₁₇;
R₇ₐ is selected from the group consisting of hydrogen, deuterium, halogen, cyano, C₁₋₄ alkyl, C₃₋₆ cycloalkyl, 3-6 membered heterocyclyl, methylsulfinyl, methylsulfonyl, isopropylsulfonyl, cyclopropylsulfonyl, aminosulfonyl, amino, methylamino, dimethylamino, C₁₋₄ alkanoyl and - C(O)NR₁₆R₁₇, the above groups are independently optionally further substituted with one or more substituents selected from the group consisting of deuterium, halogen, cyano, hydroxyl, C₁₋₄ alkyl, C₁₋₄ alkoxyl, C₃₋₆ cycloalkyl, C₃₋₆ cycloalkoxyl, 3-6 membered heterocyclyl, 3-6 membered heterocycloxyl, =O, =S, -NR₁₆R₁₇ and -C(O)NR₁₆R₁₇, the above groups are independently optionally more further substituted with one or more substituents selected from the group consisting of deuterium, halogen, cyano, hydroxyl, C₁₋₄ alkyl, C₁₋₄ alkoxyl, C₁₋₄ haloalkyl, C₁₋₄ haloalkoxyl, C₁₋₄ deuterioalkyl, C₁₋₄ deuterioalkoxyl, C₃₋₆ cycloalkyl, C₃₋₆ cycloalkoxyl, 3-6 membered heterocyclyl, 3-6 membered heterocycloxyl, =O, =S, -NR₁₆R₁₇ and -C(O)NR₁₆R₁₇;
or, R_{6b} and R₇ₐ together with the moiety to which they are directly attached form 3-8 membered heterocyclyl, the above 3-8 membered heterocyclyl is independently optionally further substituted with one or more substituents selected from the group consisting of deuterium, halogen, cyano, hydroxyl, C₁₋₄ alkyl, C₁₋₄ alkoxyl, C₁₋₄ haloalkyl, C₁₋₄ haloalkoxyl, C₁₋₄ deuterioalkyl, C₁₋₄ deuterioalkoxyl, C₃₋₆ cycloalkyl, C₃₋₆ cycloalkoxyl, 3-6 membered heterocyclyl, 3-6 membered heterocycloxyl, =O, =S and -NR₁₆R₁₇;
wherein, R₁₆ and R₁₇ are defined as in claim 13.

15. The compound of formula (I) according to claim 13, the stereoisomer or pharmaceutically acceptable salt thereof, wherein, R₆ₐ is selected from the group consisting of hydrogen, deuterium, halogen, cyano, C₁₋₄ alkyl, C₃₋₆ cycloalkyl, 3-6 membered heterocyclyl, methylsulfinyl, methylsulfonyl, isopropylsulfonyl, cyclopropylsulfonyl, aminosulfonyl, amino, methylamino, dimethylamino, C₁₋₄ alkanoyl and -C(O)NR₁₆R₁₇, the above groups are independently optionally further substituted with one or more substituents selected from the group consisting of deuterium, halogen, cyano, hydroxyl, C₁₋₄ alkyl, C₁₋₄ alkoxyl, C₃₋₆ cycloalkyl, C₃₋₆ cycloalkoxyl, 3-6 membered heterocyclyl, 3-6 membered heterocycloxyl, =O, =S, -NR₁₆R₁₇ and -C(O)NR₁₆R₁₇, the above groups are independently optionally more further substituted with one or more substituents selected from the group consisting of deuterium, halogen, cyano, hydroxyl, C₁₋₄ alkyl, C₁₋₄ alkoxyl, C₁₋₄ haloalkyl, C₁₋₄ haloalkoxyl, C₁₋₄ deuterioalkyl, C₁₋₄ deuterioalkoxyl, C₃₋₆ cycloalkyl, C₃₋₆ cycloalkoxyl, 3-6 membered heterocyclyl, 3-6 membered heterocycloxyl, =O, =S, -NR₁₆R₁₇ and -C(O)NR₁₆R₁₇;
R_{7b} is selected from the group consisting of hydrogen, deuterium, C₁₋₄ alkyl, C₃₋₆ cycloalkyl, 3-6 membered heterocyclyl, methylsulfinyl, methylsulfonyl, isopropylsulfonyl, cyclopropylsulfonyl, aminosulfonyl and C₁₋₄ alkanoyl, the above groups are independently optionally further substituted with one or more substituents selected from the group consisting of deuterium, halogen, cyano, hydroxyl, C₁₋₄ alkyl, C₁₋₄ alkoxyl, C₁₋₄ haloalkyl, C₁₋₄ haloalkoxyl, C₁₋₄ deuterioalkyl, C₁₋₄ deuterioalkoxyl, C₃₋₆ cycloalkyl, C₃₋₆ cycloalkoxyl, 3-6 membered heterocyclyl, 3-6 membered heterocycloxyl, =O, =S, -NR₁₆R₁₇ and -C(O)NR₁₆R₁₇;
or, R₆ₐ and R_{7b} together with the moiety to which they are directly attached form 3-8 membered heterocyclyl, the above 3-8 membered heterocyclyl is independently optionally further substituted with one or more substituents selected from the group consisting of deuterium, halogen, cyano, hydroxyl, C₁₋₄ alkyl, C₁₋₄ alkoxyl, C₁₋₄ haloalkyl, C₁₋₄ haloalkoxyl, C₁₋₄ deuterioalkyl, C₁₋₄ deuterioalkoxyl, C₃₋₆ cycloalkyl, C₃₋₆ cycloalkoxyl, 3-6 membered heterocyclyl, 3-6 membered heterocycloxyl, =O, =S and -NR₁₆R₁₇;
wherein, R₁₆ and R₁₇ are defined as in claim 13.

16. The compound of formula (I) according to claim 1, the stereoisomer or pharmaceutically acceptable salt thereof, wherein, each R₁₃ is independently selected from the group consisting of hydrogen, deuterium, hydroxyl, C₁₋₄ alkyl, 3-6 membered heterocyclyl and -NR₁₆R₁₇, the above groups are independently optionally further substituted with one or more substituents selected from the group consisting of deuterium, halogen, cyano, hydroxyl, =O, C₁₋₄ alkyl, C₁₋₄ alkoxyl, C₃₋₆ cycloalkyl, C₃₋₆ cycloalkoxyl, 3-6 membered heterocyclyl, 3-6 membered heterocycloxyl and -NR₁₆R₁₇;
each R₁₄ is independently selected from the group consisting of hydrogen, deuterium, C₁₋₄ alkyl and 3-6 membered heterocyclyl, the above groups are independently optionally further substituted with one or more substituents selected from the group consisting of deuterium, halogen, hydroxyl, =O, cyano, C₁₋₄ alkyl, C₁₋₄ alkoxyl, C₃₋₆ cycloalkyl, C₃₋₆ cycloalkoxyl, 3-6 membered heterocyclyl, 3-6 membered heterocycloxyl and -NR₁₆R₁₇;
each R₁₅ is independently selected from the group consisting of hydrogen, deuterium, hydroxyl, C₁₋₄ alkyl, C₁₋₄ alkoxyl, C₂₋₄ alkenyl, C₃₋₆ cycloalkyl, C₃₋₆ cycloalkoxyl, 3-6 membered heterocyclyl, 3-6 membered heterocycloxyl and -NR₁₆R₁₇, the above groups are independently optionally further substituted with one or more substituents selected from the group consisting of deuterium, halogen, hydroxyl, =O, cyano, C₁₋₄ alkyl, C₁₋₄ alkoxyl, C₃₋₆ cycloalkyl, C₃₋₆ cycloalkoxyl, 3-6 membered heterocyclyl, 3-6 membered heterocycloxyl and -NR₁₆R₁₇,
wherein, R₁₆ and R₁₇ are defined as in claim 13.

17. The compound of formula (I) according to claim 1, the stereoisomer or pharmaceutically acceptable salt thereof, wherein, each R₁₆ and each R₁₇ are independently selected from the group consisting of hydrogen, deuterium, hydroxyl, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, sulfinyl, sulfonyl, methylsulfonyl, isopropylsulfonyl, cyclopropylsulfonyl, p-toluenesulfonyl, aminosulfonyl, dimethylaminosulfonyl and C₁₋₄ alkanoyl;
or, R₁₆ and R₁₇ together with the nitrogen atom to which they are directly attached form 4-6 membered heterocyclyl, the above 4-6 membered heterocyclyl is optionally further substituted with one or more substituents selected from the group consisting of deuterium, halogen, hydroxyl, =O, C₁₋₄ alkyl, C₁₋₄ haloalkyl, C₁₋₄ deuterioalkyl, C₃₋₆ cycloalkyl and 3-6 membered heterocyclyl.

18. The compound of formula (I) according to claim 1, the stereoisomer or pharmaceutically acceptable salt thereof, wherein the compound is selected from the following compounds:

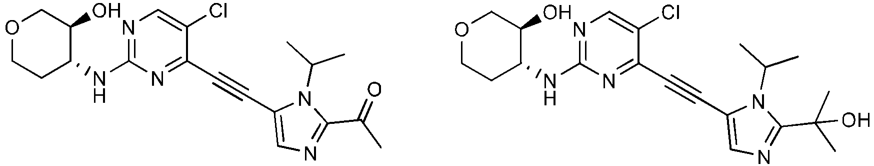

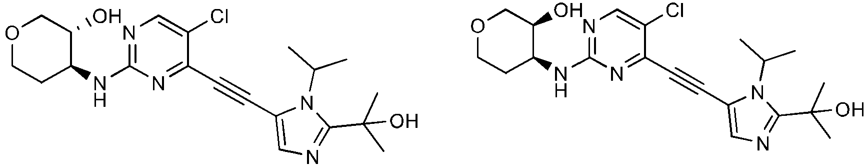

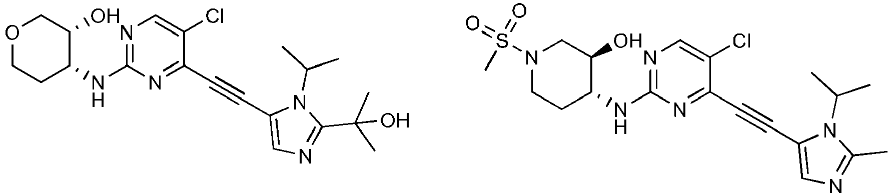

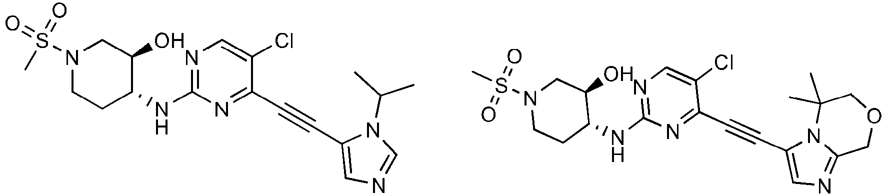

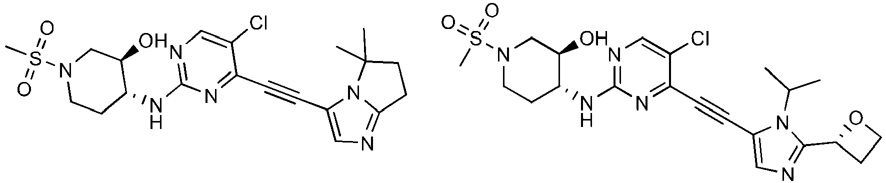

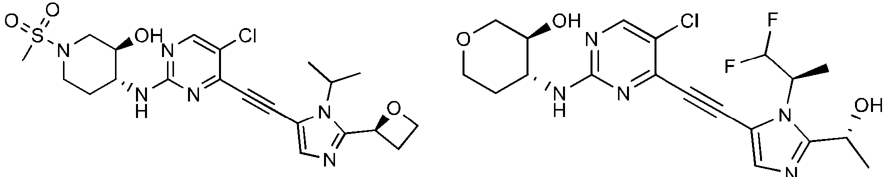

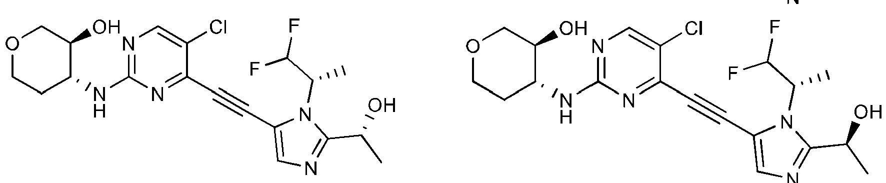

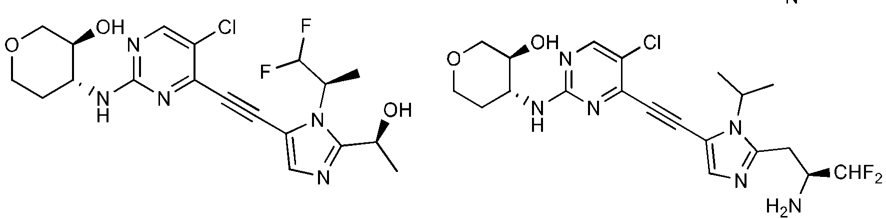

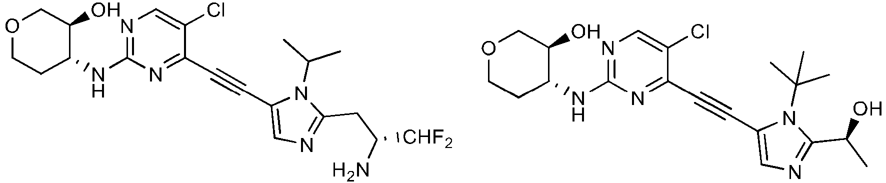

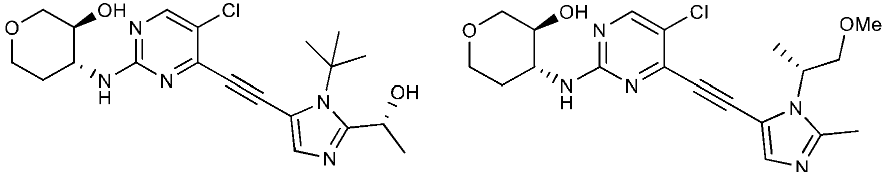

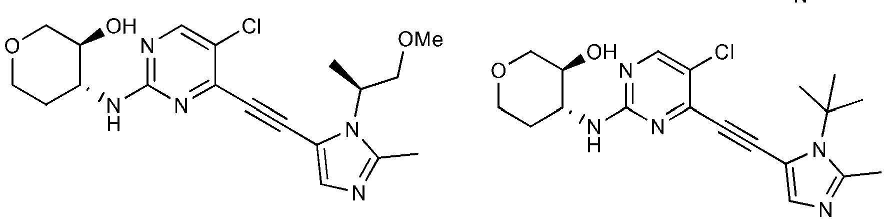

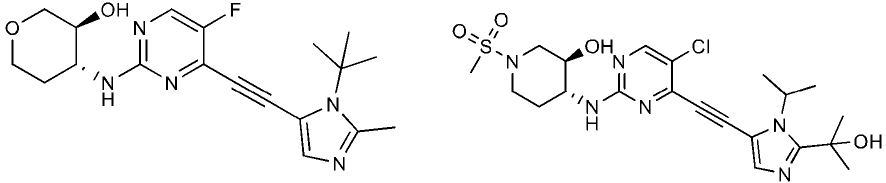

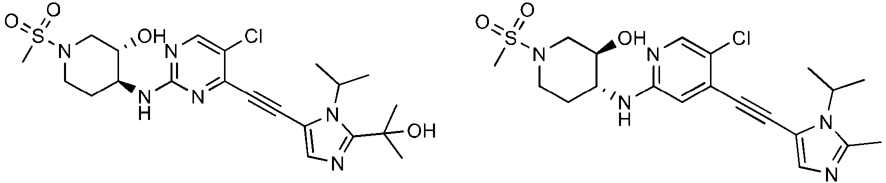

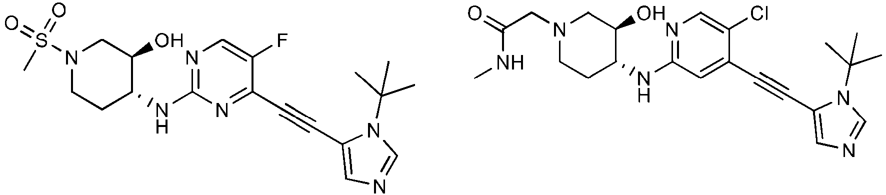

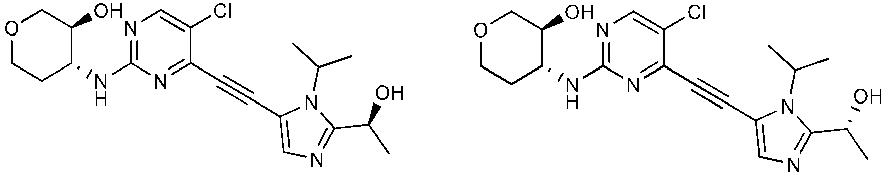

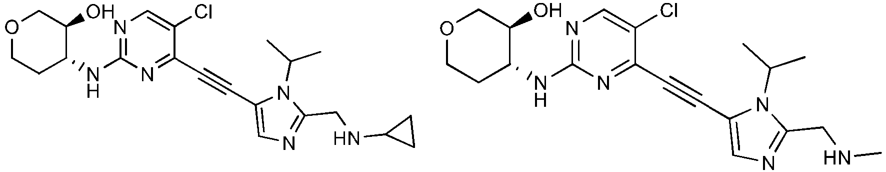

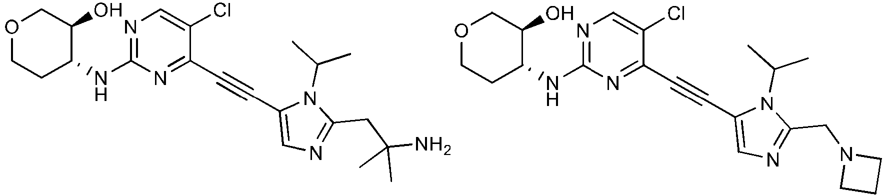

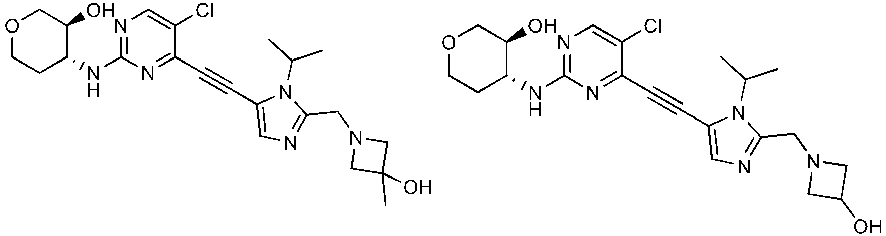

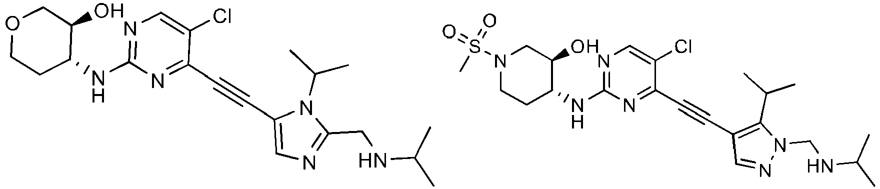

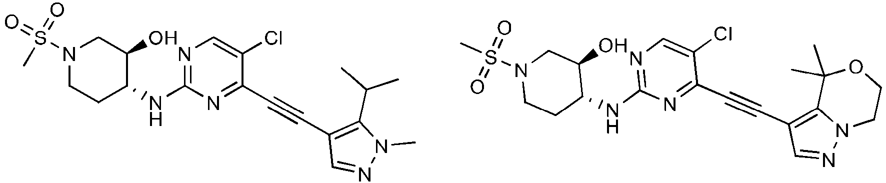

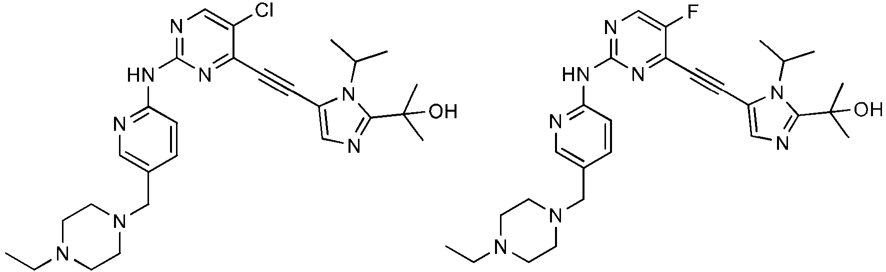

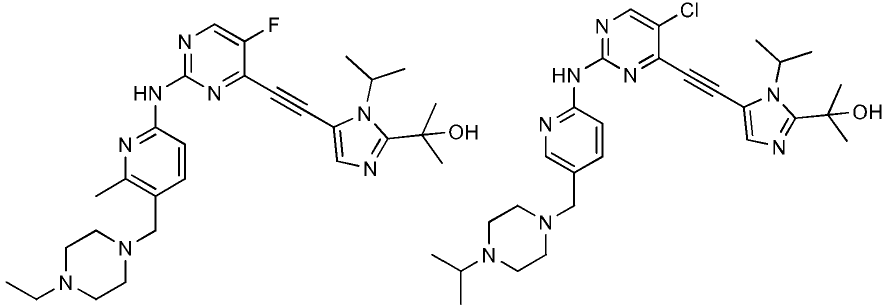

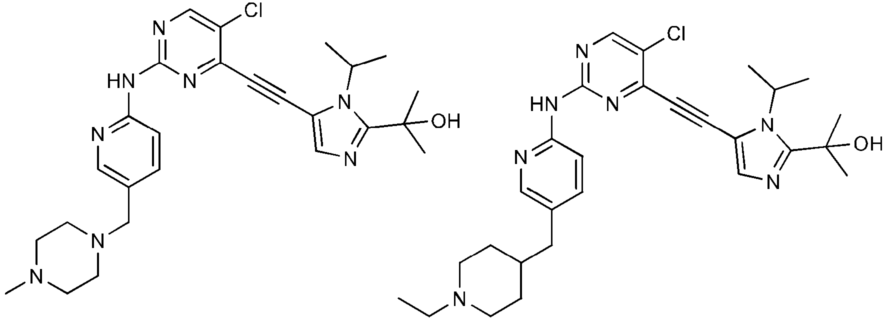

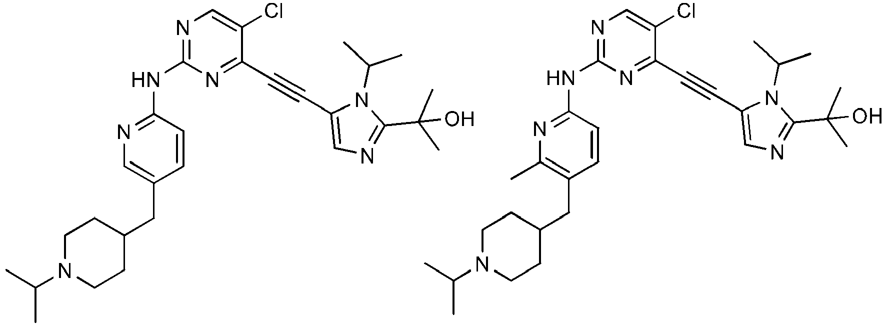

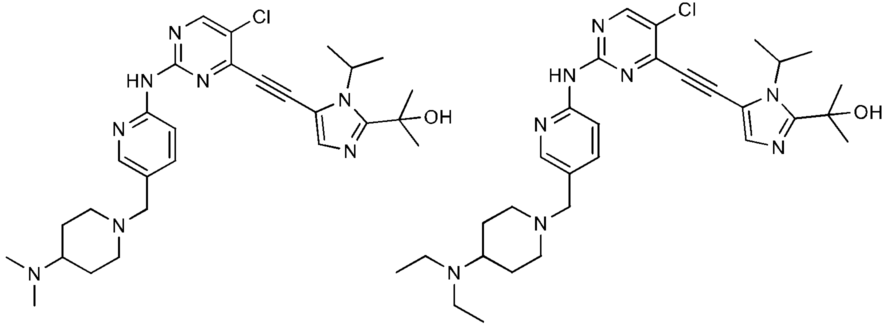

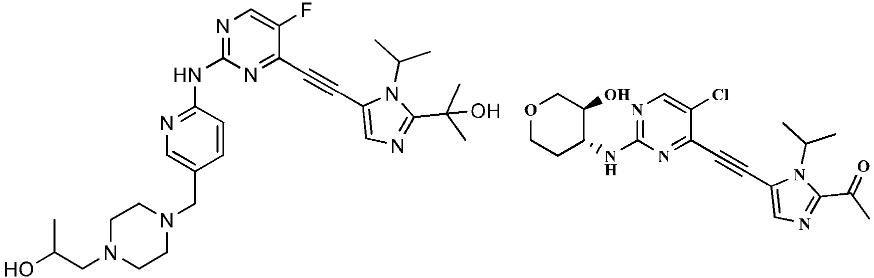

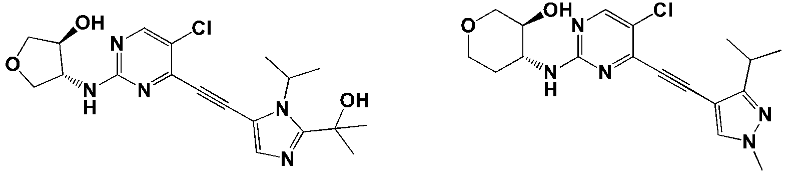

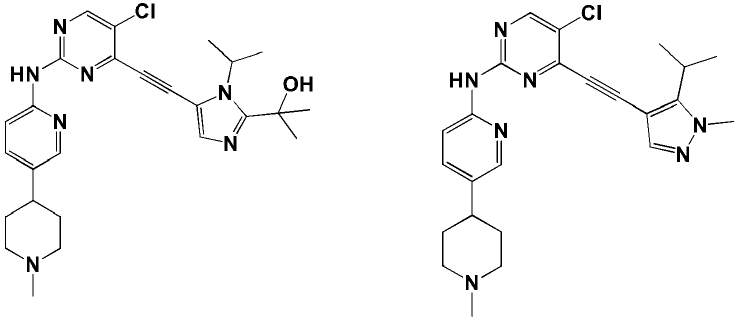

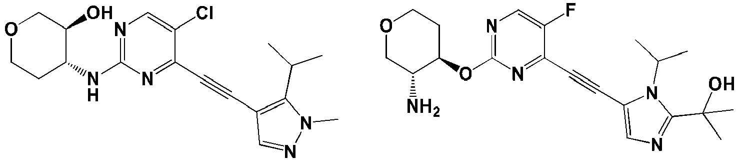

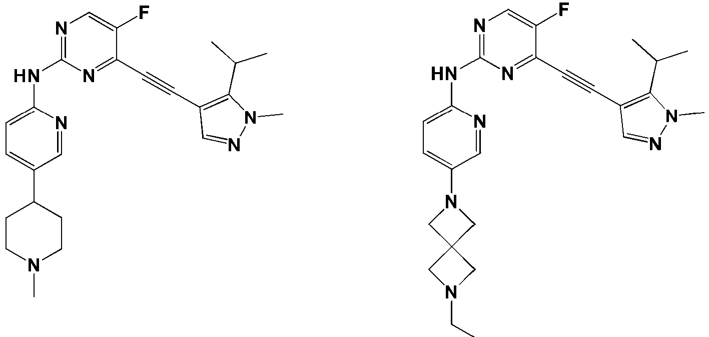

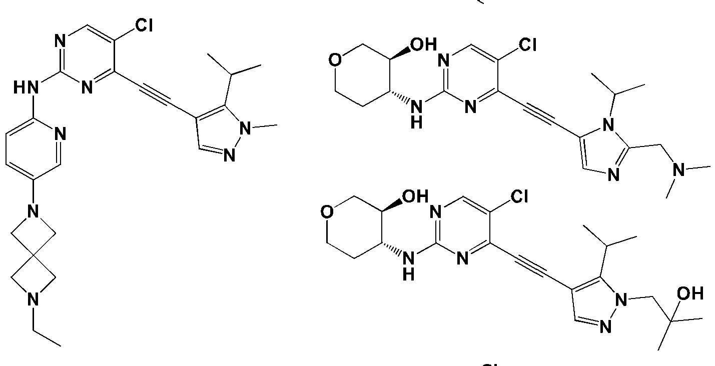

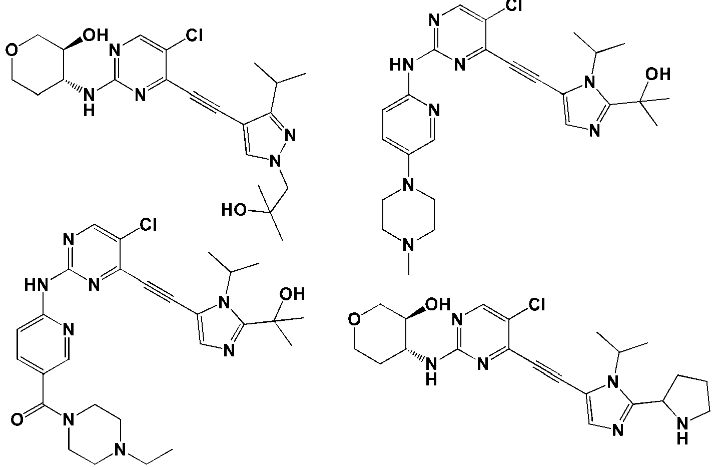

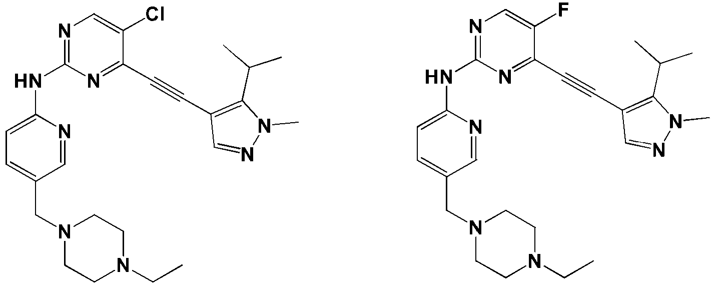

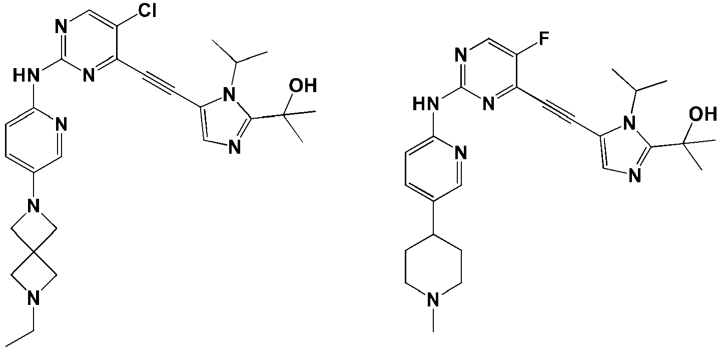

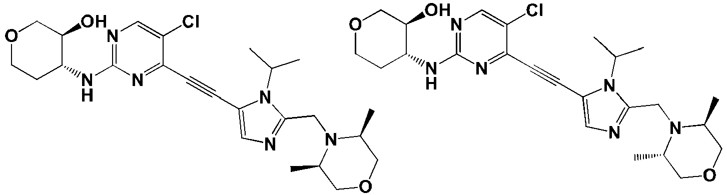

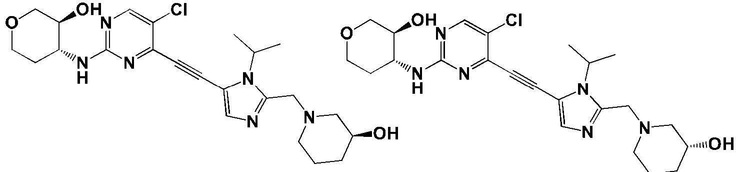

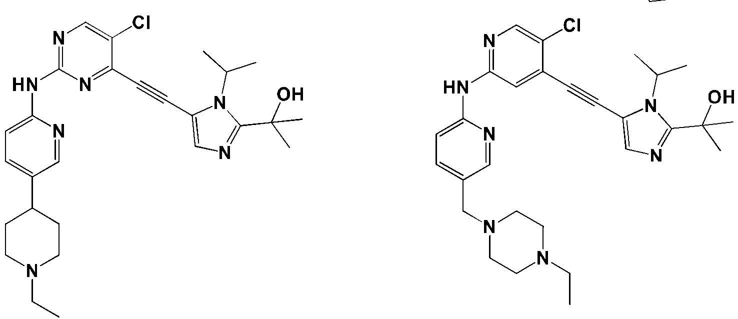

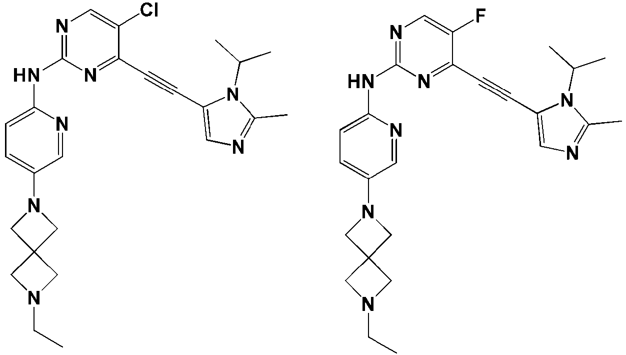

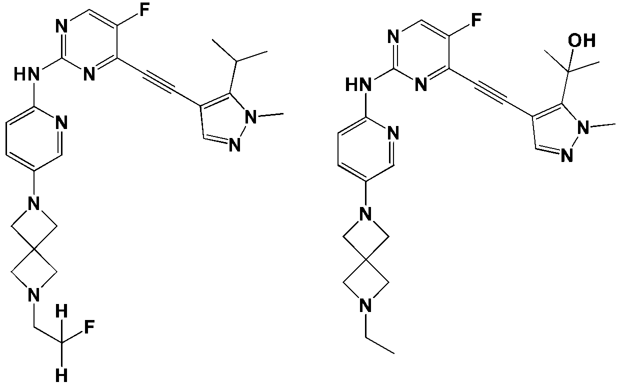

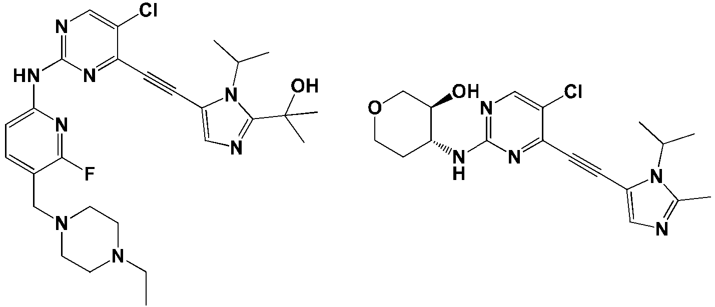

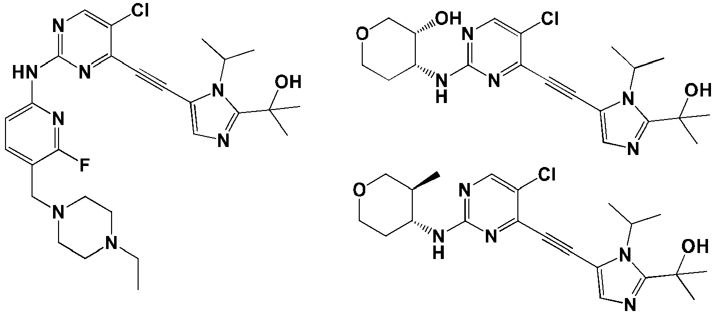

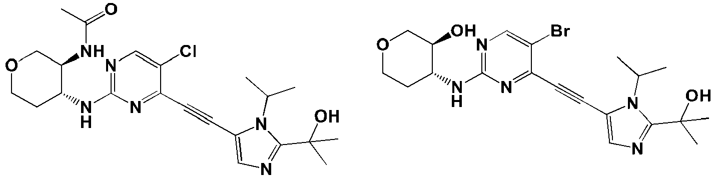

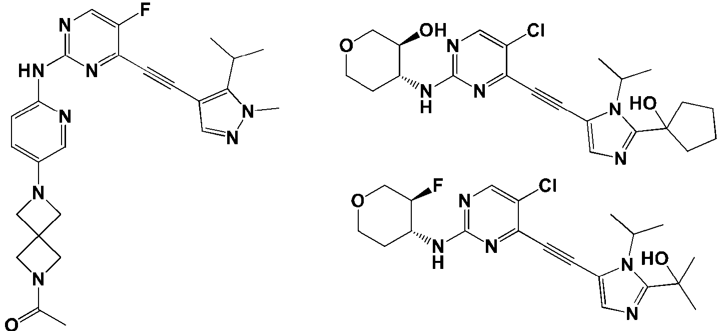

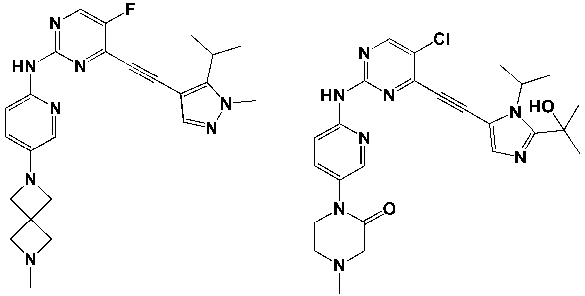

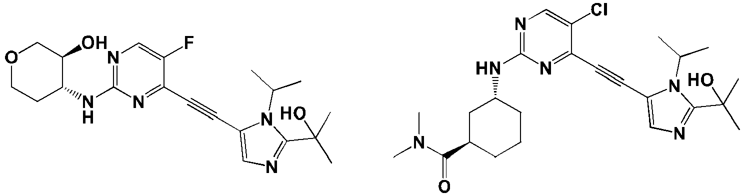

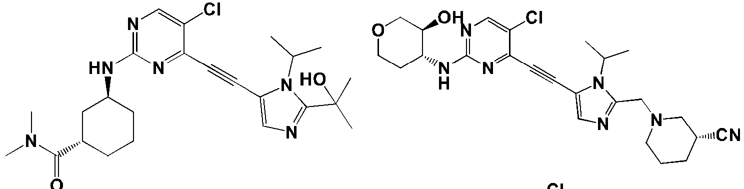

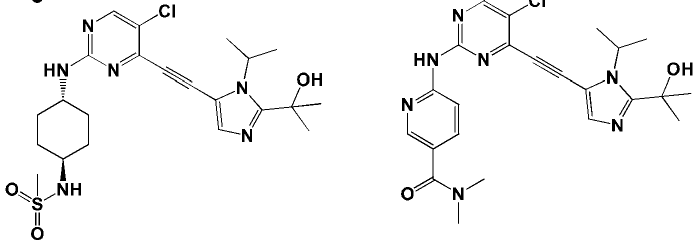

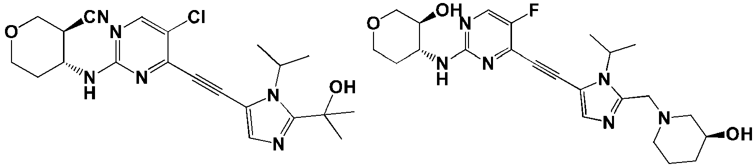

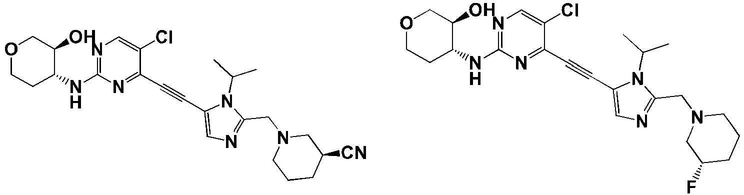

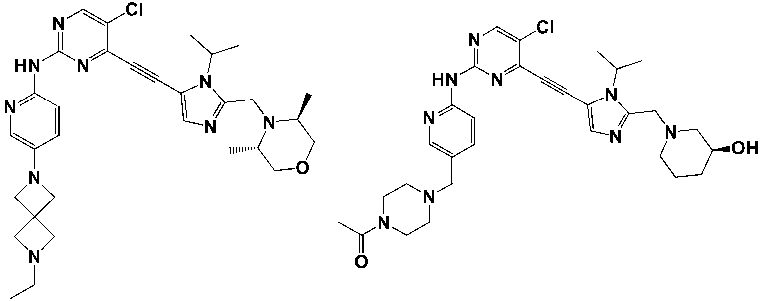

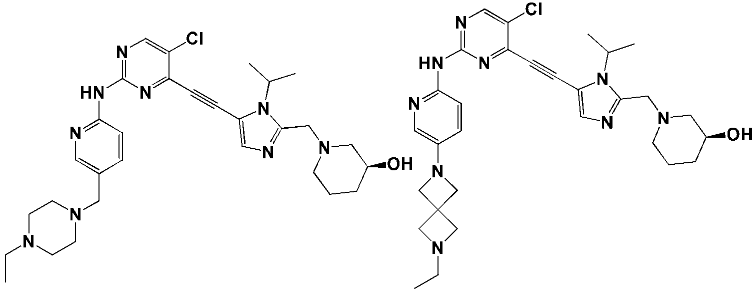

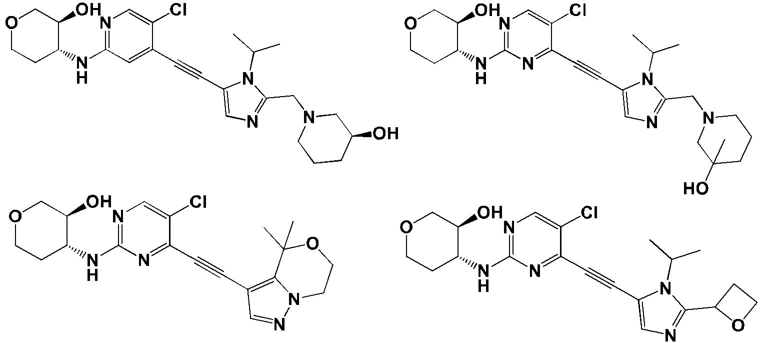

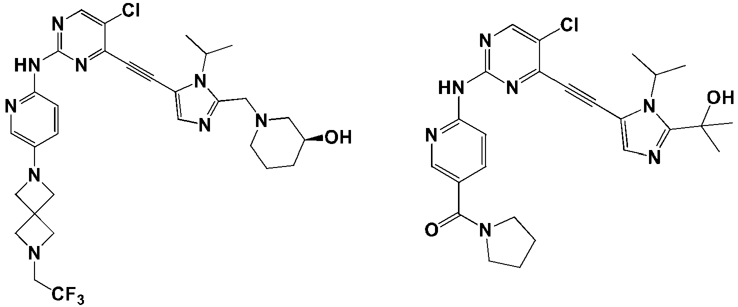

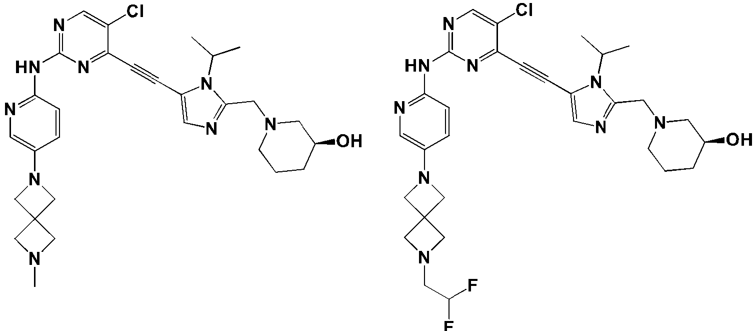

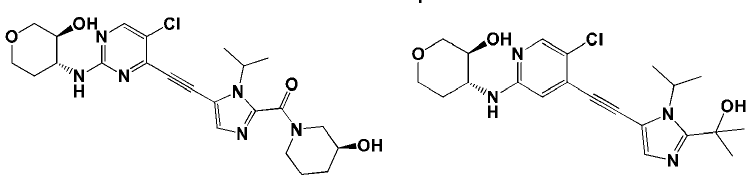

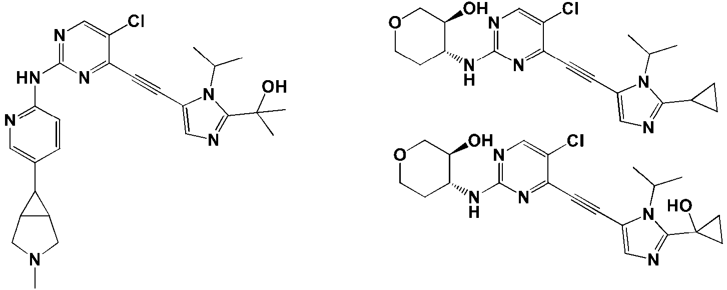

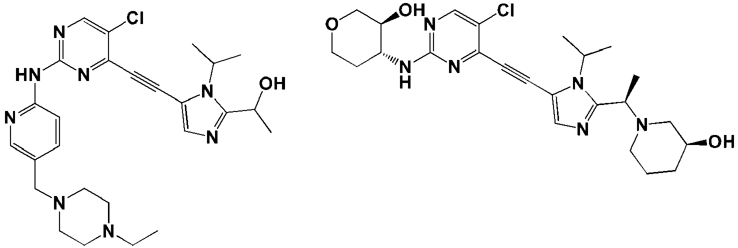

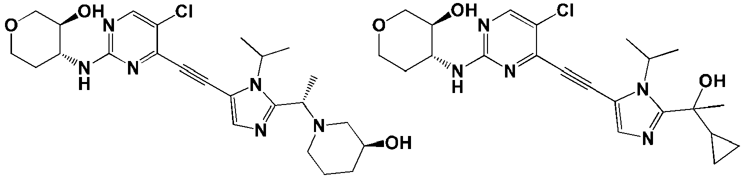

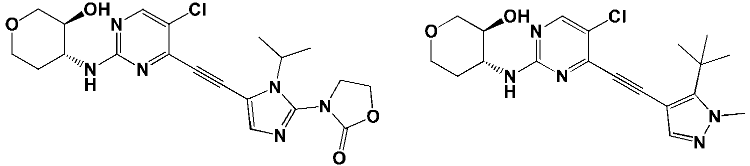

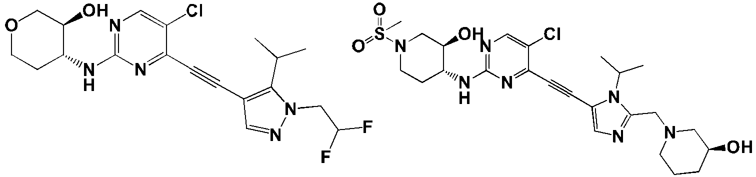

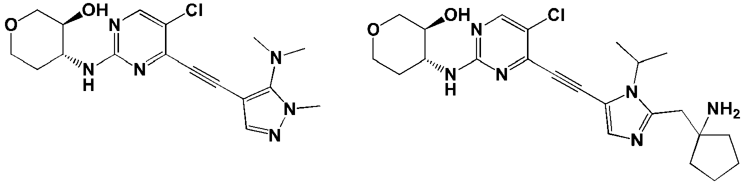

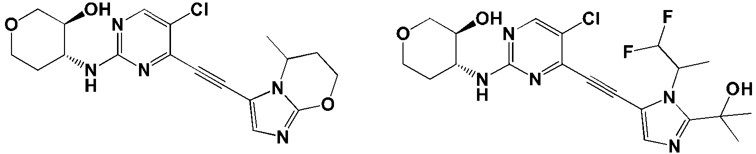

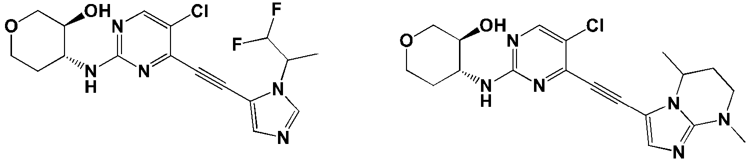

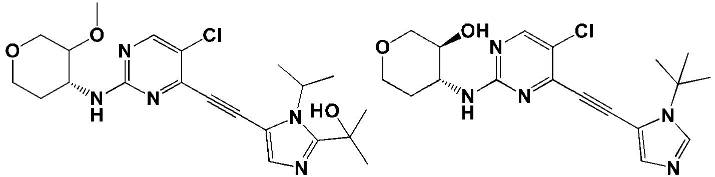

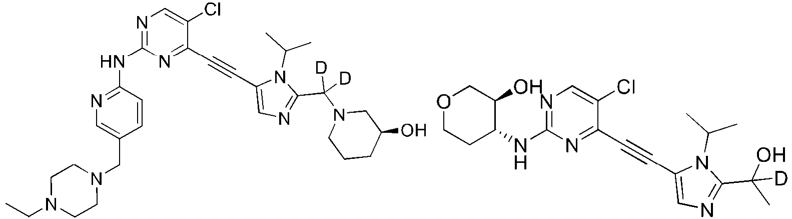

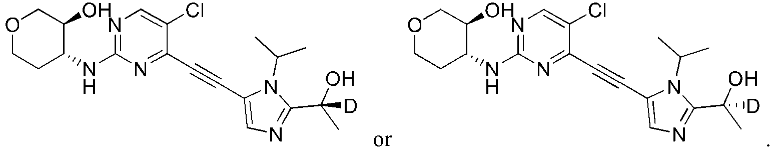

19. A pharmaceutical composition comprising the compound of formula (I) according to any one of claims 1 to 18, the stereoisomer or the pharmaceutically acceptable salt thereof, and a pharmaceutically acceptable carrier.

20. Use of the compound of formula (I) according to any one of claims 1 to 18, the stereoisomer or pharmaceutically acceptable salt thereof in the preparation of a medicament for the treatment of a tumor at least partially associated with CDK; preferably, the tumor is a cancer.

21. Use of the compound of formula (I) according to any one of claims 1 to 18, the stereoisomer or pharmaceutically acceptable salt thereof in the preparation of a medicament for the treatment of breast cancer, malignant brain tumor, colon cancer, small cell lung cancer, non-small cell lung cancer, bladder cancer, ovarian cancer, prostate cancer, chronic lymphatic leukemia, lymphoma, myeloma, acute myeloid leukemia, secondary pancreatic cancer or secondary brain metastasis at least partially associated with CDK.

22. The compound of formula (I) according to any one of claims 1 to 18, the stereoisomer or pharmaceutically acceptable salt thereof for use in the treatment of a tumor at least partially associated with CDK; preferably, the tumor is a cancer.

23. The compound of formula (I) according to any one of claims 1 to 18, the stereoisomer or pharmaceutically acceptable salt thereof for use in the treatment of breast cancer, malignant brain tumor, colon cancer, small cell lung cancer, non-small cell lung cancer, bladder cancer, ovarian cancer, prostate cancer, chronic lymphatic leukemia, lymphoma, myeloma, acute myeloid leukemia, secondary pancreatic cancer or secondary brain metastasis at least partially associated with CDK.
